# EUROPEAN PATENT APPLICATION

(11) **EP 3 763 732 A1**
(43) Date of publication of application: **13.01.2021**
(21) Application number: 20185253.0
(22) Date of filing: 24.11.2017
(51) Int. Cl.: C07K 14/47, C12Q 1/6883, C12Q 1/6886, G16B 20/00, G16B 25/00, G16H 50/20

(54) **METHOD TO DISTINGUISH TUMOR SUPPRESSIVE FOXO ACTIVITY FROM OXIDATIVE STRESS**

(30) Priority: 25.11.2016 EP 16200697
(62) Divisional of application: 17811497.1
(71) Applicant: Koninklijke Philips N.V., 5656 AG Eindhoven (NL)
(72) Inventor: VAN DE STOLPE, Anja, 5656 AE Eindhoven (NL); Velter, Rick, 5656 AE Eindhoven (NL); VERHAEGH, Wilhelmus Franciscus Johannes, 5656 AE Eindhoven (NL); VAN OOIJEN, Hendrik Jan, 5656 AE Eindhoven (NL); Akse, Martijn Theodorus Lambert, 5656 AE Eindhoven (NL)
(74) Representative: Philips Intellectual Property & Standards

(57) **Abstract**

The present invention relates to a kit of parts for the detection of certain target genes of the FOXO transcription factor family, which are markers for an oxidative stress state and can be used for inferring an oxidative stress state of a FOXO transcription factor element in the body of a medical subject. The invention further relates to the use of the kit in inferring an oxidative stress state of a FOXO transcription element and for inferring the activity of the FOXO/PI3K cellular signalling pathway based on expression levels of the target genes as well as products to perform the methods.

## Description

### FIELD OF THE INVENTION

The present invention generally relates to the field of bioinformatics, genomic/transcriptomic processing, proteomic processing, and related arts. More particularly, the present invention relates to certain target genes of the FOXO transcription factor family, which are markers for an oxidative stress state and can be used for inferring an oxidative stress state of a FOXO transcription factor element in a medical subject. The present invention also relates to a method for inferring an oxidative stress state of a FOXO transcription factor element in a medical subject based on the expression level(s) of one or more of the FOXO target genes and a method for inferring the activity of the FOXO/PI3K cellular signaling pathway in a medical subject based on expression levels of one or more target gene(s) of the FOXO/PI3K cellular signaling pathway measured in an extracted sample of the medical subject and based on the inferred oxidative stress state of the FOXO transcription factor element in the medical subject. The present invention further relates to a product comprising primers and/or probes for determining the expression levels of the FOXO target genes. The present invention further relates to an apparatus comprising a digital processor configured to perform the method, a non-transitory storage medium storing instructions that are executable by a digital processing device to perform such a method, and a computer program comprising program code means for causing a digital processing device to perform such a method.

### BACKGROUND OF THE INVENTION

Genomic/transcriptomic and proteomic analyses have substantial realized and potential promise for clinical application in medical fields such as oncology, where various cancers are known to be associated with specific combinations of genomic mutations/variations and/or high or low expression levels for specific genes, which play a role in growth and evolution of cancer, e.g., cell proliferation and metastasis.

For example, screening for an over-expression of the HER2 receptor on the membrane of cells in breast cancer samples is currently the standard test performed for identifying patients that are eligible to HER2 inhibitors such as Trastuzumab. Over-expression of the ERBB2 gene, which results in an over-expression of the HER2 receptor on the cell membrane, occurs in approximately 25% to 30% of all breast cancers and is associated with an increased disease recurrence and a poor prognosis. However, the expression of the HER2 receptor is by no means a conclusive indicator for driving tumor growth as the signaling initiated by the HER2 receptor can for instance be dampened by the downstream cellular signaling pathway. This also seems to be reflected in the initial response rate of 26% in HER2-positive breast cancer patients treated with Trastuzumab (Charles L. Vogel, et al., "Efficacy and Safety of Trastuzumab as a Single Agent in First-Line Treatment of HER2-Overexpressing Metastatic Breast Cancer", Journal of Clinical Oncology, Vol. 20, No. 3, February 2002, pages 719 to 726). Besides that, the cellular signaling pathway downstream of the HER2 receptor can also be activated by mutations/over-expression in proteins downstream of the HER2 receptor, resulting in (a) relatively aggressive tumor type(s) that will not be detected by measuring HER2 expression levels.

It has been shown that the possibilities of characterizing patients having a tumor, e.g., breast cancer, can be improved by studying effects occurring in the cellular signaling pathway downstream of the HER2 receptor. Thus, a method for inferring activity of a PI3K cellular signaling pathway using mathematical modelling of target gene expressions has been described in the published international patent application WO 2015/101635 A1 ("Assessment of the PI3K cellular signaling pathway activity using mathematical modelling of target gene expression").

According to WO 2015/101635 A1 the method for inferring activity of a PI3K cellular signaling pathway using mathematical modelling of target gene expressions, comprises:
inferring the activity of the FOXO/PI3K cellular signaling pathway in a medical subject based at least on expression levels of one or more target gene(s) of the FOXO/PI3K cellular signaling pathway measured in an extracted sample of the medical subject, wherein the inferring comprises:
determining an activity level of a FOXO transcription factor element in the extracted sample of the medical subject, the FOXO transcription factor element controlling transcription of the one or more target gene(s) of the PI3K cellular signaling pathway, the determining being based at least in part on evaluating a mathematical model relating expression levels of the one or more target gene(s) of the FOXO/PI3K cellular signaling pathway to the activity level of the FOXO transcription factor element;
inferring the activity of the PI3K cellular signaling pathway in the medical subject based on the determined activity level of the FOXO transcription factor element in the extracted sample of the medical subject.

In this context it has been realized that a suitable way of identifying effects occurring in the cellular signaling pathway downstream of the HER2 receptor, such as the PI3K cellular signaling pathway, can be based on a measurement of the signaling output of the cellular signaling pathway, which is - amongst others - the transcription of the target genes by a transcription factor (TF), such as a FOXO transcription factor element, controlled by the cellular signaling pathway. The PI3K cellular signaling pathway targeted herein is not only linked to breast cancer, but is known to be inappropriately activated in many types of cancer (Jeffrey A. Engelman, "Targeting PI3K signalling in cancer: opportunities, challenges and limitations", Nature Reviews Cancer, No. 9, August 2009, pages 550 to 562). It is thought to be regulated by the RTK receptor family, which also includes the HER-family. Subsequently, the PI3K cellular signaling pathway passes on its received signal(s) via a multitude of processes, of which the two main branches are the activation of the mTOR complexes and the inactivation of a family of transcription factors often referred to as FOXO (cf. the figure showing the PI3K cellular signaling pathway in the above article from Jeffrey A. Engelman). The method concentrates on the PI3K cellular signaling pathway and the FOXO TF family, the activity of which is substantially negatively correlated with the activity of the PI3K cellular signaling pathway, i.e., activity of FOXO is substantially correlated with inactivity of the PI3K cellular signaling pathway, whereas inactivity of FOXO is substantially correlated with activity of the PI3K cellular signaling pathway. The method makes it possible to determine the activity of the PI3K cellular signaling pathway in a medical subject by (i) determining an activity level of a FOXO transcription factor element in the extracted sample of the medical subject, wherein the determining is based at least in part on evaluating a mathematical model relating expression levels of one or more target gene(s) of the PI3K cellular signaling pathway, the transcription of which is controlled by the FOXO transcription factor element, to the transcriptional activity level of the FOXO transcription factor element, and by (ii) inferring the activity of the PI3K cellular signaling pathway in the medical subject based on the determined activity level of the FOXO transcription factor element in the extracted sample of the medical subject. This allows improving the possibilities of characterizing patients that have a tumor, e.g., breast cancer, which is at least partially driven by a deregulated PI3K cellular signaling pathway, and that are therefore likely to respond to inhibitors of the PI3K cellular signaling pathway.

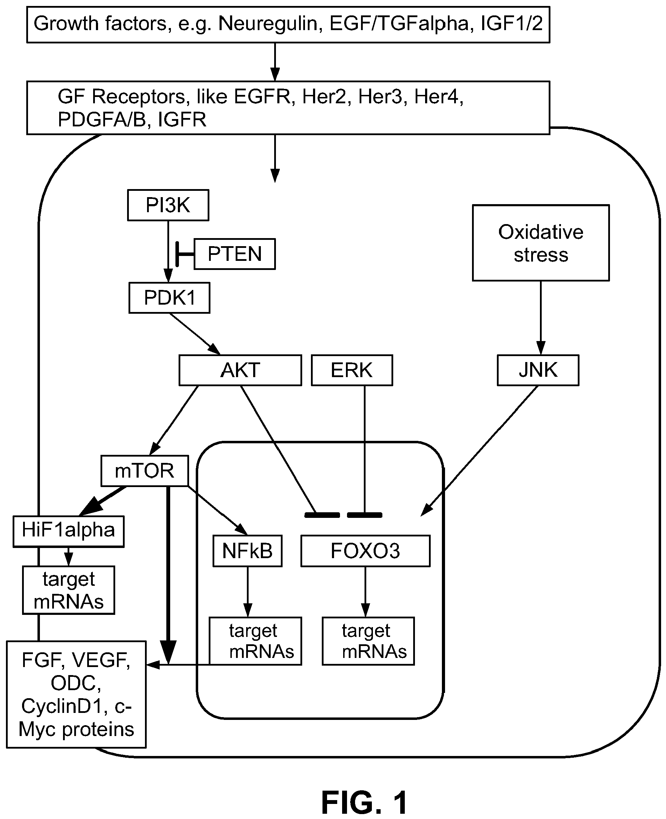

The nuclear FOXO3 transcription factor, a member of the FOXO transcription factor family, for example, can be active in normal cells but also in a situation in which the cell experiences oxidative stress, like in a cancer cell. In both situations FOXO is present in the nucleus (cf. figure 1). When the PI3K cellular signaling pathway becomes active FOXO is translocated from nucleus to cytoplasm, which is associated with inactivity of FOXO. This is not possible, however, when FOXO is activated due to oxidative stress.

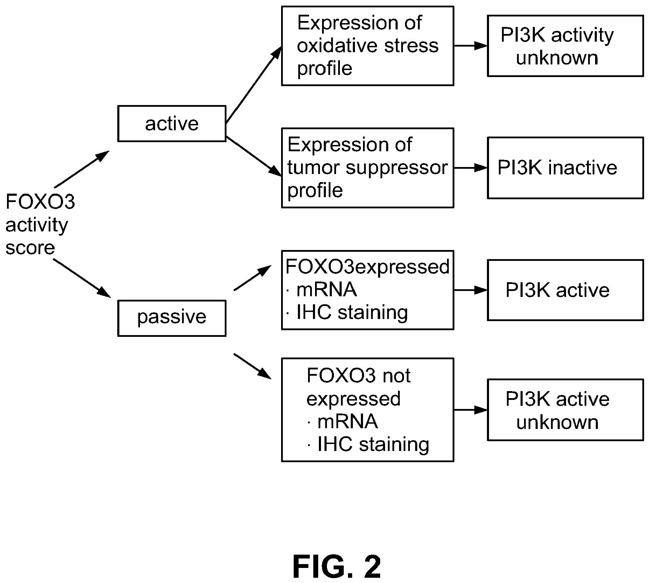

Therefore, in a situation, in which FOXO is found to be active and in the nucleus, this may either be a normal cell, in which the PI3K pathway is inactive and FOXO is in a tumor suppressive state or it may be in a situation in which the cell experiences oxidative stress and in which the PI3K pathway is active but FOXO translocation from the nucleus and inactivity is prevented. It is therefore desirable to find a way to distinguish a tumor suppressive FOXO activity from oxidative stress in order to render the inference that the PI3K pathway is active or inactive more reliable. A decision tree to infer PI3K activity is shown in figure 2.

### SUMMARY OF THE INVENTION

In accordance with a main aspect of the present invention, the above problem is solved by a FOXO target gene or set of two or more FOXO target genes for use as marker for an oxidative stress state of a FOXO transcription factor element in a medical subject based on the expression level(s) of the one or the set of two or more FOXO target gene(s) in an extracted sample of the medical subject, wherein the target gene(s) is/are selected from the group consisting of SOD2, BNIP3, MXI1, PCK1, PPARGC1A and CAT.

The present invention is based on the realization that specific FOXO target genes are differentially expressed between the "normal", i.e. the tumor suppressive state, and an "oxidative stress" state and therefore measuring their expression levels allows distinction between the two states.

Herein, a FOXO transcription factor (TF) element is defined to be a protein complex containing at least one of the FOXO TF family members, i.e., FOXO1, FOXO3, FOXO4 and FOXO6, which is capable of binding to specific DNA sequences, thereby controlling transcription of target genes.

An oxidative stress state of a FOXO transcription factor element herein refers to a state, in which FOXO is active and in the nucleus but wherein the PI3K pathway can be either active or inactive. If the PI3K pathway is active, deactivation of FOXO does not occur due to the cell experiencing oxidative stress. In particular, an oxidative stress state refers to a state of cancer or a pre- cancer stage of a cell.

In contrast, a tumor suppressive state of a FOXO transcription factor element herein refers to a state, in which FOXO is active in the nucleus and the PI3K pathway is inactive. In particular, a tumor suppressive state refers to a normal, healthy state of a cell.

A "PI3K cellular singaling pathway" or "PI3K pathway" herein preferably refers to a cellular singaling pathway that ultimately leads to transcriptional activity of the transcription factor (TF) complexes associated with the pathway. In the present case, these consist of a FOXO TF family member as mentioned above. The pathway may therefore also be referred to as the "FOXO/PI3K cellular signaling pathway" in the context of the present invention.

The "target gene(s)" may be "direct target genes" and/or "indirect target genes".

Suitable target genes are described in the following text.

SOD2 (superoxide dismutase-2) is a mitochondrial matrix enzyme that scavenges oxygen radicals produced by the extensive oxidation-reduction and electron transport reactions occurring in mitochondria.

BNIP3 (BcL-2/adenovirus E1B-19-kDA protein interacting protein 3) is normally expressed as an inactive monomer, but following toxic stimuli, it forms stable homodimers, integrates into the outer mitochondrial membrane, and causes loss of mitochondrial membrane potential and cell death (Sassone et al., "BNIP3 has a key role in the mitochondrial dysfunction induced by mutant huntigtin", Human Molecular Genetics, Vol. 24, 2015, pages 6530-6539).

PCK1 (Phosphoenolpyruvate carboxykinase) is a main target for regulation of gluconeogenesis. Transcription of the PEPCK gene is regulated by insulin, glucocorticoids, cAMP, and diet, in order to adjust glucose production to physiologic requirements.

The MXI1 gene encodes a basic helix-loop-helix leucine zipper transcription factor that bind MAX in vitro, forming a sequence-specific DNA-binding complex similar to the MYC-MAX heterodimer. MXI1 antagonizes MYC function and is a candidate tumor suppressor gene. (Delpuech O, Griffiths B, East P, Essafi A, Lam EW, Burgering B, Downward J, Schulze A. "Induction of Mxi1-SR alpha by FOXO3a contributes to repression of Myc-dependent gene expression", Molecular Cell Biology, Jul. 2007; Vol. 27(13), pages 4917-30)

PPARGC1A is a coactivator of nuclear receptors and other transcription factors that regulate metabolic processes, including mitochondrial biogenesis and respiration, hepatic gluconeogenesis, and muscle fiber-type switching (Lin et al., "Defects in adaptive energy metabolism with CNS-linked hyperactivity in PGC-1-alpha null mice", Cell, 2004, Vol. 119, pages 121-135).

According to a preferred embodiment, the present invention relates to a set of at least four FOXO target genes, preferably all target genes, selected from the group consisting of SOD2, BNIP3, MXI1, PCK1, PPARGC1A and CAT for use as marker as described above.

According to a particularly preferred embodiment, the present invention relates to a set of two or more FOXO target genes, preferably all target genes, selected from the group consisting of SOD2, BNIP3, MXI1 and PCK1 for use as marker as described above.

Another aspect of the present invention relates to the use of a FOXO target gene or of a set of two or more FOXO target genes as marker for inferring an oxidative stress state of a FOXO transcription factor element in a medical subject based on the expression levels of the one or the set of two or more FOXO target gene(s) in an extracted sample of the medical subject, wherein the target gene(s) is/are selected from the group consisting of SOD2, BNIP3, MXI1, PCK1, PPARGC1A and CAT.

The term "inferring" in the context of the present invention refers to the act of applying a created mathematical expression or model to a dataset measured in a sample, such as expression levels of specific genes, to derive information relating to a state of the sample. For example, "inferring" may involve the calculation of a score, such as an oxidative stress score, for a sample and deducing a state, such as an oxidative stress state, by e.g. applying a threshold, wherein the score is either above or below the threshold according to the state of the sample.

The "subject" or "medical subject" may be a human or an animal.

The extracted sample may be a sample of the tissue and/or the cells and/or the body fluid of the medical subject or may be from a cell line and/or a tissue culture derived from a medical subject and, if applicable, cultivated in vitro in the lab (e.g., for regenerative medicine purposes). The sample can be, e.g., a sample obtained from a cancer lesion, or from a lesion suspected for cancer, or from a metastatic tumor, or from a body cavity in which fluid is present which is contaminated with cancer cells (e.g., pleural or abdominal cavity or bladder cavity), or from other body fluids containing cancer cells, and so forth, preferably via a biopsy procedure or other sample extraction procedure. The cells of which a sample is extracted may also be tumorous cells from hematologic malignancies (such as leukemia or lymphoma). In some cases, the cell sample may also be circulating tumor cells, that is, tumor cells that have entered the bloodstream and may be extracted using suitable isolation techniques, e.g., apheresis or conventional venous blood withdrawal. Aside from blood, the body fluid of which a sample is extracted may be urine, gastrointestinal contents, or an extravasate. The term "extracted sample", as used herein, also encompasses the case where tissue and/or cells and/or body fluid of the subject have been taken from the subject and, e.g., have been put on a microscope slide, and where for performing the claimed method a portion of this sample is extracted, e.g., by means of Laser Capture Microdissection (LCM), or by scraping off the cells of interest from the slide, or by fluorescence-activated cell sorting techniques. The cells or tissue can also be from normal, non-malignant tissue, or from diseased tissue other than cancer.

Preferred is the use as described above, wherein inferring an oxidative stress state of a FOXO transcription factor element in a medical subject is based on the expression levels of at least four FOXO target genes, preferably all target genes selected from the group consisting of SOD2, BNIP3, MXI1, PCK1, PPARGC1A and CAT in an extracted sample of the medical subject.

Further preferred is the use as described above, wherein inferring an oxidative stress state of a FOXO transcription factor element in a medical subject is based on the expression levels of two or more FOXO target genes, preferably all target genes, selected from the group consisting of SOD2, BNIP3, MXI1 and PCK1, in an extracted sample of the medical subject.

According to another main aspect, the present invention relates to a method for inferring an oxidative stress state of a FOXO transcription factor element in a medical subject, wherein the inferring comprises:
determining the expression levels of one or more FOXO target gene(s) in an extracted sample of the medical subject, wherein the target gene(s) is/are selected from the group consisting of SOD2, BNIP3, MXI1, PCK1, PPARGC1A and CAT; and
inferring the oxidative stress state of the FOXO transcription factor element in the medical subject based on the determined expression levels of one or more FOXO target gene(s) in the extracted sample of the medical subject.

The distinguishing set of target genes used for determining the oxidative stress state of a FOXO transcription factor element as opposed to a tumor suppressive state was found by comparing the target gene expression profile in normal breast tissue and normal colon tissue samples in which FOXO is active, with that in samples from respectively breast cancer and colon cancer.

A preferred embodiment is a method as described above, wherein the inferring is based on the expression level(s) of at least four, preferably all of the FOXO target gene(s) selected from the group consisting of SOD2, BNIP3, MXI1, PCK1, PPARGC1A and CAT in the extracted sample of the medical subject.

A further preferred embodiment is a method as described above, wherein the inferring is based on the expression level(s) of two or more, preferably all of the FOXO target gene(s) selected from the group consisting of SOD2, BNIP3, MXI1 and PCK1 in the extracted sample of the medical subject.

The target genes recited in the paragraph above have been found to be particularly informative with respect to the oxidative stress state.

In another embodiment, the present invention relates to a method as described above, wherein an oxidative stress state of the FOXO transcription factor element is inferred, when the expression level(s) of SOD2 and/or BNIP3 are upregulated in the extracted sample of the medical subject compared to a control sample and/or when the expression level(s) of one or more target gene(s) selected from MXI1, PCK1, PPARGC1A and CAT are downregulated in the extracted sample of the medical subject compared to a control sample.

A preferred embodiment is a method as described above, wherein an oxidative stress state of the FOXO transcription factor element is inferred, when the expression level(s) of SOD2 and/or BNIP3 are upregulated in the extracted sample of the medical subject compared to a control sample and/or when the expression level(s) of MXI1 and/or PCK1 are downregulated in the extracted sample of the medical subject compared to a control sample.

As mentioned above, the distinguishing set of target genes used for determining the oxidative stress state of a FOXO transcription factor element as opposed to a tumor suppressive state was found by comparing the target gene expression profile in normal breast tissue and normal colon tissue samples in which FOXO is active, with that in samples from respectively breast cancer and colon cancer.

The results can be summarized such that a comparison of FOXO being active in normal colon versus FOXO active in colon carcinoma shows:
- increased expression levels of SOD2 and BNIP3 in colon cancer, and
- increased expression levels of MXI1, PCK1 and PPARGC1A in normal colon tissue.

The above results were reproducible in FOXO active breast cancer versus FOXO active in normal breast tissue:
- increased expression levels of SOD2 and BNIP3 in breast cancer, and
- increased expression levels of MXI1, PCK1, CAT and PPARGC1A in normal breast tissue.

The results were also at least partially reproducible in FOXO active esophageal cancer versus FOXO active in normal esophageal tissue:
- increased expression level of SOD2 in esophageal cancer, and
- increased expression levels of MXI1 and PPARGC1A in normal esophageal tissue.

Accordingly, the method of the invention can be used to indicate a cancer or pre-cancer state in a medical subject, in particular it can be used to determine the presence or absence of colon cancer, breast cancer and esophageal cancer.

The most distinguishing genes are SOD2, BNIP3 (both increased in cancer) and MXI1, PCK1 (both decreased in cancer). PPARGC1A is less informative in breast cancer and therefore less preferred.

The control sample can be a sample of "normal" tissue, cells or body fluid extracted from a healthy medical subject or it can refer to averaged expression data over collected samples from multiple healthy medical subjects. Such expression data may be derived from public databases.

The method according to the invention may also be a method for inferring the activity of a PI3K cellular signaling pathway in a subject based at least on the oxidative stress state of the subject, comprising,
inferring the oxidative stress state of the subject based on the expression level(s) of one or more gene(s) selected from the group consisting of SOD2, BNIP3, MXI1, PCK1, PPARGC1A and CAT in the subject.

The oxidative stress state of the subject is preferably the oxidative stress state of a FOXO transcription factor element in the subject.

The method is preferably based at least on expression levels of one or more target gene(s) of the PI3K cellular signaling pathway measured in the subject, which may be a medical subject. Preferably, the method is performed using an extracted sample of the subject, i.e. expression levels are measured in an extracted sample of the subject.

According to a further aspect of the present invention, the method described above can be integrated into a method for inferring the activity of the FOXO/PI3K cellular signalling pathway as e.g. described in WO 2015/101635 A1 and improve the reliability of the result as explained above.

Preferably, inferring the activity of the PI3K cellular signaling pathway in the subject is therefore based on the inferred oxidative stress state of the subject and the activity level of the FOXO transcription factor element in the subject.

The method may comprise determining the expression level(s) of the one or more genes in the subject.

The expression levels of the one or more gene(s) are preferably determined in an extracted sample of the subject.

Preferably, inferring the oxidative stress state of the subject is based on the expression level(s) of at least four, preferably all of the FOXO target gene(s) selected from the group consisting of SOD2, BNIP3, MXI1, PCK1, PPARGC1A and CAT in the subject.

Further preferably, inferring the oxidative stress state of the subject is based on the expression level(s) of two or more, preferably all of the FOXO target gene(s) selected from the group consisting of of SOD2, BNIP3, MXI1 and PCK1 in the subject.

Further preferably, inferring the oxidative stress state of the subject is based on the expression level of one FOXO target gene selected from the group consisting of SOD2, BNIP3, MXI1, PCK1, PPARGC1A and CAT in the subject.

Another preferred embodiment is a method as described above, further comprising inferring the activity of the FOXO/PI3K cellular signalling pathway in the medical subject based at least on expression levels of one or more target gene(s) of the FOXO/PI3K cellular signalling pathway measured in an extracted sample of the medical subject, wherein the inferring comprises:
determining an activity level of a FOXO transcription factor element in the extracted sample of the medical subject, the FOXO transcription factor element controlling transcription of the one or more target gene(s) of the FOXO/PI3K cellular signalling pathway, the determining being based at least in part on evaluating a mathematical model relating expression levels of the one or more target gene(s) of the FOXO/PI3K cellular signalling pathway to the activity level of the FOXO transcription factor element;
inferring the activity of the FOXO/PI3K cellular signalling pathway in the medical subject based on the determined activity level of the FOXO transcription factor element in the extracted sample of the medical subject and the inferred oxidative stress state of the FOXO transcription factor element in the medical subject,
wherein inferring the activity of the FOXO/PI3K cellular signaling pathway is performed by a digital processing device using the mathematical model.

A further preferred embodiment of the present invetion is a method for inferring the activity of the FOXO/PI3K cellular signalling pathway in a medical subject based at least on expression levels of one or more target gene(s) of the FOXO/PI3K cellular signalling pathway measured in an extracted sample of the medical subject, wherein the inferring comprises:
determining an activity level of a FOXO transcription factor element in the extracted sample of the medical subject, the FOXO transcription factor element controlling transcription of the one or more target gene(s) of the FOXO/PI3K cellular signalling pathway, the determining being based at least in part on evaluating a mathematical model relating expression levels of the one or more target gene(s) of the FOXO/PI3K cellular signalling pathway to the activity level of the FOXO transcription factor element;
inferring the oxidative stress state of the FOXO transcription factor element in the medical subject based on the expression levels of one or more FOXO target gene(s) selected from the group consisting of SOD2, BNIP3, MXI1, PCK1, PPARGC1A and CAT in the extracted sample of the medical subject;
inferring the activity of the FOXO/PI3K cellular signalling pathway in the medical subject based on the determined activity level of the FOXO transcription factor element in the extracted sample of the medical subject and the inferred oxidative stress state of the FOXO transcription factor element in the medical subject,
wherein inferring the activity of the FOXO/PI3K cellular signaling pathway is performed by a digital processing device using the mathematical model.

By relying not only on the determined activity level of the FOXO transcription factor element but including also the inferred oxidative stress state of the FOXO transcription factor element, the inferred activity of the FOXO/PI3K cellular signalling pathway becomes more reliable as explained above.

As will be understood by a person skilled in the art, determining the expression levels of the FOXO target gene(s) for both purposes, inferring the oxidative stress state of the FOXO transcription factor element as well as determining the activity level of the FOXO transcription factor element, may be done using the same or different samples from the same medical subject and/or the same or different probes and may be based on the same or different, or partially overlapping (sets) of target genes as applicable. If one, two or more, or all of the expression level(s) of the target gene(s) is/are selected from the group consisting of SOD2, BNIP3, MXI1, PCK1, PPARGC1A and CAT are determined the result can be used for both purposes. Preferably, only one sample for the medical subject is used to determine all of the expression levels needed for the method described above.

The mathematical model may be a probabilistic model, preferably a Bayesian network model as described in WO 2015/101635 A1, based at least in part on conditional probabilities relating the FOXO transcription factor element and expression levels of the one or more target gene(s) of the PI3K cellular signaling pathway measured in the extracted sample of the tissue and/or the cells and/or the body fluid of the medical subject, or the mathematical model may be based at least in part on one or more linear combination(s) of expression levels of the one or more target gene(s) of the PI3K cellular signaling pathway measured in the extracted sample of the tissue and/or the cells and/or the body fluid of the medical subject. In particular, the inferring of the activity of the PI3K cellular signaling pathway may be performed as disclosed in the published international patent application WO 2013/011479 A2 ("Assessment of cellular signaling pathway activity using probabilistic modeling of target gene expression") or as described in the published international patent application WO 2014/102668 ("Assessment of cellular signaling pathway activity using linear combination(s) of target gene expressions").

Acccording to a preferred embodiment of the present invention, an oxidative stress state is inferred, when the expression level(s) of SOD2 and/or BNIP3 are upregulated in the extracted sample of the medical subject compared to a control sample and/or when the expression level(s) of one or more target gene(s) selected from MXI1, PCK1, PPARGC1A and CAT are downregulated in the extracted sample of the medical subject compared to a control sample.

According to a further preferred embodiment of the present invention, an oxidative stress state is inferred, when the expression level(s) of SOD2 and/or BNIP3 are upregulated in the extracted sample of the medical subject compared to a control sample and/or when the expression level(s) of MXI1 and/or PCK1 are downregulated in the extracted sample of the medical subject compared to a control sample.

Preferably, the oxidative stress state is the oxidative stress state of a FOXO transcription factor element in the subject.

According to a preferred embodiment of the present invention, the target gene(s) for inferring the activity of the PI3K cellular signaling pathway is/are selected from the group consisting of the target genes below.

In a preferred method as described above, the activity level of the FOXO transcription factor element in the subject is determined based at least on expression levels of one or more, preferably at least three, target gene(s) of the PI3K cellular signaling pathway measured in an extracted sample of the subject selected from the group consisting of: AGRP, BCL2L11, BCL6, BNIP3, BTG1, CAT, CAV1, CCND1, CCND2, CCNG2, CDKN1A, CDKN1B, ESR1, FASLG, FBXO32, GADD45A, INSR, MXI1, NOS3, PCK1, POMC, PPARGC1A, PRDX3, RBL2, SOD2 and TNFSF10.

In a further preferred method as described above, the activity level of the FOXO transcription factor element is determined based at least on expression levels of one or more, preferably at least three, target gene(s) of the PI3K cellular signaling pathway measured in the extracted sample of the subject selected from the group consisting of: ATP8A1, C10orf10, CBLB, DDB1, DYRK2, ERBB3, EREG, EXT1, FGFR2, IGF1R, IGFBP1, IGFBP3, LGMN, PPM1D, SEMA3C, SEPP1, SESN1, SLC5A3, SMAD4 and TLE4 and/or selected from the group consisting of: ATG14, BIRC5, IGFBP1, KLF2, KLF4, MYOD1, PDK4, RAG1, RAG2, SESN1, SIRT1, STK11 and TXNIP.

The activtiy level of the FOXO transcription factor element is preferably inferred based on expression level(s) of the target gene(s) recited above measured in an extracted sample of the subject.

Particularly preferred is a method wherein
inferring the activity of the FOXO/PI3K cellular signaling pathway in the medical subject is based at least on expression levels of one or more, preferably at least three, target gene(s) of the FOXO/PI3K cellular signaling pathway measured in the extracted sample of the medical subject selected from the group consisting of: AGRP, BCL2L11, BCL6, BNIP3, BTG1, CAT, CAV1, CCND1, CCND2, CCNG2, CDKN1A, CDKN1B, ESR1, FASLG, FBXO32, GADD45A, INSR, MXI1, NOS3, PCK1, POMC, PPARGC1A, PRDX3, RBL2, SOD2 and TNFSF10 and/or wherein inferring the oxidative stress state of the FOXO transcription factor element is based on the expression levels of one or more, preferably all of the target genes of a FOXO transcription factor SOD2, BNIP3, MXI1 and PCK1 measured in the extracted sample of the medical subject.

Further preferred is an embodiment, wherein the inferring the activity of the FOXO/PI3K cellular signaling pathway in the medical subject is based at least on expression levels of six or more, preferably ten or more, more preferably all target gene(s) of the FOXO/PI3K cellular signaling pathway measured in the extracted sample of the medical subject selected from the group consisting of: AGRP, BCL2L11, BCL6, BNIP3, BTG1, CAT, CAV1, CCND1, CCND2, CCNG2, CDKN1A, CDKN1B, ESR1, FASLG, FBXO32, GADD45A, INSR, MXI1, NOS3, PCK1, POMC, PPARGC1A, PRDX3, RBL2, SOD2 and TNFSF10.

Further preferred is a method, wherein the inferring is further based on expression levels of at least one target gene of the PI3K cellular signaling pathway measured in the extracted sample of the tissue and/or the cells and/or the body fluid of the medical subject selected from the group consisting of: ATP8A1, C10orf10, CBLB, DDB1, DYRK2, ERBB3, EREG, EXT1, FGFR2, IGF1R, IGFBP1, IGFBP3, LGMN, PPM1D, SEMA3C, SEPP1, SESN1, SLC5A3, SMAD4 and TLE4.

Further preferred is a method, wherein the inferring is further based on expression levels of at least one target gene of the PI3K cellular signaling pathway measured in the extracted sample of the tissue and/or the cells and/or the body fluid of the medical subject selected from the group consisting of: ATG14, BIRC5, IGFBP1, KLF2, KLF4, MYOD1, PDK4, RAG1, RAG2, SESN1, SIRT1, STK11 and TXNIP.

If the inferring is further based both on expression levels of at least one target gene selected from the group specified in the preceding paragraph and on expression levels of at least one target gene selected from the group specified in the paragraph preceding the preceding paragraph, the target gene IGFBP1, which is mentioned above with respect to both groups, may only be contained in one of the groups.

Another aspect of the present invention relates to a method (as described herein), further comprising:
determining whether the PI3K cellular signaling pathway is operating abnormally in the tissue and/or the cells and/or the body fluid of the medical subject based on the inferred activity of the PI3K cellular signaling pathway in the tissue and/or the cells and/or the body fluid of the medical subject.

In a preferred embodiment, the method as described above therefore further comprises determining whether the PI3K cellular signalling pathway is operating abnormally in the subject based on the inferred activity of the PI3K cellular signalling pathway in the subj ect.

The phrase "the cellular signaling pathway is operating abnormally" refers to the case where the "activity" of the pathway is not as expected, wherein the term "activity" may refer to the activity of the transcription factor complex in driving the target genes to expression. "Normal" may be when it is inactive in tissue where it is expected to be inactive and active where it is expected to be active. Furthermore, there may be a certain level of activity that is considered normal, and anything higher or lower may be considered abnormal.

The present invention also relates to a method (as described herein) further comprising:
recommending prescribing a drug for the medical subject that corrects for abnormal operation of the PI3K cellular signaling pathway,
wherein the recommending is performed only if the PI3K cellular signaling pathway is determined to be operating abnormally in the tissue and/or the cells and/or the body fluid of the medical subject based on the inferred activity of the PI3K cellular signaling pathway.

According to a preferred embodiment the method of the present invention therfore further comprises recommending prescribing a drug for the subject that corrects for abnormal operation of the PI3K cellular signalling pathway, wherein the recommending is performed if the PI3K cellular signalling pathway is determined to be operating abnormally in the subject based on the inferred activity of the PI3K cellular signalling pathway.

Advantageously, the method as described above can be used to indicate a cancer or pre-cancer state in a medical subject, in particular it can be used to determine the presence or absence of colon cancer, breast cancer and esophageal cancer.

The present invention also relates to a method (as described herein), wherein the inferring comprises:
inferring the activity of the PI3K cellular signaling pathway in the medical subject based at least on expression levels of two, three or more target genes of a set of target genes of the PI3K cellular signaling pathway measured in the extracted sample of the tissue and/or the cells and/or the body fluid of the medical subject.

Preferably,
the set of target genes of the PI3K cellular signaling pathway includes at least nine, preferably all target genes selected from the group consisting of: AGRP, BCL2L11, BCL6, BNIP3, BTG1, CAT, CAV1, CCND1, CCND2, CCNG2, CDKN1A, CDKN1B, ESR1, FASLG, FBXO32, GADD45A, INSR, MXI1, NOS3, PCK1, POMC, PPARGC1A, PRDX3, RBL2, SOD2 and TNFSF10.

A method, wherein
the set of target genes of the PI3K cellular signaling pathway further includes at least one target gene selected from the group consisting of: ATP8A1, C10orf10, CBLB, DDB1, DYRK2, ERBB3, EREG, EXT1, FGFR2, IGF1R, IGFBP1, IGFBP3, LGMN, PPM1D, SEMA3C, SEPP1, SESN1, SLC5A3, SMAD4 and TLE4,
is particularly preferred.

A method, wherein
the set of target genes of the PI3K cellular signaling pathway further includes at least one target gene selected from the group consisting of: ATG14, BIRC5, IGFBP1, KLF2, KLF4, MYOD1, PDK4, RAG1, RAG2, SESN1, SIRT1, STK11 and TXNIP,
is also particularly preferred.

If the set of target genes further includes both at least one target gene selected from the group specified in the preceding paragraph and at least one target gene selected from the group specified in the paragraph preceding the preceding paragraph, the target gene IGFBP1, which is mentioned above with respect to both groups, may only be contained in one of the groups.

In a further aspect, the present invention also relates to a product comprising:
primers and/or probes for determining a gene expression level of one or a set of two or more FOXO target gene(s), preferably at least four FOXO target genes in an extracted sample of a medical subject, wherein the target gene(s) is/are selected from the group consisting of SOD2, BNIP3, MXI1, PCK1, PPARGC1A and CAT, preferably selected from SOD2, BNIP3, MXI1 and PCK1; and
optionally further comprising primers and/or probes for determining the expression levels of a set of genes other than the above mentioned genes in the extracted sample of a medical subject, preferably of two or more target gene(s) of the FOXO/PI3K cellular signaling pathway selected from the group consisting of: AGRP, BCL2L11, BCL6, BTG1, CAV1, CCND1, CCND2, CCNG2, CDKN1A, CDKN1B, ESR1, FASLG, FBXO32, GADD45A, INSR, NOS3, POMC, PRDX3, RBL2 and TNFSF10.

In a preferred embodiment, the product described above is a PCR kit, a RNA-sequencing kit, or a microarray kit.

The materials for use in the methods of the present invention are ideally suited for preparation of kits produced in accordance with well known procedures. The invention thus provides kits comprising agents for the detection of expression of the disclosed genes and sequences. Such kits optionally comprise the agent with an identifying description or label or instructions relating to their use in the methods of the present invention, is provided. Such a kit may comprise containers, each with one or more of the various reagents (typically in concentrated form) utilized in the methods, including, for example, pre-fabricated microarrays, buffers, the appropriate nucleotide triphosphates (e.g., dATP, dCTP, dGTP and dTTP; or rATP, rCTP, RGTP and UTP), reverse transcriptase, DNA polymerase, RNA polymerase, and one or more primers. A set of instructions will also typically be included.

In the context of the present invention, the expression level(s) may be determined by a method involving the detection of an mRNA encoded by the gene.

For example, the measurement of the nucleic acid level of marker gene(s) expression may be assessed by purification of nucleic acid molecules (e.g. RNA or cDNA) obtained from the sample, followed by hybridization with specific oligonucleotide probes as defined herein above. Comparison of expression levels may be accomplished visually or by means of an appropriate device. Methods for the detection of mRNA or expression products are known to the person skilled in the art.

Alternatively, the nucleic acid level of marker gene(s) expression may be detected in a DNA array or microarray approach. Typically, sample nucleic acids derived from patients to be tested are processed and labeled, preferably with a fluorescent label. Subsequently, such nucleic acid molecules may be used in a hybridization approach with immobilized capture probes corresponding to the marker genes of the present invention. Suitable means for carrying out microarray analyses are known to the person skilled in the art.

In a standard setup a DNA array or microarray comprises immobilized high-density probes to detect a number of genes. The probes on the array are complementary to one or more parts of the sequence of the marker genes. Typically, cDNAs, PCR products, and oligonucleotides are useful as probes.

A DNA array- or microarray-based detection method typically comprises the following steps: (1) Isolating mRNA from a sample and optionally converting the mRNA to cDNA, and subsequently labeling this RNA or cDNA. Methods for isolating RNA, converting it into cDNA and for labeling nucleic acids are described in manuals for micro array technology. (2) Hybridizing the nucleic acids from step 1 with probes for the marker genes. The nucleic acids from a sample can be labeled with a dye, such as the fluorescent dyes Cy3 (red) or Cy5 (blue). Generally, a control sample is labeled with a different dye. (3) Detecting the hybridization of the nucleic acids from the sample with the probes and determining at least qualitatively, and more particularly quantitatively, the amounts of mRNA in the sample for marker genes investigated. The difference in the expression level between sample and control can be estimated based on a difference in the signal intensity. These can be measured and analyzed by appropriate software such as, but not limited to the software provided for example by Affymetrix.

There is no limitation on the number of probes corresponding to the marker genes used, which are spotted on a DNA array. Also, a marker gene can be represented by two or more probes, the probes hybridizing to different parts of a gene. Probes are designed for each selected marker gene. Such a probe is typically an oligonucleotide comprising 5-50 nucleotide residues. Longer DNAs can be synthesized by PCR or chemically. Methods for synthesizing such oligonucleotides and applying them on a substrate are well known in the field of micro-arrays. Genes other than the marker genes may be also spotted on the DNA array. For example, a probe for a gene whose expression level is not significantly altered may be spotted on the DNA array to normalize assay results or to compare assay results of multiple arrays or different assays.

Alternatively, the nucleic acid level of marker gene(s) expression may be detected in a quantitative RT-PCR approach, preferably in a real-time PCR approach following the reverse transcription transcripts of interest. Typically, as first step, a transcript is reverse transcribed into a cDNA molecule according to any suitable method known to the person skilled in the art. A quantitative or real-time PCR approach may subsequently be carried out based on a first DNA strand obtained as described above.

Preferably, Taqman or Molecular Beacon probes as principal FRET-based probes of this type may be used for quantitative PCR detection. In both cases, the probes, serve as internal probes which are used in conjunction with a pair of opposing primers that flank the target region of interest, preferably a set of marker gene(s) specific oligonucleotides as defined herein above. Upon amplification of a target segment, the probe may selectively bind to the products at an identifying sequence in between the primer sites, thereby causing increases in FRET signaling relative to increases in target frequency.

Preferably, a Taqman probe to be used for a quantitative PCR approach according to the present invention may comprises a specific oligonucleotide as defined above of about 22 to 30 bases that is labeled on both ends with a FRET pair. Typically, the 5' end will have a shorter wavelength fluorophore such as fluorescein (e.g. FAM) and the 3' end is commonly labeled with a longer wavelength fluorescent quencher (e.g. TAMRA) or a non-fluorescent quencher compound (e.g. Black Hole Quencher). It is preferred that the probes to be used for quantitative PCR, in particular probes as defined herein above, have no guanine (G) at the 5' end adjacent to the reporter dye in order to avoid quenching of the reporter fluorescence after the probe is degraded.

A Molecular Beacon probe to be used for a quantitative PCR approach according to the present invention preferably uses FRET interactions to detect and quantify a PCR product, with each probe having a 5' fluorescent-labeled end and a 3' quencher-labeled end. This hairpin or stem-loop configuration of the probe structure comprises preferably a stem with two short self-binding ends and a loop with a long internal target-specific region of about 20 to 30 bases.

Alternative detection mechanisms which may also be employed in the context of the present invention are directed to a probe fabricated with only a loop structure and without a short complementary stem region. An alternative FRET -based approach for quantitative PCR which may also be used in the context of the present invention is based on the use of two hybridization probes that bind to adjacent sites on the target wherein the first probe has a fluorescent donor label at the 3' end and the second probe has a fluorescent acceptor label at its 5' end.

In accordance with another disclosed aspect, an apparatus comprises a digital processor configured to perform a method according to the present invention as described herein.

In accordance with another disclosed aspect, a non-transitory storage medium stores instructions that are executable by a digital processing device to perform a method according to the present invention as described herein. The non-transitory storage medium may be a computer-readable storage medium, such as a hard drive or other magnetic storage medium, an optical disk or other optical storage medium, a random access memory (RAM), read only memory (ROM), flash memory, or other electronic storage medium, a network server, or so forth. The digital processing device may be a handheld device (e.g., a personal data assistant or smartphone), a notebook computer, a desktop computer, a tablet computer or device, a remote network server, or so forth.

In accordance with another disclosed aspect, a computer program comprises program code means for causing a digital processing device to perform a method according to the present invention as described herein. The digital processing device may be a handheld device (e.g., a personal data assistant or smartphone), a notebook computer, a desktop computer, a tablet computer or device, a remote network server, or so forth.

The present invention as described herein can, e.g., also advantageously be used in connection with:
diagnosis based on the inferred activity of the PI3K cellular signaling pathway in the tissue and/or the cells and/or the body fluid of the medical subject;
prognosis based on the inferred activity of the PI3K cellular signaling pathway in the tissue and/or the cells and/or the body fluid of the medical subject;
drug prescription based on the inferred activity of the PI3K cellular signaling pathway in the tissue and/or the cells and/or the body fluid of the medical subject;
prediction of drug efficacy based on the inferred activity of the PI3K cellular signaling pathway in the tissue and/or the cells and/or the body fluid of the medical subject;
prediction of adverse effects based on the inferred activity of the PI3K cellular signaling pathway in the tissue and/or the cells and/or the body fluid of the medical subject;
monitoring of drug efficacy;
drug development;
assay development;
pathway research;
cancer staging;
enrollment of the medical subject in a clinical trial based on the inferred activity of the PI3K cellular signaling pathway in the tissue and/or the cells and/or the body fluid of the medical subject;
selection of subsequent test to be performed; and
selection of companion diagnostics tests.

According to a preferred embodiment, the method of the present invention is therfore used in at least one of the following activities:
diagnosis based on the inferred activity of the PI3K cellular signalling pathway in the subject;
prognosis based on the inferred activity of the PI3K cellular signalling pathway in the subject;
drug prescription based on the inferred activity of the PI3K cellular signalling pathway in the subject;
prediction of drug efficacy based on the inferred activity of the PI3K cellular signalling pathway in the subject;
prediction of adverse effects based on the inferred activity of the PI3K cellular signalling pathway in the subject;
monitoring of drug efficacy;
drug development;
assay development;
pathway research;
cancer staging;
enrollment of the subject in a clinical trial based on the inferred activity of the PI3K cellular signalling pathway in the subject;
selection of subsequent test to be performed; and
selection of companion diagnostics tests.

Further advantages will be apparent to those of ordinary skill in the art upon reading and understanding the attached figures, the following description and, in particular, upon reading the detailed examples provided herein below.

It shall be understood that the method, the apparatus, the non-transitory storage medium, and the computer program of the present invention have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

Fig. 1 schematically shows the FOXO/PI3K cellular signaling pathway in a cell, wherein FOXO3 is located in the nucleus.
Fig. 2 shows a schematic decision tree for inferring activity of the PI3K cellular signalling pathway.
Fig. 3 shows the oxidative stress score for each subgroup specified in the dataset using all genes and probesets shown in table 1.
Fig. 4 shows the oxidative stress score for only those samples that had FOXO activity as inferred by the FOXO activity model described in WO 2015/101635 A1
Fig. 5 shows the oxidative stress score obtained by using only the most informative oxidative stress-induced FOXO target genes SOD2, MXI1, PCK1 and BNIP3.
Fig. 6 shows a schematic FOXO model structure incorporating oxidative stress by adding an oxidative stress node. Nodes representing probesets are not shown for readability purposes.
Fig. 7 shows the results of the model described in example 2 and shown in Figure 6 tested on the dataset GS20916. The x-axis shows the probability of the transcription complex (TC) node to be active and the y-axis the probability of the OXI (oxidative stress state) node to be active. Black circles represent normal colon samples, empty circles represent adenocarcinoma samples and crosses represent colon carcinoma samples.
Fig. 8 shows a Bayesian computational model predicting FOXO activity.
   A. The Bayesian network structure used as a basis for the modeling approach shown as a simplified model of the transcriptional program of a cellular signal transduction pathway, consisting of three types of nodes: transcription factor, target gene and microarray probe sets corresponding to target genes.
   B. Training of the computational FOXO3 model on a public GEO dataset GSE16573, consisting of Affymetrix microarray 2.0 Plus expression data from HUVEC containing a 4OHT-inducible FOXO3.A3-ER expression construct. Each bar represents a sample analysis result. The vertical axis indicates the probability that FOXO is "active" (values above the horizontal axis) versus "inactive" (values below the horizontal axis).

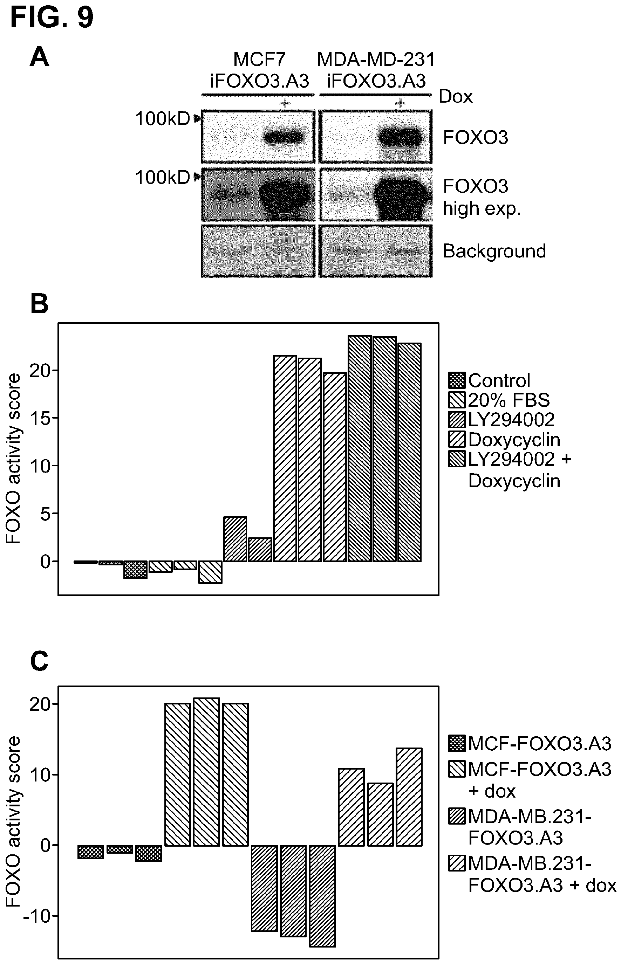

Fig. 9 shows the FOXO and PI3K activity are correctly predicted in breast cancer cell lines A. Western blot analysis of FOXO3 expression levels in MCF7-FOXO3.A3 and MDA-MB-231 cells cultured in the absence or presence of doxycycline (dox) for 16 hours. The lower FOXO3 blot represents a longer exposure of the same blot. B. Biological validation of the computational FOXO3 model using MCF7-FOXO3.A3 cells treated with 20% FBS, PI3K inhibitor LY294002, doxycycline or a combination of doxycycline and LY294002 for 16 hours. Each bar represents the analysis results of one sample. The vertical axis indicates the probability that the FOXO3 is "active" (values above the horizontal axis) versus "inactive" (values below the horizontal axis. C. Biological validation of the computational FOXO3 model using MCF7-FOXO3.A3 and MDA-MB-231 cells treated with doxycycline for 16 hours. Each bar represents the analysis results of one sample. The vertical axis indicates the probability that the FOXO3 is "active" (values above the horizontal axis) versus "inactive" (values below the horizontal axis).

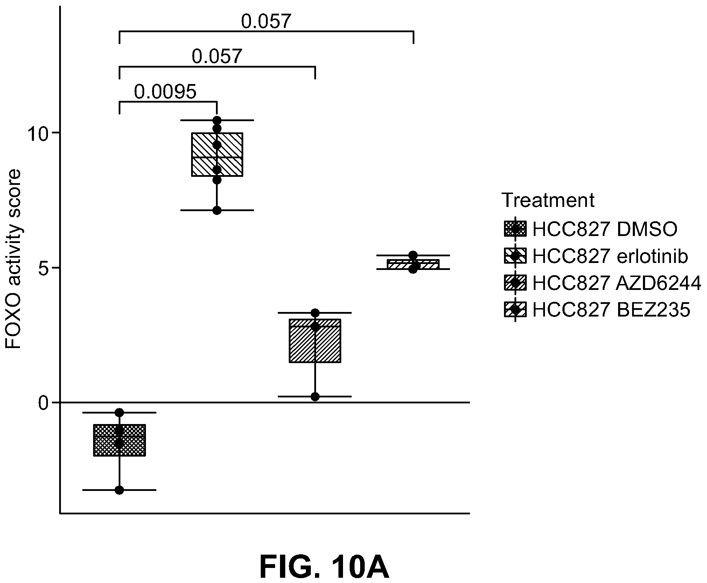

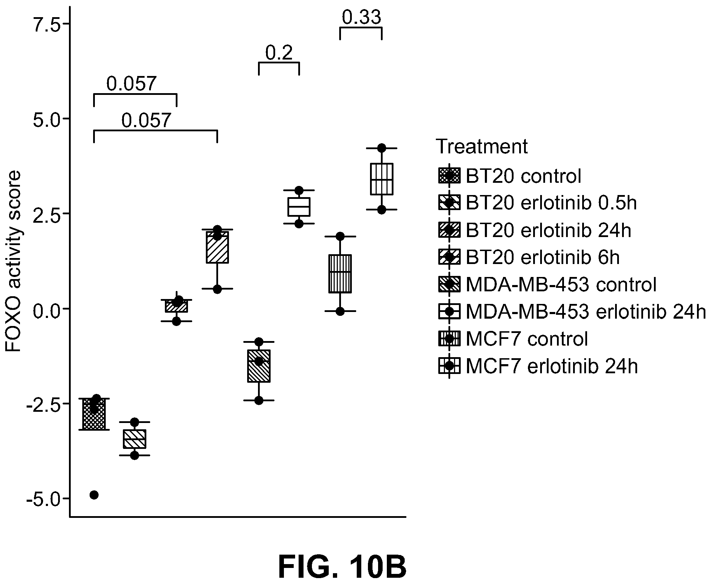

Fig. 10 shows targeted drugs which inhibit the PI3K pathway and induce FOXO activity. Public datasets from samples that were treated with drugs targeting growth factor pathways. FOXO activity score is indicated as log2odds. p values resulting from of Wilcoxon rank statistical tests are indicated in the figures. **A**. GEO GSE51212. The lung cancer cell line HCC827 was treated either with vehicle (DMSO) or with erlotinib, AZD6244 (selumetinib) or BEZ235, as indicated (from left to right). Erlotinib inhibits EGFR; selumetinib inhibits specifically MEK1/MEK2; BEZ235 is a dual inhibitor of PI3K/mTOR. **B**. GEO GSE30516 dataset. Three breast cancer cell lines representing triple-negative (BT20), ER positive (MCF7), and HER2 positive breast cancer (MDA-MB-453) were treated with Erlotinib (time periods indicated, from left to right).

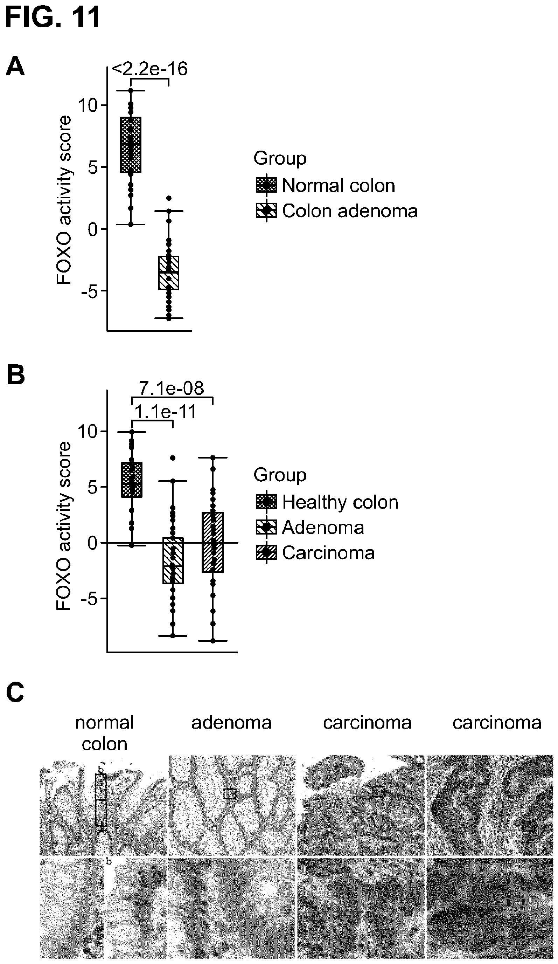

Fig. 11 shows the FOXO activity and cellular localisation in colon, colon adenoma and colon carcinoma A. Biological validation of the computational FOXO model in corresponding normal colon and colon adenoma patient samples within the GSE8671 dataset. Each bar represents the analysis result of one sample. The vertical axis indicates the probability that the FOXO3 is "active" (values above the horizontal axis) versus "inactive" (values below the horizontal axis). The bars on the left represent normal tissue, the bars on the right represent adenoma samples. B. Biological validation of the computational FOXO3 model using a public dataset (GSE20916) with samples of normal colon tissue ("normal colon, dis mucosa": normal mucosa micro-dissected from tumor tissue; "distant colon, dis mucosa": from distant healthy tissue; "normal colon, dis crypt": micro-dissected normal crypt from tumor tissue; "distant colon, dis crypt": microdissected crypt from distant healthy colon; "colon, surgery": full thickness tissue from normal colon), colon adenoma (separated in micro-dissected mucosa, micro-dissected crypt and complete surgical samples) and carcinoma (separated in micro-dissected mucosa, micro-dissected crypt and complete surgical samples) patient samples. Each bar represents the analysis results of one sample. The vertical axis indicates the probability that the FOXO3 is "active" (values above the horizontal axis) versus "inactive" (values below the horizontal axis). The bars on the left represent normal tissue, the bars in the middle represent adenoma samples, and the bars on the right represent colon carcinoma samples. C. Immunohistochemical staining of FOXO3 and haematoxylin in normal colon, colon adenoma and two carcinoma patient samples. The lower panel is a magnification of the area indicated by the black box.

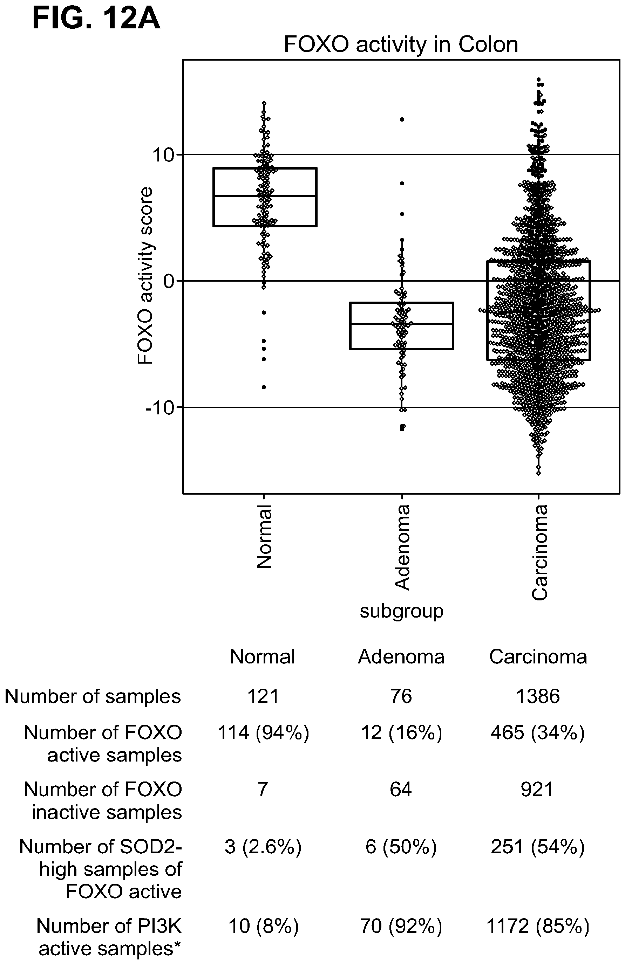

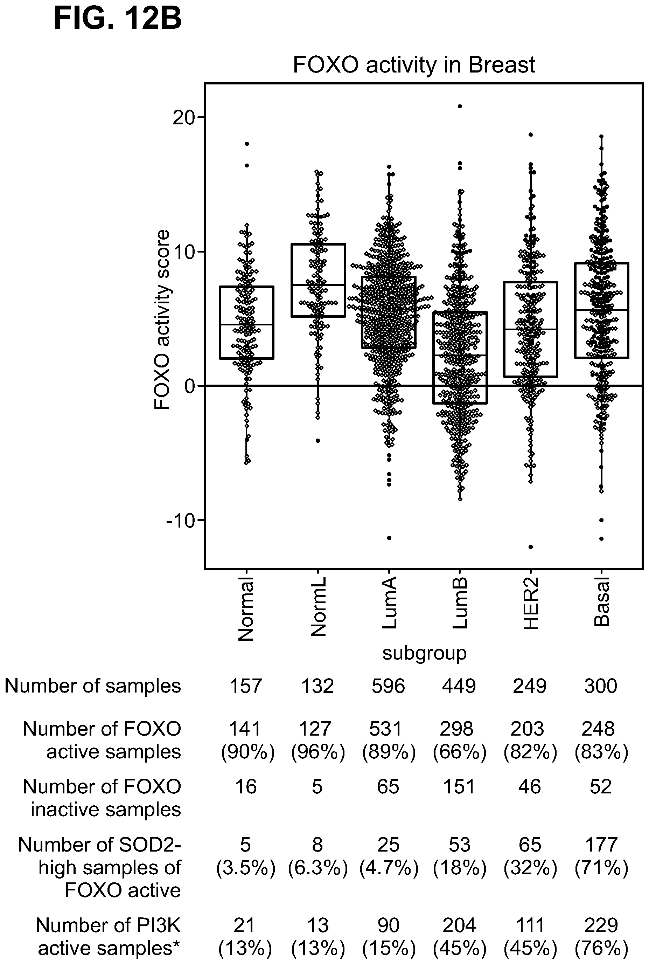

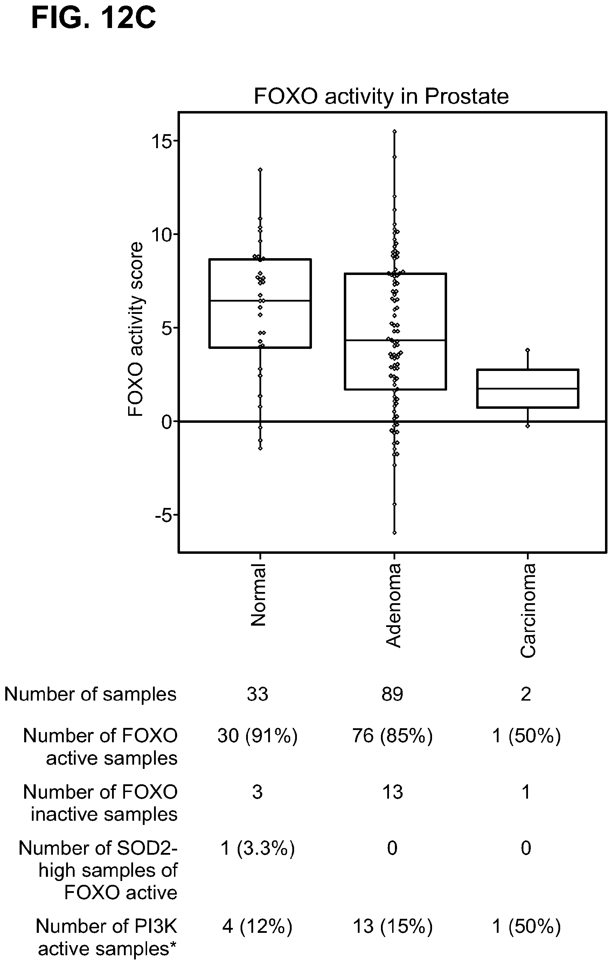

Fig. 12 shows the predicted FOXO activity and distinction between tumor suppressive and oxidative stress mode of FOXO activity. To obtain larger sample numbers for analysis multiple public Affymetrix datasets from the GEO database were compiled and analysed (Colon: GSE14333, GSE20916, GSE2109, GSE37364, GSE39084, GSE40967, GSE4183, GSE8671.Breast: EMTAB365, GSE10780, GSE12276, GSE18146, GSE21653, GSE26910, GSE42568, GSE45827, GSE6532, GSE7307, GSE20685, GSE9195, GSE17907. Prostate: GSE2109, GSE32982, GSE3325, GSE46602; GSE55945, GSE7307). For each analysed sample FOXO activity is shown as Log2odds. Although a continuous scale, FOXO is in principle considered active when the FOXO activity score is higher than 0. FOXO-active samples in solid black have a SOD2 expression level that is higher than the *mean*+*2SD* of SOD expression in FOXO-active normal healthy tissue samples, indicating high likelihood of oxidative stress-induced FOXO activity. Circles indicate FOXO-active samples in which SOD2 expression is in the normal range, indicating that FOXO is likely to be active in the tumor suppressive mode. The number of PI3K active sample represents the number of FOXO inactive samples plus the number of SOD2-high samples which are also FOXO active. For detailed explanation see text. **A.** Colon cancer. **B.** Breast cancer. For details on uniform breast cancer subtyping according to Perou, see Methods in Example 4. **C.** Prostate cancer.

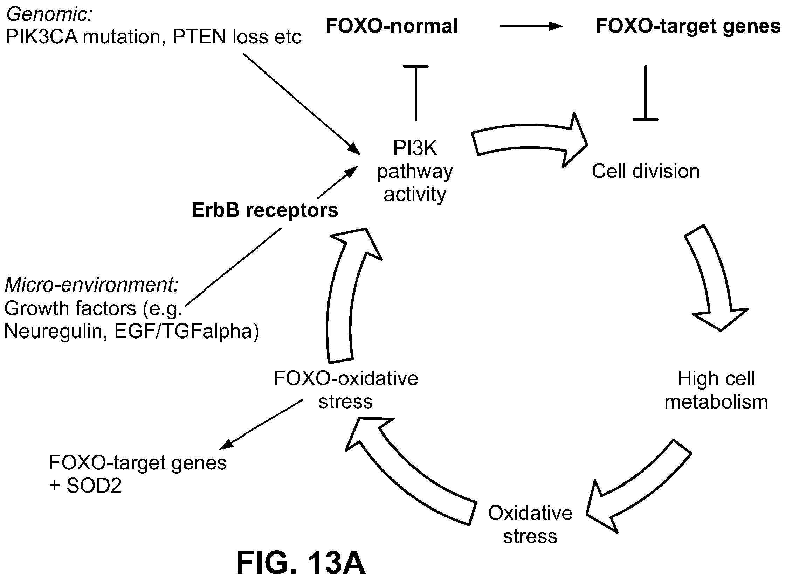

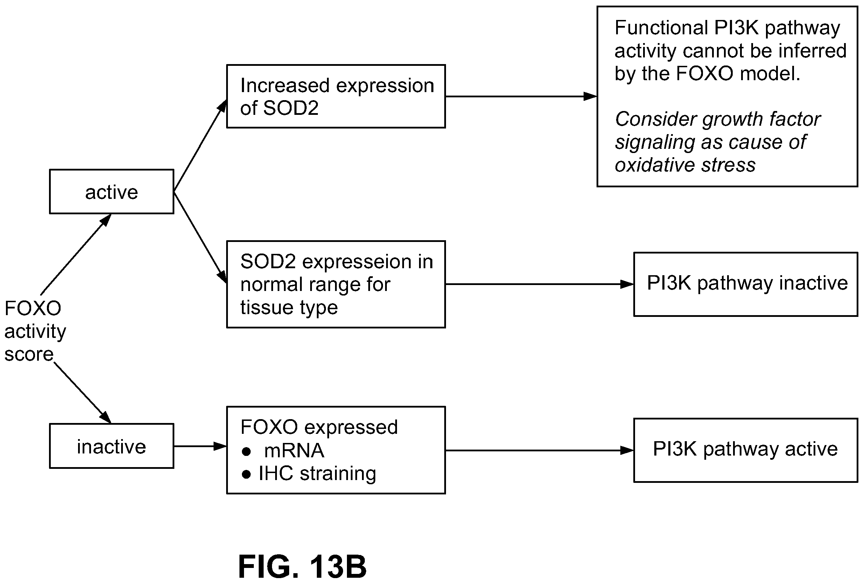

Fig. 13 shows the PI3K-FOXO pathway and distinction between tumor suppressive and oxidative stress mode of FOXO activity **A**. *PI3K-FOXO pathway and relation with SOD2 target gene expression.* In healthy normal tissue FOXO induces transcription of target genes which control cell division. When the PI3K pathway is activated, either by genomic mutations or by stimuli from the microenvironment, FOXO activity is blocked, control over cell division is lost and cell metabolism increases, associated with oxidative stress. Oxidative stress induces activation of FOXO, now with the alternative function to protect against the consequences of the stress situation in the cell. The FOXO target gene expression profile is slightly changed to include now also SOD2. **B.** Decision tree to decide on PI3K activity in a tissue sample. This simplified decision tree is valid, on the assumptions: (1) FOXO is expressed in the cancer cells and (2) limited contamination with healthy FOXO-active cells.

### DETAILED DESCRIPTION OF THE EMBODIMENTS

The following examples merely illustrate particularly preferred methods and selected aspects in connection therewith. The teaching provided therein may be used for constructing several tests and/or kits, e.g., to detect, predict and/or diagnose the abnormal activity of one or more cellular signaling pathways. Furthermore, upon using methods as described herein drug prescription can advantageously be guided, drug prediction and monitoring of drug efficacy (and/or adverse effects) can be made, drug resistance can be predicted and monitored, e.g., to select subsequent test(s) to be performed (like a companion diagnostic test). The following examples are not to be construed as limiting the scope of the present invention.

### Example 1: Exemplary embodiment using a linear score to infer an oxidative stress state of a FOXO transcription factor element

A simple linear score based on the FOXO target genes SOD2, BNIP3, PCK1, MXI1, PPARGC1A and CAT is presented here. Each target gene's expression, in this example for each probeset associated to the aforementioned genes, is compared to its expression in healthy samples for which the FOXO pathway is known to be active in its tumor-suppressive form and not in its tumor-promoting form.

In this example a threshold is defined as the average of the expression levels in healthy tissue samples plus three times the standard deviation, alternatively one can use two times the standard deviation or any other positive number, in case the gene is upregulated as a result of oxidative stress-induced activity of the FOXO pathway, which is the case for SOD2 and BNIP3. In case the gene is upregulated in the normal FOXO activity, which is the case for PCK1, MXI1, PPARGC1A and CAT, the threshold is set at the average minus three times the standard deviation of the expression in the healthy samples. Then a score is calculated for each sample by adding a point for every expression for which the expression exceeds or is lower than the set threshold for the genes upregulated by oxidative stress and upregulated in the tumor-suppressive FOXO activity, respectively.

Here the results of this score using a publically available dataset of colon samples, GSE20916, containing healthy colon samples depicted as normal colon samples, adeno(carcinomas) and colon carcinomas are shown using all genes and probesets shown in Table 1.

**Table 1: oxidative stress-induced FOXO target genes**

| | |
|---|---|
| SOD2 | 1566342_at |
| SOD2 | 215078_at |
| SOD2 | 215223_s_at |
| SOD2 | 216841_s_at |
| SOD2 | 221477_s_at |
| MXI1 | 202364_at |
| PCK1 | 208383_s_at |
| BNIP3 | 201848_s_at |
| BNIP3 | 201849_at |
| CAT | 201432_at |
| CAT | 211922_s_at |
| CAT | 215573_at |
| PPARGC1A | 1569141_a_at |
| PPARGC1A | 219195_at |

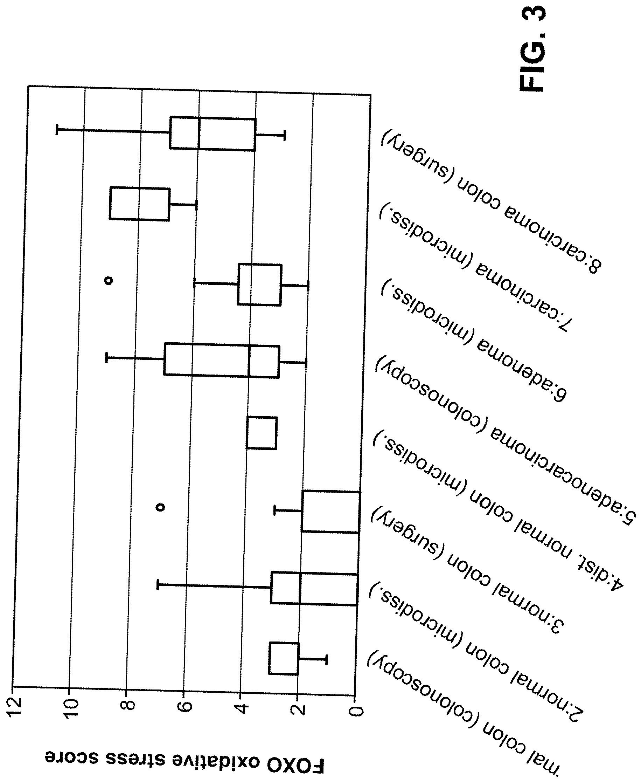

The score for each subgroup specified in GSE20916 are shown in Figure 3. As expected the scores of the carcinoma samples are higher than the normal (healthy) samples, which is an indication that one can detect tumor-promoting FOXO activity in colon carcinoma samples (group 7 and 8) using the expression levels of the aforementioned genes as their FOXO oxidative stress scores are clearly higher than the scores in the healthy colon samples (groups 1 - 4). In contrast, many of the adenoma samples (group 5 and 6) also have higher FOXO oxidative stress scores compared to normal colon tissue as is evident by the higher FOXO oxidative stress score.

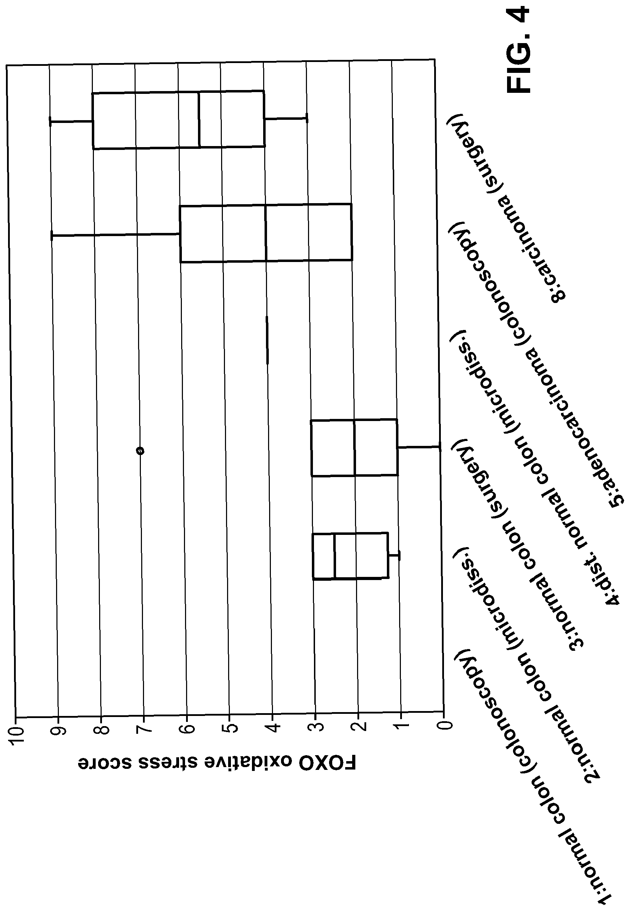

Figure 4 depicts the FOXO oxidative stress score for only those samples from GSE20916 that had FOXO activity determined using the FOXO activity model described in WO 2015/101635 A1. Also in this case the colon carcinoma samples (group 7 and 8) have a higher FOXO oxidative stress score compared to the vast majority of the normal or healthy colon samples (groups 1 - 4). Also here the adenocarcinoma samples (group 5) seem to lie in between the normal and colon carcinoma samples, however only one-third of the adenocarcinoma samples were found to have an active normal FOXO TF element.

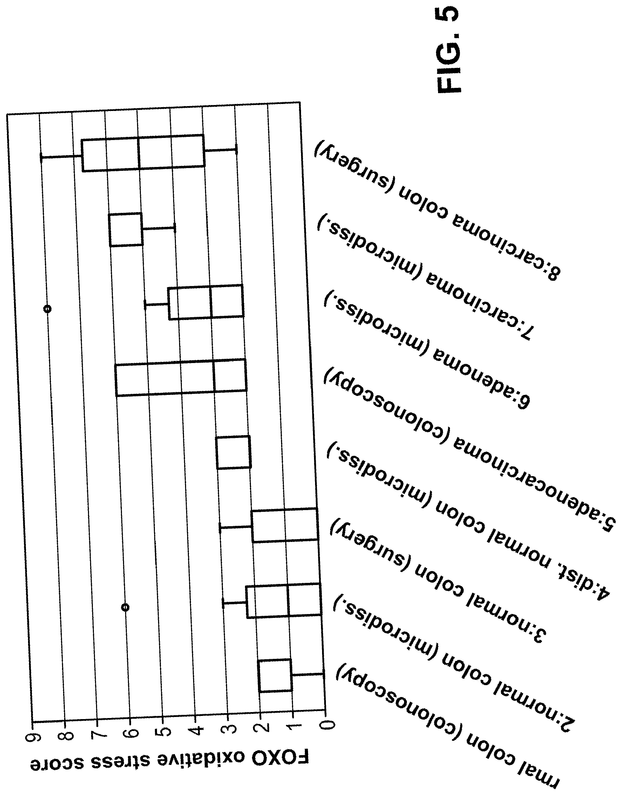

Results obtained by using only the most informative oxidative stress-induced FOXO target genes SOD2, MXI1, PCK1 and BNIP3 for all samples from GSE20916 are shown in Figure 5. A similar behavior is observed.

As an alternative to this discrete score based on the actual measured gene expression levels on a continuous scale an alternative transformations of the expression value is proposed:
- "z-score", i.e. continuous expression levels scaled such that the average across all samples is 0 and the standard deviation is 1,
- "fuzzy", i.e. the continuous expression levels are converted to values between 0 and 1 using a sigmoid function of the following format: 1 / (1 + exp((thr - expr) / se)), with expr being the continuous expression levels, thr being the threshold as mentioned before and se being a softening parameter influencing the difference between 0 and 1.

### Example 2: Exemplary embodiment improving the Bayesian network described in WO 2015/101635 A1

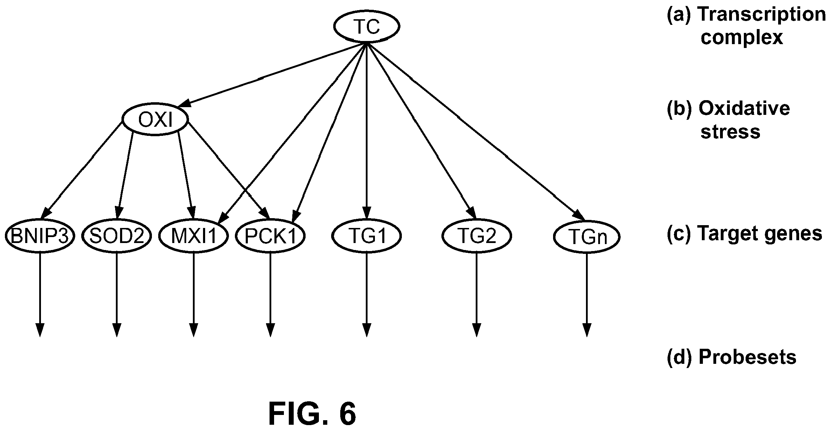

As an alternative method to model oxidative stress in the FOXO pathway, the Bayesian network described in WO 2015/101635 A1 was improved to include a separate module that represents the oxidative stress-induced FOXO activity. The structure of the Bayesian network remains the same, except for the fact that an extra node is included that defines the oxidative stress state. This node is referred to as OXI and it has two states: it is either active or inactive. Figure 6 schematically represents the FOXO model structure incorporating oxidative stress by adding an oxidative stress node. Nodes representing probesets have not been shown for readability purposes; Table 1 shows the probesets associated with BNIP3, SOD2, MXI1 and PCK1. The probesets associated to the target genes only connected to the TC (transcription complex) node are in this example embodiment the same as described in WO 2015/101635 A1. As can be seen in figure 6, there is a directed edge between the FOXO pathway and oxidative stress. This allows to incorporate the knowledge that it is not possible to have an inactive FOXO pathway and at the same time oxidative stress.

As defined earlier, there are four genes (SOD2, BNIP3, PCK1 and MXI1) that are most informative indicators for oxidative stress-induced FOXO activity. Therefore, between the node OXI and genes SOD2, BNIP3, PCK1 and MXI1 directed edges can be found. Genes SOD2 and BNIP3 have higher expression levels in case there is oxidative stress, which is why there is only a directed edge from OXI and not TC. The genes PCK1 and MXI1 are affected by both the oxidative stress FOXO and the 'normal' FOXO nodes. Therefore, these genes have directed edges from both the TC node and the OXI node. The rest of the genes in this model are affected by the FOXO pathway only and for that reason there are only directed edges between the TC state and the rest of the target genes. Here a Bayesian network with these four FOXO oxidative stress genes is shown but a person skilled in the field can easily extend this to include the remaining two genes, PPARGC1A and CAT, as well.

As can be seen in WO 2015/101635 A1, all nodes in the network have to be quantified by means of conditional probability tables (CPT) to allow for quantitative reasoning. The CPTs of the genes and the calibration of probesets as shown in Table 1 need to be altered slightly due to the addition of the oxidative stress node. In WO 2015/101635 A1 the CPT of the edges between the probesets and target gene nodes were calibrated on samples where the pathway activity is known, but now samples known to have oxidative stress-induced FOXO activity need to be used as well. The reason for this is that a distinction in expression levels for the genes affected by oxidative stress needs to be made. Therefore, probesets of genes affected by the oxidative stress node only are calibrated using samples where the pathway is known to be switched off as inactive samples and samples with oxidative stress as active ones. For the probesets of PCK1 and MXI1 the information that they have higher expression levels in samples where FOXO is active, but only in the situation, where oxidative stress is not the cause of FOXO activity, is used. Therefore, samples that are known to have oxidative stress-induced FOXO activity are chosen as inactive samples for these probesets while the normal active FOXO samples (no oxidative stress) are chosen as active ones for these probesets. For probesets belonging to genes affected by the FOXO TC node only the calibration remains the same. The CPTs between the TC and target genes and the genes that have edges from the OXI state only are the same as in WO 2015/101635 A1. The newly defined CPT between the TC and OXI is shown in Table 2. This table reflects the knowledge that oxidative stress is unlikely with an absent TC, while no prior knowledge regarding oxidative stress in case of a present TC is available. Another CPT needs to be defined for the genes that have directed edges from both the TC and the OXI node. As both TC and OXI have two states, they combine for a total of four possibilities, so this table has eight entries. This table, defined for PCK1 and MXI1 in this case, is shown in Table 3.

**Table 2: conditional probabilities for P[OXI|TC].**

| P[OXI\|TC] | OXI = inactive | OXI = active |
|---|---|---|
| TC = absent | 0.95 | 0.05 |
| TC = present | 0.5 | 0.5 |

**Table 3: conditional probabilities for the target genes affected by both TC and OXI; P[TG|TC, OXI].**

| P[TG\|OXI, TC] | 'inactive' | OXI = | | OXI = 'active' |
|---|---|---|---|---|
| | TG = 'down' | TG = 'up' | TG = 'down' | TG = 'up' |
| TC = 'absent' | 0.95 | 0.05 | 0.95 | 0.05 |
| TC = 'present' | 0.3 | 0.7 | 0.95 | 0.05 |

In the following, samples from the data set GSE20916 are used to calibrate such a Bayesian network. This data set contains normal, adenoma, adenocarcinoma and carcinoma colon samples. These samples allow to calibrate the network since experimental evidence suggests that in normal colon samples the FOXO pathway is active, but that there is no oxidative stress. We choose the samples GSM523290, GSM523314, GSM523289 and GSM523310 because these normal colon samples are predicted most active by the FOXO model of WO 2015/101635 A1. The oxidative stress state FOXO calibration samples chosen are the carcinoma colon samples that are predicted most active by the FOXO model of WO 2015/101635 A1: GSM523331, GSM523303, GSM523344 and GSM523323. Lastly, samples that have an inactive FOXO pathway are needed. These samples are chosen to be the most inactive carcinomas as predicted by the FOXO model of WO 2015/101635 A1: GSM523372, GSM523313, GSM523332 and GSM523283.

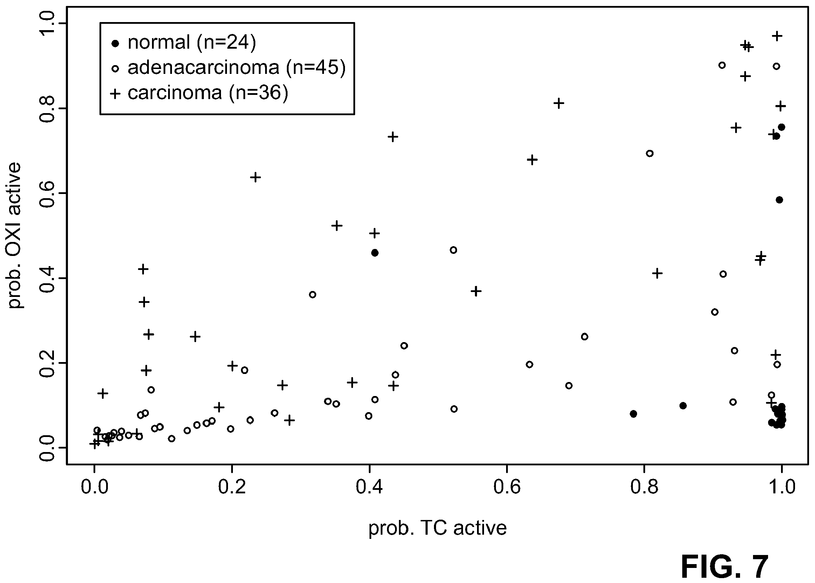

In Figure 7, the described model was tested on the dataset GSE20916. For each sample in this set, the probability of the FOXO pathway to be active was calculated, the probability of the TC node, and the probability of the FOXO pathway to be in the oxidative stress state, i.e. the probability of the OXI node. Almost all normal colon samples, shown as black dots, are predicted to have an active FOXO pathway without oxidative stress, which is in line with the expectations. The adenocarcinoma and especially the colon carcinoma samples show a noticeably different combination of the FOXO and oxidative stress-induced activity compared to the normal samples. This shows that it is possible to distinguish normal FOXO activity from oxidative stress activity.

These results demonstrate the aforementioned FOXO target genes, PCK1, MXI1, SOD2 and BNIP3, to be indicative of oxidative stress-induced activity, in other words tumor-suppressive or -promoting activity of the FOXO pathway, and can be construed in such a Bayesian network that is able to better detect the tumor-suppressive or oxidative stress associated activity of the FOXO pathway, which may be tumor promoting. This is a novel and inventive technical enablement as the addition of the OXI node dependent on the nodes of a subset of FOXO target genes, indicative of the oxidative stress-induced activity of the FOXO pathway, as well as the node of the 'normal' FOXO activity results was not mentioned or hinted upon in the prior art.

The method presented herein may be used, for instance, in diagnosing an (abnormal) activity of the PI3K cellular signaling pathway, in prognosis based on the inferred activity of the PI3K cellular signaling pathway, in the enrollment of a medical subject in a clinical trial based on the inferred activity of the PI3K cellular signaling pathway, in the selection of subsequent test(s) to be performed, in the selection of companion diagnostics tests, in clinical decision support systems, or the like. In this regard, reference is made to the published international patent application WO 2013/011479 A2 ("Assessment of cellular signaling pathway activity using probabilistic modeling of target gene expression") and to the published international patent application WO 2014/102668 A2 ("Assessment of cellular signaling pathway activity using linear combination(s) of target gene expressions"), which describe these applications in more detail.

### Example 3: Selection of the genes indicative of oxidative stress

For different tissue origins, differential mRNA expression of the oxidative stress-associated genes between cancer tissue with an active FOXO3 score as assessed by the FOXO3 model and corresponding normal tissue with an active FOXO3 score according to the model was determined. Gene expression levels as measured by gene-specific probesets on an Affymetrix microarray (GeneChip® Human Genome U133 Plus 2.0 Array) were obtained from public GEO datasets and averaged over a larger number of tissue samples from such a dataset. Subsequently, average mRNA expression levels for the oxidative stress associated genes BNIP3, MXI1, PCK1, PPARGGC1, SOD2, from non-malignant normal tissue were subtracted from the respective average level of the same mRNA measured in a cancer tissue sample, or a non-cancer condition, from the same tissue origin. E.g. normal lung average expression level for SOD2 gene (two probe sets, 215223_s and 216841_s) was subtracted from the average expression level of several different lung cancer subtypes. Subtracted tissue sample types are indicated to the left in table 4, the subtracted average mRNA expression level is indicated below each gene symbol to the right, for each probeset of the respective genes. The numbers in boxes indicate the level of differential expression and a positive value indicates that the subtraction resulted in a positive result; a negative level indicates a negative result. In the non-malignant conditions colon adenoma (A), Barrett's esophagus (C) and endometroid endometrium (E), oxidative stress-associated genes were not overexpressed. In contrast, in most cancer types (A-D, F-H), the oxidative stress-associated genes are overexpressed compared to corresponding normal tissue. Similar subtraction results are shown for FOXO3 and ESR expression levels, to indicate that these transcription factor levels were not differentially expressed. The GEO dataset containing samples from normal colon, colon adenoma and colon carcinoma (A) was used at the lead set to discover the oxidative stress-associated genes. The other GEO datasets (B-H) are presented to validate the oxidative stress gene panel in non-colon related cancer types and non-malignant conditions.

### Example 4: Use of SOD2 to distinguish between the two functional states of FOXO activity

The PI3K signal transduction pathway is commonly hyper-activated in cancer. Tumors are potentially sensitive to PI3K pathway inhibitors but reliable diagnostic tests assessing functional PI3K activity are lacking. As the PI3K pathway negatively regulates FOXO transcription factors, FOXO target gene expression is inversely correlated to PI3K activity. A knowledge-based Bayesian computational model was developed inferring PI3K activity in cancer tissue samples using FOXO target gene mRNA levels. In various cancer cell lines it was observed with this model that addition of PI3K inhibitors caused an increase in FOXO activity, confirming a decrease in PI3K pathway activity. In tissue samples FOXO activity was predicted to be active in multiple cancer types of varying aggressiveness. Cellular oxidative stress is associated with cancer and an alternative activator of FOXO, frequently associated with PI3K pathway activity. SOD2 was found differentially expressed between the two modes of FOXO activation. Threshold levels for healthy tissue SOD2 expression were defined, above which FOXO activity was considered oxidative stress-induced. In slowly growing Luminal A breast cancer and low Gleason prostate cancer FOXO was typically active in PI3K-mediated fashion, indicating an inactive PI3K pathway. In more aggressive Luminal B, HER2 and basal-like breast cancer FOXO was frequently found either inactive, or active induced by oxidative stress, indicating high likelihood of PI3K pathway activity. A decision tree facilitates assessment of PI3K pathway activity in cancer samples. This mRNA-based FOXO-model may be used for response prediction of ErbB-PI3K pathway targeting drugs.

The past decade, systemic treatment of cancer moved from conventional chemotherapy towards administration of drugs that target tumor traits chosen on an individual patient basis. This "precision medicine" approach requires biomarkers that reliably predict response to targeted drugs (1). Cancer growth and metastasis are driven by roughly ten to twelve cellular signal transduction pathways, relatively independent of the cancer cell type of origin (2-4). One of these, the PI3K pathway, one of the main cellular growth factor signaling pathways, is frequently hyperactivated in cancer as a result of genomic changes like receptor tyrosine kinase amplification, loss of PTEN, activating mutations in *PIK3CA,* or by stimuli from the cancer cell micro-environment (2). PI3K pathway inhibitors are used in cancer treatment either alone or in combinations with other targeted strategies or conventional chemotherapy (5-7). Despite selection of potentially responsive patients based on PI3K pathway mutation analysis, only a subpopulation of patients respond adequately to the drugs (8,9). To improve prediction of drug response and monitor therapy efficacy or emerging resistance, tests measuring functional PI3K activity are needed.

Previously, a knowledge-based computational approach was described to assess signal transduction pathway activity in cancer tissue samples, based on measuring target gene mRNA levels of the pathway-specific transcription factor (10,11). Now development of an mRNA-based diagnostics for quantitative assessment of PI3K pathway activity is reported that uses Forkhead Box O (FOXO) transcription factor-induced transcription as readout, based on the well-known inverse relationship between FOXO and PI3K pathway activity (12-16). The model was biologically validated using breast and lung cancer cell lines treated with PI3K pathway inhibitors or carrying doxycycline inducible active FOXO3 constructs.

In cancer tissue FOXOs can be alternatively activated by cellular oxidative stress, a common trait in cancer which interferes with the inverse relationship with PI3K pathway activity. The SOD2/MnSOD FOXO target gene level was used to distinguish between the two functional states of FOXO activity, resulting in a robust approach to assess PI3K pathway activity in individual patient cancer samples.

### Methods

### Cell culture-based model system for FOXO3 activity

MCF7 and MDA-MB-231 breast cancer cell lines were cultured in DMEM-F12 containing 10% FBS (Lonza) 100U/ml penicillin and 100 microgram/ml streptomycin (Lonza). Transfecting third generation packaging vectors using Polyethylenimine into HEK293T cells generated lentiviral particles (17). MCF7 and MDA-MB-231 cells were stably transduced with lentivirus containing pINDUCER20-FOXO3.A3, allowing doxycycline induced expression of constitutively active FOXO3 (FOXO3.A3) (13,18,19). Cells were treated with 20% FBS or 10 M PI3K inhibitor LY294002 (Selleckchem) for 16 hours to activate and inactivate the endogenous PI3K pathway, respectively. FOXO3.A3 expression was induced by 16 hours treatment with 10 ng/ml doxycycline.

### RNA isolation and Affymetrix microarray hybridization

Treated cells were harvested after 16 hours of the respective incubations as indicated, RNA was isolated using the RNeasy kit (Qiagen), and hybridized on Affymetrix HT HG-U133+ PM Array Plate by ServiceXS (GenomeScan, Leiden, The Netherlands, http://www.genomescan.nl) and Eurofins AROS Denmark (http://arosab.com/).

### Quality control on Affymetrix

On all Affymetrix microarray data, both from experiments performed for this study, as well as from datasets from the public GEO database, an extensive quality control was performed. All microarrays that were used were from either Affymetrix HG-U133Plus2.0 or Affymetrix HG-U133+PM microarrays, which have been processed with fRMA with 'random effect' summarization (23). In principle, the Affymetrix HG-U133+PM platform contains all the perfect match probes of the HG-U133Plus2.0 platform, albeit with some minor reselections. To make the processed data comparable from both microarray types, a chip description file was used that contains only the shared probes, and took the processing parameters of this subset from the HG-U133Plus2.0 frmavecs to process data from both platforms.

Quality control of the microarray samples has been performed using several quality checks. These checks include the average value of all PM probes intensities, negative or extreme (> 16-bit) intensity values, poly-A RNA (sample preparation spike-ins) and labelled cRNA (hybridization spike ins) controls, ACTB and GAPDH 3'/5' ratio, values and center of intensity of the positive and negative border controls determined by the affyQCReport package and an RNA degradation value determined by the AffyRNAdeg function from the affy package. Samples from the breast and colon cancer datasets not passing the quality criteria were removed from further analyses.

Table 5 shows the datasets that have been used and in which Figures they appear

**Table 5:**

| **Dataset** | **Preprocessing** | **Purpose** | **Appearance** |
|---|---|---|---|
| GSE16573 | fRMA | Calibration | Figure 8 |
| Proprietary data | PMfRMA | Validation | Figure 9 |
| GSE51212 | fRMA | Validation | Figure 10 |
| GSE30516 | fRMA | Validation | Figure 10 |
| GSE8671 | fRMA | Colon | Figure 11 |
| GSE20916 | fRMA | Colon | Figure 11 |
| GSE14333 | fRMA | Colon | Figure 12 |
| GSE37364 | fRMA | Colon | Figure 12 |
| GSE39084 | fRMA | Colon | Figure 12 |
| GSE40967 | fRMA | Colon | Figure 12 |
| GSE4183 | fRMA | Colon | Figure 12 |
| E-MT AB-365 | fRMA | Breast | Figure 12 |
| GSE10780 | fRMA | Breast | Figure 12 |
| GSE12276 | fRMA | Breast | Figure 12 |
| GSE17907 | fRMA | Breast | Figure 12 |
| GSE20685 | fRMA | Breast | Figure 5 |
| GSE21653 | fRMA | Breast | Figure 5 |
| GSE26910 | fRMA | Breast | Figure 5 |
| GSE42568 | fRMA | Breast | Figure 5 |
| GSE45827 | fRMA | Breast | Figure 5 |
| GSE6532 | fRMA | Breast | Figure 5 |
| GSE7307 | fRMA | Breast | Figure 5 |
| GSE9195 | fRMA | Breast | Figure 5 |

### Western Blotting

Western blot analysis was performed using standard 6-15% SDS-PAGE. Proteins were detected with primary rabbit antibodies (1:2000) for FOXO3 (H144, Santa Cruz). Blots were incubated with HRP-conjugated secondary antibodies at 4°C for 16 hours. Proteins were visualized with enhanced chemiluminescent (Biorad) using an ImageQuant LAS 4000 scanner (GE healthcare).

### Immunofluorescence and immunohistochemistry

For immunofluorescent staining, cells were grown on glass coverslips, fixed using 4% paraformaldehyde and blocked with PBS containing 2% bovine serum albumin (BSA) (Invitrogen) and 0.1% normal goat serum (Invitrogen). Cells were incubated with FOXO3 antibody (Foxo3A Rabbit MAb, 1:500 CST-75D8), secondary Alexa563 conjugated antibodies and DAPI (Sigma). Slides were imaged on a Zeiss LSM710 confocal microscope.

For FOXO3 immunohistochemistry staining 4µm sections of formalin fixed paraffin embedded (FFPE) tissue samples were deparaffinized and rehydrated. After blocking of endogenous peroxidase activity, antigen retrieval was performed with TE buffer at pH 9.0 (Dako) in a water bath at 95-96°C for 25 minutes. After cooling for at least 15 minutes and washing steps with PBS the samples were blocked with 1% BSA in PBS for 15 minutes. Then the sections were incubated with FOXO3 antibody (1:50, CST-75D8) for 1 hour at room temperature. Visualization was achieved using the Dako Envision+ TM - System anti-Rabbit-HRP (DAB). As a counterstain Gill's 2 Heamatoxilin is used. Images were generated with a 3D Histech scanner. Negative controls consisted of sections that underwent similar staining procedures without addition of the primary antibody. As a positive control non-malignant tonsil tissue is used.

### Development of the computational model for predicting FOXO activity

Development of the computational model for FOXO transcriptional activity is based on probabilistic Bayesian network inference, as previously described (11).

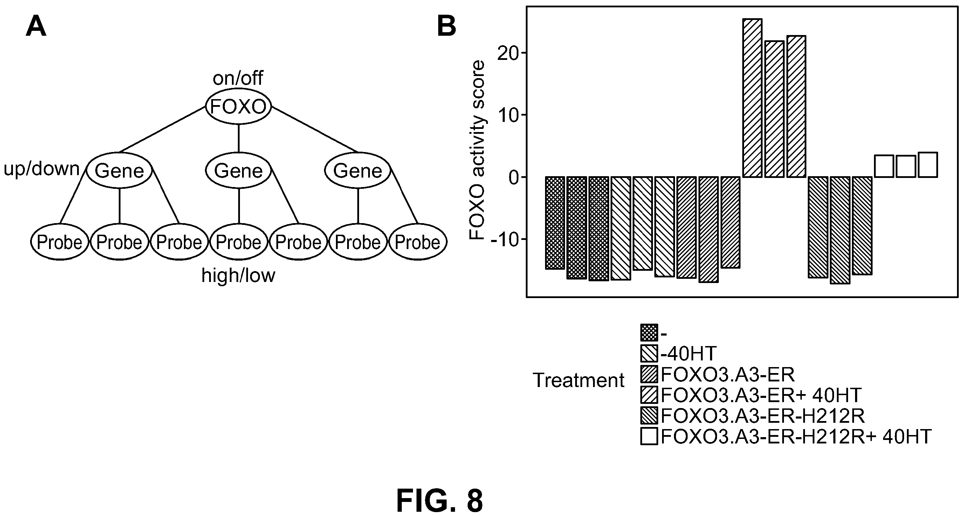

The signal transduction pathway modeling approach is based on inferring pathway activity from the expression profile of its target genes using probabilistic Bayesian network inference. Previously, such models were developed to determine the functional activity of the Wnt and ER pathways. Bayesian networks were built using the Bayes Net Toolbox for MATLAB, as described earlier. The Bayesian network structure used as a basis for the modeling approach (Figure 8A) is a simplified model of the transcriptional program of a cellular signal transduction pathway, consisting of three types of nodes: (a) transcription complex, (b) target genes and (c) microarray probesets corresponding to target genes. The model describes (i) how expression of target genes depends on transcription complex activation, and (ii) how probeset intensities in turn depend on expression of the respective target genes.

The probabilistic relations in the Bayesian network model were made quantitative using experimental data to enable quantitative reasoning on new experimental samples. Parameters describing relationships between target genes and their respective probesets (ii) were trained on HUVEC cell lines with a stable transfection of a FOXO3.A3.ER construct stimulated for 12 hours with 4-OHT resulting in an active FOXO transcriptional program, used as FOXO3 active training samples, and without stimulation, used as FOXO3 inactive training samples (public data available in GSE16573 (20). With the PI3K pathway, the activity score of the pathway being active scales inversely with the probability of the FOXO3 transcription factor being in the active transcribing state. Parameters enforcing the relationships between the transcription complex and target genes (i) were manually set as described elsewhere (11) to improve generalization behavior of the model across different tissue types.

Once the model has been calibrated, it could be used on microarray (Affymetrix HG-U133Plus2.0) data of new tumor samples by entering probeset measurements as observations in the bottom layer, and inferring backwards in the model the activity probability of the FOXO3 transcription factor. The model was frozen and applied to microarray data of cell line and tissue samples by entering probeset measurements as observations into the model, inferring backwards FOXO transcription factor activity score as the log-2 value of the FOXO transcription factor odds p/(1-p). Samples are classified as FOXO active if the pathway activity exceeds an activity score above 0, corresponding with an odds above 1 to 1 that the pathway is active, and inactive in case activity score is below 0.

Parameters enforcing relationships between the FOXO transcription complex and target genes were manually set to improve generalization behavior of the model across different tissue types (11). Parameters describing relationships between target genes and their respective probe sets were calibrated on a public data set of Human Umbilical Vein Endothelial Cells (HUVEC) carrying inducible constitutively active FOXO3.A3-ER, and a threshold for FOXO-activity was set (GSE16573) (20). The model was frozen and applied to microarray data of cell line and tissue samples by entering probeset measurements as observations into the model, inferring backwards FOXO transcription factor activity score as the log-2 value of the FOXO transcription factor odds (p/(1-p). For validation purposes, FOXO activity analysis was always performed on independent fRMA (unless otherwise indicated) preprocessed Affymetrix HG-U133Plus2.0 microarray data from described experiments and from public GEO datasets.

### Identification of direct target genes for FOXO3A

For optimal performance, across multiple different tissue types, the mathematical model should contain direct target genes of the FOXO transcription factor. Unfortunately, pathway databases such as KEGG (www.genome.jp/kegg) and Biocarta (www.biocarta.com) are incomplete and inconsistent on this aspect (23). Hence, target genes were manually selected based on extensive scientific evidence for each individual gene being a direct target gene of the respective transcription complex, including promotor region enhancer motif analysis, transcription factor binding experiments (EMSA and ChIP), gene promoter luciferase reporter experiments, and differential mRNA expression analysis. Available literature retrieved from the MEDLINE database using PubMed for FOXO target genes was extensively evaluated. In addition, target genes were extracted from Thomson-Reuters' Metacore by selecting only genes that had multiple sources of reliable evidence for being transcriptionally regulated by one or more of the FOXO family members. Ultimately, the target genes were ranked according to literature evidence using a similar methodology as described earlier (11). Only the highest ranked target genes, also included in the list published by van der Vos and Coffer (24) were selected as "bona fide" target genes.

### SOD2 level associated with oxidative stress

To investigate differences in individual FOXO target gene expression levels between FOXO-active samples of healthy and tumor tissue, publicly available microarray datasets were used from healthy and corresponding pre-malignant or malignant tumor tissue samples (Table 7).

Table 7 shows the average expression levels (standard deviation in italics) of the two FOXO3 target genes SOD2 and BNIP3 as measured by the indicated probesets on the Affymetrix microarray, in samples scored as FOXO active by the computational FOXO model. GEO datset numbers are indicated in the table. A. Samples are selected from GEO samplesets based on a FOXO3 activity probability score (according to the FOXO model) above 5.6 (probability above 0.98); B. Samples are selected from GEO samplesets based on a FOXO3 activity probability score (according to the FOXO model) above 0.

Normalized intensities on a log2-scale measured on the different gene-specific probesets on the microarray reflect gene expression levels. FOXO target gene expression levels were compared between FOXO-active tumor and healthy tissue samples.

To establish variation in SOD2 expression levels in the absence of oxidative stress, mean + 2SD was determined for Affymetrix SOD2 probeset values for different types of healthy normal tissue. In FOXO-active samples with SOD2 mRNA levels exceeding these threshold levels, FOXO was considered as oxidative stress-induced.

### Subtyping breast cancer samples from public datasets according to Perou

Intrinsic breast cancer subtypes of all breast cancer samples were determined from microarray data according to the method described by Parker and co-workers (21,22).

Intrinsic subtypes were determined from the Affymetrix microarray data using the methodology as described by Parker and co-workers (21) and the Prosigna Packet Insert **(technologies, nanoString.** Package Insert Prosigna Breast Cancer Prognostic Gene Signature Assay. s.l. : http://prosigna.com/docs/Prosigna_Packet_Insert_US.pdf, 2015). fRMA-normalized gene expression of all 50 genes included in PAM50 was extracted from the microarray data using the probesets associated with the PAM50-genes. The probeset with the highest variance was selected in case more than one probeset was associated with a single gene. Centroids for the luminal A, luminal B, HER2 enriched, basal and normal-likes were calculated using the samples from GSE21653 with given subtypes. Next, Pearson's correlation coefficients with these centroids were calculated for all samples. Each sample was assigned to the subtype with the highest correlation.

### Results

### Development of a computational model for PI3K-FOXO activity

A Bayesian network-based computational model for FOXO activity was created which infers FOXO transcriptional activity from 26 FOXO target gene mRNA levels in a tissue sample (Fig 8A). While inferring transcription factor activity from the mRNA level of one target gene is not sufficiently specific, inferring activity from expression levels of a larger number of target genes, typically between 20 and 30, appears to be a highly specific way to quantify associated transcription factor activity. For optimal performance across multiple different tissue types, direct target genes are selected. Since pathway databases such as KEGG (www.genome.jp/kegg) and Biocarta (www.biocarta.com) are inconsistent on this aspect (23), genes were manually selected based on scientific evidence from literature (PubMed) and Thomson-Reuters' Metacore, and ranked using a similar methodology as described earlier (11). Highest ranked genes were selected to build the computational FOXO pathway model (16,24) (Table 6). HUVECs with inducible FOXO activity provided "ground truth" evidence for FOXO activity status in an untransformed setting, and were used to calibrate the model, prior to model freeze (Figure 8B).

Predictions of the Bayesian-model are consistent with known experimental FOXO activity status in the complete HUVEC data set including independent sample data (Figure 8B). HUVECs, HUVECs treated with 4OHT, and HUVEC-FOXO3.A3-ER were predicted to have low FOXO activity and therefore active PI3K signaling, as expected in proliferating cells. HUVEC-FOXO3.A3-ER treated with 4OHT are predicted to have highly active FOXO, in line with the induction of constitutively active FOXO3.A3. In HUVECs expressing FOXO3.A3-ER-H212R, a mutant version of FOXO3 with reduced DNA binding capacity, the model predicted FOXO to be inactive in untreated cells and low FOXO3 activity in cells treated with 4OHT (20). These observations confirm that the model specifically detects transcriptional changes induced by FOXOs, and is sensitive to low levels of FOXO activity.

### Biological validation of the PI3K-FOXO model in breast cancer cell lines

Following calibration, the model was biologically validated in independent breast cancer cell lines. ER positive, PIK3CA^{E545K} mutant MCF7 and triple negative MDA-MB-231 cells were stably transduced with a doxycycline inducible FOXO3.A3 expression vector, allowing rapid and controlled induction of FOXO3 protein expression and transcriptional activity upon treatment with doxycycline for 16 hours (Figure 9A). FOXO3 protein was predominantly detected in the cytoplasm in untreated and 20% FBS stimulated cells; switching to dominant nuclear localization in cells treated with doxycycline, PI3K inhibitor LY294002, and doxycycline in combination with LY294002. This shows that nuclear translocation of FOXO3 was induced in a controlled manner in this experimental cell culture system. On Affymetrix mRNA expression data from this cell model, the FOXO model predicted respectively low FOXO activity in untreated (PI3K pathway active), and high FOXO activity in doxycycline-treated (PI3K pathway inactive) MCF-FOXO3.A3 and MDA-MB-231-FOXO3.A3 cells; low FOXO activity in 20% FBS treated MCF7 cells (PI3K pathway active), and high activity in doxycycline, LY294002 and combined doxycycline + LY294002 treated cells (all PI3K pathway inactive) (Figure 9B/C). Together these results confirm that the computational FOXO model predicted FOXO activity as expected in independent cancer cell line samples.

### Use of the pathway model to predict and monitor response to drugs.

On the premise that FOXO activity is inversely related to PI3K activity in cell lines, it was investigated whether the FOXO model was capable of predicting response to drugs targeting receptor tyrosine kinase activity in independent cancer cell line data sets (GSE51212, GSE30516). FOXO3 activity was scored in an EGFR-mutant HCC827 lung cancer cell line either treated with Erlotinib (EGFR inhibitor), Selumetinib (MEK inhibitor) or BEZ235 (PI3K/TOR dual inhibitor). FOXO scored inactive in untreated samples indicating the active PI3K pathway; upon treatment with any of the three drugs FOXO scored active, maximal with the EGFR inhibitor, confirming that all three drugs were effective in directly and/or indirectly blocking activity of the PI3K pathway (Figure 10A). In three breast cancer cell lines representing triple-negative (BT20), ER positive (MCF7), and HER2 positive breast cancer (MDA-MB-453) the FOXO activity score increased as expected when treated with Erlotinib (Figure 10B).

### FOXO activity in healthy colon and colorectal cancer tissue samples

For evaluation of the model when used on patient tissue samples, a number of selected public datasets were used. First the FOXO activity model was applied on tissue samples derived from patient biopsies of 32 normal colon tissues and 32 adenoma tissues (GSE8671). A clear difference in FOXO activity score was observed between healthy colon and adenoma tissue, showing FOXOs to be active and inactive respectively, indicating expected activation of the PI3K pathway in colon adenoma (Fig. 11A).

Second, the FOXO model was applied on a patient tissue set containing normal colon, benign colon adenoma and colon carcinoma tissues (GSE20916) (Figure 11B). In normal colon tissue samples FOXO was predicted active. In the majority of adenoma tissue samples FOXO was predicted to be inactive, in line with the findings in the first data set, and indicating PI3K pathway activity. However, in half of the colon carcinoma tissue samples FOXOs were predicted to be active. Since colon carcinomas are thought to arise from colon adenomas, at least the same frequency of PI3K pathway activity was expected.

Thus, in up to about one third of the cancer tissue samples a high FOXO activity score would be expected to indicate an inactive PI3K pathway. In the rest of the FOXO active cancer samples there may be another cause of high FOXO activity, one being admixture of healthy cells in which FOXO is generally active. To investigate this, a FOXO3 immunohistochemistry (IHC) staining was developed to determine cytoplasmic and nuclear FOXO3 localization in tissue samples. FOXO3 was found mainly cytoplasmic in healthy colon crypt cells but present in the nucleus in healthy mucosa cells, and in other non-tumor healthy cells, in line with the mRNA-based FOXO-activity score seen in the public dataset GSE20916 (but not the same samples) (Figure 11B/C). These results suggest that admixture of normal cells may cause a false positive FOXO score. In colon adenoma cells FOXO3 showed cytoplasmic localization while colon carcinoma cells explicitly displayed heterogeneous FOXO3 localization with areas of nuclear and other areas with cytoplasmic staining.

Another well-described cause for FOXO activity likely to be present in aggressive cancer tissue is cellular oxidative stress (12,25). However, the function of FOXO activity in normal tissue is clearly different from the function during oxidative stress, and is therefore likely to be reflected in a difference in transcribed target genes.

To determine which genes within the used FOXO target gene set would discriminate best between FOXO activity in normal and in carcinoma samples, target gene expression levels were compared between normal colon and colon cancer tissue samples in which FOXOs were predicted to be active by the model. *SOD2* and *BNIP3* gene expression was strongly increased in FOXO-active cancer tissue samples compared to normal colon tissue (Table 7). Both genes play a role in response to oxidative stress and are transcribed by FOXOs under these circumstances, making them prime candidates to distinguish between the two modes of FOXO activity (26,27).
Indeed, comparing SOD2 and BNIP gene expression levels between various FOXO active normal tissues and corresponding pre-malignant or malignant tumor tissue samples (colon cancer, breast cancer, Barrett's esophagus, esophageal cancer, bladder cancer, and gliomas) confirmed increased expression levels of SOD2 and to a lesser extent BNIP3 in FOXO-active samples from aggressive cancer types (Table 7). In remarkable contrast, in FOXO active samples of two benign hyperproliferative conditions, colon adenoma and Barrett's esophagus, FOXO activity was not associated with increased SOD2 and BNIP3 expression, indicating that in these benign tumors FOXO was activated in a PI3K-regulated manner comparable to healthy tissue. Since SOD2 showed the most generalized and profound differential expression between FOXO-active normal tissue samples and corresponding FOXO-active cancer types, this gene was selected as the most reliable parameter to distinguish between the two modes of FOXO activity. The SOD2 upper threshold level for PI3K-regulated (non-oxidative stress) FOXO activity was defined as two standard deviations above the mean expression level in normal tissue, and was calculated for healthy colon, breast and prostate tissue.
Subsequently, the FOXO model in combination with the SOD2 expression threshold was applied to independent publicly available data sets with data from individual patients with colon, breast, and prostate cancer. In FOXO-active samples with elevated SOD2 expression PI3K pathway activity cannot be directly inferred from FOXO activity. Also, in case FOXO is scored inactive by the model, knowledge on FOXO expression is required for a conclusion with regard to PI3K activity. FOXO3 is considered the most relevant FOXO gene in cancer and was consistently expressed in all samples from all cancer types that were analysed to date, including from breast, colon, prostate, brain, bladder, and esophagus (Table 7).

### Prediction of FOXO activity mode (canonical or oxidative stress) in primary colon adenoma and carcinoma tissue samples

Analysis of a large set of healthy colon tissue samples (n = 121) allowed a threshold setting for normal SOD2 mRNA levels in FOXO-active samples (Figure 12A). Subsequently an extended independent set of patient colon adenoma and carcinoma sample data was compiled and FOXO activity and SOD2 expression levels determined. In normal colon samples only 2.6% of FOXO active samples had SOD2 expression exceeding the threshold level. In the few adenoma samples that were FOXO active (n=12, 16%), SOD2 levels were elevated (over threshold) in half. In the carcinoma samples one third of the samples were scored FOXO active, of which 53.9% with elevated SOD2 expression.

### Prediction of FOXO activity mode (canonical versus oxidative stress) in primary breast cancer tissue samples

Similarly a compiled set of data from breast cancer patients was analyzed. Prior to FOXO activity analysis, breast cancer tumor subtyping in all cancer data sets was performed using the PAM50 algorithm to ensure that the subtypes in all data sets were similarly determined (21). In line with the findings in healthy colon tissue FOXO was predicted to be generally active in normal breast tissue (85%) (Figure 12B). In the luminal B, HER2 and basal like subtypes respectively 37%, 23% and 20% scored low for FOXO, indicating PI3K pathway activity. An increasing percentage of FOXO active samples with elevated SOD2 (over threshold in healthy breast cancer) was observed with increasing cancer subtype aggressiveness: from 4.7% in luminal A to 71.4% in basal like breast cancer.

### Prediction of FOXO activity mode (canonical versus oxidative stress) in primary prostate cancer samples

In normal prostate tissue and primary prostate cancer samples from compiled public patient datasets FOXO activity analysis showed that FOXO is active in 91% of patients with lower Gleason score tumors (Gleason 4-7). Too few samples of patients with a high Gleason score (Gleason 8-9) were available to draw any comparative conclusions (Figure 12C). Interestingly, in none of the FOXO-active primary prostate cancer samples SOD2 expression was increased over the threshold level set for normal tissue, indicating that for prostate cancer PI3K pathway activity can be safely inferred from the FOXO activity score. In the majority of primary prostate cancer samples the PI3K pathway was inactive.

### Identifying tumors with an active PI3K pathway

To facilitate inference of functional PI3K pathway activity in a tissue sample, a simplified decision tree was created, based on the premises (1) that FOXO is expressed in the cancer cells from the sample and (2) measured FOXO activity derives from cancer cells (Figure 13B). Assuming the PI3K pathway is active in case of active FOXO-oxidative stress, for each investigated cancer type these samples can be added (Figure 12, FOXO active black dots) to the number of samples with inactive FOXO (indicating active PI3K pathway) to calculate the total number/percentage of patients likely to have a tumor with an active PI3K pathway (Fig 12, indicated in the table with asterix). The thus calculated percentage of PI3K pathway active samples was between 8 and 13% in normal tissue (colon, breast, prostate), and quite similar in Luminal A and Normal-like breast cancer and low Gleason score prostate cancer (respectively 13%, 15%, 15%); in Luminal B, HER2, and basal-like breast cancer the percentage samples with PI3K pathway activity was much higher, respectively 45%, 45%, and 76%; while in colon adenoma and carcinoma nearly all samples were scored as PI3K pathway active (respectively 92% and 85% of samples).

### Discussion

### A knowledge-based Bayesian model for predicting FOXO and PI3K activity

The PI3K pathway is an important proliferation and survival pathway in cancer and is a core signaling part of the ErbB growth factor signal transduction machinery. Many targeted drugs aim at blocking PI3K pathway activity at multiple locations in the pathway. Improving response rates to PI3K pathway inhibitors requires tests that reliably assess PI3K pathway activity in cancer samples. FOXO transcription factors are negatively regulated by the PI3K pathway, and can in principle be used as an inverse readout for PI3K pathway activity (5,12,28). To measure PI3K pathway activity in cancer tissue samples, a computational knowledge-based Bayesian network was developed inferring FOXO transcriptional activity from established FOXO target gene mRNA expression levels to be measured in the tissue sample at hand (11). The different FOXO members are redundant and a comparative analysis of FOXO1, FOXO3 and FOXO4-induced gene regulation indicates a large overlap between the transcriptional profiles of each FOXO member (16,24,29). Therefore the FOXO activity model, which incorporates knowledge on regulation of direct FOXO target genes, performs as a general FOXO activity predictor.

Calibrating this Bayesian model on FOXO-inducible HUVECs resulted in a computational FOXO model that predicted FOXO activity as expected in breast cancer cell lines with either constitutively active FOXO3, or incubated with PI3K pathway targeting drugs. The observation in MCF7 cells that FOXO is scored less active in cells treated with the PI3K inhibitor drug LY294002 compared to ectopic FOXO activation is readily explained by higher FOXO3 protein levels induced by ectopic expression, and/or as a result of positive growth factor signaling feedback in the case of treatment with LY294002. Pharmacological inhibition of the PI3K pathway may initiate a growth factor feedback response that can reestablish growth factor signaling which constitutes a major component of drug resistance development in cancer cells (6,7,30). In contrast to MCF7 cells, in untreated MDA-MB-231 cells some nuclear FOXO protein was detected. However the FOXO activity model scored this FOXO as transcriptionally inactive - as expected in this rapidly dividing cell line. Thus the nuclear presence of FOXO cannot always be used to infer transcriptional activity.

In three breast cancer cell lines as well as in lung cancer cells with mutated hyperactive EGFR, the model identified increased FOXO activity, associated with effective inhibition of PI3K pathway activity by the EGFR inhibitor *erlotinib.* In addition, treatment of the lung cancer cell line with a dual mTOR-PI3K inhibitor *(BEZ235),* and a MEK1/2 inhibitor (*selumetinib)* similarly resulted in increased FOXO activity, and thus reduced inferred PI3K pathway activity. Signal transduction initiated by the ErbB family of growth factor receptors (EGFR, HER2, HER3, HER4) leads to inactivation of FOXO via PI3K-AKT and RAS-MEK-ERK-MDM2 (31).

Thus the Bayesian model robustly predicted FOXO and PI3K activity in these cell culture-based models for PI3K pathway activation, providing biological validation of the model. However, the difference in response of the lung cancer cell line to the three drugs targeting different elements of the signaling pathways downstream of EGFR illustrates that to make an optimal choice with respect to a targeted drug, additional analysis (e.g. genomic mutation analysis) may be required to establish the underlying cause of the PI3K pathway activity.

### A computational model predicting FOXO activity in tissue samples

Applying the model to tissue material to infer PI3K activity proved to be more challenging. In healthy tissue samples from a variety of tissue types (colon, breast, prostate, esophagus, bladder, brain) FOXO was scored active, as expected. In colon adenoma samples FOXO activity was frequently lost and PI3K pathway inferred as active, once more emphasizing the role of FOXO in controlling cell division. In this situation growth factor-induced activation of the PI3K pathway initiates mitogenic signaling and blocks FOXO transcriptional activity (12,32,33,34).

### Distinguishing between different mechanisms regulating FOXO activity

In cancer the situation in which FOXOs can be active becomes more complex. Both in colon carcinoma and luminal B, HER2 and basal like breast carcinoma subtypes, where the PI3K pathway is frequently activated, the majority of the samples were predicted to have an active FOXO transcription factor (2,35). This suggested that simple inversion of FOXO activity status to infer PI3K pathway activity was not valid in all cancer tissue samples. A differentiator between canonical FOXO activity associated with control of cell division and FOXO activity in the presence of growth factor/PI3K activity-induced cell proliferation was necessary.

While considered tumor suppressors, FOXOs also function as regulators of cellular homeostasis and respond to various adverse cellular conditions including DNA damage, high levels of reactive oxygen species and low nutrient availability (12,36,37). Indeed, in cancer samples, an active PI3K pathway may be present in combination with active FOXO transcription since FOXO can be activated in an alternative manner via oxidative stress to protect cells against ROS (Figure 13A). Such an oxidative stress state is a frequent phenomenon in hypoxic and rapidly proliferating cancer tissues (12,38-40).

FOXO is post-translational modified to adapt its transcriptional function to different intracellular conditions (41). It was hypothesized that the two very different functional roles of FOXO would be reflected in changes in FOXO target mRNA expression profiles. *BNIP3* and *SOD2* are two FOXO3 target genes used by the computational model and known to be induced in the presence of cellular oxidative stress to protect the cell against the consequences of this toxic state (26,27,42). Indeed, high expression of both *SOD2* and *BNIP3* was found by comparing FOXO target gene expression level between multiple FOXO-active cancer samples and corresponding healthy tissue samples, in contrast to FOXO-active healthy tissue and benign hyper-proliferative colon adenoma. These results underpin the concept that oxidative stress in parallel to growth factor signalling can by an alternative pathway induce FOXO activity in rapidly proliferating cancer tissue. Of the two genes, *SOD2* expression appeared to be universally elevated in FOXO-active samples from a large number of cancer types when compared to healthy tissues. To enable distinction between canonical FOXO activity and oxidative stress-associated FOXO activity, threshold levels for *SOD2* expression in healthy tissues were defined. Adding this information to the FOXO model improves reliability of inferring PI3K pathway activity in various types of cancer.

The application of this rule to breast and prostate cancer subtypes that are well-defined with respect to aggressive behaviour and prognosis allowed initial clinical validation. Indeed, adding information on *SOD2* expression level to the FOXO activity score classified FOXO activity in luminal A breast cancer and lower Gleason score prostate cancer in general as canonical FOXO activity, with an inferred inactive PI3K pathway. Indeed, these cancer types are typically more differentiated and slow growing and driven by the ER pathway in the absence of an active HER2-PI3K pathway. In contrast, in breast cancer subtypes with more aggressive behaviour, that is luminal B, HER2 and basal like, samples where either inactive for FOXO (indicating an active PI3K pathway) or FOXO activity was increasingly classified as FOXO oxidative stress activity. Summarizing, in well differentiated slow growing breast and prostate cancer FOXOs were generally active in the canonical mode to control cell division, while in more aggressive cancer FOXOs were either inactivated by PI3K pathway activation, or active in the oxidative stress mode. Interestingly, oxidative stress-associated FOXO activity may actually function to support tumor growth by both protecting the cell from oxidative stress damage which normally leads to cell death, and stimulating the growth factor PI3K-AKT pathway (43).

### Use of the computational FOXO model to identify PI3K pathway activity in an individual cancer tissue sample

The use of the computational model is to assess functional PI3K pathway activity in an individual cancer tissue samples to support in the decision on the choice of a targeted drug directed at ErbB growth factor signaling pathways, e.g. HER2-PI3K and EGFR pathways, and/or to monitor response and detect emerging resistance against the chosen drug.

Using the FOXO computational model to assess FOXO activity in combination with the oxidative stress *SOD2* gene marker which indicates the mode of FOXO activity, the likelihood that the PI3K pathway is active can be derived from highly specified gene expression data using a (somewhat simplified) decision tree (Figure 13B). In case FOXO is scored active, expression levels of *SOD2* are interpreted to decide on PI3K-regulated versus oxidative stress induced FOXO activity. If FOXO activity is caused by oxidative stress, underlying information on activity of the PI3K pathway is hidden, and formally a decision cannot be made with respect to activity of the PI3K pathway. However, cellular oxidative stress associated FOXO activity frequently (if not always) occurs concurrently with growth factor pathway activity in high-grade cancers (4,38). Thus, in the presence of FOXO active/SOD2 high, there is a high likelihood of the PI3K pathway being active. Adding up samples that were scored as either FOXO inactive or FOXO active-oxidative stress provided an indication of the total number of patients within each cancer subtype group with a PI3K active tumor which might have benefited from PI3K pathway inhibitor treatment. In the low grade Luminal A and lower Gleason score prostate cancer groups the thus calculated percentage of PI3K-active tumors was close to that seen in healthy tissue (roughly around 10%), while in higher grade breast cancer this increased up to three quarters in the basal-like breast cancer group. Indeed, the PI3K pathway is probably the most frequently activated pathway in cancer.

When in a tissue sample FOXOs are predicted to be active and expression levels of SOD2 fall within the normal range, complementary IHC staining may be necessary to exclude that FOXO activity is caused by healthy tissue cell contamination of the cancer sample. Taking this into account, the FOXO model provides a robust method for determining functional PI3K pathway activity in tumor samples. Targeted genomic analysis to identify a mutational cause of abnormal PI3K pathway activity can be limited to patients in which an active PI3K pathway was inferred. This approach is expected to improve decision taking on the use of drugs targeting the ErbB PI3K-AKT-mTOR pathway aiming at increased treatment efficacy rates, and is expected to be applicable to many different tumor types. Monitoring of therapy response and resistance, for example in a neo-adjuvant or "window-of-opportunity" setting, is another envisioned application, as is quantitative assessment of drug response during drug development.

### References:

1. Ashley EA. Towards precision medicine. Nat Rev Genet 2016;17(9):507-22.
2. Vogelstein B, Papadopoulos N, Velculescu VE, Zhou S, Diaz LA, Jr., Kinzler KW. Cancer genome landscapes. Science 2013;339(6127):1546-58.
3. van de Stolpe A. On the origin and destination of cancer stem cells: a conceptual evaluation. Am J Cancer Res 2013;3(1):107-16.
4. Hanahan D, Weinberg RA. Hallmarks of cancer: the next generation. Cell 2011;144(5):646-74.
5. Fruman DA, Rommel C. PI3K and cancer: lessons, challenges and opportunities. Nat Rev Drug Discov 2014;13(2):140-56.
6. Engelman JA. Targeting PI3K signalling in cancer: opportunities, challenges and limitations. Nat Rev Cancer 2009;9(8):550-62.
7. Arnedos M, Vicier C, Loi S, Lefebvre C, Michiels S, Bonnefoi H, et al. Precision medicine for metastatic breast cancer-limitations and solutions. Nature reviews Clinical oncology 2015.
8. Rodon J, Dienstmann R, Serra V, Tabernero J. Development of PI3K inhibitors: lessons learned from early clinical trials. Nat Rev Clin Oncol 2013;10(3):143-53.
9. Kwiatkowski DJ, Wagle N. mTOR Inhibitors in Cancer: What Can We Learn from Exceptional Responses? EBioMedicine 2015;2(1):2-4.
10. Verhaegh W, Van de Stolpe A. Knowledge-based computational models. Oncotarget 2014;5(14):5196-7.
11. Verhaegh W, van Ooijen H, Inda MA, Hatzis P, Versteeg R, Smid M, et al. Selection of personalized patient therapy through the use of knowledge-based computational models that identify tumor-driving signal transduction pathways. Cancer Res 2014;74(11):2936-45.
12. Eijkelenboom A, Burgering BM. FOXOs: signalling integrators for homeostasis maintenance. Nat Rev Mol Cell Biol 2013;14(2):83-97.
13. Brunet A, Bonni A, Zigmond MJ, Lin MZ, Juo P, Hu LS, et al. Akt promotes cell survival by phosphorylating and inhibiting a Forkhead transcription factor. Cell 1999;96(6):857-68.
14. Kops GJ, de Ruiter ND, De Vries-Smits AM, Powell DR, Bos JL, Burgering BM. Direct control of the Forkhead transcription factor AFX by protein kinase B. Nature 1999;398(6728):630-4.
15. Eijkelenboom A, Mokry M, de Wit E, Smits LM, Polderman PE, van Triest MH, et al. Genome-wide analysis of FOXO3 mediated transcription regulation through RNA polymerase II profiling. Mol Syst Biol 2013;9:638.
16. Webb AE, Kundaje A, Brunet A. Characterization of the direct targets of FOXO transcription factors throughout evolution. Aging Cell 2016;15(4):673-85.
17. Dull T, Zufferey R, Kelly M, Mandel RJ, Nguyen M, Trono D, et al. A third-generation lentivirus vector with a conditional packaging system. J Virol 1998;72(11):8463-71.
18. Meerbrey KL, Hu G, Kessler JD, Roarty K, Li MZ, Fang JE, et al. The pINDUCER lentiviral toolkit for inducible RNA interference in vitro and in vivo. Proc Natl Acad Sci U S A 2011;108(9):3665-70.
19. Hornsveld M, Tenhagen M, van de Ven RA, Smits AM, van Triest MH, van Amersfoort M, et al. Restraining FOXO3-dependent transcriptional BMF activation underpins tumour growth and metastasis of E-cadherin-negative breast cancer. Cell Death Differ 2016.
20. Czymai T, Viemann D, Sticht C, Molema G, Goebeler M, Schmidt M. FOXO3 modulates endothelial gene expression and function by classical and alternative mechanisms. J Biol Chem 2010;285(14):10163-78.
21. Parker JS, Mullins M, Cheang MC, Leung S, Voduc D, Vickery T, et al. Supervised risk predictor of breast cancer based on intrinsic subtypes. J Clin Oncol 2009;27(8):1160-7.
22. Perou CM, Sorlie T, Eisen MB, van de Rijn M, Jeffrey SS, Rees CA, et al. Molecular portraits of human breast tumours. Nature 2000;406(6797):747-52.
23. Shmelkov E, Tang Z, Aifantis I, Statnikov A. Assessing quality and completeness of human transcriptional regulatory pathways on a genome-wide scale. Biol Direct 2011;6:15.
24. van der Vos KE, Coffer PJ. The extending network of FOXO transcriptional target genes. Antioxid Redox Signal 2011;14(4):579-92.
25. van den Berg MCW, Burgering BMT. Integrating opposing signals toward forkhead box o. Antioxidants & redox signaling 2011;14(4):607-21.
26. Kops GJPL, Dansen TB, Polderman PE, Saarloos I, Wirtz KWA, Coffer PJ, et al. Forkhead transcription factor FOXO3a protects quiescent cells from oxidative stress. Nature 2002;419(6904):316-21.
27. Mammucari C, Milan G, Romanello V, Masiero E, Rudolf R, Del Piccolo P, et al. FoxO3 controls autophagy in skeletal muscle in vivo. Cell Metab 2007;6(6):458-71.
28. Kim HJ, Lee SY, Kim CY, Kim YH, Ju W, Kim SC. Subcellular localization of FOXO3a as a potential biomarker of response to combined treatment with inhibitors of PI3K and autophagy in PIK3CA-mutant cancer cells. Oncotarget 2017;8(4):6608-22.
29. Paik JH, Kollipara R, Chu G, Ji H, Xiao Y, Ding Z, et al. FoxOs are lineage-restricted redundant tumor suppressors and regulate endothelial cell homeostasis. Cell 2007;128(2):309-23.
30. Chandarlapaty S, Sawai A, Scaltriti M, Rodrik-Outmezguine V, Grbovic-Huezo O, Serra V, et al. AKT inhibition relieves feedback suppression of receptor tyrosine kinase expression and activity. Cancer Cell 2011;19(1):58-71.
31. Yang JY, Zong CS, Xia W, Yamaguchi H, Ding Q, Xie X, et al. ERK promotes tumorigenesis by inhibiting FOXO3a via MDM2-mediated degradation. Nat Cell Biol 2008;10(2):138-48.
32. Sheng H, Shao J, Townsend CM, Jr., Evers BM. Phosphatidylinositol 3-kinase mediates proliferative signals in intestinal epithelial cells. Gut 2003;52(10):1472-8.
33. Clevers H. The intestinal crypt, a prototype stem cell compartment. Cell 2013;154(2):274-84.
34. Clemons NJ, Phillips WA, Lord RV. Signaling pathways in the molecular pathogenesis of adenocarcinomas of the esophagus and gastroesophageal junction. Cancer Biol Ther 2013;14(9):782-95.
35. Vanhaesebroeck B, Stephens L, Hawkins P. PI3K signalling: the path to discovery and understanding. Nat Rev Mol Cell Biol 2012;13(3):195-203.
36. van der Horst A, Burgering BM. Stressing the role of FoxO proteins in lifespan and disease. Nat Rev Mol Cell Biol 2007;8(6):440-50.
37. Webb AE, Brunet A. FOXO transcription factors: key regulators of cellular quality control. Trends Biochem Sci 2014;39(4):159-69.
38. Hornsveld M, Dansen TB. The Hallmarks of Cancer from a Redox Perspective. Antioxid Redox Signal 2016;25(6):300-25.
39. Klotz LO, Sanchez-Ramos C, Prieto-Arroyo I, Urbanek P, Steinbrenner H, Monsalve M. Redox regulation of FoxO transcription factors. Redox Biol 2015;6:51-72.
40. van den Berg MC, van Gogh IJ, Smits AM, van Triest M, Dansen TB, Visscher M, et al. The small GTPase RALA controls c-Jun N-terminal kinase-mediated FOXO activation by regulation of a JIP1 scaffold complex. J Biol Chem 2013;288(30):21729-41.
41. Calnan DR, Brunet A. The FoxO code. Oncogene 2008;27(16):2276-88.
42. Lin A, Yao J, Zhuang L, Wang D, Han J, Lam EW, et al. The FoxO-BNIP3 axis exerts a unique regulation of mTORC1 and cell survival under energy stress. Oncogene 2014;33(24):3183-94.
43. Coomans de Brachene A, Demoulin JB. FOXO transcription factors in cancer development and therapy. Cell Mol Life Sci 2016;73(6):1159-72.

**Table 4: Expression data of target genes in different samples**

| **A: Colon** | | | | | | | | | |
|---|---|---|---|---|---|---|---|---|---|
| | **Colon** | BNIP3 | BNIP3 | MXI1 | PCK1 | PPARGC1A | SOD2 | SOD2 | Foxo3 |
| Nr of samples: sub type - normal | | 201849_at | 201848_s_at | 202364_at | 208383_s_at | 219195_at | 215223_s_at | 216841_s_at | 204132_s_at |
| 10-10 | Adenoma active - normal active | 0,273798 | 0,269074 | -1,48303 | -3,13745 | -1,67616 | 0,836813 | 0,236897 | -0,25657 |
| 20-10 | Adenocarcinoma active-normal active | 1,9587 | 1,710284 | -1,22683 | -3,37404 | -2,15956 | 1,85336 | 1,628937 | -0,34943 |
| | GSE37364, GSE39084, GSE40967, GSE2109, GSE4183, GSE8671, GSE1433, GSE20916 | | | | | | | | |

| **B: Breast cancer subtypes** | | | | | | | | | |
|---|---|---|---|---|---|---|---|---|---|
| | **Breast** | BNIP3 | BNIP3 | MXI1 | PCK1 | PPARGC1A | SOD2 | SOD2 | Foxo3 |
| Nr of samples: sub type - normal | | 201849_at | 201848_s_at | 202364_at | 208383_s_at | 219195_at | 215223_s_at | 216841_s_at | 204132 s at |
| 15-15 | Basal active-Normal active | 1,667574 | 1,863761 | -0,53552 | -1,13966 | -0,49688 | 3,044671 | 3,04274 | 0,351846 |
| 15-15 | HER2 active - Normal active | 1,299157 | 1,300141 | -1,06429 | -1,25087 | -0,59512 | 2,030035 | 2,445361 | 0,098959 |
| 15-15 | LumA active - Normal active | 1,192817 | 1,04744 | -0,63432 | -0,97879 | -0,85001 | 1,13798 | 1,29578 | 0,115024 |
| 15-15 | LumB active - Normal active | 1,659292 | 1,666756 | -0,55229 | -1,06282 | -0,94702 | 1,844972 | 2,066248 | -0,15813 |
| 15-15 | NormL active - Normal active | 0,379788 | 0,199025 | -0,29916 | -0,1721 | -0,45272 | 0,873948 | 1,067476 | 0,495257 |
| | GSE6532, GSE10780, GSE12276, GSE21653, GSE26910, GSE42568, GSE45827,GSE58697, EMTAB365, GSE7307, GSE54002 | | | | | | | | |

| **C: Endometrioid endometrium (non-malignant)** | | | | | | | | | |
|---|---|---|---|---|---|---|---|---|---|
| | **Endometrium** | BNIP3 | BNIP3 | MXI1 | PCK1 | PPARGC1A | SOD2 | SOD2 | Foxo3 |
| Nr of samples: sub type - normal | | 201849_at | 201848_s_at | 202364_at | 208383_s_at | 219195_at | 215223_s_at | 216841_s_at | 204132_s_at |
| 5-10 | Grade 3 Endometrium endometrioid active - Normal active | 1,056748 | 0,942018 | 0,218929 | 0,78093 | -0,53813 | -0,27305 | 0,115214 | 0,327355 |
| 5-10 | Grade 2 Endometrium endometrioid active - Normal active | 0,883182 | 0,515255 | -0,25033 | -0,29361 | -1,20879 | -0,54561 | -0,00248 | 0,321328 |
| 5-10 | Grade 1 Endometrium endometrioid active - Normal active | -0,46113 | -0,83605 | -0,43501 | 1,758022 | -0,84941 | -1,32275 | -0,93531 | 0,130646 |
| | GSE65986, GSE56026, GSE51981, GSE39099, GSE29437, GSE29436, GSE20854, GSE17025, GSE7307, GSE7305, GSE4888, GSE3526 | | | | | | | | |

| **D: Bladder cancer** | | | | | | | | | |
|---|---|---|---|---|---|---|---|---|---|
| | **Bladder** | BNIP3 | BNIP3 | MXI1 | PCK1 | PPARGC1A | SOD2 | SOD2 | Foxo3 |
| Nr of samples: sub type - normal | | 201849_at | 201848_s_at | 202364_at | 208383_s_at | 219195_at | 215223_s_at | 216841_s_at | 204132_s_at |
| 15-10 | Urothelial cell carcinoma active - normal bladder active | 0,246742 | 0,634363 | -0,26445 | -0,05666 | -0,33966 | 1,536777 | 1,668267 | 0,08466 |
| | GSE31684, GSE31189, GSE30522, GSE7476, GSE11839 | | | | | | | | |

| **E: Barrett's esophagus (non-malignant) and esophageal cancer** | | | | | | | | | |
|---|---|---|---|---|---|---|---|---|---|
| | **Esopahagael** | BNIP3 | BNIP3 | MXI1 | PCK1 | PPARGC1A | SOD2 | SOD2 | Foxo3 |
| Nr of samples: sub type - normal | | 201849_at | 201848_s_at | 202364_at | 208383_s_at | 219195_at | 215223_s_at | 216841_s_at | 204132_s_at |
| 5-8 | Barrett's esophagus active-Normal esophagus active | -1,16364 | -3,01546 | -1,25124 | 0,180396 | -0,52154 | -1,06208 | -0,42729 | -1,73071 |
| 5-8 | Esophageal adenocarcinoma active - Normal esophagus active | -2,56948 | -2,27944 | -1,23093 | 1,887236 | -0,59335 | 0,29449 | 0,752051 | -0,78151 |
| 8-8 | Esophageal carcinoma ESCC active - Normal esophagus active | -0,12874 | -0,17861 | -0,42882 | -1,46369 | -1,52075 | 0,407411 | 0,966236 | -1,32294 |
| 8-8 | Esophageal carcinoma unclear type active - Normal esophagus active | 0,185132 | -1,55941 | -1,48575 | -1,44444 | -0,86866 | 0,311418 | 1,63532 | -2,8173 |
| | GSE26886,GSE32701, GSE42363, GSE45670, GSE7307, GSE40220, GSE14827, GSE17351,GSE33810 | | | | | | | | |

| **F: Brain tumors** | | | | | | | | | |
|---|---|---|---|---|---|---|---|---|---|
| | **Brain** | BNIP3 | BNIP3 | MXI1 | PCK1 | PPARGC1A | SOD2 | SOD2 | Foxo3 |
| Nr of samples: sub type - normal | | 201849_at | 201848_s_at | 202364_at | 208383_s_at | 219195_at | 215223_s_at | 216841_s_at | 204132_s_at |
| 15-15 | Astrocytoma Active - normal active | 0,146884 | 0,011696 | -0,12663 | -1,27425 | 0,15012 | 0,885691 | 1,335549 | -0,16905 |
| 15-15 | Ependymoma Active - normal active | 1,366425 | 1,335516 | 0,200653 | -1,35681 | 1,754186 | 1,674704 | 1,819496 | -0,05665 |
| 15-15 | Glioblastoma Active - normal active | 0,432824 | 0,588991 | -0,28271 | -0,90651 | -0,31045 | 2,443816 | 2,540678 | -0,3791 |
| 5-15 | Glioma Active - normal active | 1,025915 | 0,826212 | 0,235253 | -1,14941 | 0,273865 | 1,628326 | 1,999869 | -0,94771 |
| 5-15 | Oligoastrocytoma Active - normal active | -0,39944 | -0,97413 | -0,86394 | -1,32875 | -0,29759 | 0,485354 | 0,862014 | -0,80434 |
| 15-15 | Medulloblastoma Active - normal active | -0,3592 | -0,33424 | 0,353031 | -1,17693 | 0,374427 | -1,3727 | -1,36469 | -0,14824 |
| 15-15 | Meningioma Active - normal active | -1,88971 | -1,41137 | 0,044613 | -1,0159 | 0,284705 | 0,487543 | 0,844608 | 0,155723 |
| 7-15 | PNET Pediatric Active - normal active | -0,46105 | -0,27148 | -0,19149 | 0,249276 | 0,796817 | -0,72253 | -0,52649 | -0,04015 |
| 10-15 | ETMR pediatric Active - normal active | -1,05009 | -0,99756 | -0,7645 | -1,22548 | -0,08409 | -1,27778 | -1,03669 | -1,14498 |
| 4-15 | Atypical teratoid/rhabdoid Active - normal active | 0,127491 | 0,421646 | -0,11917 | -1,33723 | -0,06481 | -0,86283 | -0,73715 | -0,4772 |
| 15-15 | Adamantinomatous craniopharyngioma Active - normal active | -1,01281 | -0,92224 | -0,3828 | -0,09534 | -0,16296 | -0,20932 | -0,22166 | -0,99084 |
| 6-15 | Malignant peripheral nerve sheath Active - normal active | -1,69854 | -1,20554 | -1,58464 | 1,0569 | -2,07102 | -1,28197 | -1,39175 | 0,329751 |
| 5-15 | Papilloma Active - normal active | -1,91215 | -1,26686 | -1,99798 | -1,52666 | 1,835479 | -0,75212 | -1,08724 | -0,68474 |
| | GSE50161, GSE16581, GSE44971, GSE15824, GSE19350, GSE68015, GSE11882, GSE7307, GSE4780, GSSE9438, GSE73066, GSE5675, GSE33331, GSE22927 | | | | | | | | |
| | GSE53733, GSE45921, GSE50774, GSE34824, GSE36245, GSE13041, GSE43378, GSE4290, GSE73038, 37418, GSE67850, GSE 12992, GSE74195, GSE50161, | | | | | | | | |
| | GSE66354, GSE68015, GSE66354, GSE50385, GSE16155, GSE21687, GSE45437, GSE12141,GSE70678, GSE9832, GSE51455, GSE19348, GSE16910, GSE18180 | | | | | | | | |

| **G: Lung tissues and carcinomas** | | | | | | | | | |
|---|---|---|---|---|---|---|---|---|---|
| | **Lung** | BNIP3 | BNIP3 | MXI1 | PCK1 | PPARGC1A | SOD2 | SOD2 | Foxo3 |
| Nr of samples: sub type - normal | | 201849_at | 201848_s_at | 202364_at | 208383_s_at | 219195_at | 215223_s_at | 216841_s_at | 204132_s_at |
| 15-10 | Lung adenocarcinoma Active - Normal Lung active | 0,862772 | 1,15117 | 0,146792 | 0,04439 | -1,46338 | 0,340791 | 0,537944 | -0,12852 |
| 15-10 | Lung carcinoma Active-Normal Lung active | 1,368504 | 1,523603 | 0,063306 | 0,834799 | -1,13767 | 0,080692 | 0,346262 | 0,115441 |
| 5-10 | Lung carcinoma basaloid Active - Normal Lung active | 1,412481 | 1,779473 | 0,477305 | 1,438382 | -1,55802 | -0,27336 | 0,093518 | 0,369853 |
| 5-10 | Lung carcinoma carcinoid Active - Normal Lung active | 1,757095 | 1,681628 | -0,21827 | 2,739352 | 1,139666 | -2,50947 | -2,52514 | -0,51509 |
| 10-10 | Lung carcinoma large cell Active - Normal Lung active | 1,52916 | 1,588956 | 0,191703 | 0,540628 | -0,43365 | 0,046743 | 0,240385 | -0,12804 |
| 5-10 | Lung carcinoma Malignant Pleural Mesothelioma Active - Normal Lung active | 0,248672 | -0,93022 | -1,03017 | 0,574851 | -1,55522 | -0,74182 | 0,055374 | -1,4961 |
| 15 - 10 | Lung carcinoma non small cell Active - Normal Lung active | 0,90711 | 1,028795 | -0,05802 | -0,02026 | -0,65816 | 0,071709 | 0,337043 | -0,20114 |
| 5-10 | Lung carcinoma small cell Active - Normal Lung active | 1,938049 | 2,003627 | 0,354586 | 2,643471 | -1,56829 | -1,4894 | -0,88207 | -0,10044 |
| 10-10 | Lung carcinoma squamous cell Active - Normal Lung active | 1,409648 | 1,776817 | 0,167414 | 0,212526 | -1,82582 | 0,411398 | 0,6163 | 0,487339 |
| | | | | | | | | | |
| 15-5 | Lung adenocarcinoma Active - Normal lung Parenchyma active | 0,181774 | 2,513496 | 0,921171 | -0,09567 | -2,33804 | 0,204362 | -0,10273 | 0,512917 |
| 15-5 | Lung carcinoma Active-Normal lung Parenchyma active | 0,687506 | 2,885929 | 0,837686 | 0,694743 | -2,01234 | -0,05574 | -0,29441 | 0,756882 |
| 5-5 | Lung carcinoma basaloid Active - Normal lung Parenchyma active | 0,731483 | 3,141799 | 1,251684 | 1,298326 | -2,43268 | -0,40979 | -0,54716 | 1,011294 |
| 5-5 | Lung carcinoma carcinoid Active - Normal lung Parenchyma active | 1,076097 | 3,043954 | 0,556111 | 2,599296 | 0,265004 | -2,6459 | -3,16581 | 0,12635 |
| 10-5 | Lung carcinoma large cell Active - Normal lung Parenchyma active | 0,848162 | 2,951282 | 0,966083 | 0,400572 | -1,30831 | -0,08969 | -0,40029 | 0,513398 |
| 5-5 | Lung carcinoma Malignant Pleural Mesothelioma Active - Normal lung Parenchyma active | -0,43233 | 0,432109 | -0,25579 | 0,434795 | -2,42988 | -0,87825 | -0,5853 | -0,85466 |
| 15-5 | Lung carcinoma non small cell Active - Normal lung Parenchyma active | 0,226112 | 2,39112 | 0,716357 | -0,16031 | -1,53282 | -0,06472 | -0,30363 | 0,440297 |
| 5-5 | Lung carcinoma small cell Active - Normal lung Parenchyma active | 1,257051 | 3,365953 | 1,128966 | 2,503415 | -2,44296 | -1,62583 | -1,52274 | 0,541004 |
| 10-5 | Lung carcinoma squamous cell Active - Normal lung Parenchyma active | 0,72865 | 3,139143 | 0,941793 | 0,07247 | -2,70048 | 0,274968 | -0,02438 | 1,12878 |
| 15-5 | Lung adenocarcinoma Active - Normal trachea Active | 1,199132 | 1,473595 | 0,073019 | 0,00497 | -0,07875 | 0,864836 | 1,319405 | -0,46545 |
| 15-5 | Lung carcinoma Active-Normal trachea Active | 1,704864 | 1,846028 | -0,01047 | 0,795379 | 0,24696 | 0,604736 | 1,127722 | -0,22149 |
| 5-5 | Lung carcinoma basaloid Active - Normal trachea Active | 1,748841 | 2,101898 | 0,403532 | 1,398963 | -0,17339 | 0,250685 | 0,874979 | 0,032925 |
| 5-5 | Lung carcinoma carcinoid Active - Normal trachea Active | 2,093455 | 2,004053 | -0,29204 | 2,699932 | 2,524299 | -1,98542 | -1,74368 | -0,85202 |
| 10-5 | Lung carcinoma large cell Active - Normal trachea Active | 1,86552 | 1,911381 | 0,117931 | 0,501208 | 0,950987 | 0,570787 | 1,021845 | -0,46497 |
| 5-5 | Lung carcinoma Malignant Pleural Mesothelioma Active - Normal trachea Active | 0,585033 | -0,60779 | -1,10394 | 0,535431 | -0,17059 | -0,21778 | 0,836834 | -1,83303 |
| 15-5 | Lung carcinoma non small cell Active - Normal trachea Active | 1,24347 | 1,351219 | -0,1318 | -0,05968 | 0,726472 | 0,595754 | 1,118503 | -0,53807 |
| 5-5 | Lung carcinoma small cell Active - Normal trachea Active | 2,27441 | 2,326052 | 0,280814 | 2,604051 | -0,18366 | -0,96536 | -0,10061 | -0,43737 |
| 10-5 | Lung carcinoma squamous cell Active - Normal trachea Active | 1,746008 | 2,099242 | 0,093641 | 0,173106 | -0,44118 | 0,935442 | 1,39776 | 0,15041 |
| | | | | | | | | | |
| 15 - 8 | Lung adenocarcinoma Active - Normal large airway Active | 1,328557 | 1,475149 | 0,23599 | 0,063051 | -0,42398 | 2,604758 | 2,681653 | -0,06196 |
| 15 - 8 | Lung carcinoma Active-Normal large airway Active | 1,834289 | 1,847582 | 0,152505 | 0,85346 | -0,09827 | 2,344659 | 2,489971 | 0,182004 |
| 5-8 | Lung carcinoma basaloid Active - Normal large airway Active | 1,878267 | 2,103452 | 0,566504 | 1,457043 | -0,51861 | 1,990608 | 2,237227 | 0,436416 |
| 5-8 | Lung carcinoma carcinoid Active - Normal large airway Active | 2,22288 | 2,005607 | -0,12907 | 2,758013 | 2,179072 | -0,2455 | -0,38143 | -0,44853 |
| 10-8 | Lung carcinoma large cell Active - Normal large airway Active | 1,994945 | 1,912935 | 0,280902 | 0,559289 | 0,60576 | 2,31071 | 2,384093 | -0,06148 |
| 5-8 | Lung carcinoma Malignant Pleural Mesothelioma Active - Normal large airway Active | 0,714458 | -0,60624 | -0,94097 | 0,593512 | -0,51581 | 1,522143 | 2,199083 | -1,42954 |
| 15 - 8 | Lung carcinoma non small cell Active - Normal large airway Active | 1,372895 | 1,352773 | 0,031176 | -0,0016 | 0,381245 | 2,335676 | 2,480751 | -0,13458 |
| 5-8 | Lung carcinoma small cell Active - Normal large airway Active | 2,403835 | 2,327606 | 0,443785 | 2,662132 | -0,52889 | 0,774565 | 1,261639 | -0,03387 |
| 10-8 | Lung carcinoma squamous cell Active - Normal large airway Active | 1,875433 | 2,100796 | 0,256613 | 0,231187 | -0,78641 | 2,675364 | 2,760008 | 0,553902 |
| 15-15 | Lung adenocarcinoma Active - Normal small airway Active | 0,627512 | 1,009811 | -0,20951 | -0,01811 | -0,05601 | 1,666712 | 1,958862 | -0,1256 |
| 15-15 | Lung carcinoma Active - Normal small airway Active | 1,133244 | 1,382243 | -0,29299 | 0,772296 | 0,2697 | 1,406613 | 1,76718 | 0,118366 |
| 5-15 | Lung carcinoma basaloid Active - Normal small airway Active | 1,177222 | 1,638113 | 0,121005 | 1,37588 | -0,15065 | 1,052562 | 1,514437 | 0,372778 |
| 5-15 | Lung carcinoma carcinoid Active - Normal small airway Active | 1,521835 | 1,540268 | -0,57457 | 2,676849 | 2,547039 | -1,18355 | -1,10422 | -0,51217 |
| 10-15 | Lung carcinoma large cell Active - Normal small airway Active | 1,293901 | 1,447597 | -0,1646 | 0,478125 | 0,973727 | 1,372664 | 1,661303 | -0,12512 |
| 5-15 | Lung carcinoma Malignant Pleural Mesothelioma Active - Normal small airway Active | 0,013413 | -1,07158 | -1,38647 | 0,512348 | -0,14785 | 0,584097 | 1,476292 | -1,49318 |
| 15-15 | Lung carcinoma non small cell Active - Normal small airway Active | 0,67185 | 0,887435 | -0,41432 | -0,08276 | 0,749212 | 1,39763 | 1,757961 | -0,19822 |
| 5-15 | Lung carcinoma small cell Active - Normal small airway Active | 1,70279 | 1,862267 | -0,00171 | 2,580968 | -0,16092 | -0,16348 | 0,538849 | -0,09751 |
| 10-15 | Lung carcinoma squamous cell Active - Normal small airway Active | 1,174388 | 1,635458 | -0,18889 | 0,150023 | -0,41844 | 1,737319 | 2,037218 | 0,490264 |
| | GSE63074, | | | | | | | | |
| | GSE63074, GSE30219, GSE28582, GSE50081, GSE77803, GSE31210, GSE33532, GSE51024, GSE19804, GSE10006, GSE19667, GSE13933, GSE43346, GSE7307. | | | | | | | | |

| **H: Prostate cancer** | | | | | | | | | |
|---|---|---|---|---|---|---|---|---|---|
| | **Prostate** | BNIP3 | BNIP3 | MXI1 | PCK1 | PPARGC1A | SOD2 | SOD2 | Foxo3 |
| Nr of samples: sub type - normal | | 201849_at | 201848_s_at | 202364_at | 208383_s_at | 219195_at | 215223_s_at | 216841_s_at | 204132_s_at |
| 10-5 | Prostate adenocarcinoma Active - Normal prostate active | 0,882 | 0,787338 | 0,270096 | 0,232785 | -0,75766 | 0,621324 | 0,444623 | -0,45114 |
| 15-5 | Prostate Carcinoma Active-Normal prostate active | -0,89432 | -0,53046 | -0,00952 | 0,106124 | 0,196289 | -0,72156 | -1,10879 | -0,33564 |
| 4-5 | Prostate carcinoma high grade Active - Normal prostate active | -0,84998 | -0,65283 | -0,62984 | -0,0771 | -1,79734 | -2,79272 | -1,79146 | -1,70778 |
| 5-5 | Prostate carcinoma metastatic Active - Normal prostate active | 0,766682 | 0,048211 | 0,3789 | 0,603609 | -0,20119 | -2,41498 | -2,09149 | -0,49558 |
| | GSE21887, GSE15392, GSE17482, GSE17906, GSE17951, GSE18676, GSE22606, GSE26910, GSE28403, GSE30304, GSE32967, GSE32982, | | | | | | | | |
| | GSE3325, GSE33316, GSE34043, GSE40794, GSE45016, GSE46602, GSE56352, GSE73044, GSE9951, GSE2109, GSE7307 | | | | | | | | |

**Table 6: Selected FOXO target genes**

| **Gene** | **Probeset** | **References** |
|---|---|---|
| ***AGRP*** | 207193_at | (Kim et al., 2006; Kitamura et al., 2006) |
| ***BCL2L11*** | 1553096_s_at/ 1555372_at/ 1558143_a_at/ 208536_s_at/ 222343_at/ 225606_at | (Dijkers et al., 2000a; Gilley et al., 2003) |
| ***BCL6*** | 203140_at/ 215990_s_at | (Fernandez de Mattos et al., 2004; Tang et al., 2002) |
| ***BNIP3*** | 201848_s_at/ 201849_at | (Mammucari et al., 2007; Zhao et al., 2007) |
| ***BTG1*** | 1559975_at/ 200920_s_at/ 200921_s_at | (Bakker et al., 2004) |
| ***CAT*** | 201432_at/ 211922_s_at/215573_at | (Nemoto and Finkel, 2002) |
| ***CAV1*** | 203065_s_at/ 212097_at | (Roy et al., 2008; van den Heuvel et al., 2005) |
| ***CCND1*** | 208711_s_at/ 208712_at/ 214019_at | (Schmidt et al., 2002) |
| ***CCND2*** | 200951_s_at/ 200952_s_at/ 200953_s_at/ 231259_s_at | (Schmidt et al., 2002) |
| ***CCNG2*** | 1555056_at/ 202769_at/ 202770_s_at/ 211559_s_at/ 228081_at | (Chen et al., 2006; Martinez-Gac et al., 2004) |
| ***CDKN1A*** | 1555186_at/ 202284_s_at | (Nakae et al., 2003; Seoane et al., 2004) |
| ***CDKN1B*** | 209112_at | (Dijkers et al., 2000b; Medema et al., 2000; Stahl et al., 2002) |
| ***ESR1*** | 205225_at/ 211233_x_at/ 211234_x_at/ 211235_s_at/ 211627_x_at/ 215551_at/ 215552_s_at/ 217190_x_at/ 207672_at | (Guo and Sonenshein, 2004) |
| ***FASLG*** | 210865_at/ 211333_s_at | (Brunet et al., 1999; Ciechomska et al., 2003) |
| ***FBX032*** | 225801_at/ 225803_at/ 225345_s_at/ 225328_at | (Sandri et al., 2004) |
| ***GADD45A*** | 203725_at | (Furukawa-Hibi, 2002; Tran et al., 2002) |
| ***INSR*** | 207851_s_at/ 213792_s_at/ 226212_s_at/ 226216_at/226450_at | (Puig and Tjian, 2005) |
| ***MXI1*** | 202364_at | (Delpuech et al., 2007) |
| *NOS3* | 205581_s_at | (Potente et al., 2005) |
| ***PCK1*** | 208383_s_at | (Sekine et al., 2007) |
| **POMC** | 205720_at | (Harada et al., 2006; Kim et al., 2006) |
| ***PPARGC1A*** | 1569141_a_at/ 219195_at | (Daitoku et al., 2003) |
| ***PRDX3*** | 201619_at/209766_at | (Chiribau et al., 2008) |
| ***RBL2*** | 212331_at/212332_at | (Chen et al., 2006; Kops et al., 2002b) |
| ***SOD2*** | 215078_at/ 215223_s_at/ 216841_s_at/ 221477_s_at | (Kops et al., 2002a) |
| ***TNFSF10*** | 202687_s_at/202688_at/214329_x_at | (Modur et al., 2002) |

**Table 7: SOD2 and BNIP3 expression leves in various normal and tumor types**

| A: | | | | | | | | |
|---|---|---|---|---|---|---|---|---|
| GEO dataset | Total Nr. of samples | Samples used to calculate average | Tissue type | BNIP3 Probe average intensity and StDv | BNIP3 Probe average intensity and StDv | SOD2 Probe average intensity and StDv | SOD2 Probe average intensity and StDv | FOXO3 average intensity and StDv |
| | | | | *201849_at* | *201848_s_at* | *215223_s_at* | 216841_*s_at* | *204132_s_at* |
| GSE37364, GSE39084, GSE40967, GSE2109, GSE4183, GSE8671, GSE1433, GSE20916 | 121 | 10 | Normal colon active | 6.437889 *0.447166* | 5.869242 *0.373749* | 8.542018 *0.446035* | 7.84363 *0.537961* | 8.588602 *0.372467* |
| | 67 | 10 | Colon adenoma active | 6.711687 *1.426655* | 6.138316 *1.252235* | 9.378831 *1.461469* | 8.080526 *1.439315* | 8.332033 *0.395177* |
| | 1394 | 20 | Colon adenocarcinoma active | 8.396589 *1.112439* | 7.579526 *1.126591* | 10.39538 *0.881171* | 9.472567 *0.847033* | 8.239174 *0.514963* |
| GSE6532, GSE10780, GSE12276, GSE21653, GSE26910, GSE42568, GSE45827,GSE58697, EMTAB365, GSE7307, GSE54002 | 161 | 15 | Normal breast active | 8.319051 *0.28339* | 7.357265 *0.27218* | 7.219094 *0.553115* | 5.77076 *0.533596* | 8.241385 *0.764766* |
| | 440 | 15 | Luminal A breast cancer active | 9.511868 *0.800135* | 8.404704 *0.925308* | 8.357074 *0.823774* | 7.06654 *0.792626* | 8.356409 *0.398693* |
| | 325 | 15 | Luminal B breast cancer active | 9.978344 *0.663878* | 9.024021 *0.779164* | 9.064066 *0.637656* | 7.837008 *0.728711* | 8.083252 *0.885695* |
| | 159 | 15 | HER2 breast cancer active | 9.618208 *1.07878* | 8.657405 *1.224734* | 9.249129 *0.860431* | 8.216121 *0.980804* | 8.340344 *0.548851* |
| | 233 | 15 | Basal like breast cancer active | 9.316237 *0.874281* | 8.305644 *0.897085* | 10.26377 *0.92037* | 8.8135 *0.825788* | 8.593231 *0.566783* |
| GSE31684, GSE31189, GSE30522, GSE7476, GSE11839 | 43 | 10 | Normal bladder active | 8.577845 *0.607024* | 6.271738 *0.894274* | 8.365665 *1.405524* | 8.032263 *2.304808* | 6.757359 *1.061791* |
| | 145 | 15 | Urothelial cell carcinoma active | 8.824586 *0.984354* | 6.906101 *1.109759* | 9.902442 *1.550475* | 9.70053 *1.682397* | 6.842019 *0.963735* |
| GSE26886,GSE32701, GSE42363, GSE45670, GSE7307, GSE40220, GSE14827, GSE17351,GSE33810 | 42 | 8 | Normal esophagus active | 9.511993 *0.521287* | 8.643755 *0.556569* | 9.754773 *0.805589* | 8.51123 *0.655757* | 8.663209 *0.585179* |
| | 20 | 5 | Barrett's esophagus active | 8.348352 *1.608622* | 5.628293 *1.997879* | 8.692696 *0.954547* | 8.083941 *0.973802* | 6.932503 *0.327309* |
| | 45 | 8 | Esophageal Squamous cell carcinoma active | 9.383256 *1.483722* | 8.465146 *1.511957* | 10.16218 *0.82945* | 9.477466 *0.806687* | 7.340267 *1.020953* |
| | 35 | 5 | Esophageal adenocarcinoma active | 6.942516 *1.183051* | 6.364311 *0.832998* | 10.04926 *1.028668* | 9.263281 *0.987494* | 7.8817 *0.361725* |
| GSE50161, GSE16581, GSE44971, GSE15824, GSE19350, GSE68015, GSE11882, GSE7307, GSE4780, GSSE9438, | | 15 | Normal brain active | 10.571 *0.93017* | 9.165685 *0.918522* | 8.420073 *1.815166* | 7.401808 *2.073445* | 8.411185 *0.742852* |
| | | 15 | Astrocytoma active | 10.79248 *0.954059* | 9.2328 *1.07454* | 8.983583 *1.271324* | 8.608461 *1.514669* | 8.04843 *0.540728* |
| GSE73066, GSE5675, GSE33331, GSE22927, GSE53733, GSE45921, GSE50774, GSE34824, GSE36245, GSE13041, GSE43378, GSE4290, GSE73038, GSE37418, GSE67850, GSE12992, GSE74195, GSE50161, GSE66354, GSE68015, GSE66354, GSE50385, GSE16155, GSE21687, GSE45437, GSE12141,GSE70678, GSE9832, GSE51455, GSE19348, GSE16910, GSE18180 | | | | | | | | |
| | | 15 | Ependymoma active | 11.93806 *0.426621* | 10.55883 *0.467185* | 10.10958 *1.631254* | 9.270556 *1.613944* | 7.992423 *0.785697* |
| | | 5 | Glioma active | 11.60068 *0.513624* | 10.003 *0.737599* | 10.38833 *0.873492* | 9.938395 *1.100576* | 7.217868 *0.627584* |
| | | 15 | Glioblastoma active | 11.21654 *0.733984 201849_at* | 10.02933 *0.901195 201848_s_at* | 10.80697 *0.949478 215223_s_at* | 9.990391 *0.960017 216841_s_at* | 7.959677 *0.600796 204132_s_at* |

| **B:** | | | | | | | | |
|---|---|---|---|---|---|---|---|---|
| GEO dataset | Total Nr. of samples | Samples used to calculate average | Tissue type | BNIP3 Probe average intensity and StDv | BNIP3 Probe average intensity and StDv | SOD2 Probe average intensity and StDv | SOD2 Probe average intensity and StDv | FOXO3 average intensity and StDv |
| GSE37364, GSE39084, GSE40967, GSE2109, GSE4183, GSE8671, GSE1433, GSE20916 | 121 | 114 | Normal colon active | 6.766300 *0.551473* | 6.026765 *0.507432* | 8.196375983 *0.570831* | 6.995536285 *0.790092* | 8.415143741 *0.431592* |
| | 76 | 12 | Colon adenoma active | 6.904867 *1.383773* | 6.305086 *1.205611* | 9.439642059 *1.370847* | 8.100363041 *1.318199* | 8.30777867 *0.368975* |
| | 1341 | 450 | Colon adenocarcinoma active | 7.417266 *1.412652* | 6.773805 *1.18832* | 9.715393514 *0.918502* | 8.650113556 *0.955991* | 8.014610253 *0.701335* |
| GSE6532, GSE10780, GSE12276, GSE21653, GSE26910, GSE42568, GSE45827, GSE58697, EMTAB365, GSE7307, GSE54002 | 175 | 149 | Normal breast active | 8.580632 *0.705929* | 7.502588 0.623842 | 7.105725 *0.763677* | 5.838791 *0.945085* | 8.122807 *0.818499* |
| | 624 | 536 | Luminal A breast cancer active | 8.967998 *0.74231* | 7.772701 *0.693106* | 7.603739 *0.715028* | 6.430053 *0.835848* | 8.185212 *0.603764* |
| | 486 | 304 | Luminal B breast cancer active | 9.394412 *0.934612* | 8.228799 *0.950355* | 8.127528 *0.875258* | 6.954036 *0.952059* | 8.048243 *0.784529* |
| | 267 | 206 | HER2 breast cancer active | 9.306159 *0.967564* | 8.168413 *1.029658* | 8.580925 *0.846655* | 7.311002 *1.018047* | 8.275227 *0.636811* |
| | 319 | 255 | Basal like breast cancer active | 9.289813 *1.065399* | 8.300672 *1.082328* | 9.710912 *1.095402* | 8.375586 *1.186299* | 8.36859 *0.665996* |
| GSE31684, GSE31189, GSE30522, GSE7476, GSE11839 | 43 | 10 | Normal bladder active | 8.578437 *0.607002* | 6.272345 *0.893837* | 8.365931 *1.40522* | 8.03247 *2.303875* | 6.758705 *1.060427* |
| | 145 | 34 | Urothelial cell carcinoma active | 8.376082 *1.336647* | 6.953712 *1.230363* | 9.394505 *1.605892* | 8.772433 *2.090078* | 6.779379 *0.935848* |
| GSE26886, GSE32701, GSE42363, GSE45670, GSE7307, GSE40220, GSE14827, GSE17351, GSE33810. | 42 | 35 | Normal esophagus active | 10.31495 *0.521287* | 8.398459 *0.746881* | 9.566248 *0.805589* | 8.839943 *1.158115* | 7.529135 *0.910611* |
| | 20 | 4 | Barrett's esophagus active | 8.721536 *1.608622* | 5.915304 *2.184687* | 8.924126 *0.954547* | 8.287561 *0.993971* | 7.061635 *0.177972* |
| | 44 | 37 | Esophageal Squamous cell carcinoma active | 9.907335 *1.483722* | 8.888424 *1.134621* | 9.80937 *0.82945* | 9.353739 *0.609105* | 6.88251 *0.741576* |
| | 35 | 12 | Esophageal adenocarcinoma active | 6.511577 *1.183051* | 5.959288 *1.352522* | 9.744908 *1.028668* | 9.155751 *0.942176* | 7.734009 *0.77448* |
| GSE50161, GSE16581, GSE44971, GSE15824, GSE19350, GSE68015, GSE11882, GSE7307, | 515 | 73 | Normal brain active | 10.69179 *0.823843* | 9.183911 *0.938014* | 7.41698 *1.410068* | 6.147368 *1.682804* | 8.298635 *0.619204* |
| | 221 | 94 | Astrocytoma active | 10.42371 *0.697635* | 8.827601 *0.752459* | 8.171173 *1.309707* | 7.642736 *1.45043* | 7.760604 *0.691973* |
| GSE4780, GSSE9438, GSE73066, GSE5675, GSE53733, GSE45921, GSE50774, GSE34824, GSE36245, GSE13041, GSE43378, GSE4290, GSE73038, GSE37418, GSE67850, GSE12992, GSE66354, GSE68015, GSE66354, GSE50385, GSE16155, GSE21687, GSE45437, GSE12141, GSE70678, GSE9832, GSE51455, GSE19348, GSE33331, GSE22927, GSE74195, GSE50161, GSE16910, GSE18180 | 120 | 50 | Ependymoma active | 11.18481 *0.801885* | 9.983857 *0.68127* | 9.51132 *1.496878* | 8.602994 *1.529031* | 7.933142 *0.797787* |
| | 39 | 20 | Glioma active | 10.93251 *0.935365* | 9.44152 *1.044345* | 8.81291 *1.891867* | 8.088357 *1.994029* | 7.554503 *0.640079* |
| | 289 | 179 | Glioblastoma active | 10.66393 *0.807012* | 9.005034 *1.162699* | 9.234986 *1.331689* | 8.592844 *1.434318* | 7.662142 *0.889288* |

**SEQUENCE LISTING:**

| Seq. No.: | Gene : |
|---|---|
| Seq. 1 | AGRP |
| Seq. 2 | ATG14 |
| Seq. 3 | ATP8A1 |
| Seq. 4 | BCL2L11 |
| Seq. 5 | BCL6 |
| Seq. 6 | BIRC5 |
| Seq. 7 | BNIP3 |
| Seq. 8 | BTG1 |
| Seq. 9 | C10orf10 |
| Seq. 10 | CAT |
| Seq. 11 | CAV1 |
| Seq. 12 | CBLB |
| Seq. 13 | CCND1 |
| Seq. 14 | CCND2 |
| Seq. 15 | CCNG2 |
| Seq. 16 | CDKN1A |
| Seq. 17 | CDKN1B |
| Seq. 18 | DDB1 |
| Seq. 19 | DYRK2 |
| Seq. 20 | ERBB3 |
| Seq. 21 | EREG |
| Seq. 22 | ESR1 |
| Seq. 23 | EXT1 |
| Seq. 24 | FASLG |
| Seq. 25 | FBXO32 |
| Seq. 26 | FGFR2 |
| Seq. 27 | GADD45A |
| Seq. 28 | IGF1R |
| Seq. 29 | IGFBP1 |
| Seq. 30 | IGFBP3 |
| Seq. 31 | INSR |
| Seq. 32 | KLF2 |
| Seq. 33 | KLF4 |
| Seq. 34 | LGMN |
| Seq. 35 | MXI1 |
| Seq. 36 | MYOD1 |
| Seq. 37 | NOS3 |
| Seq. 38 | PCK1 |
| Seq. 39 | PDK4 |
| Seq. 40 | POMC |
| Seq. 41 | PPARGC1A |
| Seq. 42 | PPM1D |
| Seq. 43 | PRDX3 |
| Seq. 44 | RAG1 |
| Seq. 45 | RAG2 |
| Seq. 46 | RBL2 |
| Seq. 47 | SEMA3C |
| Seq. 48 | SEPP1 |
| Seq. 49 | SESN1 |
| Seq. 50 | SIRT1 |
| Seq. 51 | SLC5A3 |
| Seq. 52 | SMAD4 |
| Seq. 53 | SOD2 |
| Seq. 54 | STK11 |
| Seq. 55 | TLE4 |
| Seq. 56 | TNFSF10 |
| Seq. 57 | TXNIP |

## Claims

1. A product comprising:
primers and probes for determining a gene expression level of two or more, preferably at least four, FOXO target genes in an extracted sample of a medical subject, wherein the target genes are selected from the group consisting of SOD2, BNIP3, MXI1, PCK1, PPARGC1A and CAT, preferably selected from SOD2, BNIP3, MXI1 and PCK1.

2. Product according to claim 1 further comprising primers and probes for determining the expression levels of two or more target genes of the FOXO/PI3K cellular signaling pathway selected from the group consisting of: AGRP, BCL2L11, BCL6, BTG1, CAV1, CCND1, CCND2, CCNG2, CDKN1A, CDKN1B, ESR1, FASLG, FBXO32, GADD45A, INSR, NOS3, POMC, PRDX3, RBL2 and TNFSF10.

3. Product according to any one of the preceding claims, wherein the product is a PCR kit, a RNA-sequencing kit, or a microarray kit.

4. Product according to any one of the preceding claims wherein the product is a kit.

5. Kit according to claim 4 comprising containers, each with one or more of the various reagents selected from pre-fabricated microarrays, buffers, the appropriate nucleotide triphosphates, reverse transcriptase, DNA polymerase, RNA polymerase, and one or more primers.

6. Use of a product or kit according to any one of the preceding claims for inferring the activity of a PI3K cellular signaling pathway in a subject based at least on the oxidative stress state of the subject, preferably the oxidative stress state of a FOXO transcription factor element in the subject, comprising,
inferring the oxidative stress state of the subject, preferably the oxidative stress state of the FOXO transcription factor element in the subject, based on the expression levels of two or more genes selected from the group consisting of SOD2, BNIP3, MXI1, PCK1, PPARGC1A and CAT in an extracted sample from the subject.

7. Use according to claim 6, wherein an oxidative stress state is inferred, when the expression level(s) of SOD2 and/or BNIP3 are upregulated in an extracted sample of the subject compared to a control sample and/or when the expression level(s) of one or more target gene(s) selected from MXI1, PCK1, PPARGC1A and CAT are downregulated in an extracted sample of the subject compared to a control sample

8. Use according to claim 6 or 7, wherein said inferring the activity of the PI3K cellular signaling pathway in the subject is based on the inferred oxidative stress state of the subject and the activity level of the FOXO transcription factor element in the subj ect,

9. Use according to any one of claims 6 to 8, wherein the activity level of the FOXO transcription factor element in the subject is determined based at least on expression levels of one or more, preferably at least three, target gene(s) of the PI3K cellular signaling pathway measured in an extracted sample of the subject selected from the group consisting of: AGRP, BCL2L11, BCL6, BNIP3, BTG1, CAT, CAV1, CCND1, CCND2, CCNG2, CDKN1A, CDKN1B, ESR1, FASLG, FBXO32, GADD45A, INSR, MXI1, NOS3, PCK1, POMC, PPARGC1A, PRDX3, RBL2, SOD2 and TNFSF10.

10. Use according to any one of claims 6 to 9, wherein the activity level of the FOXO transcription factor element is determined further based at least on expression levels of one or more, preferably at least three, target gene(s) of the PI3K cellular signaling pathway measured in the extracted sample of the subject selected from the group consisting of: ATP8A1, C10orf10, CBLB, DDB1, DYRK2, ERBB3, EREG, EXT1, FGFR2, IGF1R, IGFBP1, IGFBP3, LGMN, PPM1D, SEMA3C, SEPP1, SESN1, SLC5A3, SMAD4 and TLE4 and/or selected from the group consisting of: ATG14, BIRC5, IGFBP1, KLF2, KLF4, MYOD1, PDK4, RAG1, RAG2, SESN1, SIRT1, STK11 and TXNIP.

11. Use according to any one of claims 6 to 10, further comprising:
determining whether the PI3K cellular signalling pathway is operating abnormally in the subject based on the inferred activity of the PI3K cellular signalling pathway in the subject.

12. Use according to any one of claims 6 to 11, further comprising:
recommending prescribing a drug for the subject that corrects for abnormal operation of the PI3K cellular signalling pathway, wherein the recommending is performed if the PI3K cellular signalling pathway is determined to be operating abnormally in the subject based on the inferred activity of the PI3K cellular signalling pathway.

13. Use according to any one of claims 6 to 12 to indicate a cancer or pre-cancer state in a subject.

14. Use according to any one of claims 6 to 13 in at least one of the following activities:
diagnosis based on the inferred activity of the PI3K cellular signalling pathway in the subject;
prognosis based on the inferred activity of the PI3K cellular signalling pathway in the subject;
drug prescription based on the inferred activity of the PI3K cellular signalling pathway in the subject;
prediction of drug efficacy based on the inferred activity of the PI3K cellular signalling pathway in the subject;
prediction of adverse effects based on the inferred activity of the PI3K cellular signalling pathway in the subject;
monitoring of drug efficacy;
drug development;
assay development;
pathway research;
cancer staging;
enrollment of the subject in a clinical trial based on the inferred activity of the PI3K cellular signalling pathway in the subject;
selection of subsequent test to be performed; and
selection of companion diagnostics tests.

15. Method for inferring the activity of a PI3K cellular signaling pathway in a subject based at least on the oxidative stress state of a FOXO transcription factor element in the subject, comprising,
inferring the oxidative stress state of the subject, the oxidative stress state of the FOXO transcription factor element in the subject, based on the expression level(s) of two or more, preferably four or more, more preferably all, of the FOXO target genes selected from the group consisting of SOD2, BNIP3, MXI1, PCK1, PPARGC1A and CAT in an extracted sample from the subject,
wherein an oxidative stress state is inferred, when the expression level(s) of SOD2 and/or BNIP3 are upregulated in an extracted sample of the subject compared to a control sample and/or when the expression level(s) of one or more target gene(s) selected from MXI1, PCK1, PPARGC1A and CAT are downregulated in an extracted sample of the subject compared to a control sample,
wherein said inferring the activity of the PI3K cellular signaling pathway in the subject is based on the inferred oxidative stress state of the subject and the activity level of the FOXO transcription factor element in the subject,
wherein the activity level of the FOXO transcription factor element in the subject is determined based at least on expression levels of one or more, preferably at least three, target gene(s) of the PI3K cellular signaling pathway measured in an extracted sample of the subject selected from the group consisting of: AGRP, BCL2L11, BCL6, BNIP3, BTG1, CAT, CAV1, CCND1, CCND2, CCNG2, CDKN1A, CDKN1B, ESR1, FASLG, FBXO32, GADD45A, INSR, MXI1, NOS3, PCK1, POMC, PPARGC1A, PRDX3, RBL2, SOD2 and TNFSF10.


SEQUENCE LISTING

<110>  Koninklijke Philips N.V.

<120>  Method to distinguish tumor suppressive FOXO activity from
       oxidative stress

<130>  2016PF01211

<160>  57

<170>  PatentIn version 3.5

<210>  1
<211>  783
<212>  DNA
<213>  Homo sapiens

<400>  1
agctcctagg tccctgtcct gtggaaattt gtggaccctg ggcaccctct cttgctccca      60

aattttaatc ggctcctgga aacctcaccc caaattggag ataggcactc ctcttgtaga     120

acaaaaggct caggttcagg gagtgagggc ctgaactgtg cccccaccct ccaggaaggg     180

tccttcacgg cctggctgca gggatcagtc acgtgtggcc cttcattagg ccctgccata     240

taagccaagg gcacggggtg gccgggaact ctctaggcaa gaatcccgga ggcagaggcc     300

atgctgaccg cagcggtgct gagctgtgcc ctgctgctgg cactgcctgc cacgcgagga     360

gcccagatgg gcttggcccc catggagggc atcagaaggc ctgaccaggc cctgctccca     420

gagctcccag gcctgggcct gcgggcccca ctgaagaaga caactgcaga acaggcagaa     480

gaggatctgt tgcaggaggc tcaggccttg gcagaggtac tagacctgca ggaccgcgag     540

ccccgctcct cacgtcgctg cgtaaggctg catgagtcct gcctgggaca gcaggtgcct     600

tgctgtgacc catgtgccac gtgctactgc cgcttcttca atgccttctg ctactgccgc     660

aagctgggta ctgccatgaa tccctgcagc cgcacctagc tggccaacgt cagggtcggg     720

gctagggtag gggcaaggaa actcgaataa aggatgggac caacaaaaaa aaaaaaaaaa     780

aaa                                                                   783


<210>  2
<211>  4760
<212>  DNA
<213>  Homo sapiens

<400>  2
   aaaatcccac gtgactggct ctcctctcag gccatcatgg cgtctcccag tgggaaggga      60
   gcccgggcgc tggaggctcc tggctgcggg ccccggccgc tcgcccggga cctggtggac     120
   tccgtggacg atgcggaggg gctgtacgtg gctgtggagc gctgcccgct gtgcaacact     180
   acccgccggc ggctgacctg cgccaaatgc gttcagagcg gcgatttcgt ctacttcgac     240
   ggccgcgacc gggagaggtt tatcgacaag aaggaaaggt taagccgact taagagcaag     300
   caagaagaat ttcagaaaga agtgttaaaa gctatggaag gaaaatggat aacagatcag 360
   ttgagatgga aaataatgtc ctgcaagatg aggattgaac agttaaaaca aacaatatgt 420
   aaaggaaatg aagaaatgga gaaaaattct gaaggccttc tcaaaaccaa ggaaaagaat 480
   cagaagcttt acagtcgagc acaacggcac caagagaaaa aggagaagat tcagaggcat 540
   aatcgcaaac ttggtgacct ggtagaaaaa aagaccattg acttaagaag tcattatgag 600
   cgtctggcaa atcttcgacg atcccatata ttagagctca cctctgtcat ttttccaatc 660
   gaggaagtaa agacgggtgt gagagacccc gcagatgtgt cttcagagag tgacagtgcc 720
   atgacctcca gcactgtgag caagcttgct gaagcccgga ggacaactta cctctcagga 780
   cgatgggtct gtgacgatca caacggagac accagcatta gcattacagg gccttggatt 840
   agcctcccta acaatgggga ctactctgcc tactacagct gggtggagga gaagaaaaca 900
   acccaggggc ctgacatgga gcagagtaac cctgcctaca ccatcagtgc tgcgctgtgc 960
   tatgcaactc agctggtcaa cattctgtct catatacttg atgtaaatct tcccaaaaag 1020
   ctctgcaaca gtgaattttg tggcgaaaat ctaagcaagc agaaatttac tcgagcagtg 1080
   aagaaactga atgcaaatat tctttacctt tgtttttctc agcatgtaaa tttagatcaa 1140
   ttacaaccac tgcataccct caggaatcta atgtacctgg tcagtccaag ctctgaacac 1200
   ctaggcaggt cagggccctt tgaagtacga gcagaccttg aggagtccat ggaatttgtg 1260
   gatcccggag ttgctggaga atcagatgag agcggagatg agcgcgtcag cgatgaagaa 1320
   accgacctgg gcacagactg ggagaacttg cctagtcccc ggttttgtga tatcccttcc 1380
   cagtctgtgg aagtctccca gagtcagagc acccaggcgt ccccacccat cgcgagcagc 1440
   agtgcaggtg ggatgatctc ctctgcagca gcctcggtga cctcctggtt taaagcttac 1500
   actggacacc gttaacgagc atggaccaaa acataccaaa tctgcatcaa gaaagttctt 1560
   ctcccactac actctagtaa acattttctg tttaagttaa gatagtgtct ggaacaaaga 1620
   ggttaaagtg ttgttttgtt ttgtcttttt aagcagggag acaaacattt ctatttgcca 1680
   agtggcctgt gatggtgacc aacatgctta tgataattaa gagaacaggg gtcgaaggtc 1740
   tttctaccca gaccagtgct ggtggaagga ggacctgtgc gtgtggccag ttctgccaag 1800
   gaagcagttg atttgggttc cctctgggcc cgggccaccg ggcccacaga tatgggtcag 1860
   tgtgctggtc cttgcggtgc tgagactgtt cctgacactt taagttttag aggttggttg 1920
   aatcacaaga ggtgattctt gattattagg acatgaaaga taaaagctct ttaataagag 1980
   tttttctgcc attgtttttt gtatgagaac cagcaggcaa tttaaaattt ctaatttggt 2040
   cctttgattt tgtttgggag gggtgagtta cacgtatttt attcatgctg ctctgtcgta 2100
   gtttgtcaga cattcctgtt tttctttccc ccacacacca aagaaaatga aagtcttttt 2160
   ctttaggacc cacatccata aatggaagaa atcctggctg caataatgtc tagagagttt 2220
   ttaactattt tcttgtattc tgaggggaat taagcttatt cttacctagt tgaattcctg 2280
   ccatccacac tatgagcatt ttgaaattga acttatattt tctgggtgaa aataagtcat 2340
   gaaggtcatt cccttatgta agctcaatgc ctgcctgggc acaggggaaa agccacttag 2400
   ttaagtggcc tctggtcatt cttgtggtgt ccactttctt tctatgggat tgagtaggtg 2460
   gcaggtgttt tcaggggaaa ccatcctact tgtttccccg aactctttgt tgctctgagg 2520
   acacagcttt gctcagaaat gcagcgcaga tccttacggc tgatgctact ctgctctgtt 2580
   ctggggaaag cacaatataa agaaagaatt tcccagccag gcgcagtggc tcacgcctgt 2640
   aatcccagca ctttaggagg ccgaggcagg cggatcactt gaggtcagga gtttgagagc 2700
   agcctggcta acatggtgaa accctgtttc tactaaaaat acaaaaaatt accgggtgtg 2760
   gtggcgcacg cctgtaatcc cagctactcg ggaggctgcg gcaggagaat cgcttgaacc 2820
   gggaggcaga ggttgcagtg agccgagatt gtgccattgc actccagcct gggcaacaag 2880
   agcgaaactc cgtctcaaaa aaaaaaagaa tttccctcag caggagatca ttttcagctc 2940
   acgtgtcttg tcattctttt agtgacaatc ttacaagaaa actataatga gagaggcatt 3000
   atgtacaaat atgtaagtag tttattttta ataactgcaa aaaaatccta tgtaacaact 3060
   accaaaagaa atcctatgaa agagtcctaa caggcattat taccatatct tatgtgattg 3120
   gcatgatagc acctctgata aatcattcag aggtttgcca tgccccagct tcttttctca 3180
   tcataataat tgtagttgat actttgcctc caagtccgag gtgctatata gcttttgcta 3240
   atggtatatt tggtgttttg tatagttttg ggtagagttg cagaacggag tttatttcta 3300
   tccggtagtc acaaattcct tggctctatg aattttccat gaaaggagga agtaggcttt 3360
   tctcgttgtg ggtggtcttt tttttttttg gagacggagt ctcactcagc tgcccaggct 3420
   ggagtgtagt ggcaccatct ccgctcactg caaccaccat ctcctgggtt caagcaattc 3480
   ttccatctca acctcccgag tagctgggat tataggcacc tgccatcatg cccagctaat 3540
   ttttgtattt tagtaaagac ggggggtttc accatgttgg ccaggctggt cttgaactcc 3600
   tgacttcagg tgatccgctt gccttggcct cctaaagtgc taggattaca ggcctgagcc 3660
   accgcgcccg gccccttatg ggttcttcta cactgctggg atctctgttt taagtgctca 3720
   gcttcatgat tgattgctgg gcttccattt tcccatccag ttctggagtt cgtagagagt 3780
   gaagatggta gacttgaaca gataaataaa cttaacgatc ttgtaagagt tgtctagcta 3840
   cttaaaaccc tcagaagtaa gagcttagtc tcacgagttg taagagtggg atttggagct 3900
   tggtggtgga gactgacttc agctgagaga tgcacaacag tcatggtttt cttaagcctc 3960
   ttatgaaacc atgaatgaga gatgaagcta aagaatagaa tccagagatc acaaactcat 4020
   ctagagtact tccacaaaat ttacaaagat gtgggaactt tatggatagg atatattttg 4080
   tttgttgttg ttaatatcaa ctagaggcac tttacatagg gttaagtgat cgaacccttt 4140
   tgtggttttg aacaccaaca tactggctta cactgctgaa atattttggg tttcattatt 4200
   ttgcactgga tccaccctgt aaatactctt aagtatacat ttcaaccact gttttttcta 4260
   ctctttttgc tgctcattaa aatctttcat gtaggtgcca gaaccatatg taaacagctt 4320
   tttaaaaaat tgaagctggt attttgttta aacaaaaagc catagaactt ggtcatgttt 4380
   tccattttaa aatgatttac tgaaacaaag taatactaat aaaaacccac aggcaccaaa 4440
   caggctgctt aaaatggtct gttaaagaca ttttttggtt atggaatata agaaaagttt 4500
   tgcacatctg taagggggaa aaacagtata tcaccattgg gtagagtgga cgggactcat 4560
   gtaaggactc aatttgggga agagcattca gtggcatgct gttagaggac tagtgtccga 4620
   gaatctcctc acagtatcat gttgcaggaa ttccccattg ctctgcaact tccaaaccag 4680
   tttgagtcat acaaatgttt tctaaacttt tattgtatta ctgcaataaa tcttttaaca 4740
   gtaaaaaaaa aaaaaaaaaa 4760

<210> 3
<211> 8270
<212> DNA
<213> Homo sapiens

<400> 3
   aagagctcgc ccagctctgc gggcgccgcc accttcgccg ccaccgctgc ctttctcctc 60
   ctcctgtcgg cgtgcggggg ccgcgcccgg cggcagctct gccctaggtg ggcggcggcg 120
   cggcccaggc tgcagctgag cgctctgcgc ggcgcagccg ggtctcccgc gtgtaccacg 180
   ccgtgacagg tgcagagtcc gggctgagga cccacctgca gccgccgccg cgatgcccac 240
   catgcggagg accgtgtcgg agatccgctc gcgcgccgaa ggttatgaga agacagatga 300
   tgtttcagag aagacctcac tggctgacca ggaggaagta aggactattt tcatcaacca 360
   gccccagctg acaaaattct gcaataacca tgtcagcact gcaaaataca acataatcac 420
   attccttcca agatttctct actctcagtt cagaagagct gctaattcat tttttctctt 480
   tattgcactg ctgcagcaaa tacctgatgt gtcaccaaca ggtcgttata caacactggt 540
   tcctctctta tttattttag ctgtggcagc tatcaaagag ataatagaag atattaaacg 600
   acataaagct gataatgcag tgaacaagaa acaaacgcaa gttttgagaa atggtgcttg 660
   ggaaattgtc cactgggaaa aggtggcagt aggggagata gtgaaagtga ccaatgggga 720
   acatctccca gcagatctca tcagtctgtc ctcaagtgag ccccaagcca tgtgctacat 780
   tgaaacatcc aacttagatg gtgaaacaaa cttgaaaatt agacagggct taccagcaac 840
   atcagatatc aaagacgttg acagtttgat gaggatttct ggcagaattg agtgtgaaag 900
   tccaaacaga catctctacg attttgttgg aaacataagg cttgatggac atggcaccgt 960
   tccactggga gcagatcaga ttcttcttcg aggagctcag ttgagaaata cacagtgggt 1020
   tcatggaata gttgtctaca ctggacatga caccaagctg atgcagaatt caacaagtcc 1080
   accacttaag ctctcaaatg tggaacggat tacaaatgta caaattttga ttttattttg 1140
   tatcttaatt gccatgtctc ttgtctgttc tgtgggctca gccatttgga atcgaaggca 1200
   ttctggaaaa gactggtatc tcaatctaaa ctatggtggc gctagtaatt ttggactgaa 1260
   tttcttgacc ttcatcatcc ttttcaacaa tctcattcct atcagcttat tggttacatt 1320
   agaagttgtg aaatttaccc aggcatactt cataaattgg gatcttgaca tgcactatga 1380
   acccacagac actgctgcta tggctcgaac atctaatctg aatgaggaac ttggccaggt 1440
   taaatacata ttttctgaca aaactggtac tctgacatgc aatgtaatgc agtttaagaa 1500
   gtgcaccata gcgggagttg cttatggcca tgtccctgaa cctgaggatt atggctgctc 1560
   tcctgatgaa tggcagaact cacagtttgg agatgaaaaa acatttagtg attcatcatt 1620
   gctggaaaat ctccaaaata atcatccaac tgcacctata atatgtgaat ttcttacaat 1680
   gatggcagtc tgtcacacag cagtgccaga gcgagaaggt gacaagatta tttatcaagc 1740
   agcatctcca gatgagggag cattggtcag agcagccaag caattgaatt ttgttttcac 1800
   tggaagaaca cccgactcgg tgattataga ttcactgggg caggaagaaa gatatgaatt 1860
   gctcaatgtc ttggagttta ccagtgctag gaaaagaatg tcagtgattg ttcgcactcc 1920
   atctggaaag ttacgactct actgcaaagg agctgacact gtaatttatg atcgactggc 1980
   agagacgtca aaatacaaag aaattaccct aaaacattta gagcagtttg ctacagaagg 2040
   gttaagaact ttatgttttg ctgtggctga gatttcagag agcgactttc aggagtggcg 2100
   agcagtctat cagcgagcat ctacatctgt gcagaacagg ctactcaaac tcgaagagag 2160
   ttatgagttg attgaaaaga atcttcagct acttggagca acagccattg aggataaatt 2220
   acaagatcaa gtgcctgaaa ccatagaaac gctaatgaaa gcagacatca aaatctggat 2280
   ccttacaggg gacaagcaag aaactgccat taacatcgga cactcctgca aactgttgaa 2340
   gaagaacatg ggaatgattg ttataaatga aggctctctt gatggaacaa gggaaactct 2400
   cagtcgtcac tgtactaccc ttggtgatgc tctccggaaa gagaatgatt ttgctcttat 2460
   aattgatggg aaaaccctca aatatgcctt aacctttgga gtacgacagt atttcctgga 2520
   cttagctttg tcatgcaaag ctgtcatttg ctgtcgggtt tctcctcttc aaaaatctga 2580
   agttgttgag atggttaaga aacaagtcaa agtcgtaacg cttgcaatcg gtgatggagc 2640
   aaatgatgtc agcatgatac agacagcgca cgttggtgtt ggtatcagtg gcaatgaagg 2700
   cctgcaggca gctaattcct ctgactactc catagctcag ttcaaatatt tgaagaattt 2760
   actgatgatt catggtgcct ggaactataa cagagtctcc aagtgcatct tatactgctt 2820
   ctacaagaat atagtgctct atattatcga gatctggttt gcctttgtta atggcttttc 2880
   tggacagatc ctctttgaaa gatggtgtat aggtctctat aacgtgatgt ttacagcaat 2940
   gcctccttta actcttggaa tatttgagag atcatgcaga aaagagaaca tgttgaagta 3000
   ccctgaatta tacaaaacat ctcagaatgc cctggacttc aacaccaagg ttttctgggt 3060
   tcattgttta aatggcctct tccactcagt tattctgttt tggtttccac taaaagccct 3120
   tcagtatggt actgcatttg gaaatggaaa aacctcggat tatctgctac tgggaaactt 3180
   tgtgtacact tttgtggtga taactgtgtg tttgaaagct ggattggaga catcatattg 3240
   gacatggttc agccacatag cgatatgggg gagcatcgca ctctgggtgg tgttttttgg 3300
   aatctactca tctctgtggc ctgccattcc gatggcccct gatatgtcag gagaggcagc 3360
   catgttgttc agttctggag tcttttggat gggcttgtta ttcatccctg tggcatctct 3420
   gctccttgat gtggtgtaca aggttatcaa gaggactgct tttaaaacat tggtcgatga 3480
   agttcaggag ctggaggcaa aatctcaaga cccaggagca gttgtacttg gaaaaagcct 3540
   gaccgagagg gcgcaactgc tcaagaacgt ctttaagaag aaccacgtga acttgtaccg 3600
   ctctgaatcc ttgcaacaaa atctgctcca tgggtatgcg ttctctcaag atgaaaatgg 3660
   aatcgtttca cagtctgaag tgataagagc atatgatacc acgaaacaga ggcccgacga 3720
   atggtgatgg ggagagcctg aaaggcaggc tctgttacct ctctaaggag agctaccagg 3780
   ttgtcaccgc agtctgctaa ccaattccag tctggtccat gaagaggaaa ggtagatctg 3840
   agctcatctc gctgatggac attcagattc atgtatatta tagacataag cactgtgcaa 3900
   ctgtactgta acaccatctc ttttggattt ttttaaggta tttgctaagt ctttgtaaac 3960
   ggaaattgaa aatgacctgg tatcttgcca gagggctttc ttaaacggag aataagtcag 4020
   tattcttatg ccattactgt ggggctgtaa ctgactgtca gtttattggc tgtaccacaa 4080
   ggtaaccaac cattaaaaaa ctctaaatga tatttagtta aaggactctt tggtatccag 4140
   acttagattt caggatatgc tgaaacaaac cagcattctt aaggaactga ctcaccttcc 4200
   tgagcaaaat ttctaaacaa gcatttgtgt ccaaaattgt cttgataaat gtttgccaaa 4260
   gaggttcagt aagtgttttt ctagttcagt agtcatatgc ccagaaatgt aagagaaagt 4320
   ttacttccag ttccgctgta agatctgcat gcctgacttt ccaaatgtaa gagtgattta 4380
   caaaaatgaa tatttcaagg catttgctac taaaatcggt gatgttgcac ctttggcctt 4440
   acaaatgctt ctttgttgtt tgtcgtgttt atttgttaga ggacacacgt gttaatgtga 4500
   ctctgttgtt atgacactga tttttcaaac tatgtatgtt tcaggtattt ctgatgaagt 4560
   ttcatcatca tttagatttt tctaaaaatc tggctaatgc agtagattga gtgatgtcat 4620
   tttgtcttaa agtttttcct cttaagaaac atatgctacg tatttacgtg ggatttccaa 4680
   agcttctgtt gcaatatttg gaataacatg tcagataaat gcatgggctt ttgtcctgtg 4740
   ttccagttcc cactagagat gcctgtgtct tgtgtagcac acccagtgtt atggtgactg 4800
   ccccctatac tgaagactga aaattatttc acagttcact catcaaatag ttcccaaaat 4860
   tcgtcacatg ctgcttattg ggacaaatag gtagtacatt ttccccattt aaaaaatgcg 4920
   gattttactc aggccggtaa ctttacagtc agaggacacg ttcatcatga gtagcttttg 4980
   ttagtatgtt ttaaaatgta tcttcagttc aattattttc agcatttaca agacatctga 5040
   aaatggctat tttgctacca acagtaaatg aaggggctgt ttaaaaacca caaccagttt 5100
   tctacactat tttttaaata atactttcat ttgaaaaaaa ggaattagtt ttcagataca 5160
   cttcagagat tgaagcaaac tatttgcctt ttactcaaaa gcctgcttgc ctttacatgg 5220
   acttaccagc aaaataggta gaactttctc ttttaaaaaa agtcaactag aattgagaag 5280
   aggtgatttt ttttcagatc gcttctcgag tttaatattt tcacattctt ttcacccttt 5340
   ttctcaatct agatttaaaa ttaggatata tgtcatttcc ttgtctgtat ttgtagctcc 5400
   ttagttacca gtatgcctct ccatttctta caaataagag gttataacac atatacataa 5460
   ttctaacctt aagggaacac acgtttacat actttacttc ccaagccctt cctgtttggg 5520
   gtacagattg agagagtcat gaatcaacac atctagcaag accacaggtg taagagtcta 5580
   agatcgtctt caaaattctg aagtcccagt ctttacctgt ccagtgaatg aatattcaga 5640
   gcagcttttc ctgggcttcc cagtggtgat agctgaggtc aaaccacaaa aaataagaaa 5700
   gcaagagtga aatgcacccc tccagagaaa cactttgtag tgtttaattc tgttaataga 5760
   gaagagctgc ttctgtttgc gctcacttca tcagtggcac ccttctgcag aattttaata 5820
   taaaaacatt atggatataa tagaactgga ttttctgact taaaaatgta agttttattt 5880
   taatcttgaa acgtggattg tttctgtgga gctcttaaac atgagaagaa tacttacggt 5940
   tgataatgtg taacatgatc tgaaatgtga ctaatttgag cctctttgtc ccatcgtcct 6000
   gtttttgaat tattgacatt gtcagtctct ttgcttcctg ggtgagactt ggggtttgag 6060
   ggacagggaa tgaccttctt ggtgaaactt aaaatataac attgcaattg cagtgacttt 6120
   acagtgttaa attagagaaa atagtctgat tttttaaacc ttccttaact ggaaaaaagt 6180
   cacatggttt taccaggatt gaaataaaca gtcaatgtga cttttaacat gtgttttttt 6240
   gaaataaagg gcacgtactc ttcaattaaa aagttcctta tagggactct ggcaaatgct 6300
   aacacagttg ctttacaatg tttacaattc agacaatacg acttataata gaaaatcctc 6360
   attcatttag cattgaaaag ctggaagttg cttctttaat gttgaatagt atacagtggt 6420
   attgagcatg gactttctaa atgttttata tatacatata aaaatatatt ggtgtctcac 6480
   acccagaaag atgttatatt gtagatatta ttaggaaaac agtgtttctc aggaacgttg 6540
   taaattttaa atgatatatg tacttcccgt cctcccacct ccactctgtg ctctaatgtg 6600
   agactgcttc agcagtgttg ctaagttaat ggaaaacttt ttctaatcaa gtcaggtgaa 6660
   tgtgtattct gctaaataat gttagccatt tacatgaatt gtatggtcat taaatggaat 6720
   cagtgattcc tctttaattt ccagagggga aatgaattat ggaaatcagt cagcattctg 6780
   atcattaaat tttatacttt aattttgccg ttcagcattc taaatatcca atgtgaaagt 6840
   cacatgataa tttgttttgc attgcgtgca ctgtacaaca cttacaactt gtcatttaaa 6900
   atgttttctc gggaaatgaa tgctagtcag aaagtaatag attgtattat tcatagtttt 6960
   aaaattatga caatgtcata attactacaa agctaaataa tcgtgtttat ttttgtgcag 7020
   ttgccctttg atagttcctg gttttaaaac ctattaagtg tataatctta caaatagtca 7080
   tctacaaaat ttatggagaa agtgcccagc ccattcacat cacatggacc aggaattctt 7140
   ttgtaaatga cttaaggtaa catcatgcag ttcagtgcct aataaatgct ttttaatgat 7200
   gaacatttct ataatgactc gtaagatacc atagtctgat ttttctcaca ttaaaataac 7260
   tgaagtcact tgtgtaacgt agttatactt tgctgcattt taattaacct tcaacagcta 7320
   ttaaagtgga atgtaagtta aattttgaag gaaaggaaat aaatgttttc catatttcgt 7380
   cttgatttac tttctgtatg agaacagctg tgtttttgat aggtttatgg tttgcatgag 7440
   ttcatattta aagtgatcca ggccaatgca tggctattgc tgtaaatctt gatgtttatt 7500
   tctgccttgt aaagttctat cacggcctac ctggaattta aaattcagta gacaaattaa 7560
   ttggtcctct gcacaacttt tttaataagt agattatttt acaaagaaat ttgaacaaat 7620
   ttaattgaat cttttgttta gcttgcctct aagaactttt cttaataaag ctcccaaaac 7680
   ttctcagcaa ataaatctcc cttaagtagg aaagctagat ttcatatttg cttactttga 7740
   attaacagca actttccaca ggtaaatctg ttcttgcaaa gatgtgagca gaatagttaa 7800
   aaataatatt tttatgtttc atggttctaa atggaagcca taaatgcagt aaatactatc 7860
   tgttgtttaa ctactttaat cgtcattttt tacattttca agtttattag gttaagaaaa 7920
   acagggcagc cttggaaggc agctactaca gaaaactgca gttttgcgtt aaagataaag 7980
   tagtattttc agctccctga aaaaccattc ctgctgaaac tgctgtagaa attgtgaagc 8040
   tgcatgagtg gagagtattg aatctgtggt tatagtagtt ttctcaggtt tgtttatctt 8100
   gatgtttgat gcactgtgtt ttatagttat taaaattgag taatattatt tctatgcagt 8160
   gttatgtgtc attggccttt tgtgaatgtg catgttttaa actgcaaatt ttaaacattt 8220
   tgtcctctaa ttgttattaa aaatgaaata aactttacca ttacttaaaa 8270

<210> 4
<211> 417
<212> DNA
<213> Homo sapiens

<400> 4

atggcaaagc aaccttctga tgtaagttct gagtgtgacc gagaaggtag acaattgcag 60

cctgcggaga ggcctcccca gctcagacct ggggccccta cctccctaca gacagagcca 120

caagacagga gcccagcacc catgagttgt gacaaatcaa cacaaacccc aagtcctcct 180

tgccaggcct tcaaccacta tctcagtgca atggcttcca tgaggcaggc tgaacctgca 240

gatatgcgcc cagagatatg gatcgcccaa gagttgcggc gtatcggaga cgagtttaac 300

gcttactatg caaggagggt atttttgaat aattaccaag cagccgaaga ccacccacga 360

atggttatct tacgactgtt acgttacatt gtccgcctgg tgtggagaat gcattga 417

<210> 5
<211> 3575
<212> DNA
<213> Homo sapiens

<400> 5
accatcgtct tgggcccggg gagggagagc caccttcagg cccctcgagc ctcgaaccgg 60

aacctccaaa tccgagacgc tctgcttatg aggacctcga aatatgccgg ccagtgaaaa 120

aatcttgtgg ctttgagggc ttttggttgg ccaggggcag taaaaatctc ggagagctga 180

caccaagtcc tcccctgcca cgtagcagtg gtaaagtccg aagctcaaat tccgagaatt 240

gagctctgtt gattcttaga actggggttc ttagaagtgg tgatgcaaga agtttctagg 300

aaaggccgga caccaggttt tgagcaaaat tttggactgt gaagcaaggc attggtgaag 360

acaaaatggc ctcgccggct gacagctgta tccagttcac ccgccatgcc agtgatgttc 420

ttctcaacct taatcgtctc cggagtcgag acatcttgac tgatgttgtc attgttgtga 480

gccgtgagca gtttagagcc cataaaacgg tcctcatggc ctgcagtggc ctgttctata 540

gcatctttac agaccagttg aaatgcaacc ttagtgtgat caatctagat cctgagatca 600

accctgaggg attctgcatc ctcctggact tcatgtacac atctcggctc aatttgcggg 660

agggcaacat catggctgtg atggccacgg ctatgtacct gcagatggag catgttgtgg 720

acacttgccg gaagtttatt aaggccagtg aagcagagat ggtttctgcc atcaagcctc 780

ctcgtgaaga gttcctcaac agccggatgc tgatgcccca agacatcatg gcctatcggg 840

gtcgtgaggt ggtggagaac aacctgccac tgaggagcgc ccctgggtgt gagagcagag 900

cctttgcccc cagcctgtac agtggcctgt ccacaccgcc agcctcttat tccatgtaca 960

gccacctccc tgtcagcagc ctcctcttct ccgatgagga gtttcgggat gtccggatgc 1020

ctgtggccaa ccccttcccc aaggagcggg cactcccatg tgatagtgcc aggccagtcc 1080

ctggtgagta cagccggccg actttggagg tgtcccccaa tgtgtgccac agcaatatct 1140

attcacccaa ggaaacaatc ccagaagagg cacgaagtga tatgcactac agtgtggctg 1200

agggcctcaa acctgctgcc ccctcagccc gaaatgcccc ctacttccct tgtgacaagg 1260

ccagcaaaga agaagagaga ccctcctcgg aagatgagat tgccctgcat ttcgagcccc 1320

ccaatgcacc cctgaaccgg aagggtctgg ttagtccaca gagcccccag aaatctgact 1380

gccagcccaa ctcgcccaca gagtcctgca gcagtaagaa tgcctgcatc ctccaggctt 1440

ctggctcccc tccagccaag agccccactg accccaaagc ctgcaactgg aagaaataca 1500

agttcatcgt gctcaacagc ctcaaccaga atgccaaacc agaggggcct gagcaggctg 1560

agctgggccg cctttcccca cgagcctaca cggccccacc tgcctgccag ccacccatgg 1620

agcctgagaa ccttgacctc cagtccccaa ccaagctgag tgccagcggg gaggactcca 1680

ccatcccaca agccagccgg ctcaataaca tcgttaacag gtccatgacg ggctctcccc 1740

gcagcagcag cgagagccac tcaccactct acatgcaccc cccgaagtgc acgtcctgcg 1800

gctctcagtc cccacagcat gcagagatgt gcctccacac cgctggcccc acgttccctg 1860

aggagatggg agagacccag tctgagtact cagattctag ctgtgagaac ggggccttct 1920

tctgcaatga gtgtgactgc cgcttctctg aggaggcctc actcaagagg cacacgctgc 1980

agacccacag tgacaaaccc tacaagtgtg accgctgcca ggcctccttc cgctacaagg 2040

gcaacctcgc cagccacaag accgtccata ccggtgagaa accctatcgt tgcaacatct 2100

gtggggccca gttcaaccgg ccagccaacc tgaaaaccca cactcgaatt cactctggag 2160

agaagcccta caaatgcgaa acctgcggag ccagatttgt acaggtggcc cacctccgtg 2220

cccatgtgct tatccacact ggtgagaagc cctatccctg tgaaatctgt ggcacccgtt 2280

tccggcacct tcagactctg aagagccacc tgcgaatcca cacaggagag aaaccttacc 2340

attgtgagaa gtgtaacctg catttccgtc acaaaagcca gctgcgactt cacttgcgcc 2400

agaagcatgg cgccatcacc aacaccaagg tgcaataccg cgtgtcagcc actgacctgc 2460

ctccggagct ccccaaagcc tgctgaagca tggagtgttg atgctttcgt ctccagcccc 2520

ttctcagaat ctacccaaag gatactgtaa cactttacaa tgttcatccc atgatgtagt 2580

gcctctttca tccactagtg caaatcatag ctgggggttg ggggtggtgg gggtcggggc 2640

ctgggggact gggagccgca gcagctcccc ctcccccact gccataaaac attaagaaaa 2700

tcatattgct tcttctccta tgtgtaaggt gaaccatgtc agcaaaaagc aaaatcattt 2760

tatatgtcaa agcaggggag tatgcaaaag ttctgacttg actttagtct gcaaaatgag 2820

gaatgtatat gttttgtggg aacagatgtt tcttttgtat gtaaatgtgc attcttttaa 2880

aagacaagac ttcagtatgt tgtcaaagag agggctttaa tttttttaac caaaggtgaa 2940

ggaatatatg gcagagttgt aaatatataa atatatatat atataaaata aatatatata 3000

aacctaaaaa agatatatta aaaatataaa actgcgttaa aggctcgatt ttgtatctgc 3060

aggcagacac ggatctgaga atctttattg agaaagagca cttaagagaa tattttaagt 3120

attgcatctg tataagtaag aaaatatttt gtctaaaatg cctcagtgta tttgtatttt 3180

tttgcaagtg aaggtttaca atttacaaag tgtgtattaa aaaaaacaaa aagaacaaaa 3240

aaatctgcag aaggaaaaat gtgtaatttt gttctagttt tcagtttgta tatacccgta 3300

caacgtgtcc tcacggtgcc ttttttcacg gaagttttca atgatgggcg agcgtgcacc 3360

atcccttttt gaagtgtagg cagacacagg gacttgaagt tgttactaac taaactctct 3420

ttgggaatgt ttgtctcatc ccattctgcg tcatgcttgt gttataacta ctccggagac 3480

agggtttggc tgtgtctaaa ctgcattacc gcgttgtaaa atatagctgt acaaatataa 3540

gaataaaatg ttgaaaagtc aaactggaaa aaaaa 3575

<210> 6
<211> 2655
<212> DNA
<213> Homo sapiens

<400> 6
cccagaaggc cgcggggggt ggaccgccta agagggcgtg cgctcccgac atgccccgcg 60

gcgcgccatt aaccgccaga tttgaatcgc gggacccgtt ggcagaggtg gcggcggcgg 120

catgggtgcc ccgacgttgc cccctgcctg gcagcccttt ctcaaggacc accgcatctc 180

tacattcaag aactggccct tcttggaggg ctgcgcctgc accccggagc ggatggccga 240

ggctggcttc atccactgcc ccactgagaa cgagccagac ttggcccagt gtttcttctg 300

cttcaaggag ctggaaggct gggagccaga tgacgacccc atagaggaac ataaaaagca 360

ttcgtccggt tgcgctttcc tttctgtcaa gaagcagttt gaagaattaa cccttggtga 420

atttttgaaa ctggacagag aaagagccaa gaacaaaatt gcaaaggaaa ccaacaataa 480

gaagaaagaa tttgaggaaa ctgcggagaa agtgcgccgt gccatcgagc agctggctgc 540

catggattga ggcctctggc cggagctgcc tggtcccaga gtggctgcac cacttccagg 600

gtttattccc tggtgccacc agccttcctg tgggcccctt agcaatgtct taggaaagga 660

gatcaacatt ttcaaattag atgtttcaac tgtgctcttg ttttgtcttg aaagtggcac 720

cagaggtgct tctgcctgtg cagcgggtgc tgctggtaac agtggctgct tctctctctc 780

tctctctttt ttgggggctc atttttgctg ttttgattcc cgggcttacc aggtgagaag 840

tgagggagga agaaggcagt gtcccttttg ctagagctga cagctttgtt cgcgtgggca 900

gagccttcca cagtgaatgt gtctggacct catgttgttg aggctgtcac agtcctgagt 960

gtggacttgg caggtgcctg ttgaatctga gctgcaggtt ccttatctgt cacacctgtg 1020

cctcctcaga ggacagtttt tttgttgttg tgtttttttg tttttttttt tttggtagat 1080

gcatgacttg tgtgtgatga gagaatggag acagagtccc tggctcctct actgtttaac 1140

aacatggctt tcttattttg tttgaattgt taattcacag aatagcacaa actacaatta 1200

aaactaagca caaagccatt ctaagtcatt ggggaaacgg ggtgaacttc aggtggatga 1260

ggagacagaa tagagtgata ggaagcgtct ggcagatact ccttttgcca ctgctgtgtg 1320

attagacagg cccagtgagc cgcggggcac atgctggccg ctcctccctc agaaaaaggc 1380

agtggcctaa atccttttta aatgacttgg ctcgatgctg tgggggactg gctgggctgc 1440

tgcaggccgt gtgtctgtca gcccaacctt cacatctgtc acgttctcca cacgggggag 1500

agacgcagtc cgcccaggtc cccgctttct ttggaggcag cagctcccgc agggctgaag 1560

tctggcgtaa gatgatggat ttgattcgcc ctcctccctg tcatagagct gcagggtgga 1620

ttgttacagc ttcgctggaa acctctggag gtcatctcgg ctgttcctga gaaataaaaa 1680

gcctgtcatt tcaaacactg ctgtggaccc tactgggttt ttaaaatatt gtcagttttt 1740

catcgtcgtc cctagcctgc caacagccat ctgcccagac agccgcagtg aggatgagcg 1800

tcctggcaga gacgcagttg tctctgggcg cttgccagag ccacgaaccc cagacctgtt 1860

tgtatcatcc gggctccttc cgggcagaaa caactgaaaa tgcacttcag acccacttat 1920

ttctgccaca tctgagtcgg cctgagatag acttttccct ctaaactggg agaatatcac 1980

agtggttttt gttagcagaa aatgcactcc agcctctgta ctcatctaag ctgcttattt 2040

ttgatatttg tgtcagtctg taaatggata cttcacttta ataactgttg cttagtaatt 2100

ggctttgtag agaagctgga aaaaaatggt tttgtcttca actcctttgc atgccaggcg 2160

gtgatgtgga tctcggcttc tgtgagcctg tgctgtgggc agggctgagc tggagccgcc 2220

cctctcagcc cgcctgccac ggcctttcct taaaggccat ccttaaaacc agaccctcat 2280

ggctaccagc acctgaaagc ttcctcgaca tctgttaata aagccgtagg cccttgtcta 2340

agtgcaaccg cctagacttt ctttcagata catgtccaca tgtccatttt tcaggttctc 2400

taagttggag tggagtctgg gaagggttgt gaatgaggct tctgggctat gggtgaggtt 2460

ccaatggcag gttagagccc ctcgggccaa ctgccatcct ggaaagtaga gacagcagtg 2520

cccgctgccc agaagagacc agcaagccaa actggagccc ccattgcagg ctgtcgccat 2580

gtggaaagag taactcacaa ttgccaataa agtctcatgt ggttttatct aaaaaaaaaa 2640

aaaaaaaaaa aaaaa 2655

<210> 7
<211> 789
<212> DNA
<213> Homo sapiens

<400> 7
gccaccgccc gcagctgaag cacatccgca gcccggcgcg actccgatcg ccgcagttgc 60

cctctggcgc catgtccgag aacggagcgc ccgggatgca ggaggagagc ctgcagggct 120

cctgggtaga actgcacttc agcaataatg ggaacggggg cagcgttcca gcctcggttt 180

ctatttataa tggagacatg gaaaaaatac tgctggacgc acagcatgag tctggacgga 240

gtagctccaa gagctctcac tgtgacagcc cacctcgctc gcagacacca caagatacca 300

acagggcttc tgaaacagat acccatagca ttggagagaa aaacagctca cagtctgagg 360

aagatgatat tgaaagaagg aaagaagttg aaagcatctt gaagaaaaac tcagattgga 420

tatgggattg gtcaagtcgg ccggaaaata ttccccccaa ggagttcctc tttaaacacc 480

cgaagcgcac ggccaccctc agcatgagga acacgagcgt catgaagaaa gggggcatat 540

tctctgcaga atttctgaaa gttttccttc catctctgct gctctctcat ttgctggcca 600

tcggattggg gatctatatt ggaaggcgtc tgacaacctc caccagcacc ttttgatgaa 660

gaactggagt ctgacttggt tcgttagtgg attacttctg agcttgcaac atagctcact 720

gaagagctgt tagatcctgg gccttcgtgg ctcgagagac tagaatcgca gatacgaaaa 780

ccccgcagc 789

<210> 8
<211> 2251
<212> DNA
<213> Homo sapiens

<400> 8
ctgctgaagc gggaaggagg agctagggct gggggcggag ctttcacacg cgcaccctct 60

gttccctccc tccctccctc gacacaagca actgggtctc cagccgccac tccgggttta 120

tttgtttaca agcggattac gtcagctcct ccctctcttc cctatctctg gacccgcctc 180

ctgaactctt ttcccgcccc tttcggctcc gaaccggctt gcgtcacaat ggtgcgatat 240

tcggattggc tggagtcggc catcacgctc cagctacgcc acttcctttt cgtggcacta 300

taaagggtgc tgcacggcgc ttgcatctct tcgcctctcg gagctggaaa tgcagctatt 360

gagatcttcg aatgctgcgg agctggaggc ggaggcagct ggggaggtcc gagcgatgtg 420

accaggccgc catcgctcgt ctcttcctct ctcctgccgc ctcctgtctc gaaaataact 480

tttttagtct aaagaaagaa agacaaaagt agtcgtccgc ccctcacgcc ctctcttcct 540

ctcagccttc cgcccggtga ggaagcccgg ggtggctgct ccgccgtcgg ggccgcgccg 600

ccgagcccca gccgccccgg gccgcccccg cacgccgccc ccatgcatcc cttctacacc 660

cgggccgcca ccatgatagg cgagatcgcc gccgccgtgt ccttcatctc caagtttctc 720

cgcaccaagg ggctcacgag cgagcgacag ctgcagacct tcagccagag cctgcaggag 780

ctgctggcag gtgagcaggg gagtcctaag cgttgtttct ctgttcttct tttcttctat 840

agaacattat aaacatcact ggttcccaga aaagccatgc aagggatcgg gttaccgttg 900

tattcgcatc aaccataaaa tggatcctct gattggacag gcagcacagc ggattggact 960

gagcagtcag gagctgttca ggcttctccc aagtgaactc acactctggg ttgaccccta 1020

tgaagtgtcc tacagaattg gagaggatgg ctccatctgt gtgctgtatg aagcctcacc 1080

agcaggaggt agcactcaaa acagcaccaa cgtgcaaatg gtagacagcc gaatcagctg 1140

taaggaggaa cttctcttgg gcagaacgag cccttccaaa aactacaata tgatgactgt 1200

atcaggttaa gatatagtct gtggatggat catctgatga tgatggataa atttgatttt 1260

tgctttgggt gggctcctct tggggatgga ttatggaatt taaaccatgt cacagctgtg 1320

aagatctggc acaagataga atggtaaaaa aaaaaaaaaa ttttaagtga cagtgccata 1380

gtttggacag tacctttcaa tgattaattt taatagcctg tgagtccaag taaatgatca 1440

ctttatttgc tagggaggga agtcctaggg tggtttcagt ttctcccaga catacctaaa 1500

tttttacatc aatcctttta aagaaaatct gtatttcaaa gaatctttct ctgcagtaaa 1560

tctcgcaggg gaatttgcac tattacactt gaaagttgtt attgttaacc ttttcggcag 1620

cttttaatag gaaagttaaa cgttttaaac atggtagtac tggaaatttt acaagacttt 1680

tacctagcac ttaaatatgt ataaatgtac ataaagacaa actagtaagc atgacctggg 1740

gaaatggtca gaccttgtat tgtgtttttg gccttgaaag tagcaagtga ccagaatctg 1800

ccatggcaac aggctttaaa aaagaccctt aaaaagacac tgtctcaact gtggtgttag 1860

caccagccag ctctctgtac atttgctagc ttgtagtttt ctaagactga gtaaacttct 1920

tatttttaga aagtggaggt ctggtttgta actttccttg tacttaattg ggtaaaagtc 1980

ttttccacaa accaccatct attttgtgaa ctttgttagt catcttttat ttggtaaatt 2040

atgaactggt gtaaatttgt acagttcatg tatattgatt gtggcaaagt tgtacagatt 2100

tctatatttt ggatgagaaa tttttcttct ctctataata aatcgtttct tatcttggca 2160

ttttaatcaa tctctgtcat gatagaggtt gctaaagtat tttctagaga acggttctat 2220

aaactgaata tctgttgcac actggtcatg c 2251

<210> 9
<211> 2060
<212> DNA
<213> Homo sapiens

<400> 9
atttttggcc ctcgtgacag tgattgatag ctgctgggaa ggtataaaag cagcttgcct 60

gcgaaggttc ttcacactgc tcagggaaga gcctgctacg gtggactgtg agactcagtg 120

cactgtcctc ctcccagcga ccccacgctg gaccccctgc cggaccctcc acccttcggc 180

ccccaagctt cccaggggct tcctttggac tggactgtcc ctgctcatcc attctcctgc 240

cacccccaga cctcctcagc tccaggttgc cacctcctct cgccagagtg atgaggtccc 300

ggcttctgct ctccgtggcc catctgccca caattcggga gaccacggag gagatgctgc 360

ttgggggtcc tggacaggag cccccaccct ctcctagcct ggatgactac gtgaggtcta 420

tatctcgact ggcacagccc acctctgtgc tggacaaggc cacggcccag ggccaaccca 480

ggccacccca caggccagcc caggcctgcc ggaagggccg ccctgctgtg tccctgcgag 540

acatcaccgc acgtttcagt ggccagcagc ccacactgcc catggctgat actgtggacc 600

ccctggactg gctttttggg gagtcccagg aaaagcagcc aagccagagg gacctgccaa 660

ggaggactgg cccctctgct ggcctctggg gtccacatag acagatggac agcagcaagc 720

ccatgggggc ccccagaggg aggctctgtg aagccaggat gcctgggcat tccctggcaa 780

gaccaccgca ggatgggcag cagagctctg acctaagaag ctggactttt gggcagtctg 840

cccaagccat ggcctcccgc caccgccccc gccccagcag tgtcctcaga acactctact 900

cgcacctccc ggtgatccat gaactctgac ccctccccag taaaggcttc tgtagagagc 960

atgctgggtc tgcatctcct ctcgtctcct ccatggtggt cactgcccct ggcaggtctc 1020

tgaaagggaa atgcttttct gcagaggccc ctgcttgggc agttcacagt gagaccgacc 1080

ccctctgaat atgataacag cctgtttcac atgaggagat gttaccaatc ccgttcgctc 1140

tgacccttgc tggctgatca ccttgagcaa cttacttaac atctgtgttc ctcagtttct 1200

catgggtaat atagggataa ttactggcac ctgcctccca ggccattctg acgtgtaacc 1260

gcatatagga gcccactggc tgagtagcta ccatcatcgc tggtggggaa actggtggta 1320

ggggtgtgag ggtagtgggg gtgtcagccc cccaggtgtt tcagaacaag gcctcgggca 1380

ctcccaagtc tgcctcttgg ctcccaccct caaagcccat gttctgtgag gcccaagaga 1440

acacatggag tcttagcaaa tgcactaatg tattccgggg gactgtcacc tggcaccact 1500

ggggcactct gctggctaca actcatacgt cctgtggtgg cattgggaga gttcccccat 1560

gatgagggcc aagatagaat ctgtaccact cagtgctacc atccccaccc ctacaccact 1620

tccacacagg ggcctcatgg catggtcagg gtcccagctg tgggtgagag cagggcactg 1680

tccagctgtc cactggggaa gtcaagatgt cctaaggccc aggtcagggc atctggagtc 1740

tgaaggaccc tagttcctag aggcatctgg cagcaagaag gtgaggcatc agggaacggg 1800

aatcaggctg ggactgatca gaggtgaagg gacagagaga ggagaggagg aagattgagc 1860

tgggggcaac agccaagctc acctgggcag gtctctgcca cctccttgct ctgtgagctg 1920

tcagtctagg ttattctctt tttttgtggc tatttttaat tgctttggat ttgttaaatg 1980

ttttctgtct tctgttaagt gtgttttctc tggagataga atgtaaacca tattaaaagg 2040

aaaaagtttc agacaagcaa 2060

<210> 10
<211> 2300
<212> DNA
<213> Homo sapiens

<400> 10
actcggggca acaggcagat ttgcctgctg agggtggaga cccacgagcc gaggcctcct 60

gcagtgttct gcacagcaaa ccgcacgcta tggctgacag ccgggatccc gccagcgacc 120

agatgcagca ctggaaggag cagcgggccg cgcagaaagc tgatgtcctg accactggag 180

ctggtaaccc agtaggagac aaacttaatg ttattacagt agggccccgt gggccccttc 240

ttgttcagga tgtggttttc actgatgaaa tggctcattt tgaccgagag agaattcctg 300

agagagttgt gcatgctaaa ggagcagggg cctttggcta ctttgaggtc acacatgaca 360

ttaccaaata ctccaaggca aaggtatttg agcatattgg aaagaagact cccatcgcag 420

ttcggttctc cactgttgct ggagaatcgg gttcagctga cacagttcgg gaccctcgtg 480

ggtttgcagt gaaattttac acagaagatg gtaactggga tctcgttgga aataacaccc 540

ccattttctt catcagggat cccatattgt ttccatcttt tatccacagc caaaagagaa 600

atcctcagac acatctgaag gatccggaca tggtctggga cttctggagc ctacgtcctg 660

agtctctgca tcaggtttct ttcttgttca gtgatcgggg gattccagat ggacatcgcc 720

acatgaatgg atatggatca catactttca agctggttaa tgcaaatggg gaggcagttt 780

attgcaaatt ccattataag actgaccagg gcatcaaaaa cctttctgtt gaagatgcgg 840

cgagactttc ccaggaagat cctgactatg gcatccggga tctttttaac gccattgcca 900

caggaaagta cccctcctgg actttttaca tccaggtcat gacatttaat caggcagaaa 960

cttttccatt taatccattc gatctcacca aggtttggcc tcacaaggac taccctctca 1020

tcccagttgg taaactggtc ttaaaccgga atccagttaa ttactttgct gaggttgaac 1080

agatagcctt cgacccaagc aacatgccac ctggcattga ggccagtcct gacaaaatgc 1140

ttcagggccg cctttttgcc tatcctgaca ctcaccgcca tcgcctggga cccaattatc 1200

ttcatatacc tgtgaactgt ccctaccgtg ctcgagtggc caactaccag cgtgacggcc 1260

cgatgtgcat gcaggacaat cagggtggtg ctccaaatta ctaccccaac agctttggtg 1320

ctccggaaca acagccttct gccctggagc acagcatcca atattctgga gaagtgcgga 1380

gattcaacac tgccaatgat gataacgtta ctcaggtgcg ggcattctat gtgaacgtgc 1440

tgaatgagga acagaggaaa cgtctgtgtg agaacattgc cggccacctg aaggatgcac 1500

aaattttcat ccagaagaaa gcggtcaaga acttcactga ggtccaccct gactacggga 1560

gccacatcca ggctcttctg gacaagtaca atgctgagaa gcctaagaat gcgattcaca 1620

cctttgtgca gtccggatct cacttggcgg caagggagaa ggcaaatctg tgaggccggg 1680

gccctgcacc tgtgcagcga agcttagcgt tcatccgtgt aacccgctca tcactggatg 1740

aagattctcc tgtgctagat gtgcaaatgc aagctagtgg cttcaaaata gagaatccca 1800

ctttctatag cagattgtgt aacaatttta atgctatttc cccaggggaa aatgaaggtt 1860

aggatttaac agtcatttaa aaaaaaaatt tgttttgacg gatgattgga ttattcattt 1920

aaaatgatta gaaggcaagt ttctagctag aaatatgatt ttatttgaca aaatttgttg 1980

aaattatgta tgtttacata tcacctcatg gcctattata ttaaaatatg gctataaata 2040

tataaaaaga aaagataaag atgatctact cagaaatttt tatttttcta aggttctcat 2100

aggaaaagta catttaatac agcagtgtca tcagaagata acttgagcac cgtcatggct 2160

taatgtttat tcctgataat aattgatcaa attcattttt ttcactggag ttacattaat 2220

gttaattcag cactgatttc acaacagatc aatttgtaat tgcttacatt tttacaataa 2280

ataatctgta cgtaagaaca 2300

<210> 11
<211> 2723
<212> DNA
<213> Homo sapiens

<400> 11
gggagaaacg ttctcactcg ctctctgctc gctgcgggcg ctccccgccc tctgctgcca 60

gaaccttggg gatgtgccta gacccggcgc agcacacgtc cgggccaacc gcgagcagaa 120

caaacctttg gcgggcggcc aggaggctcc ctcccagcca ccgcccccct ccagcgcctt 180

tttttccccc catacaatac aagatcttcc ttcctcagtt cccttaaagc acagcccagg 240

gaaacctcct cacagttttc atccagccac gggccagcat gtctgggggc aaatacgtag 300

actcggaggg acatctctac accgttccca tccgggaaca gggcaacatc tacaagccca 360

acaacaaggc catggcagac gagctgagcg agaagcaagt gtacgacgcg cacaccaagg 420

agatcgacct ggtcaaccgc gaccctaaac acctcaacga tgacgtggtc aagattgact 480

ttgaagatgt gattgcagaa ccagaaggga cacacagttt tgacggcatt tggaaggcca 540

gcttcaccac cttcactgtg acgaaatact ggttttaccg cttgctgtct gccctctttg 600

gcatcccgat ggcactcatc tggggcattt acttcgccat tctctctttc ctgcacatct 660

gggcagttgt accatgcatt aagagcttcc tgattgagat tcagtgcatc agccgtgtct 720

attccatcta cgtccacacc gtctgtgacc cactctttga agctgttggg aaaatattca 780

gcaatgtccg catcaacttg cagaaagaaa tataaatgac atttcaagga tagaagtata 840

cctgattttt tttcctttta attttcctgg tgccaatttc aagttccaag ttgctaatac 900

agcaacaatt tatgaattga attatcttgg ttgaaaataa aaagatcact ttctcagttt 960

tcataagtat tatgtctctt ctgagctatt tcatctattt ttggcagtct gaatttttaa 1020

aacccattta aatttttttc cttacctttt tatttgcatg tggatcaacc atcgctttat 1080

tggctgagat atgaacatat tgttgaaagg taatttgaga gaaatatgaa gaactgagga 1140

ggaaaaaaaa aaaaaagaaa agaaccaaca acctcaactg cctactccaa aatgttggtc 1200

attttatgtt aagggaagaa ttccagggta tggccatgga gtgtacaagt atgtgggcag 1260

attttcagca aactcttttc ccactgttta aggagttagt ggattactgc cattcacttc 1320

ataatccagt aggatccagt gatccttaca agttagaaaa cataatcttc tgccttctca 1380

tgatccaact aatgccttac tcttcttgaa attttaacct atgatatttt ctgtgcctga 1440

atatttgtta tgtagataac aagacctcag tgccttcctg tttttcacat tttccttttc 1500

aaatagggtc taactcagca actcgcttta ggtcagcagc ctccctgaag accaaaatta 1560

gaatatccat gacctagttt tccatgcgtg tttctgactc tgagctacag agtctggtga 1620

agctcacttc tgggcttcat ctggcaacat ctttatccgt agtgggtatg gttgacacta 1680

gcccaatgaa atgaattaaa gtggaccaat agggctgagc tctctgtggg ctggcagtcc 1740

tggaagccag ctttccctgc ctctcatcaa ctgaatgagg tcagcatgtc tattcagctt 1800

cgtttatttt caagaataat cacgctttcc tgaatccaaa ctaatccatc accggggtgg 1860

tttagtggct caacattgtg ttcccatttc agctgatcag tgggcctcca aggaggggct 1920

gtaaaatgga ggccattgtg tgagcctatc agagttgctg caaacctgac ccctgctcag 1980

taaagcactt gcaaccgtct gttatgctgt gacacatggc ccctccccct gccaggagct 2040

ttggacctaa tccaagcatc cctttgccca gaaagaagat gggggaggag gcagtaataa 2100

aaagattgaa gtattttgct ggaataagtt caaattcttc tgaactcaaa ctgaggaatt 2160

tcacctgtaa acctgagtcg tacagaaagc tgcctggtat atccaaaagc tttttattcc 2220

tcctgctcat attgtgattc tgcctttggg gacttttctt aaaccttcag ttatgatttt 2280

tttttcatac acttattgga actctgcttg atttttgcct cttccagtct tcctgacact 2340

ttaattacca acctgttacc tactttgact ttttgcattt aaaacagaca ctggcatgga 2400

tatagtttta cttttaaact gtgtacataa ctgaaaatgt gctatactgc atacttttta 2460

aatgtaaaga tatttttatc tttatatgaa gaaaatcact taggaaatgg ctttgtgatt 2520

caatctgtaa actgtgtatt ccaagacatg tctgttctac atagatgctt agtccctcat 2580

gcaaatcaat tactggtcca aaagattgct gaaattttat atgcttactg atatatttta 2640

caatttttta tcatgcatgt cctgtaaagg ttacaagcct gcacaataaa aatgtttaac 2700

ggttaaacag tcaaaaaaaa aaa 2723

<210> 12
<211> 3976
<212> DNA
<213> Homo sapiens

<400> 12
ctgggtcctg tgtgtgccac aggggtgggg tgtccagcga gcggtctcct cctcctgcta 60

gtgctgctgc ggcgtcccgc ggcctccccg agtcgggcgg gaggggagag cgggtgtgga 120

tttgtcttga cggtaattgt tgcgtttcca cgtctcggag gcctgcgcgc tgggttgctc 180

cttcttcggg agcgagctgt tctcagcgat cccactccca gccggggctc cccacacaca 240

ctgggctgcg tgcgtgtgga gtgggacccg cgcacacgcg tgtctctgga cagctacggc 300

gccgaaagaa ctaaaattcc agatggcaaa ctcaatgaat ggcagaaacc ctggtggtcg 360

aggaggaaat ccccgaaaag gtcgaatttt gggtattatt gatgctattc aggatgcagt 420

tggaccccct aagcaagctg ccgcagatcg caggaccgtg gagaagactt ggaagctcat 480

ggacaaagtg gtaagactgt gccaaaatcc caaacttcag ttgaaaaata gcccaccata 540

tatacttgat attttgcctg atacatatca gcatttacga cttatattga gtaaatatga 600

tgacaaccag aaacttgccc aactcagtga gaatgagtac tttaaaatct acattgatag 660

ccttatgaaa aagtcaaaac gggcaataag actctttaaa gaaggcaagg agagaatgta 720

tgaagaacag tcacaggaca gacgaaatct cacaaaactg tcccttatct tcagtcacat 780

gctggcagaa atcaaagcaa tctttcccaa tggtcaattc cagggagata actttcgtat 840

cacaaaagca gatgctgctg aattctggag aaagtttttt ggagacaaaa ctatcgtacc 900

atggaaagta ttcagacagt gccttcatga ggtccaccag attagctctg gcctggaagc 960

aatggctcta aaatcaacaa ttgatttaac ttgcaatgat tacatttcag tttttgaatt 1020

tgatattttt accaggctgt ttcagccttg ggctctattt ttgcggaatt ggaatttctt 1080

agctgtgaca catccaggtt acatggcatt tctcacatat gatgaagtta aagcacgact 1140

acagaaatat agcaccaaac ccggaagcta tattttccgg ttaagttgca ctcgattggg 1200

acagtgggcc attggctatg tgactgggga tgggaatatc ttacagacca tacctcataa 1260

caagccctta tttcaagccc tgattgatgg cagcagggaa ggattttatc tttatcctga 1320

tgggaggagt tataatcctg atttaactgg attatgtgaa cctacacctc atgaccatat 1380

aaaagttaca caggaacaat atgaattata ttgtgaaatg ggctccactt ttcagctctg 1440

taagatttgt gcagagaatg acaaagatgt caagattgag ccttgtgggc atttgatgtg 1500

cacctcttgc cttacggcat ggcaggagtc ggatggtcag ggctgccctt tctgtcgttg 1560

tgaaataaaa ggaactgagc ccataatcgt ggaccccttt gatccaagag atgaaggctc 1620

caggtgttgc agcatcattg acccctttgg catgccgatg ctagacttgg acgacgatga 1680

tgatcgtgag gagtccttga tgatgaatcg gttggcaaac gtccgaaagt gcactgacag 1740

gcagaactca ccagtcacat caccaggatc ctctcccctt gcccagagaa gaaagccaca 1800

gcctgaccca ctccagatcc cacatctaag cctgccaccc gtgcctcctc gcctggatct 1860

aattcagaaa ggcatagtta gatctccctg tggcagccca acgggttcac caaagtcttc 1920

tccttgcatg gtgagaaaac aagataaacc actcccagca ccacctcctc ccttaagaga 1980

tcctcctcca ccgccacctg aaagacctcc accaatccca ccagacaata gactgagtag 2040

acacatccat catgtggaaa gcgtgccttc cagagacccg ccaatgcctc ttgaagcatg 2100

gtgccctcgg gatgtgtttg ggactaatca gcttgtggga tgtcgactcc taggggaggg 2160

ctctccaaaa cctggaatca cagcgagttc aaatgtcaat ggaaggcaca gtagagtggg 2220

ctctgaccca gtgcttatgc ggaaacacag acgccatgat ttgcctttag aaggagctaa 2280

ggtcttttcc aatggtcacc ttggaagtga agaatatgat gttcctcccc ggctttctcc 2340

tcctcctcca gttaccaccc tcctccctag cataaagtgt actggtccgt tagcaaattc 2400

tctttcagag aaaacaagag acccagtaga ggaagatgat gatgaataca agattccttc 2460

atcccaccct gtttccctga attcacaacc atctcattgt cataatgtaa aacctcctgt 2520

tcggtcttgt gataatggtc actgtatgct gaatggaaca catggtccat cttcagagaa 2580

gaaatcaaac atccctgact taagcatata tttaaaggga gatgtttttg attcagcctc 2640

tgatcccgtg ccattaccac ctgccaggcc tccaactcgg gacaatccaa agcatggttc 2700

ttcactcaac aggacgccct ctgattatga tcttctcatc cctccattag gtgaagatgc 2760

ttttgatgcc ctccctccat ctctcccacc tcccccacct cctgcaaggc atagtctcat 2820

tgaacattca aaacctcctg gctccagtag ccggccatcc tcaggacagg atctttttct 2880

tcttccttca gatccctttg ttgatctagc aagtggccaa gttcctttgc ctcctgctag 2940

aaggttacca ggtgaaaatg tcaaaactaa cagaacatca caggactatg atcagcttcc 3000

ttcatgttca gatggttcac aggcaccagc cagaccccct aaaccacgac cgcgcaggac 3060

tgcaccagaa attcaccaca gaaaacccca tgggcctgag gcggcattgg aaaatgtcga 3120

tgcaaaaatt gcaaaactca tgggagaggg ttatgccttt gaagaggtga agagagcctt 3180

agagatagcc cagaataatg tcgaagttgc ccggagcatc ctccgagaat ttgccttccc 3240

tcctccagta tccccacgtc taaatctata gcagccagaa ctgtagacac caaaatggaa 3300

agcaatcgat gtattccaag agtgtggaaa taaagagaac tgagatggaa ttcaagagag 3360

aagtgtctcc tcctcgtgta gcagcttgag aagaggcttg ggagtgcagc ttctcaaagg 3420

agaccgatgc ttgctcagga tgtcgacagc tgtggcttcc ttgtttttgc tagccatatt 3480

tttaaatcag ggttgaactg acaaaaataa tttaaagacg tttacttccc ttgaactttg 3540

aacctgtgaa atgctttacc ttgtttacag tttggcaaag ttgcagtttg ttcttgtttt 3600

tagtttagtt ttgttttggt gttttgatac ctgtactgtg ttcttcacag accctttgta 3660

gcgtggtcag gtctgctgta acatttccca ccaactctct tgctgtccac atcaacagct 3720

aaatcattta ttcatatgga tctctaccat ccccatgcct tgcccaggtc cagttccatt 3780

tctctcattc acaagatgct ttgaaggttc tgattttcaa ctgatcaaac taatgcaaaa 3840

aaaaaaaagt atgtattctt cactactgag tttcttcttt ggaaaccatc actattgaga 3900

gatgggaaaa acctgaatgt ataaagcatt tatttgtcaa taaactgcct tttgtaaggg 3960

gttttcacat aacata                                                    3976

<210>  13
<211>  4304
<212>  DNA
<213>  Homo sapiens

<400>  13
cacacggact acaggggagt tttgttgaag ttgcaaagtc ctggagcctc cagagggctg      60

tcggcgcagt agcagcgagc agcagagtcc gcacgctccg gcgaggggca gaagagcgcg     120

agggagcgcg gggcagcaga agcgagagcc gagcgcggac ccagccagga cccacagccc     180

tccccagctg cccaggaaga gccccagcca tggaacacca gctcctgtgc tgcgaagtgg     240

aaaccatccg ccgcgcgtac cccgatgcca acctcctcaa cgaccgggtg ctgcgggcca     300

tgctgaaggc ggaggagacc tgcgcgccct cggtgtccta cttcaaatgt gtgcagaagg     360

aggtcctgcc gtccatgcgg aagatcgtcg ccacctggat gctggaggtc tgcgaggaac     420

agaagtgcga ggaggaggtc ttcccgctgg ccatgaacta cctggaccgc ttcctgtcgc     480

tggagcccgt gaaaaagagc cgcctgcagc tgctgggggc cacttgcatg ttcgtggcct     540

ctaagatgaa ggagaccatc cccctgacgg ccgagaagct gtgcatctac accgacaact     600

ccatccggcc cgaggagctg ctgcaaatgg agctgctcct ggtgaacaag ctcaagtgga     660

acctggccgc aatgaccccg cacgatttca ttgaacactt cctctccaaa atgccagagg     720

cggaggagaa caaacagatc atccgcaaac acgcgcagac cttcgttgcc ctctgtgcca     780

cagatgtgaa gttcatttcc aatccgccct ccatggtggc agcggggagc gtggtggccg     840

cagtgcaagg cctgaacctg aggagcccca acaacttcct gtcctactac cgcctcacac     900

gcttcctctc cagagtgatc aagtgtgacc cggactgcct ccgggcctgc caggagcaga     960

tcgaagccct gctggagtca agcctgcgcc aggcccagca gaacatggac cccaaggccg    1020

ccgaggagga ggaagaggag gaggaggagg tggacctggc ttgcacaccc accgacgtgc    1080

gggacgtgga catctgaggg cgccaggcag gcgggcgcca ccgccacccg cagcgagggc    1140

ggagccggcc ccaggtgctc ccctgacagt ccctcctctc cggagcattt tgataccaga    1200

agggaaagct tcattctcct tgttgttggt tgttttttcc tttgctcttt cccccttcca     1260

tctctgactt aagcaaaaga aaaagattac ccaaaaactg tctttaaaag agagagagag    1320

aaaaaaaaaa tagtatttgc ataaccctga gcggtggggg aggagggttg tgctacagat    1380

gatagaggat tttatacccc aataatcaac tcgtttttat attaatgtac ttgtttctct    1440

gttgtaagaa taggcattaa cacaaaggag gcgtctcggg agaggattag gttccatcct    1500

ttacgtgttt aaaaaaaagc ataaaaacat tttaaaaaca tagaaaaatt cagcaaacca    1560

tttttaaagt agaagagggt tttaggtaga aaaacatatt cttgtgcttt tcctgataaa    1620

gcacagctgt agtggggttc taggcatctc tgtactttgc ttgctcatat gcatgtagtc 1680

actttataag tcattgtatg ttattatatt ccgtaggtag atgtgtaacc tcttcacctt 1740

attcatggct gaagtcacct cttggttaca gtagcgtagc gtgcccgtgt gcatgtcctt 1800

tgcgcctgtg accaccaccc caacaaacca tccagtgaca aaccatccag tggaggtttg 1860

tcgggcacca gccagcgtag cagggtcggg aaaggccacc tgtcccactc ctacgatacg 1920

ctactataaa gagaagacga aatagtgaca taatatattc tatttttata ctcttcctat 1980

ttttgtagtg acctgtttat gagatgctgg ttttctaccc aacggccctg cagccagctc 2040

acgtccaggt tcaacccaca gctacttggt ttgtgttctt cttcatattc taaaaccatt 2100

ccatttccaa gcactttcag tccaataggt gtaggaaata gcgctgtttt tgttgtgtgt 2160

gcagggaggg cagttttcta atggaatggt ttgggaatat ccatgtactt gtttgcaagc 2220

aggactttga ggcaagtgtg ggccactgtg gtggcagtgg aggtggggtg tttgggaggc 2280

tgcgtgccag tcaagaagaa aaaggtttgc attctcacat tgccaggatg ataagttcct 2340

ttccttttct ttaaagaagt tgaagtttag gaatcctttg gtgccaactg gtgtttgaaa 2400

gtagggacct cagaggttta cctagagaac aggtggtttt taagggttat cttagatgtt 2460

tcacaccgga aggtttttaa acactaaaat atataattta tagttaaggc taaaaagtat 2520

atttattgca gaggatgttc ataaggccag tatgatttat aaatgcaatc tccccttgat 2580

ttaaacacac agatacacac acacacacac acacacacaa accttctgcc tttgatgtta 2640

cagatttaat acagtttatt tttaaagata gatcctttta taggtgagaa aaaaacaatc 2700

tggaagaaaa aaaccacaca aagacattga ttcagcctgt ttggcgtttc ccagagtcat 2760

ctgattggac aggcatgggt gcaaggaaaa ttagggtact caacctaagt tcggttccga 2820

tgaattctta tcccctgccc cttcctttaa aaaacttagt gacaaaatag acaatttgca 2880

catcttggct atgtaattct tgtaattttt atttaggaag tgttgaaggg aggtggcaag 2940

agtgtggagg ctgacgtgtg agggaggaca ggcgggagga ggtgtgagga ggaggctccc 3000

gaggggaagg ggcggtgccc acaccgggga caggccgcag ctccattttc ttattgcgct 3060

gctaccgttg acttccaggc acggtttgga aatattcaca tcgcttctgt gtatctcttt 3120

cacattgttt gctgctattg gaggatcagt tttttgtttt acaatgtcat atactgccat 3180

gtactagttt tagttttctc ttagaacatt gtattacaga tgcctttttt gtagtttttt 3240

ttttttttat gtgatcaatt ttgacttaat gtgattactg ctctattcca aaaaggttgc 3300

tgtttcacaa tacctcatgc ttcacttagc catggtggac ccagcgggca ggttctgcct 3360

gctttggcgg gcagacacgc gggcgcgatc ccacacaggc tggcgggggc cggccccgag 3420

gccgcgtgcg tgagaaccgc gccggtgtcc ccagagacca ggctgtgtcc ctcttctctt 3480

ccctgcgcct gtgatgctgg gcacttcatc tgatcggggg cgtagcatca tagtagtttt 3540

tacagctgtg ttattctttg cgtgtagcta tggaagttgc ataattatta ttattattat 3600

tataacaagt gtgtcttacg tgccaccacg gcgttgtacc tgtaggactc tcattcggga 3660

tgattggaat agcttctgga atttgttcaa gttttgggta tgtttaatct gttatgtact 3720

agtgttctgt ttgttattgt tttgttaatt acaccataat gctaatttaa agagactcca 3780

aatctcaatg aagccagctc acagtgctgt gtgccccggt cacctagcaa gctgccgaac 3840

caaaagaatt tgcaccccgc tgcgggccca cgtggttggg gccctgccct ggcagggtca 3900

tcctgtgctc ggaggccatc tcgggcacag gcccaccccg ccccacccct ccagaacacg 3960

gctcacgctt acctcaacca tcctggctgc ggcgtctgtc tgaaccacgc ggggggcttg 4020

agggacgctt tgtctgtcgt gatggggcaa gggcacaagt cctggatgtt gtgtgtatcg 4080

agaggccaaa ggctggtggc aagtgcacgg ggcacagcgg agtctgtcct gtgacgcgca 4140

agtctgaggg tctgggcggc gggcggctgg gtctgtgcat ttctggttgc accgcggcgc 4200

ttcccagcac caacatgtaa ccggcatgtt tccagcagaa gacaaaaaga caaacatgaa 4260

agtctagaaa taaaactggt aaaaccccaa aaaaaaaaaa aaaa 4304

<210> 14
<211> 6531
<212> DNA
<213> Homo sapiens

<400> 14
gcccagccag cttgcgtcac cgcttcagag cggagaagag cgagcagggg agagcgagac 60

cagttttaag ggaggaccgg gtgcgagtga ggcagccccg aggctctgct cgcccaccac 120

ccaatcctcg cctcccttct gctccacctt ctctctctgc cctcacctct cccccgaaaa 180

ccccctattt agccaaagga aggaggtcag gggaacgctc tcccctcccc ttccaaaaaa 240

caaaaacaga aaaacccttt tccaggccgg ggaaagcagg agggagaggg gccgccgggc 300

tggccatgga gctgctgtgc cacgaggtgg acccggtccg cagggccgtg cgggaccgca 360

acctgctccg agacgaccgc gtcctgcaga acctgctcac catcgaggag cgctaccttc 420

cgcagtgctc ctacttcaag tgcgtgcaga aggacatcca accctacatg cgcagaatgg 480

tggccacctg gatgctggag gtctgtgagg aacagaagtg cgaagaagag gtcttccctc 540

tggccatgaa ttacctggac cgtttcttgg ctggggtccc gactccgaag tcccatctgc 600

aactcctggg tgctgtctgc atgttcctgg cctccaaact caaagagacc agcccgctga 660

ccgcggagaa gctgtgcatt tacaccgaca actccatcaa gcctcaggag ctgctggagt 720

gggaactggt ggtgctgggg aagttgaagt ggaacctggc agctgtcact cctcatgact 780

tcattgagca catcttgcgc aagctgcccc agcagcggga gaagctgtct ctgatccgca 840

agcatgctca gaccttcatt gctctgtgtg ccaccgactt taagtttgcc atgtacccac 900

cgtcgatgat cgcaactgga agtgtgggag cagccatctg tgggctccag caggatgagg 960

aagtgagctc gctcacttgt gatgccctga ctgagctgct ggctaagatc accaacacag 1020

acgtggattg tctcaaagct tgccaggagc agattgaggc ggtgctcctc aatagcctgc 1080

agcagtaccg tcaggaccaa cgtgacggat ccaagtcgga ggatgaactg gaccaagcca 1140

gcacccctac agacgtgcgg gatatcgacc tgtgaggatg ccagttgggc cgaaagagag 1200

agacgcgtcc ataatctggt ctcttcttct ttctggttgt tttgttcttt tgtgttttag 1260

ggtgaaactt aaaaaaaaaa ttctgccccc acctagatca tatttaaaga tcttttagaa 1320

gtgagagaaa aaggtcctac gaaaacggaa taataaaaag catttggtgc ctatttgaag 1380

tacagcataa gggaatccct tgtatatgcg aacagttatt gtttgattat gtaaaagtaa 1440

tagtaaaatg cttacaggaa aacctgcaga gtagttagag aatatgtatg cctgcaatat 1500

gggaacaaat tagaggagac tttttttttt catgttatga gctagcacat acaccccctt 1560

gtagtataat ttcaaggaac tgtgtacgcc atttatggca tgattagatt gcaaagcaat 1620

gaactcaaga aggaattgaa ataaggaggg acatgatggg gaaggagtac aaaacaatct 1680

ctcaacatga ttgaaccatt tgggatggag aagcaccttt gctctcagcc acctgttact 1740

aagtcaggag tgtagttgga tctctacatt aatgtcctct tgctgtctac agtagctgct 1800

acctaaaaaa agatgtttta ttttgccagt tggacacagg tgattggctc ctgggtttca 1860

tgttctgtga catcctgctt cttcttccaa atgcagttca ttgcagacac caccatattg 1920

ctatctaatg gggaaatgta gctatgggcc ataaccaaaa ctcacatgaa acggaggcag 1980

atggagacca agggtgggat ccagaatgga gtcttttctg ttattgtatt taaaagggta 2040

atgtggcctt ggcatttctt cttagaaaaa aactaatttt tggtgctgat tggcatgtct 2100

ggttcacagt ttagcattgt tataaaccat tccattcgaa aagcactttg aaaaattgtt 2160

cccgagcgat agatgggatg gtttatgcaa gtcatgctga atactcctcc cctcttctct 2220

tttgccccct cccttcctgc ccccagtctg ggttactctt cgcttctggt atctggcgtt 2280

ctttggtaca cagttctggt gttcctacca ggactcaaga gacacccctt cctgctgaca 2340

ttcccatcac aacattcctc agacaagcct gtaaactaaa atctgttacc attctgatgg 2400

cacagaagga tcttaattcc catctctata cttctccttt ggacatggaa agaaaagtta 2460

ttgctggtgc aaagatagat ggctgaacat cagggtgtgg cattttgttc ccttttccgt 2520

tttttttttt ttattgttgt tgttaatttt attgcaaagt tgtattcagc gtacttgaat 2580

ttttcttcct ctccacttct tagaggcatt cagttagcaa agaggttgga gcaacaactt 2640

tttttttttt ttttgcacaa ttgtaattga caggtaatga agctatttgt taaaatattt 2700

gcctttttaa gtaaaaaaga aaaatcagaa caggctattt tgaagaatta ttttatacac 2760

agattctgcc ttgtttcata gtatgagggt tgaagacgga aaacaatcta agggtctctc 2820
atttttttaa ttttgttttg ttcagtttgg tttttttttt tttttgcgct gctaagaagc 2880
taaagtcatc catccttatt cacgttgaca gtacctagct gtaatgtttc acagagtgtg 2940
ctgctatttt ataaacattt ttataatata ttattttact gcttaaattc caagtcctga 3000
agtagatggt tgagatatga gttcttcgta ctggaaaagc ccttccgtag tttgttttct 3060
tctggtagca tattcatggt tgtttttttt tttctttttt ggttttttgg tttttttttt 3120
ttcctctgat cacattcttc aaagacggag tattctttac ctcaggttta ctggacaaaa 3180
tcaataacta caaaaggcaa tgattcacgc ttttgttttc ataatacctc acaaccgtac 3240
agtttctgct tgggagccca ttcgcatgag gaatacagaa gcagtgtgag cagggctgac 3300
tccctctcag gtggaaggca gggcggtctc actcccaggg acctttttgg tcatggaggc 3360
catcgggctc ccagttagac cctggtatcc tcatcatgat ggaaaaaata cattgaacca 3420
agggatcctc cctccccttc aaggcagacg ttcagtacaa acatttatgc ggtaggctca 3480
gatgtcgtaa tttgcactta ggtaccaggt gtcaggaaac agactaaaaa gaattccacc 3540
aggctgtttg gagatcctca tcttggagct ttttcaaaag cggggcttca tctgcaaagg 3600
gccctttcat cttgaagttt ttcccctccg tctttcccct cccctggcat ggacaccttg 3660
tgtttaggat catctctgca ggtttcctag gtctgaatct gcgagtagat gaacctgcag 3720
caagcagcgt ttatggtgct tccttctccc tcctctgtct caaactgcgc aggcaagcac 3780
tatgcaagcc caggccctct gctgagcggt actaaacggt cgggttttca atcacactga 3840
attggcagga taagaaaaat aggtcagata agtatgggat gatagttgaa gggaggtgaa 3900
gaggctgctt ctctacagag gtgaaattcc agatgagtca gtctcttggg aagtgtgttt 3960
agaagggttc aggactttgt gagttagcat gaccctaaaa ttctagggga tttctggtgg 4020
gacaatgggt ggtgaattct gaagttttgg agagggaagt ggagcagcca gcaagtaagc 4080
tagccagagt tttctcaaga gccagctttg ctcagcacac tctcctgggc cccaaggagt 4140
cccacggaat ggggaaagcg ggaaccctgg agttcttggg aatcttggag cctaaagaga 4200
aaccgaggtg caaattcatt tcatggtgac tgacccttga gcttaaacag aagcagcaaa 4260
tgaaagaacc ggacaaataa ggaagggcac aagcctaccc gactctattt acagtctgta 4320
actttccact cttcctgtag tcccgaggcc cctgggtcct tctagctttt ctctttccca 4380
tccttggggc cttgtgtgat gatgggtgtg gggctgccga tgggaaagtc gggggttgtt 4440
aggcttttct gcctgctcct gcttaaacac aagaaggaat cctggatttt gccctctcct 4500
tagctcttag tctctttggt aggagttttg ttccagagga gctctccccc ttggatttga 4560
acttgctctt tttgttgttg ttgttctttc tcttcttttt cttacctccc actaaagggg 4620
ttccaaatta tcctggtctt tttctacctt gttgtgtttc tatctcgtct ttacttccat 4680

ctgtttgttt ttttctccat cagtgggggc cgagttgttc ccccagcctg ccaaattttg 4740

atccttcccc tcttttggcc aaatcctagg gggaagaaat cctagtatgc caaaaatata 4800

tgctaagcat aattaaactc catgcgggtc cataacagcc aagaagcctg caggagaaag 4860

ccaagggcag ttccctccgc agaacacccc atgcgtgctg agaggcgagc tccttgaaga 4920

aggggctgtt cttccaggag gccttatttt gaactgcctc aggaccccac tggagagcac 4980

agcatgcctt actactgggt catccttggt ctatgtgctc tgtactggag gctctgttct 5040

gcctcttatc agccaggtca ggggcacaca tggcttaagt gacaaagcca gaggagaaga 5100

caaccctgac agcatcacgc tgcatcccat tgctagcagg attggcaact cttcagacgg 5160

agctgcgctt ccctgcagtc tagcacctct agggcctctc cagactgtgc cctgggagct 5220

ctgggactga aaggttaaga acataaggca ggatcagatg actctctcca agagggcagg 5280

ggaattttct ctccatgggc cacaggggac agggctggga gaagaaatag acttgcacct 5340

tatgtcatgt aaataattga ttttctagtt caagaagata atattggtag tgtgggaatt 5400

ggaggtagga aggggaggaa gtctgagtaa gccagttggc ttctaagcca aaaggattcc 5460

tctttgttta tctctgagac agtccaacct tgagaatagc tttaaaaggg aaattaatgc 5520

tgagatgata aagtcccctt aagccaacaa accctctgta gctatagaat gagtgcaggt 5580

ttctattggt gtggactcag agcaatttac aagagctgtt catgcagcca tccatttgtg 5640

caaaataggg taagaagatt caagaggata tttattactt cctcatacca catggctttt 5700

gatgattctg gattctaaac aacccagaat ggtcatttca ggcacaacga tactacattc 5760

gtgtgtgtct gcttttaaac ttggctgggc tatcagaccc tattctcggc tcaggttttg 5820

agaagccatc agcaaatgtg tacgtgcatg ctgtagctgc agcctgcatc ccttcgcctg 5880

cagcctactt tggggaaata aagtgcctta ctgactgtag ccattacagt atccaatgtc 5940

ttttgacagg tgcctgtcct tgaaaaacaa agtttctatt tttattttta attggtttag 6000

ttcttaactg ctggccaact cttacatccc cagcaaatca tcgggccatt ggattttttc 6060

cattatgttc atcaccctta tatcatgtac ctcagatctc tctctctctc ctctctctca 6120

gttatgtagt ttcttgtctt ggactttttt ttttcttttc tttttctttt tttttttgct 6180

ttaaaacaag tgtgatgcca tatcaagtcc atgttattct ctcacagtgt actctataag 6240

aggtgtgggt gtctgtttgg tcaggatgtt agaaagtgct gataagtagc atgatcagtg 6300

tatgcgaaaa ggtttttagg aagtatggca aaaatgttgt attggctatg atggtgacat 6360

gatatagtca gctgcctttt aagaggtctt atctgttcag tgttaagtga tttaaaaaaa 6420

taataacctg ttttctgact agtttaaaga tggatttgaa aatggttttg aatgcaatta 6480

ggttatgcta tttggacaat aaactcacct tgacctaaat taaaaaaaaa a 6531

<210>  15
<211>  5489
<212>  DNA
<213>  Homo sapiens

<400>  15
gaaactctta acaaaaacaa ggggctcggg gaggtttccg ctgaggcggc gggggtgcgg      60

cggtgggctg gtcttccgcg gccggcgttg cgccgcggcg gagggtgggc gcgcggggag     120

cgggatggag ccggggctgt gaggccgagg cggcggtgcc tgggaggaag ggtcggatgc     180

cggaccgggg gcaccgctga ggcggtgggt ccccgacctg cgagacaggt ttggaagccc     240

ccgctgcgcc cagtccgtgc ggaccgcgag gccgcgggcg ggtggaggcg cgtctccggc     300

acgatgaagg atttgggggc agagcacttg gcaggtcatg aaggggtcca acttctcggg     360

ttgttgaacg tctacctgga acaagaagag agattccaac ctcgagaaaa agggctgagt     420

ttgattgagg ctaccccgga gaatgataac actttgtgtc caggattgag aaatgccaaa     480

gttgaagatt taaggagttt agccaacttt tttggatctt gcactgaaac ttttgtcctg     540

gctgtcaata ttttggacag gttcttggct cttatgaagg tgaaacctaa acatttgtct     600

tgcattggag tctgttcttt tttgctggct gctagaatag ttgaagaaga ctgcaatatt     660

ccatccactc atgatgtgat ccggattagt cagtgtaaat gtactgcttc tgacataaaa     720

cggatggaaa aaataatttc agaaaaattg cactatgaat tggaagctac tactgcctta     780

aactttttgc acttatacca tactattata ctttgtcata cttcagaaag gaaagaaata     840

ctgagccttg ataaactaga agctcagctg aaagcttgca actgccgact catcttttca     900

aaagcaaaac catctgtatt agccttgtgc cttctcaatt tggaagtgga aactttgaaa     960

tctgttgaat tactggaaat tctcttgcta gttaaaaaac attccaagat taatgacact    1020

gagttcttct actggagaga gttggtttct aaatgcctag ccgagtattc ttctcctgaa    1080

tgttgcaaac cagatcttaa gaagttggtt tggatcgttt caaggcgcac agcccagaac    1140

ctccacaaca gctactatag tgttcctgag ctgccaacga tacctgaggg gggttgtttt    1200

gatgaaagtg aaagtgagga ctcttgtgaa gatatgagtt gtggagagga gagtctcagc    1260

agctctcctc ccagtgatca agagtgcacc ttctttttca acttcaaagt ggcacaaaca    1320

ctgtgctttc catcttagaa atctgattgt tctgtcagaa tttatattta caggtttcaa    1380

agcaataaat gggggaatag gtagtttcct ggtttagccc ccatctagtc aggaattaat    1440

atactggaat acctaccttc tatttgttat tcagatcaga tctggcctat tttcatattt    1500

atcctaagcc atcaaatggg gtagtgcctc ttaaaccatt aacagtactt tagacattgg    1560

cactttattt ttctcgtaga tctttagcta ctttggggag gagggaaggt gctgatacct    1620

tcaatttgtt acttttcaag atttttaaaa ataactagtg tagcttatct taaacatttt    1680

ataaaacctt cagatgtctt taagcagatt ggaagtatgc aagtgcttcc ttagcaggga 1740

cagtggataa tccttaatgg tttatcatag atttcaccct ccccccttct cagaagagtg 1800

agtatgctct taaatgtcaa acacattttt gttgttttgt tttttaaatg atcagtgtct 1860

atttgatgtg atgcagatct tataaatttg ggaattataa tattgacatt tctgtgattt 1920

ttatatatgt aatgtcttaa ttgagatttc tgttaaggca gaaataatta ggctagggct 1980

cttagttttc attcctattg cccaagtatt gtcaaactat ggtattattt taatgttact 2040

ttaaaaatcc ataatctgct agttttgcat gtacttatat gaaaacagtg cagtaagttg 2100

aaaactcagt atctatggaa ttgataaatg ttgatctggt gtagtatatt ttatcgcatt 2160

ttcttatatt aaaaaatgtc tgcatgatta cattttattt cctttgtaat ttacatttca 2220

gaatagtgta ttgctatatg ggtgccaaga ttgaatatga agaacccgag tgtttgtagt 2280

attatagttt taagcaaatc tgtgtggtga tacagccata agaatggggc ttatataaac 2340

tctgtacatg taagattttg tacagagaat ttttaacttt ataaattgta tatgaacatg 2400

taaatctttt aaaatgtaca taaaatactg tattttttta ccttgtgtgt gatagtctag 2460

tcattgcatg taaatataat ttattatgta ttctgtagta taaatcatac attgatgact 2520

tacattttta ctggtaagtc aacatccgtt ggatgttttc tgaagtggct ctttttgaag 2580

tgataataga ttgtaattca aaataaaatt attaatgaat tctccttgtt tgggatcaca 2640

tcttaatttt taatctgtta aaagttcttg atgtatttta atgagaagac tttaggtgag 2700

gctacagtga ttccagagtg agccttctaa ctggctagca gaagttctct aggtttggca 2760

tctgtgcctt ggagatactg aaagagaatc tgtcatttga caattgacct ctttgtggga 2820

tggactcatt aagtatgctc tcagagactg gtatattacc agaatgccta ttaattttca 2880

gtgagaggca acaggtatta agtagaacag aatgctcagg ttggcagatt agaacgatct 2940

ttcaggagac aaagcaagtt ttaatcagtt gtttggttaa taagtatggg gtgttcgctg 3000

tgatagggcc ccgccagctt ctggctcttg tggacctcaa aagtatcagg tggttttgca 3060

agtggtggtc ctttcccctg ccccacccca ataggttccc catctgtcta gtttgatttt 3120

tgtagacctt tgttttctct agttagaaaa tcaggtacac tgaatatggt tttcatgtaa 3180

cacctcttct ctggagatag gggtatgttt tcctaccctt ctagtggaga atcctacttg 3240

aggatgacct ttcctctctt actaaataat attagtaaat agtgggcaat atattctgct 3300

ttcagatttt gatttgttga gatgtaaaag ttgtttgggg cttaccaaat ctcaagactc 3360

tctttagctc ctgcaggatt gtattgcttt tcttactgga tatttttcct gggtaagcat 3420

ctttgtggct tcatctcttc cccctgtggt tttcagtgta tttagtcgag acctctctgc 3480

tgagcttgca acctgtttat tcacatggcc tgccatgcca cttggaggtt tctgattact 3540

cccaaacctg ctggttcttt atgtctttct cagcgaataa ttccatctat tcatgttgga 3600

aacttaggtg atatgctcat ctccttttgc ctgtttatgg aggtcaccag cctctatcat 3660

ttgtatgatt tcgtttacac tgtttatatc tctctgtccc ccctttttct gccattggca 3720

tggtttagac ctgtactctt tatcagcaga ggtactgtaa tatatttgtg atccctcagc 3780

ttccaggctt actcctggtc tctgccttcc tatctacata tccttttaaa ataaaatttt 3840

aactatctcc tgaaaaattg ttgagtaggt cacgcacaat caggagaaaa atctattcat 3900

gacatacaag tctctgtcta atctgaacac tgcacctgtc tctggccttt ttttcttgtc 3960

atttcctaga ccttaaaaaa tgtgtattga gaaagaactc tgttagctat acagaagatg 4020

aactgggcaa tatagagtag cagcatggag accagtctga ctgaactaag gcagtggaag 4080

tgtggatgag gaagagaggt gaaaattgag aagcgctatc ctttctcttt gggcattatt 4140

aggaggctca cagacaagtc caggagcctg gttataccct cctgtgccat tcaaccaggt 4200

ggctttccca tgactgtgat gaataaaatt gagaagcccc tgcccttttc agagcagagg 4260

gtgaggagaa agctaccatt ttgtcctcat ccttaccccc gttgacttgg cgagagattt 4320

gacctttcag gttttgatcc tgtcattttc taggatgtgg tgcacgcact ttgctgttgc 4380

gcatggtgaa gtattgtgcc taggtcctgg gtcttcatct gtttggctct gctactgttt 4440

cctcctccca ggaagtgtgg ttagacaaat aatgtgtttt aattacctgt cacactcagg 4500

attaatacat actcaggtta actgtagaga ggcattggct tcagaacact cctcgtgaca 4560

attttaacca ttttctttgt ctagagtctg cctttttctt ttttacaatt tcttttattt 4620

caacactagg tttcaatatg gtgttcctgc tacctcccac ctccctcctc cctcatcaca 4680

catgcaaatt gtcagcttat tgagacaacc cacttagatt catatatgga caaggacaag 4740

gtattttgca tttgttactg gaattcagtt ttcctaacta tttactacca gaaatggtca 4800

ataacttact ttgtgtttag caaatcaaat tgtgtgatag atagtttccc agtatgatgg 4860

ccagtcagtc tttccatccc tgtgcctaca tgctgctctt cccgtccaca agtggagtct 4920

gtttctcttg agttttggct ggccttatga atggctttgc ttactgaagt gcagcagaag 4980

aaatttagta tatgtccaag cctaggcttt aagagactgg cagctttcct tttatccttt 5040

ttggaagcta gccaccatgc tgcaaagaag ctcagctgga ttactgaaag atgagaggcc 5100

atgtggagag agactcttga ggatgagaga ttatcttgga tgttccagcc ttaagctccc 5160

agctgaatgt gggtgtatcc tcagctacac cacagaaaac agaggaacta ctcagtcgat 5220

cccaatcaac ccacagactc actagaaata acaaattatt gttttaagcc acgaggtttt 5280

gggggagggt tgttaaacag taatagataa gtgagacaga ttgcttgtta tttatggtca 5340

aatggtgatt atctctggtg agattacagg tgatgttttt tttaagttat gcctatctgt 5400

agtttccttt ttttcctaaa attgatttga attattagtg tattaacaga ataaagaatg 5460

aactttaaaa cacaaaaaaa aaaaaaaaa 5489

<210> 16
<211> 2122
<212> DNA
<213> Homo sapiens

<400> 16
ggtggctatt ttgtccttgg gctgcctgtt ttcagctgct gcaaccacag ggatttcttc 60

tgttcaggcg ccatgtcaga accggctggg gatgtccgtc agaacccatg cggcagcaag 120

gcctgccgcc gcctcttcgg cccagtggac agcgagcagc tgagccgcga ctgtgatgcg 180

ctaatggcgg gctgcatcca ggaggcccgt gagcgatgga acttcgactt tgtcaccgag 240

acaccactgg agggtgactt cgcctgggag cgtgtgcggg gccttggcct gcccaagctc 300

taccttccca cggggccccg gcgaggccgg gatgagttgg gaggaggcag gcggcctggc 360

acctcacctg ctctgctgca ggggacagca gaggaagacc atgtggacct gtcactgtct 420

tgtacccttg tgcctcgctc aggggagcag gctgaagggt ccccaggtgg acctggagac 480

tctcagggtc gaaaacggcg gcagaccagc atgacagatt tctaccactc caaacgccgg 540

ctgatcttct ccaagaggaa gccctaatcc gcccacagga agcctgcagt cctggaagcg 600

cgagggcctc aaaggcccgc tctacatctt ctgccttagt ctcagtttgt gtgtcttaat 660

tattatttgt gttttaattt aaacacctcc tcatgtacat accctggccg ccccctgccc 720

cccagcctct ggcattagaa ttatttaaac aaaaactagg cggttgaatg agaggttcct 780

aagagtgctg ggcattttta ttttatgaaa tactatttaa agcctcctca tcccgtgttc 840

tccttttcct ctctcccgga ggttgggtgg gccggcttca tgccagctac ttcctcctcc 900

ccacttgtcc gctgggtggt accctctgga ggggtgtggc tccttcccat cgctgtcaca 960

ggcggttatg aaattcaccc cctttcctgg acactcagac ctgaattctt tttcatttga 1020

gaagtaaaca gatggcactt tgaaggggcc tcaccgagtg ggggcatcat caaaaacttt 1080

ggagtcccct cacctcctct aaggttgggc agggtgaccc tgaagtgagc acagcctagg 1140

gctgagctgg ggacctggta ccctcctggc tcttgatacc cccctctgtc ttgtgaaggc 1200

agggggaagg tggggtcctg gagcagacca ccccgcctgc cctcatggcc cctctgacct 1260

gcactgggga gcccgtctca gtgttgagcc ttttccctct ttggctcccc tgtacctttt 1320

gaggagcccc agctaccctt cttctccagc tgggctctgc aattcccctc tgctgctgtc 1380

cctccccctt gtcctttccc ttcagtaccc tctcagctcc aggtggctct gaggtgcctg 1440

tcccaccccc acccccagct caatggactg gaaggggaag ggacacacaa gaagaagggc 1500

accctagttc tacctcaggc agctcaagca gcgaccgccc cctcctctag ctgtgggggt 1560

gagggtccca tgtggtggca caggccccct tgagtggggt tatctctgtg ttaggggtat 1620

atgatggggg agtagatctt tctaggaggg agacactggc ccctcaaatc gtccagcgac 1680

cttcctcatc caccccatcc ctccccagtt cattgcactt tgattagcag cggaacaagg 1740

agtcagacat tttaagatgg tggcagtaga ggctatggac agggcatgcc acgtgggctc 1800

atatggggct gggagtagtt gtctttcctg gcactaacgt tgagcccctg gaggcactga 1860

agtgcttagt gtacttggag tattggggtc tgaccccaaa caccttccag ctcctgtaac 1920

atactggcct ggactgtttt ctctcggctc cccatgtgtc ctggttcccg tttctccacc 1980

tagactgtaa acctctcgag ggcagggacc acaccctgta ctgttctgtg tctttcacag 2040

ctcctcccac aatgctgaat atacagcagg tgctcaataa atgattctta gtgactttac 2100

ttgtaaaaaa aaaaaaaaaa aa 2122

<210> 17
<211> 2413
<212> DNA
<213> Homo sapiens

<400> 17
cttcttcgtc agcctccctt ccaccgccat attgggccac taaaaaaagg gggctcgtct 60

tttcggggtg tttttctccc cctcccctgt ccccgcttgc tcacggctct gcgactccga 120

cgccggcaag gtttggagag cggctgggtt cgcgggaccc gcgggcttgc acccgcccag 180

actcggacgg gctttgccac cctctccgct tgcctggtcc cctctcctct ccgccctccc 240

gctcgccagt ccatttgatc agcggagact cggcggccgg gccggggctt ccccgcagcc 300

cctgcgcgct cctagagctc gggccgtggc tcgtcggggt ctgtgtcttt tggctccgag 360

ggcagtcgct gggcttccga gaggggttcg ggctgcgtag ggcgctttgg ttttgttcgg 420

ttttgttttt ttgagagtgc gagagaggcg gtcgtgcaga cccgggagaa agatgtcaaa 480

cgtgcgagtg tctaacggga gccctagcct ggagcggatg gacgccaggc aggcggagca 540

ccccaagccc tcggcctgca ggaacctctt cggcccggtg gaccacgaag agttaacccg 600

ggacttggag aagcactgca gagacatgga agaggcgagc cagcgcaagt ggaatttcga 660

ttttcagaat cacaaacccc tagagggcaa gtacgagtgg caagaggtgg agaagggcag 720

cttgcccgag ttctactaca gacccccgcg gccccccaaa ggtgcctgca aggtgccggc 780

gcaggagagc caggatgtca gcgggagccg cccggcggcg cctttaattg gggctccggc 840

taactctgag gacacgcatt tggtggaccc aaagactgat ccgtcggaca gccagacggg 900

gttagcggag caatgcgcag gaataaggaa gcgacctgca accgacgatt cttctactca 960

aaacaaaaga gccaacagaa cagaagaaaa tgtttcagac ggttccccaa atgccggttc 1020

tgtggagcag acgcccaaga agcctggcct cagaagacgt caaacgtaaa cagctcgaat 1080

taagaatatg tttccttgtt tatcagatac atcactgctt gatgaagcaa ggaagatata 1140

catgaaaatt ttaaaaatac atatcgctga cttcatggaa tggacatcct gtataagcac 1200

tgaaaaacaa caacacaata acactaaaat tttaggcact cttaaatgat ctgcctctaa 1260

aagcgttgga tgtagcatta tgcaattagg tttttcctta tttgcttcat tgtactacct 1320

gtgtatatag tttttacctt ttatgtagca cataaacttt ggggaaggga gggcagggtg 1380

gggctgagga actgacgtgg agcggggtat gaagagcttg ctttgattta cagcaagtag 1440

ataaatattt gacttgcatg aagagaagca attttgggga agggtttgaa ttgttttctt 1500

taaagatgta atgtcccttt cagagacagc tgatacttca tttaaaaaaa tcacaaaaat 1560

ttgaacactg gctaaagata attgctattt atttttacaa gaagtttatt ctcatttggg 1620

agatctggtg atctcccaag ctatctaaag tttgttagat agctgcatgt ggctttttta 1680

aaaaagcaac agaaacctat cctcactgcc ctccccagtc tctcttaaag ttggaattta 1740

ccagttaatt actcagcaga atggtgatca ctccaggtag tttggggcaa aaatccgagg 1800

tgcttgggag ttttgaatgt taagaattga ccatctgctt ttattaaatt tgttgacaaa 1860

attttctcat tttcttttca cttcgggctg tgtaaacaca gtcaaaataa ttctaaatcc 1920

ctcgatattt ttaaagatct gtaagtaact tcacattaaa aaatgaaata ttttttaatt 1980

taaagcttac tctgtccatt tatccacagg aaagtgttat ttttcaagga aggttcatgt 2040

agagaaaagc acacttgtag gataagtgaa atggatacta catctttaaa cagtatttca 2100

ttgcctgtgt atggaaaaac catttgaagt gtacctgtgt acataactct gtaaaaacac 2160

tgaaaaatta tactaactta tttatgttaa aagatttttt ttaatctaga caatatacaa 2220

gccaaagtgg catgttttgt gcatttgtaa atgctgtgtt gggtagaata ggtttccccc 2280

tcttttgtta aataatatgg ctatgcttaa aaggttgcat actgagccaa gtataatttt 2340

ttgtaatgtg tgaaaaagat gccaattatt gttacacatt aagtaatcaa taaagaaaac 2400

ttccatagct att 2413

<210> 18
<211> 4372
<212> DNA
<213> Homo sapiens

<400> 18
ggtgcctccg ggggcggggc ctccttcggt tggcggcctc gggcttcggg agtcctccaa 60

gaggccaggt gaggccgtcc cgtgatgccc cgcgccccgg ccgctctggc ctgcaacgtg 120

tctctggggc ggaggcagcg gcagtggagt tcgctgcgcg ctgttggggg ccacctgtct 180

tttcgcttgt gtccctcttt ctagtgtcgc gctcgagtcc cgacgggccg ctccaagcct 240

cgacatgtcg tacaactacg tggtaacggc ccagaagccc accgccgtga acggctgcgt 300

gaccggacac tttacttcgg ccgaagactt aaacctgttg attgccaaaa acacgagatt 360

agagatctat gtggtcaccg ccgaggggct tcggcccgtc aaagaggtgg gcatgtatgg 420

gaagattgcg gtcatggagc ttttcaggcc caagggggag agcaaggacc tgctgtttat 480

cttgacagcg aagtacaatg cctgcatcct ggagtataaa cagagtggcg agagcattga 540

catcattacg cgagcccatg gcaatgtcca ggaccgcatt ggccgcccct cagagaccgg 600

cattattggc atcattgacc ctgagtgccg gatgattggc ctgcgtctct atgatggcct 660

tttcaaggtt attccactag atcgcgataa taaagaactc aaggccttca acatccgcct 720

ggaggagctg catgtcattg atgtcaagtt cctatatggt tgccaagcac ctactatttg 780

ctttgtctac caggaccctc aggggcggca cgtaaaaacc tatgaggtgt ctctccgaga 840

aaaggaattc aataagggcc cttggaaaca ggaaaatgtc gaagctgaag cttccatggt 900

gatcgcagtc ccagagccct ttgggggggc catcatcatt ggacaggagt caatcaccta 960

tcacaatggt gacaaatacc tggctattgc ccctcctatc atcaagcaaa gcacgattgt 1020

gtgccacaat cgagtggacc ctaatggctc aagatacctg ctgggagaca tggaaggccg 1080

gctcttcatg ctgcttttgg agaaggagga acagatggat ggcaccgtca ctctcaagga 1140

tctccgtgta gaactccttg gagagacctc tattgctgag tgcttgacat accttgataa 1200

tggtgttgtg tttgtcgggt ctcgcctggg tgactcccag cttgtgaagc tcaacgttga 1260

cagtaatgaa caaggctcct atgtagtggc catggaaacc tttaccaact taggacccat 1320

tgtcgatatg tgcgtggtgg acctggagag gcaggggcag gggcagctgg tcacttgctc 1380

tggggctttc aaggaaggtt ctttgcggat catccggaat ggaattggaa tccacgagca 1440

tgccagcatt gacttaccag gcatcaaagg attatggcca ctgcggtctg accctaatcg 1500

tgagactgat gacactttgg tgctctcttt tgtgggccag acaagagttc tcatgttaaa 1560

tggagaggag gtagaagaaa ccgaactgat gggtttcgtg gatgatcagc agactttctt 1620

ctgtggcaac gtggctcatc agcagcttat ccagatcact tcagcatcgg tgaggttggt 1680

ctctcaagaa cccaaagctc tggtcagtga atggaaggag cctcaggcca agaacatcag 1740

tgtggcctcc tgcaatagca gccaggtggt ggtggctgta ggcagggccc tctactatct 1800

gcagatccat cctcaggagc tccggcagat cagccacaca gagatggaac atgaagtggc 1860

ttgcttggac atcaccccat taggagacag caatggactg tcccctcttt gtgccattgg 1920

cctctggacg gacatctcgg ctcgtatctt gaagttgccc tcttttgaac tactgcacaa 1980

ggagatgctg ggtggagaga tcattcctcg ctccatcctg atgaccacct ttgagagtag 2040

ccattacctc ctttgtgcct tgggagatgg agcgcttttc tactttgggc tcaacattga 2100

gacaggtctg ttgagcgacc gtaagaaggt gactttgggc acccagccca ccgtattgag 2160

gacttttcgt tctctttcta ccaccaacgt ctttgcttgt tctgaccgcc ccactgtcat 2220

ctatagcagc aaccacaaat tggtcttctc aaatgtcaac ctcaaggaag tgaactacat 2280

gtgtcccctc aattcagatg gctatcctga cagcctggcg ctggccaaca atagcaccct 2340

caccattggc accatcgatg agatccagaa gctgcacatt cgcacagttc ccctctatga 2400

gtctccaagg aagatctgct accaggaagt gtcccagtgt ttcggggtcc tctccagccg 2460

cattgaagtc caagacacga gtgggggcac gacagccttg aggcccagcg ctagcaccca 2520

ggctctgtcc agcagtgtaa gctccagcaa gctgttctcc agcagcactg ctcctcatga 2580

gacctccttt ggagaagagg tggaggtgca caacctactt atcattgacc aacacacctt 2640

tgaagtgctt catgcccacc agtttctgca gaatgaatat gccctcagtc tggtttcctg 2700

caagctgggc aaagacccca acacttactt cattgtgggc acagcaatgg tgtatcctga 2760

agaggcagag cccaagcagg gtcgcattgt ggtctttcag tattcggatg gaaaactaca 2820

gactgtggct gaaaaggaag tgaaaggggc cgtgtactct atggtggaat ttaacgggaa 2880

gctgttagcc agcatcaata gcacggtgcg gctctatgag tggacaacag agaaggagct 2940

gcgcactgag tgcaaccact acaacaacat catggccctc tacctgaaga ccaagggcga 3000

cttcatcctg gtgggcgacc ttatgcgctc agtgctgctg cttgcctaca agcccatgga 3060

aggaaacttt gaagagattg ctcgagactt taatcccaac tggatgagtg ctgtggaaat 3120

cttggatgat gacaattttc tgggggctga aaatgccttt aacttgtttg tgtgtcaaaa 3180

ggatagcgct gccaccactg acgaggagcg gcagcacctc caggaggttg gtcttttcca 3240

cctgggcgag tttgtcaatg tcttttgcca cggctctctg gtaatgcaga atctgggtga 3300

gacttccacc cccacacaag gctcggtgct cttcggcacg gtcaacggca tgatagggct 3360

ggtgacctca ctgtcagaga gctggtacaa cctcctgctg gacatgcaga atcgactcaa 3420

taaagtcatc aaaagtgtgg ggaagatcga gcactccttc tggagatcct ttcacaccga 3480

gcggaagaca gaaccagcca caggtttcat cgacggtgac ttgattgaga gtttcctgga 3540

tattagccgc cccaagatgc aggaggtggt ggcaaaccta cagtatgacg atggcagcgg 3600

tatgaagcga gaggccactg cagacgacct catcaaggtt gtggaggagc taactcggat 3660

ccattagcca agggcagggg gcccctttgc tgaccctccc caaaggcttt gccctgctgc 3720

cctccccctc ctctccacca tcgtcttctt ggccatggga ggcctttccc taagccagct 3780

gcccccagag ccacagttcc cctatgtgga agtggggcgg gcttcataga gacttgggaa 3840

tgagctgaag gtgaaacatt ttctccctgg atttttacca gtctcacatg attccagcca 3900

tcaccttaga ccaccaagcc ttgattggtg ttgccagttg tcctccttcc ggggaaggat 3960

tttgcagttc tttggctgaa aggaagctgt gcgtgtgtgt gtgtgtatgt gtgtgtgtgt 4020

atgtgtatct cacactcatg cattgtcctc tttttattta gattggcagt gtagggagtt 4080

gtgggtagtg ggaagagggg ttaggagggt ttcattgtct gtgaagtgag accttccttt 4140

tacttttctt ctattgcctc tgagagcatc aggcctagag gcctgactgc caagccatgg 4200

gtagcctggg tgtaaaacct ggagatggtg gatgatcccc acgccacagc ccttttgtct 4260

ctgcaaactg ccttcttcgg aaagaagaag gtgggaggat gtgaattgtt agtttctgag 4320

ttttaccaaa taaagtagaa tataagaaga aaggtaaaaa aaaaaaaaaa aa 4372

<210> 19
<211> 827
<212> DNA
<213> Homo sapiens

<400> 19
gtgatccttg gggccaggta tggcatgccc attgatatgt ggagcctggg ctgcatttta 60

gcagagctcc tgacgggtta ccccctcttg cctggggaag atgaagggga ccagctggcc 120

tgtatgattg aactgttggg catgccctca cagaaactgc tggatgcatc caaacgagcc 180

aaaaattttg tgagctccaa gggttatccc cgttactgca ctgtcacgac tctctcagat 240

ggctctgtgg tcctaaacgg aggccgttcc cggaggggga aactgagggg cccaccggag 300

agcagagagt gggggaacgc gctgaagggg tgtgatgatc cccttttcct tgacttctta 360

aaacagtgtt tagagtggga tcctgcagtg cgcatgaccc caggccaggc tttgcggcac 420

ccctggctga ggaggcggtt gccaaagcct cccaccgggg agaaaacgtc agtgaaaagg 480

ataactgaga gcaccggtgc tatcacatct atatccaagt tacctccacc ttctagctca 540

gcttccaaac tgaggactaa tttggcgcag atgacagatg ccaatgggaa tattcagcag 600

aggacagtgt tgccaaaact tgttagctga gctcacgtcc cctgatgctg gtaacctgaa 660

agatacgaca ttgctgagcc ttactgggtt gaaaaggagt agctcagacc tgtttttatt 720

tgctcaataa ctctactcat ttgtatcttt tcagcactta attttaatgt aagaaagttg 780

ttcattttgt ttttataaaa tacatgagga caatgaaaaa aaaaaaa 827

<210> 20
<211> 4975
<212> DNA
<213> Homo sapiens

<400> 20
ctctcacaca cacacacccc tcccctgcca tccctccccg gactccggct ccggctccga 60

ttgcaatttg caacctccgc tgccgtcgcc gcagcagcca ccaattcgcc agcggttcag 120

gtggctcttg cctcgatgtc ctagcctagg ggcccccggg ccggacttgg ctgggctccc 180

ttcaccctct gcggagtcat gagggcgaac gacgctctgc aggtgctggg cttgcttttc 240

agcctggccc ggggctccga ggtgggcaac tctcaggcag tgtgtcctgg gactctgaat 300

ggcctgagtg tgaccggcga tgctgagaac caataccaga cactgtacaa gctctacgag 360

aggtgtgagg tggtgatggg gaaccttgag attgtgctca cgggacacaa tgccgacctc 420

tccttcctgc agtggattcg agaagtgaca ggctatgtcc tcgtggccat gaatgaattc 480
tctactctac cattgcccaa cctccgcgtg gtgcgaggga cccaggtcta cgatgggaag 540
tttgccatct tcgtcatgtt gaactataac accaactcca gccacgctct gcgccagctc 600
cgcttgactc agctcaccga gattctgtca gggggtgttt atattgagaa gaacgataag 660
ctttgtcaca tggacacaat tgactggagg gacatcgtga gggaccgaga tgctgagata 720
gtggtgaagg acaatggcag aagctgtccc ccctgtcatg aggtttgcaa ggggcgatgc 780
tggggtcctg gatcagaaga ctgccagaca ttgaccaaga ccatctgtgc tcctcagtgt 840
aatggtcact gctttgggcc caaccccaac cagtgctgcc atgatgagtg tgccgggggc 900
tgctcaggcc ctcaggacac agactgcttt gcctgccggc acttcaatga cagtggagcc 960
tgtgtacctc gctgtccaca gcctcttgtc tacaacaagc taactttcca gctggaaccc 1020
aatccccaca ccaagtatca gtatggagga gtttgtgtag ccagctgtcc ccataacttt 1080
gtggtggatc aaacatcctg tgtcagggcc tgtcctcctg acaagatgga agtagataaa 1140
aatgggctca agatgtgtga gccttgtggg ggactatgtc ccaaagcctg tgagggaaca 1200
ggctctggga gccgcttcca gactgtggac tcgagcaaca ttgatggatt tgtgaactgc 1260
accaagatcc tgggcaacct ggactttctg atcaccggcc tcaatggaga cccctggcac 1320
aagatccctg ccctggaccc agagaagctc aatgtcttcc ggacagtacg ggagatcaca 1380
ggttacctga acatccagtc ctggccgccc cacatgcaca acttcagtgt tttttccaat 1440
ttgacaacca ttggaggcag aagcctctac aaccggggct tctcattgtt gatcatgaag 1500
aacttgaatg tcacatctct gggcttccga tccctgaagg aaattagtgc tgggcgtatc 1560
tatataagtg ccaataggca gctctgctac caccactctt tgaactggac caaggtgctt 1620
cgggggccta cggaagagcg actagacatc aagcataatc ggccgcgcag agactgcgtg 1680
gcagagggca aagtgtgtga cccactgtgc tcctctgggg gatgctgggg cccaggccct 1740
ggtcagtgct tgtcctgtcg aaattatagc cgaggaggtg tctgtgtgac ccactgcaac 1800
tttctgaatg gggagcctcg agaatttgcc catgaggccg aatgcttctc ctgccacccg 1860
gaatgccaac ccatgggggg cactgccaca tgcaatggct cgggctctga tacttgtgct 1920
caatgtgccc attttcgaga tgggccccac tgtgtgagca gctgccccca tggagtccta 1980
ggtgccaagg gcccaatcta caagtaccca gatgttcaga atgaatgtcg gccctgccat 2040
gagaactgca cccaggggtg taaaggacca gagcttcaag actgtttagg acaaacactg 2100
gtgctgatcg gcaaaaccca tctgacaatg gctttgacag tgatagcagg attggtagtg 2160
attttcatga tgctgggcgg cacttttctc tactggcgtg ggcgccggat tcagaataaa 2220
agggctatga ggcgatactt ggaacggggt gagagcatag agcctctgga ccccagtgag 2280

aaggctaaca aagtcttggc cagaatcttc aaagagacag agctaaggaa gcttaaagtg 2340

cttggctcgg gtgtctttgg aactgtgcac aaaggagtgt ggatccctga gggtgaatca 2400

atcaagattc cagtctgcat taaagtcatt gaggacaaga gtggacggca gagttttcaa 2460

gctgtgacag atcatatgct ggccattggc agcctggacc atgcccacat tgtaaggctg 2520

ctgggactat gcccagggtc atctctgcag cttgtcactc aatatttgcc tctgggttct 2580

ctgctggatc atgtgagaca acaccggggg gcactggggc cacagctgct gctcaactgg 2640

ggagtacaaa ttgccaaggg aatgtactac cttgaggaac atggtatggt gcatagaaac 2700

ctggctgccc gaaacgtgct actcaagtca cccagtcagg ttcaggtggc agattttggt 2760

gtggctgacc tgctgcctcc tgatgataag cagctgctat acagtgaggc caagactcca 2820

attaagtgga tggcccttga gagtatccac tttgggaaat acacacacca gagtgatgtc 2880

tggagctatg gtgtgacagt ttgggagttg atgaccttcg gggcagagcc ctatgcaggg 2940

ctacgattgg ctgaagtacc agacctgcta gagaaggggg agcggttggc acagccccag 3000

atctgcacaa ttgatgtcta catggtgatg gtcaagtgtt ggatgattga tgagaacatt 3060

cgcccaacct ttaaagaact agccaatgag ttcaccagga tggcccgaga cccaccacgg 3120

tatctggtca taaagagaga gagtgggcct ggaatagccc ctgggccaga gccccatggt 3180

ctgacaaaca agaagctaga ggaagtagag ctggagccag aactagacct agacctagac 3240

ttggaagcag aggaggacaa cctggcaacc accacactgg gctccgccct cagcctacca 3300

gttggaacac ttaatcggcc acgtgggagc cagagccttt taagtccatc atctggatac 3360

atgcccatga accagggtaa tcttgggggg tcttgccagg agtctgcagt ttctgggagc 3420

agtgaacggt gcccccgtcc agtctctcta cacccaatgc cacggggatg cctggcatca 3480

gagtcatcag aggggcatgt aacaggctct gaggctgagc tccaggagaa agtgtcaatg 3540

tgtagaagcc ggagcaggag ccggagccca cggccacgcg gagatagcgc ctaccattcc 3600

cagcgccaca gtctgctgac tcctgttacc ccactctccc cacccgggtt agaggaagag 3660

gatgtcaacg gttatgtcat gccagataca cacctcaaag gtactccctc ctcccgggaa 3720

ggcacccttt cttcagtggg tctcagttct gtcctgggta ctgaagaaga agatgaagat 3780

gaggagtatg aatacatgaa ccggaggaga aggcacagtc cacctcatcc ccctaggcca 3840

agttcccttg aggagctggg ttatgagtac atggatgtgg ggtcagacct cagtgcctct 3900

ctgggcagca cacagagttg cccactccac cctgtaccca tcatgcccac tgcaggcaca 3960

actccagatg aagactatga atatatgaat cggcaacgag atggaggtgg tcctgggggt 4020

gattatgcag ccatgggggc ctgcccagca tctgagcaag ggtatgaaga gatgagagct 4080

tttcaggggc ctggacatca ggccccccat gtccattatg cccgcctaaa aactctacgt 4140

agcttagagg ctacagactc tgcctttgat aaccctgatt actggcatag caggcttttc 4200

cccaaggcta atgcccagag aacgtaactc ctgctccctg tggcactcag ggagcattta 4260

atggcagcta gtgcctttag agggtaccgt cttctcccta ttccctctct ctcccaggtc 4320

ccagcccctt ttccccagtc ccagacaatt ccattcaatc tttggaggct tttaaacatt 4380

ttgacacaaa attcttatgg tatgtagcca gctgtgcact ttcttctctt tcccaacccc 4440

aggaaaggtt ttccttattt tgtgtgcttt cccagtccca ttcctcagct tcttcacagg 4500

cactcctgga gatatgaagg attactctcc atatcccttc ctctcaggct cttgactact 4560

tggaactagg ctcttatgtg tgcctttgtt tcccatcaga ctgtcaagaa gaggaaaggg 4620

aggaaaccta gcagaggaaa gtgtaatttt ggtttatgac tcttaacccc ctagaaagac 4680

agaagcttaa aatctgtgaa gaaagaggtt aggagtagat attgattact atcataattc 4740

agcacttaac tatgagccag gcatcatact aaacttcacc tacattatct cacttagtcc 4800

tttatcatcc ttaaaacaat tctgtgacat acatattatc tcattttaca caaagggaag 4860

tcgggcatgg tggctcatgc ctgtaatctc agcactttgg gaggctgagg cagaaggatt 4920

acctgaggca aggagtttga gaccagctta gccaacatag taagaccccc atctc 4975

<210> 21
<211> 4627
<212> DNA
<213> Homo sapiens

<400> 21
tcacttgcct gatatttcca gtgtcagagg gacacagcca acgtggggtc ccttctaggc 60

tgacagccgc tctccagcca ctgccgcgag cccgtctgct cccgccctgc ccgtgcactc 120

tccgcagccg ccctccgcca agccccagcg cccgctccca tcgccgatga ccgcggggag 180

gaggatggag atgctctgtg ccggcagggt ccctgcgctg ctgctctgcc tgggtttcca 240

tcttctacag gcagtcctca gtacaactgt gattccatca tgtatcccag gagagtccag 300

tgataactgc acagctttag ttcagacaga agacaatcca cgtgtggctc aagtgtcaat 360

aacaaagtgt agctctgaca tgaatggcta ttgtttgcat ggacagtgca tctatctggt 420

ggacatgagt caaaactact gcaggtgtga agtgggttat actggtgtcc gatgtgaaca 480

cttcttttta accgtccacc aacctttaag caaagagtat gtggctttga ccgtgattct 540

tattattttg tttcttatca cagtcgtcgg ttccacatat tatttctgca gatggtacag 600

aaatcgaaaa agtaaagaac caaagaagga atatgagaga gttacctcag gggatccaga 660

gttgccgcaa gtctgaatgg cgccatcaaa cttatgggca gggataacag tgtgcctggt 720

taatattaat attccatttt attaataata tttatgttgg gtcaagtgtt aggtcaataa 780

cactgtattt taatgtactt gaaaaatgtt tttatttttg ttttattttt gacagactat 840

ttgctaatgt ataatgtgca gaaaatattt aatatcaaaa gaaaattgat atttttatac 900

aagtaatttc ctgagctaaa tgcttcattg aaagcttcaa agtttatatg cctggtgcac 960

agtgcttaga agtaagcaat tcccaggtca tagctcaaga attgttagca aatgacagat 1020

ttctgtaagc ctatatatat agtcaaatcg atttagtaag tatgtttttt atgttcctca 1080

aatcagtgat aattggtttg actgtaccat ggtttgatat gtagttggca ccatggtatc 1140

atatattaaa acaataatgc aattagaatt tgggagaagc aaatataggt cctgtgttaa 1200

acactacaca tttgaaacaa gctaaccctg gggagtctat ggtctcttca ctcaggtctc 1260

agctataatt ctgttatatg aggggcagtg gacagttccc tatgccaact cacgactcct 1320

acaggtacta gtcactcatc taccagattc tgcctatgta aaatgaattg aaaaacaatt 1380

ttctgtaatc ttttatttaa gtagtgggca tttcatagct tcacaatgtt ccttttttgt 1440

atattacaac atttatgtga ggtaattatt gctcaacaga caattagaaa aaagtccaca 1500

cttgaagcct aaatttgtgc tttttaagaa tatttttaga ctatttcttt ttataggggc 1560

tttgctgaat tctaacatta aatcacagcc caaaatttga tggactaatt attattttaa 1620

aatatatgaa gacaataatt ctacatgttg tcttaagatg gaaatacagt tatttcatct 1680

tttattcaag gaagttttaa ctttaataca gctcagtaaa tggcttcttc tagaatgtaa 1740

agttatgtat ttaaagttgt atcttgacac aggaaatggg aaaaaactta aaaattaata 1800

tggtgtattt ttccaaatga aaaatctcaa ttgaaagctt ttaaaatgta gaaacttaaa 1860

cacaccttcc tgtggaggct gagatgaaaa ctagggctca ttttcctgac atttgtttat 1920

tttttggaag agacaaagat ttcttctgca ctctgagccc ataggtctca gagagttaat 1980

aggagtattt ttgggctatt gcataaggag ccactgctgc caccactttt ggattttatg 2040

ggaggctcct tcatcgaatg ctaaaccttt gagtagagtc tccctggatc acataccagg 2100

tcagggagga tctgttcttc ctctacgttt atcctggcat gtgctagggt aaacgaaggc 2160

ataataagcc atggctgacc tctggagcac caggtgccag gacttgtctc catgtgtatc 2220

catgcattat ataccctggt gcaatcacac gactgtcatc taaagtcctg gccctggccc 2280

ttactattag gaaaataaac agacaaaaac aagtaaatat atatggtcct atacatattg 2340

tatatatatt catatacaaa catgtatgta tacatgacct taatggatca tagaattgca 2400

gtcatttggt gctctgctaa ccatttatat aaaacttaaa aacaagagaa aagaaaaatc 2460

aattagatct aaacagttat ttctgtttcc tatttaatat agctgaagtc aaaatatgta 2520

agaacacatt ttaaatactc tacttacagt tggccctctg tggttagttc cacatctgtg 2580

gattcaacca accaaggacg gaaaatgctt aaaaaataat acaacaacaa caaaaaatac 2640

attataacaa ctatttactt tttttttttt ctttttgaga tggagtctcg ctctgttgcc 2700

caggttggag tgcagtggca cgatctcggc tcactgcaac ctcacctccc gggttcaaga 2760

gatcctcctg cctcagcctc ctgagcagct gggactacag gcgcatgcca ccatgcccag 2820

ctaatttttg tatttttagt agaggcgggg tttcaccatg ttggccagga tggtctcaat 2880

ctcctaacct tgagatccac cctccacagc ctcccaaact gctgggatta caggcgtgag 2940

ccaccgcacg tagcatttac attaggtatt acaagtaatg taaagatgat ttaagtatac 3000

aggaggatgt gaataggtta tatgcaagca ctatgccctt ttatataagt gacttgaaca 3060

tctgtgcccg attttagtat gtgcaggggg gcgatctggg aatcagtccc ctgtggatac 3120

caaggtacaa ctgtatttat taacgcttac tagatgtgag gagagtctga atattttcag 3180

tgatcttggc tgtttcaaaa aaatctattg acttttcaat aaatcagctg caatccattt 3240

atttcattta caaaagattt attgtaagcc tctcaatctt ggtttttcag ttgatcttaa 3300

gcatgtcaat tcataaaaac aagtcatttt tgtatttttc atctttaaga atgcttaaaa 3360

aagctaatcc ctaaaatagt tagatctttg taaatgcata ttaaataata aagtatgacc 3420

cacattactt tttatgggtg aaaataagac aaaaataata gttttagtga ggatggtgct 3480

gagtaaacat aaaaactgat ttgctctcag ctgatgtgtc ctgtacacag tgggaagatt 3540

ttagttcaca cttagtctaa ctcccccatt ttacagattt ctcactatat atatttctag 3600

aaggggctat gcatattcaa tgtattgaga accaaagcaa ccacaaatgc ataaatgcat 3660

aatttatggt cttcaaccaa ggccacataa taacccagtt aacttactct ttaaccagga 3720

atattaagtt ctataactag tactcaaggt ttaaccttaa aattaagatt tccttaacct 3780

taaccttaaa attgatatta tattaaacat acataataca atgtaactcc actgttctcc 3840

tgaatatttt ttgctctaat ctctctgccg aaagtcaaag tgatgggaga attggtatac 3900

tggtatgact acgtcttaag tcagattttt atttatgagt ctttgagact aaattcaatc 3960

accaccaggt atcaaatcaa cttttatgca gcaaatatat gattctagtg tctgactttt 4020

gttaaattca gtaatgcagt ttttaaaaac ctgtatctga cccactttgt aatttttgct 4080

ccaatatcca ttctgtagac ttttgaaaaa aaagttttta atttgatgcc caatatattc 4140

tgaccgttaa aaaattcttg ttcatatggg agaaggggga gtaatgactt gtacaaacag 4200

tatttctggt gtatatttta atgtttttaa aaagagtaat ttcatttaaa tatctgttat 4260

tcaaatttga tgatgttaaa tgtaatataa tgtattttct ttttattttg cactctgtaa 4320

ttgcactttt taagtttgaa gagccatttt ggtaaacggt ttttattaaa gatgctatgg 4380

aacataaagt tgtattgcat gcaatttaaa gtaacttatt tgactatgaa tattatcgga 4440

ttactgaatt gtatcaattt gtttgtgttc aatatcagct ttgataattg tgtaccttaa 4500

gatattgaag gagaaaatag ataatttaca agatattatt aatttttatt tatttttctt 4560

gggaattgaa aaaaattgaa ataaataaaa atgcattgaa catcttgcat tcaaaatctt 4620

cactgac 4627

<210>  22
<211>  6450
<212>  DNA
<213>  Homo sapiens

<400>  22
gagttgtgcc tggagtgatg tttaagccaa tgtcagggca aggcaacagt ccctggccgt      60

cctccagcac ctttgtaatg catatgagct cgggagacca gtacttaaag ttggaggccc     120

gggagcccag gagctggcgg agggcgttcg tcctgggagc tgcacttgct ccgtcgggtc     180

gccggcttca ccggaccgca ggctcccggg gcagggccgg ggccagagct cgcgtgtcgg     240

cgggacatgc gctgcgtcgc ctctaacctc gggctgtgct ctttttccag gtggcccgcc     300

ggtttctgag ccttctgccc tgcggggaca cggtctgcac cctgcccgcg gccacggacc     360

atgaccatga ccctccacac caaagcatct gggatggccc tactgcatca gatccaaggg     420

aacgagctgg agcccctgaa ccgtccgcag ctcaagatcc ccctggagcg gcccctgggc     480

gaggtgtacc tggacagcag caagcccgcc gtgtacaact accccgaggg cgccgcctac     540

gagttcaacg ccgcggccgc cgccaacgcg caggtctacg gtcagaccgg cctcccctac     600

ggccccgggt ctgaggctgc ggcgttcggc tccaacggcc tgggggtttt ccccccactc     660

aacagcgtgt ctccgagccc gctgatgcta ctgcacccgc cgccgcagct gtcgcctttc     720

ctgcagcccc acggccagca ggtgccctac tacctggaga acgagcccag cggctacacg     780

gtgcgcgagg ccggcccgcc ggcattctac aggccaaatt cagataatcg acgccagggt     840

ggcagagaaa gattggccag taccaatgac aagggaagta tggctatgga atctgccaag     900

gagactcgct actgtgcagt gtgcaatgac tatgcttcag gctaccatta tggagtctgg     960

tcctgtgagg gctgcaaggc cttcttcaag agaagtattc aaggacataa cgactatatg    1020

tgtccagcca ccaaccagtg caccattgat aaaaacagga ggaagagctg ccaggcctgc    1080

cggctccgca aatgctacga agtgggaatg atgaaaggtg ggatacgaaa agaccgaaga    1140

ggagggagaa tgttgaaaca caagcgccag agagatgatg gggagggcag gggtgaagtg    1200

gggtctgctg gagacatgag agctgccaac ctttggccaa gcccgctcat gatcaaacgc    1260

tctaagaaga acagcctggc cttgtccctg acggccgacc agatggtcag tgccttgttg    1320

gatgctgagc cccccatact ctattccgag tatgatccta ccagaccctt cagtgaagct    1380

tcgatgatgg gcttactgac caacctggca gacagggagc tggttcacat gatcaactgg    1440

gcgaagaggg tgccaggctt tgtggatttg accctccatg atcaggtcca ccttctagaa    1500

tgtgcctggc tagagatcct gatgattggt ctcgtctggc gctccatgga gcacccagtg    1560

aagctactgt ttgctcctaa cttgctcttg gacaggaacc agggaaaatg tgtagagggc    1620

atggtggaga tcttcgacat gctgctggct acatcatctc ggttccgcat gatgaatctg    1680

cagggagagg agtttgtgtg cctcaaatct attattttgc ttaattctgg agtgtacaca 1740

tttctgtcca gcaccctgaa gtctctggaa gagaaggacc atatccaccg agtcctggac 1800

aagatcacag acactttgat ccacctgatg gccaaggcag gcctgaccct gcagcagcag 1860

caccagcggc tggcccagct cctcctcatc ctctcccaca tcaggcacat gagtaacaaa 1920

ggcatggagc atctgtacag catgaagtgc aagaacgtgg tgcccctcta tgacctgctg 1980

ctggagatgc tggacgccca ccgcctacat gcgcccacta gccgtggagg ggcatccgtg 2040

gaggagacgg accaaagcca cttggccact gcgggctcta cttcatcgca ttccttgcaa 2100

aagtattaca tcacggggga ggcagagggt ttccctgcca cagtctgaga gctccctggc 2160

tcccacacgg ttcagataat ccctgctgca ttttaccctc atcatgcacc actttagcca 2220

aattctgtct cctgcataca ctccggcatg catccaacac caatggcttt ctagatgagt 2280

ggccattcat ttgcttgctc agttcttagt ggcacatctt ctgtcttctg ttgggaacag 2340

ccaaagggat tccaaggcta aatctttgta acagctctct ttcccccttg ctatgttact 2400

aagcgtgagg attcccgtag ctcttcacag ctgaactcag tctatgggtt ggggctcaga 2460

taactctgtg catttaagct acttgtagag acccaggcct ggagagtaga cattttgcct 2520

ctgataagca ctttttaaat ggctctaaga ataagccaca gcaaagaatt taaagtggct 2580

cctttaattg gtgacttgga gaaagctagg tcaagggttt attatagcac cctcttgtat 2640

tcctatggca atgcatcctt ttatgaaagt ggtacacctt aaagctttta tatgactgta 2700

gcagagtatc tggtgattgt caattcactt ccccctatag gaatacaagg ggccacacag 2760

ggaaggcaga tcccctagtt ggccaagact tattttaact tgatacactg cagattcaga 2820

gtgtcctgaa gctctgcctc tggctttccg gtcatgggtt ccagttaatt catgcctccc 2880

atggacctat ggagagcaac aagttgatct tagttaagtc tccctatatg agggataagt 2940

tcctgatttt tgtttttatt tttgtgttac aaaagaaagc cctccctccc tgaacttgca 3000

gtaaggtcag cttcaggacc tgttccagtg ggcactgtac ttggatcttc ccggcgtgtg 3060

tgtgccttac acaggggtga actgttcact gtggtgatgc atgatgaggg taaatggtag 3120

ttgaaaggag caggggccct ggtgttgcat ttagccctgg ggcatggagc tgaacagtac 3180

ttgtgcagga ttgttgtggc tactagagaa caagagggaa agtagggcag aaactggata 3240

cagttctgag cacagccaga cttgctcagg tggccctgca caggctgcag ctacctagga 3300

acattccttg cagaccccgc attgcctttg ggggtgccct gggatccctg gggtagtcca 3360

gctcttattc atttcccagc gtggccctgg ttggaagaag cagctgtcaa gttgtagaca 3420

gctgtgttcc tacaattggc ccagcaccct ggggcacggg agaaggtggg ggaccgttgc 3480

tgtcactact caggctgact ggggcctggt cagattacgt atgcccttgg tggtttagag 3540

ataatccaaa atcagggttt ggtttgggga agaaaatcct cccccttcct cccccgcccc 3600

gttccctacc gcctccactc ctgccagctc atttccttca atttcctttg acctataggc 3660

taaaaaagaa aggctcattc cagccacagg gcagccttcc ctgggccttt gcttctctag 3720

cacaattatg ggttacttcc tttttcttaa caaaaaagaa tgtttgattt cctctgggtg 3780

accttattgt ctgtaattga aaccctattg agaggtgatg tctgtgttag ccaatgaccc 3840

aggtagctgc tcgggcttct cttggtatgt cttgtttgga aaagtggatt tcattcattt 3900

ctgattgtcc agttaagtga tcaccaaagg actgagaatc tgggagggca aaaaaaaaaa 3960

aaaaagtttt tatgtgcact taaatttggg gacaatttta tgtatctgtg ttaaggatat 4020

gcttaagaac ataattcttt tgttgctgtt tgtttaagaa gcaccttagt ttgtttaaga 4080

agcaccttat atagtataat atatattttt ttgaaattac attgcttgtt tatcagacaa 4140

ttgaatgtag taattctgtt ctggatttaa tttgactggg ttaacatgca aaaaccaagg 4200

aaaaatattt agtttttttt tttttttttg tatacttttc aagctacctt gtcatgtata 4260

cagtcattta tgcctaaagc ctggtgatta ttcatttaaa tgaagatcac atttcatatc 4320

aacttttgta tccacagtag acaaaatagc actaatccag atgcctattg ttggatattg 4380

aatgacagac aatcttatgt agcaaagatt atgcctgaaa aggaaaatta ttcagggcag 4440

ctaattttgc ttttaccaaa atatcagtag taatattttt ggacagtagc taatgggtca 4500

gtgggttctt tttaatgttt atacttagat tttcttttaa aaaaattaaa ataaaacaaa 4560

aaaaatttct aggactagac gatgtaatac cagctaaagc caaacaatta tacagtggaa 4620

ggttttacat tattcatcca atgtgtttct attcatgtta agatactact acatttgaag 4680

tgggcagaga acatcagatg attgaaatgt tcgcccaggg gtctccagca actttggaaa 4740

tctctttgta tttttacttg aagtgccact aatggacagc agatattttc tggctgatgt 4800

tggtattggg tgtaggaaca tgatttaaaa aaaaaactct tgcctctgct ttcccccact 4860

ctgaggcaag ttaaaatgta aaagatgtga tttatctggg gggctcaggt atggtgggga 4920

agtggattca ggaatctggg gaatggcaaa tatattaaga agagtattga aagtatttgg 4980

aggaaaatgg ttaattctgg gtgtgcacca aggttcagta gagtccactt ctgccctgga 5040

gaccacaaat caactagctc catttacagc catttctaaa atggcagctt cagttctaga 5100

gaagaaagaa caacatcagc agtaaagtcc atggaatagc tagtggtctg tgtttctttt 5160

cgccattgcc tagcttgccg taatgattct ataatgccat catgcagcaa ttatgagagg 5220

ctaggtcatc caaagagaag accctatcaa tgtaggttgc aaaatctaac ccctaaggaa 5280

gtgcagtctt tgatttgatt tccctagtaa ccttgcagat atgtttaacc aagccatagc 5340

ccatgccttt tgagggctga acaaataagg gacttactga taatttactt ttgatcacat 5400

taaggtgttc tcaccttgaa atcttataca ctgaaatggc cattgattta ggccactggc 5460

ttagagtact ccttcccctg catgacactg attacaaata ctttcctatt catactttcc 5520

aattatgaga tggactgtgg gtactgggag tgatcactaa caccatagta atgtctaata 5580

ttcacaggca gatctgcttg gggaagctag ttatgtgaaa ggcaaataaa gtcatacagt 5640

agctcaaaag gcaaccataa ttctctttgg tgcaagtctt gggagcgtga tctagattac 5700

actgcaccat tcccaagtta atcccctgaa aacttactct caactggagc aaatgaactt 5760

tggtcccaaa tatccatctt ttcagtagcg ttaattatgc tctgtttcca actgcatttc 5820

ctttccaatt gaattaaagt gtggcctcgt ttttagtcat ttaaaattgt tttctaagta 5880

attgctgcct ctattatggc acttcaattt tgcactgtct tttgagattc aagaaaaatt 5940

tctattcatt tttttgcatc caattgtgcc tgaactttta aaatatgtaa atgctgccat 6000

gttccaaacc catcgtcagt gtgtgtgttt agagctgtgc accctagaaa caacatactt 6060

gtcccatgag caggtgcctg agacacagac ccctttgcat tcacagagag gtcattggtt 6120

atagagactt gaattaataa gtgacattat gccagtttct gttctctcac aggtgataaa 6180

caatgctttt tgtgcactac atactcttca gtgtagagct cttgttttat gggaaaaggc 6240

tcaaatgcca aattgtgttt gatggattaa tatgcccttt tgccgatgca tactattact 6300

gatgtgactc ggttttgtcg cagctttgct ttgtttaatg aaacacactt gtaaacctct 6360

tttgcacttt gaaaaagaat ccagcgggat gctcgagcac ctgtaaacaa ttttctcaac 6420

ctatttgatg ttcaaataaa gaattaaact 6450

<210> 23
<211> 3376
<212> DNA
<213> Homo sapiens

<400> 23
ggcgaccgaa cgcggcggtc ggcagcgttc gcgcgggggc ctgcgaagcg ctgctcgggg 60

ccggcactgc ccgcggggag gacgcgccgc cgccgccacc cagcgccgcc gccgccgccg 120

cctccagccg ggccgccgcg cgtcccgggg gccggccccg cgagcgcagg agtaaacacc 180

gccggagtct tggagccgct gcagaaggga ataaagagag atgcagggat ttgtgaggtt 240

acggcgcccc agctgcaaga tgcactagcc ggctgaaccc gggatcggct gacttgttgg 300

aaccggagtg ctctgcacgg agagtggtgg atgagttgaa gttgccttcc cggggctcat 360

tttccacgct gccgagagga atccgagagg caaggcaatc acttcgtctt gccattgatt 420

gggtatcggg agcttttttt ttctcccctc tctctttctt ttcctccgtc ttgttgcatg 480

caagaaaatt acagtccgct gctcgcccgc cctgggtgcg agatattcag ccccgctctc 540

tcccgtgcat tgtgcaaccc aaagatgaaa gaccgaaggg gagaaagtta aagaaatcgc 600

ccacatgcgc tggatcagtc cacggcttgg ggaaaggcat ccagagaagg tgggagcgga 660

gagtttgaag tctttacagg cgggaagatg gcggactgga gctgaaagtg ttgattggga 720

aacttgggtg attcttgtgt ttatttacaa tcctcttgac ccaggcagga cacatgcagg 780

ccaaaaaacg ctatttcatc ctgctctcag ctggctcttg tctcgccctt ttgttttatt 840

tcggaggctt gcagtttagg gcatcgagga gccacagccg gagagaagaa cacagcggta 900

ggaatggctt gcaccacccc agtccggatc atttctggcc ccgcttcccg gacgctctgc 960

gccccttcgt tccttgggat caattggaaa acgaggattc cagcgtgcac atttcccccc 1020

ggcagaagcg agatgccaac tccagcatct acaaaggcaa gaagtgccgc atggagtcct 1080

gcttcgattt caccctttgc aagaaaaacg gcttcaaagt ctacgtatac ccacagcaaa 1140

aaggggagaa aatcgccgaa agttaccaaa acattctagc ggccatcgag ggctccaggt 1200

tctacacctc ggaccccagc caggcgtgcc tctttgtcct gagtctggat actttagaca 1260

gagaccagtt gtcacctcag tatgtgcaca atttgagatc caaagtgcag agtctccact 1320

tgtggaacaa tggtaggaat catttaattt ttaatttata ttccggcact tggcctgact 1380

acaccgagga cgtggggttt gacatcggcc aggcgatgct ggccaaagcc agcatcagta 1440

ctgaaaactt ccgacccaac tttgatgttt ctattcccct cttttctaag gatcatccca 1500

ggacaggagg ggagaggggg tttttgaagt tcaacaccat ccctcctctc aggaagtaca 1560

tgctggtatt caaggggaag aggtacctga cagggatagg atcagacacc aggaatgcct 1620

tatatcacgt ccataacggg gaggacgttg tgctcctcac cacctgcaag catggcaaag 1680

actggcaaaa gcacaaggat tctcgctgtg acagagacaa caccgagtat gagaagtatg 1740

attatcggga aatgctgcac aatgccactt tctgtctggt tcctcgtggt cgcaggcttg 1800

ggtccttcag attcctggag gctttgcagg ctgcctgcgt ccctgtgatg ctcagcaatg 1860

gatgggagtt gccattctct gaagtgatta attggaacca agctgccgtc ataggcgatg 1920

agagattgtt attacagatt ccttctacaa tcaggtctat tcatcaggat aaaatcctag 1980

cacttagaca gcagacacaa ttcttgtggg aggcttattt ttcttcagtt gagaagattg 2040

tattaactac actagagatt attcaggaca gaatattcaa gcacatatca cgtaacagtt 2100

taatatggaa caaacatcct ggaggattgt tcgtactacc acagtattca tcttatctgg 2160

gagattttcc ttactactat gctaatttag gtttaaagcc cccctccaaa ttcactgcag 2220

tcatccatgc ggtgaccccc ctggtctctc agtcccagcc agtgttgaag cttctcgtgg 2280

ctgcagccaa gtcccagtac tgtgcccaga tcatagttct atggaattgt gacaagcccc 2340

taccagccaa acaccgctgg cctgccactg ctgtgcctgt cgtcgtcatt gaaggagaga 2400

gcaaggttat gagcagccgt tttctgccct acgacaacat catcacagac gccgtgctca 2460

gccttgacga ggacacggtg ctttcaacaa cagaggtgga tttcgccttc acagtgtggc 2520

agagcttccc tgagaggatt gtggggtacc ccgcgcgcag ccacttctgg gataactcta 2580

aggagcggtg gggatacaca tcaaagtgga cgaacgacta ctccatggtg ttgacaggag 2640

ctgctattta ccacaaatat tatcactacc tatactccca ttacctgcca gccagcctga 2700

agaacatggt ggaccaattg gccaattgtg aggacattct catgaacttc ctggtgtctg 2760

ctgtgacaaa attgcctcca atcaaagtga cccagaagaa gcagtataag gagacaatga 2820

tgggacagac ttctcgggct tcccgttggg ctgaccctga ccactttgcc cagcgacaga 2880

gctgcatgaa tacgtttgcc agctggtttg gctacatgcc gctgatccac tctcagatga 2940

ggctcgaccc cgtcctcttt aaagaccagg tctctatttt gaggaagaaa taccgagaca 3000

ttgagcgact ttgaggaatc cggctgagtg gggaggggaa agcaagaagg gatgggggtc 3060

aagctgctct ctcttcccag tgcagatcca ctcatcagca gagccagatt gtgccaacta 3120

tccaaaaact tagatgagca gaatgacaaa aaaaaaaagg ccaatgagaa ctcaactcct 3180

ggctcctggg actgcaccag actgctccaa actcacctca ctggcttctg tgtcccaaga 3240

ctaggttgtg tacagtttaa ttatggaaca ttaaataatt attttgaaaa tgattgctat 3300

gcaggtttaa acttttttaa tgatcaaaac tattaaaaac cagagttctt tgtttaatca 3360

aaaaaaaaaa aaaaaa 3376

<210> 24
<211> 972
<212> DNA
<213> Homo sapiens

<400> 24
tctagactca ggactgagaa gaagtaaaac cgtttgctgg ggctggcctg actcaccagc 60

tgccatgcag cagcccttca attacccata tccccagatc tactgggtgg acagcagtgc 120

cagctctccc tgggcccctc caggcacagt tcttccctgt ccaacctctg tgcccagaag 180

gcctggtcaa aggaggccac caccaccacc gccaccgcca ccactaccac ctccgccgcc 240

gccgccacca ctgcctccac taccgctgcc acccctgaag aagagaggga accacagcac 300

aggcctgtgt ctccttgtga tgtttttcat ggttctggtt gccttggtag gattgggcct 360

ggggatgttt cagctcttcc acctacagaa ggagctggca gaactccgag agtctaccag 420

ccagatgcac acagcatcat ctttggagaa gcaaataggc caccccagtc caccccctga 480

aaaaaaggag ctgaggaaag tggcccattt aacaggcaag tccaactcaa ggtccatgcc 540

tctggaatgg gaagacacct atggaattgt cctgctttct ggagtgaagt ataagaaggg 600

tggccttgtg atcaatgaaa ctgggctgta ctttgtatat tccaaagtat acttccgggg 660

tcaatcttgc aacaacctgc ccctgagcca caaggtctac atgaggaact ctaagtatcc 720

ccaggatctg gtgatgatgg aggggaagat gatgagctac tgcactactg ggcagatgtg 780

ggcccgcagc agctacctgg gggcagtgtt caatcttacc agtgctgatc atttatatgt 840

caacgtatct gagctctctc tggtcaattt tgaggaatct cagacgtttt tcggcttata 900

taagctctaa gagaagcact ttgggattct ttccattatg attctttgtt acaggcaccg 960

agatgttcta ga 972

<210> 25
<211> 1391
<212> DNA
<213> Homo sapiens

<400> 25
tgcaccccga gcatccgccc cgggtggcac gtccccgagc ccaccaggcc ggccccgtct 60

ccccatccgt ctagtccgct cgcggtgcca tgccattcct cgggcaggac tggcggtccc 120

ccgggcagaa ctgggtgaag acggccgacg gctggaagcg cttcctggat gagaagagcg 180

gcagtttcgt gagcgacctc agcagttact gcaacaagga ggtatacaat aaggagaatc 240

ttttcaacag cctgaactat gatgttgcag ccaagaagag aaagaaggac atgctgaata 300

gcaaaaccaa aactcagtat ttccaccaag aaaaatggat ctatgttcac aaaggaagta 360

ctaaagagcg ccatggatat tgcaccctgg gggaagcttt caacagactg gacttctcaa 420

ctgccattct ggattccaga agatttaact acgtggtccg gctgttggag ctgatagcaa 480

agtcacagct cacatccctg agtggcatcg cccaaaagaa cttcatgaat attttggaaa 540

aagtggtact gaaagtcctt gaagaccagc aaaacattag actaataagg gaactactcc 600

agaccctcta cacatcctta tgtacactgg tccaaagagt cggcaagtct gtgctggtcg 660

ggaacattaa catgtgggtg tatcggatgg agacgattct ccactggcag cagcagctga 720

acaacattca gatcaccagg cctgccttca aaggcctcac cttcactgac ctgcctttgt 780

gcctacaact gaacatcatg cagaggctga gcgacgggcg ggacctggtc agcctgggcc 840

aggctgcccc cgacctgcac gtgctcagcg aagaccggct gctgtggaag aaactctgcc 900

agtaccactt ctccgagcgg cagatccgca aacgattaat tctgtcagac aaagggcagc 960

tggattggaa gaagatgtat ttcaaacttg tccgatgtta cccaaggaaa gagcagtatg 1020

gagataccct tcagctctgc aaacactgtc acatcctttc ctggaagggc actgaccatc 1080

cgtgcactgc caataaccca gagagctgct ccgtttcact ttcaccccag gactttatca 1140

acttgttcaa gttctgaatc ccagcacatg acaacacttc agaaggtccc ccctgctgac 1200

tggagagctg ggaatatggc atttggacac ttcatttgta aatagtgtac attttaaaca 1260

ttggctcgaa acttcagaga taagtcatgg agaggacatt ggaggggaga aatgcagttg 1320

ctgactggga atttaagaat gtgaacttct cactagaatt ggtatggaaa agcaaaatac 1380

tgtaaataaa c 1391

<210>  26
<211>  4654
<212>  DNA
<213>  Homo sapiens

<400>  26
ggcggcggct ggaggagagc gcggtggaga gccgagcggg cgggcggcgg gtgcggagcg      60

ggcgagggag cgcgcgcggc cgccacaaag ctcgggcgcc gcggggctgc atgcggcgta     120

cctggcccgg cgcggcgact gctctccggg ctggcggggg ccggccgcga gccccggggg     180

ccccgaggcc gcagcttgcc tgcgcgctct gagccttcgc aactcgcgag caaagtttgg     240

tggaggcaac gccaagcctg agtcctttct tcctctcgtt ccccaaatcc gagggcagcc     300

cgcgggcgtc atgcccgcgc tcctccgcag cctggggtac gcgtgaagcc cgggaggctt     360

ggcgccggcg aagacccaag gaccactctt ctgcgtttgg agttgctccc cgcaaccccg     420

ggctcgtcgc tttctccatc ccgacccacg cggggcgcgg ggacaacaca ggtcgcggag     480

gagcgttgcc attcaagtga ctgcagcagc agcggcagcg cctcggttcc tgagcccacc     540

gcaggctgaa ggcattgcgc gtagtccatg cccgtagagg aagtgtgcag atgggattaa     600

cgtccacatg gagatatgga agaggaccgg ggattggtac cgtaaccatg gtcagctggg     660

gtcgtttcat ctgcctggtc gtggtcacca tggcaacctt gtccctggcc cggccctcct     720

tcagtttagt tgaggatacc acattagagc cagaagagcc accaaccaaa taccaaatct     780

ctcaaccaga agtgtacgtg gctgcgccag gggagtcgct agaggtgcgc tgcctgttga     840

aagatgccgc cgtgatcagt tggactaagg atggggtgca cttggggccc aacaatagga     900

cagtgcttat tggggagtac ttgcagataa agggcgccac gcctagagac tccggcctct     960

atgcttgtac tgccagtagg actgtagaca gtgaaacttg gtacttcatg gtgaatgtca    1020

cagatgccat ctcatccgga gatgatgagg atgacaccga tggtgcggaa gattttgtca    1080

gtgagaacag taacaacaag agagcaccat actggaccaa cacagaaaag atggaaaagc    1140

ggctccatgc tgtgcctgcg gccaacactg tcaagtttcg ctgcccagcc ggggggaacc    1200

caatgccaac catgcggtgg ctgaaaaacg ggaaggagtt taagcaggag catcgcattg    1260

gaggctacaa ggtacgaaac cagcactgga gcctcattat ggaaagtgtg gtcccatctg    1320

acaagggaaa ttatacctgt gtagtggaga atgaatacgg gtccatcaat cacacgtacc    1380

acctggatgt tgtggagcga tcgcctcacc ggcccatcct ccaagccgga ctgccggcaa    1440

atgcctccac agtggtcgga ggagacgtag agtttgtctg caaggtttac agtgatgccc    1500

agccccacat ccagtggatc aagcacgtgg aaaagaacgg cagtaaatac gggcccgacg    1560

ggctgcccta cctcaaggtt ctcaaggccg ccggtgttaa caccacggac aaagagattg    1620

aggttctcta tattcggaat gtaacttttg aggacgctgg ggaatatacg tgcttggcgg    1680

gtaattctat tgggatatcc tttcactctg catggttgac agttctgcca gcgcctggaa 1740

gagaaaagga gattacagct tccccagact acctggagat agccatttac tgcatagggg 1800

tcttcttaat cgcctgtatg gtggtaacag tcatcctgtg ccgaatgaag aacacgacca 1860

agaagccaga cttcagcagc cagccggctg tgcacaagct gaccaaacgt atccccctgc 1920

ggagacaggt aacagtttcg gctgagtcca gctcctccat gaactccaac accccgctgg 1980

tgaggataac aacacgcctc tcttcaacgg cagacacccc catgctggca ggggtctccg 2040

agtatgaact tccagaggac ccaaaatggg agtttccaag agataagctg acactgggca 2100

agcccctggg agaaggttgc tttgggcaag tggtcatggc ggaagcagtg ggaattgaca 2160

aagacaagcc caaggaggcg gtcaccgtgg ccgtgaagat gttgaaagat gatgccacag 2220

agaaagacct ttctgatctg gtgtcagaga tggagatgat gaagatgatt gggaaacaca 2280

agaatatcat aaatcttctt ggagcctgca cacaggatgg gcctctctat gtcatagttg 2340

agtatgcctc taaaggcaac ctccgagaat acctccgagc ccggaggcca cccgggatgg 2400

agtactccta tgacattaac cgtgttcctg aggagcagat gaccttcaag gacttggtgt 2460

catgcaccta ccagctggcc agaggcatgg agtacttggc ttcccaaaaa tgtattcatc 2520

gagatttagc agccagaaat gttttggtaa cagaaaacaa tgtgatgaaa atagcagact 2580

ttggactcgc cagagatatc aacaatatag actattacaa aaagaccacc aatgggcggc 2640

ttccagtcaa gtggatggct ccagaagccc tgtttgatag agtatacact catcagagtg 2700

atgtctggtc cttcggggtg ttaatgtggg agatcttcac tttagggggc tcgccctacc 2760

cagggattcc cgtggaggaa ctttttaagc tgctgaagga aggacacaga atggataagc 2820

cagccaactg caccaacgaa ctgtacatga tgatgaggga ctgttggcat gcagtgccct 2880

cccagagacc aacgttcaag cagttggtag aagacttgga tcgaattctc actctcacaa 2940

ccaatgagga atacttggac ctcagccaac ctctcgaaca gtattcacct agttaccctg 3000

acacaagaag ttcttgttct tcaggagatg attctgtttt ttctccagac cccatgcctt 3060

acgaaccatg ccttcctcag tatccacaca taaacggcag tgttaaaaca tgaatgactg 3120

tgtctgcctg tccccaaaca ggacagcact gggaacctag ctacactgag cagggagacc 3180

atgcctccca gagcttgttg tctccacttg tatatatgga tcagaggagt aaataattgg 3240

aaaagtaatc agcatatgtg taaagattta tacagttgaa aacttgtaat cttccccagg 3300

aggagaagaa ggtttctgga gcagtggact gccacaagcc accatgtaac ccctctcacc 3360

tgccgtgcgt actggctgtg gaccagtagg actcaaggtg gacgtgcgtt ctgccttcct 3420

tgttaatttt gtaataattg gagaagattt atgtcagcac acacttacag agcacaaatg 3480

cagtatatag gtgctggatg tatgtaaata tattcaaatt atgtataaat atatattata 3540

tatttacaag gagttatttt ttgtattgat tttaaatgga tgtcccaatg cacctagaaa 3600

attggtctct ctttttttaa tagctatttg ctaaatgctg ttcttacaca taatttctta 3660

attttcaccg agcagaggtg gaaaaatact tttgctttca gggaaaatgg tataacgtta 3720

atttattaat aaattggtaa tatacaaaac aattaatcat ttatagtttt ttttgtaatt 3780

taagtggcat ttctatgcag gcagcacagc agactagtta atctattgct tggacttaac 3840

tagttatcag atcctttgaa aagagaatat ttacaatata tgactaattt ggggaaaatg 3900

aagttttgat ttatttgtgt ttaaatgctg ctgtcagacg attgttctta gacctcctaa 3960

atgccccata ttaaaagaac tcattcatag gaaggtgttt cattttggtg tgcaaccctg 4020

tcattacgtc aacgcaacgt ctaactggac ttcccaagat aaatggtacc agcgtcctct 4080

taaaagatgc cttaatccat tccttgagga cagaccttag ttgaaatgat agcagaatgt 4140

gcttctctct ggcagctggc cttctgcttc tgagttgcac attaatcaga ttagcctgta 4200

ttctcttcag tgaattttga taatggcttc cagactcttt ggcgttggag acgcctgtta 4260

ggatcttcaa gtcccatcat agaaaattga aacacagagt tgttctgctg atagttttgg 4320

ggatacgtcc atctttttaa gggattgctt tcatctaatt ctggcaggac ctcaccaaaa 4380

gatccagcct catacctaca tcagacaaaa tatcgccgtt gttccttctg tactaaagta 4440

ttgtgttttg ctttggaaac acccactcac tttgcaatag ccgtgcaaga tgaatgcaga 4500

ttacactgat cttatgtgtt acaaaattgg agaaagtatt taataaaacc tgttaatttt 4560

tatactgaca ataaaaatgt ttctacagat attaatgtta acaagacaaa ataaatgtca 4620

cgcaacttat tttttaata aaaaaaaaa aaaa 4654

<210> 27
<211> 1398
<212> DNA
<213> Homo sapiens

<400> 27
ggagagcggg gccctttgtc ctccagtggc tggtaggcag tggctgggag gcagcggccc 60

aattagtgtc gtgcggcccg tggcgaggcg aggtccgggg agcgagcgag caagcaaggc 120

gggaggggtg gccggagctg cggcggctgg cacaggagga ggagcccggg cgggcgaggg 180

gcggccggag agcgccaggg cctgagctgc cggagcggcg cctgtgagtg agtgcagaaa 240

gcaggcgccc gcgcgctagc cgtggcagga gcagcccgca cgccgcgctc tctccctggg 300

cgacctgcag tttgcaatat gactttggag gaattctcgg ctggagagca gaagaccgaa 360

aggatggata aggtggggga tgccctggag gaagtgctca gcaaagccct gagtcagcgc 420

acgatcactg tcggggtgta cgaagcggcc aagctgctca acgtcgaccc cgataacgtg 480

gtgttgtgcc tgctggcggc ggacgaggac gacgacagag atgtggctct gcagatccac 540

ttcaccctga tccaggcgtt ttgctgcgag aacgacatca acatcctgcg cgtcagcaac 600

ccgggccggc tggcggagct cctgctcttg gagaccgacg ctggccccgc ggcgagcgag 660

ggcgccgagc agcccccgga cctgcactgc gtgctggtga cgaatccaca ttcatctcaa 720

tggaaggatc ctgccttaag tcaacttatt tgtttttgcc gggaaagtcg ctacatggat 780

caatgggttc cagtgattaa tctccctgaa cggtgatggc atctgaatga aaataactga 840

accaaattgc actgaagttt ttgaaatacc tttgtagtta ctcaagcagt tactccctac 900

actgatgcaa ggattacaga aactgatgcc aaggggctga gtgagttcaa ctacatgttc 960

tgggggcccg gagatagatg actttgcaga tggaaagagg tgaaaatgaa gaaggaagct 1020

gtgttgaaac agaaaaataa gtcaaaagga acaaaaatta caaagaacca tgcaggaagg 1080

aaaactatgt attaatttag aatggttgag ttacattaaa ataaaccaaa tatgttaaag 1140

tttaagtgtg cagccatagt ttgggtattt ttggtttata tgccctcaag taaaagaaaa 1200

gccgaaaggg ttaatcatat ttgaaaacca tattttattg tattttgatg agatattaaa 1260

ttctcaaagt tttattataa attctactaa gttattttat gacatgaaaa gttatttatg 1320

ctataaattt tttgaaacac aatacctaca ataaactggt atgaataatt gcatcatttc 1380

aaaaaaaaaa aaaaaaaa 1398

<210> 28
<211> 11242
<212> DNA
<213> Homo sapiens

<400> 28
   tttttttttt ttttttttga gaaaggggaa tttcatccca aataaaagga atgaagtctg 60
   gctccggagg agggtccccg acctcgctgt gggggctcct gtttctctcc gccgcgctct 120
   cgctctggcc gacgagtgga gaaatctgcg ggccaggcat cgacatccgc aacgactatc 180
   agcagctgaa gcgcctggag aactgcacgg tgatcgaggg ctacctccac atcctgctca 240
   tctccaaggc cgaggactac cgcagctacc gcttccccaa gctcacggtc attaccgagt 300
   acttgctgct gttccgagtg gctggcctcg agagcctcgg agacctcttc cccaacctca 360
   cggtcatccg cggctggaaa ctcttctaca actacgccct ggtcatcttc gagatgacca 420
   atctcaagga tattgggctt tacaacctga ggaacattac tcggggggcc atcaggattg 480
   agaaaaatgc tgacctctgt tacctctcca ctgtggactg gtccctgatc ctggatgcgg 540
   tgtccaataa ctacattgtg gggaataagc ccccaaagga atgtggggac ctgtgtccag 600
   ggaccatgga ggagaagccg atgtgtgaga agaccaccat caacaatgag tacaactacc 660
   gctgctggac cacaaaccgc tgccagaaaa tgtgcccaag cacgtgtggg aagcgggcgt 720
   gcaccgagaa caatgagtgc tgccaccccg agtgcctggg cagctgcagc gcgcctgaca 780
   acgacacggc ctgtgtagct tgccgccact actactatgc cggtgtctgt gtgcctgcct 840
   gcccgcccaa cacctacagg tttgagggct ggcgctgtgt ggaccgtgac ttctgcgcca 900
   acatcctcag cgccgagagc agcgactccg aggggtttgt gatccacgac ggcgagtgca 960
   tgcaggagtg cccctcgggc ttcatccgca acggcagcca gagcatgtac tgcatccctt 1020
   gtgaaggtcc ttgcccgaag gtctgtgagg aagaaaagaa aacaaagacc attgattctg 1080
   ttacttctgc tcagatgctc caaggatgca ccatcttcaa gggcaatttg ctcattaaca 1140
   tccgacgggg gaataacatt gcttcagagc tggagaactt catggggctc atcgaggtgg 1200
   tgacgggcta cgtgaagatc cgccattctc atgccttggt ctccttgtcc ttcctaaaaa 1260
   accttcgcct catcctagga gaggagcagc tagaagggaa ttactccttc tacgtcctcg 1320
   acaaccagaa cttgcagcaa ctgtgggact gggaccaccg caacctgacc atcaaagcag 1380
   ggaaaatgta ctttgctttc aatcccaaat tatgtgtttc cgaaatttac cgcatggagg 1440
   aagtgacggg gactaaaggg cgccaaagca aaggggacat aaacaccagg aacaacgggg 1500
   agagagcctc ctgtgaaagt gacgtcctgc atttcacctc caccaccacg tcgaagaatc 1560
   gcatcatcat aacctggcac cggtaccggc cccctgacta cagggatctc atcagcttca 1620
   ccgtttacta caaggaagca ccctttaaga atgtcacaga gtatgatggg caggatgcct 1680
   gcggctccaa cagctggaac atggtggacg tggacctccc gcccaacaag gacgtggagc 1740
   ccggcatctt actacatggg ctgaagccct ggactcagta cgccgtttac gtcaaggctg 1800
   tgaccctcac catggtggag aacgaccata tccgtggggc caagagtgag atcttgtaca 1860
   ttcgcaccaa tgcttcagtt ccttccattc ccttggacgt tctttcagca tcgaactcct 1920
   cttctcagtt aatcgtgaag tggaaccctc cctctctgcc caacggcaac ctgagttact 1980
   acattgtgcg ctggcagcgg cagcctcagg acggctacct ttaccggcac aattactgct 2040
   ccaaagacaa aatccccatc aggaagtatg ccgacggcac catcgacatt gaggaggtca 2100
   cagagaaccc caagactgag gtgtgtggtg gggagaaagg gccttgctgc gcctgcccca 2160
   aaactgaagc cgagaagcag gccgagaagg aggaggctga ataccgcaaa gtctttgaga 2220
   atttcctgca caactccatc ttcgtgccca gacctgaaag gaagcggaga gatgtcatgc 2280
   aagtggccaa caccaccatg tccagccgaa gcaggaacac cacggccgca gacacctaca 2340
   acatcaccga cccggaagag ctggagacag agtacccttt ctttgagagc agagtggata 2400
   acaaggagag aactgtcatt tctaaccttc ggcctttcac attgtaccgc atcgatatcc 2460
   acagctgcaa ccacgaggct gagaagctgg gctgcagcgc ctccaacttc gtctttgcaa 2520
   ggactatgcc cgcagaagga gcagatgaca ttcctgggcc agtgacctgg gagccaaggc 2580
   ctgaaaactc catcttttta aagtggccgg aacctgagaa tcccaatgga ttgattctaa 2640
   tgtatgaaat aaaatacgga tcacaagttg aggatcagcg agaatgtgtg tccagacagg 2700
   aatacaggaa gtatggaggg gccaagctaa accggctaaa cccggggaac tacacagccc 2760
   ggattcaggc cacatctctc tctgggaatg ggtcgtggac agatcctgtg ttcttctatg 2820
   tccaggccaa aacaggatat gaaaacttca tccatctgat catcgctctg cccgtcgctg 2880
   tcctgttgat cgtgggaggg ttggtgatta tgctgtacgt cttccataga aagagaaata 2940
   acagcaggct ggggaatgga gtgctgtatg cctctgtgaa cccggagtac ttcagcgctg 3000
   ctgatgtgta cgttcctgat gagtgggagg tggctcggga gaagatcacc atgagccggg 3060
   aacttgggca ggggtcgttt gggatggtct atgaaggagt tgccaagggt gtggtgaaag 3120
   atgaacctga aaccagagtg gccattaaaa cagtgaacga ggccgcaagc atgcgtgaga 3180
   ggattgagtt tctcaacgaa gcttctgtga tgaaggagtt caattgtcac catgtggtgc 3240
   gattgctggg tgtggtgtcc caaggccagc caacactggt catcatggaa ctgatgacac 3300
   ggggcgatct caaaagttat ctccggtctc tgaggccaga aatggagaat aatccagtcc 3360
   tagcacctcc aagcctgagc aagatgattc agatggccgg agagattgca gacggcatgg 3420
   catacctcaa cgccaataag ttcgtccaca gagaccttgc tgcccggaat tgcatggtag 3480
   ccgaagattt cacagtcaaa atcggagatt ttggtatgac gcgagatatc tatgagacag 3540
   actattaccg gaaaggaggg aaagggctgc tgcccgtgcg ctggatgtct cctgagtccc 3600
   tcaaggatgg agtcttcacc acttactcgg acgtctggtc cttcggggtc gtcctctggg 3660
   agatcgccac actggccgag cagccctacc agggcttgtc caacgagcaa gtccttcgct 3720
   tcgtcatgga gggcggcctt ctggacaagc cagacaactg tcctgacatg ctgtttgaac 3780
   tgatgcgcat gtgctggcag tataacccca agatgaggcc ttccttcctg gagatcatca 3840
   gcagcatcaa agaggagatg gagcctggct tccgggaggt ctccttctac tacagcgagg 3900
   agaacaagct gcccgagccg gaggagctgg acctggagcc agagaacatg gagagcgtcc 3960
   ccctggaccc ctcggcctcc tcgtcctccc tgccactgcc cgacagacac tcaggacaca 4020
   aggccgagaa cggccccggc cctggggtgc tggtcctccg cgccagcttc gacgagagac 4080
   agccttacgc ccacatgaac gggggccgca agaacgagcg ggccttgccg ctgccccagt 4140
   cttcgacctg ctgatccttg gatcctgaat ctgtgcaaac agtaacgtgt gcgcacgcgc 4200
   agcggggtgg ggggggagag agagttttaa caatccattc acaagcctcc tgtacctcag 4260
   tggatcttca gaactgccct tgctgcccgc gggagacagc ttctctgcag taaaacacat 4320
   ttgggatgtt ccttttttca atatgcaagc agctttttat tccctgccca aacccttaac 4380
   tgacatgggc ctttaagaac cttaatgaca acacttaata gcaacagagc acttgagaac 4440
   cagtctcctc actctgtccc tgtccttccc tgttctccct ttctctctcc tctctgcttc 4500
   ataacggaaa aataattgcc acaagtccag ctgggaagcc ctttttatca gtttgaggaa 4560
   gtggctgtcc ctgtggcccc atccaaccac tgtacacacc cgcctgacac cgtgggtcat 4620
   tacaaaaaaa cacgtggaga tggaaatttt tacctttatc tttcaccttt ctagggacat 4680
   gaaatttaca aagggccatc gttcatccaa ggctgttacc attttaacgc tgcctaattt 4740
   tgccaaaatc ctgaactttc tccctcatcg gcccggcgct gattcctcgt gtccggaggc 4800
   atgggtgagc atggcagctg gttgctccat ttgagagaca cgctggcgac acactccgtc 4860
   catccgactg cccctgctgt gctgctcaag gccacaggca cacaggtctc attgcttctg 4920
   actagattat tatttggggg aactggacac aataggtctt tctctcagtg aaggtgggga 4980
   gaagctgaac cggcttccct gccctgcctc cccagccccc tgcccaaccc ccaagaatct 5040
   ggtggccatg ggccccgaag cagcctggcg gacaggcttg gagtcaaggg gccccatgcc 5100
   tgcttctctc ccagccccag ctcccccgcc cgcccccaag gacacagatg ggaaggggtt 5160
   tccagggact cagccccact gttgatgcag gtttgcaagg aaagaaattc aaacaccaca 5220
   acagcagtaa gaagaaaagc agtcaatgga ttcaagcatt ctaagctttg ttgacatttt 5280
   ctctgttcct aggacttctt catgggtctt acagttctat gttagaccat gaaacatttg 5340
   catacacatc gtctttaatg tcacttttat aactttttta cggttcagat attcatctat 5400
   acgtctgtac agaaaaaaaa aagctgctat tttttttgtt cttgatcttt gtggatttaa 5460
   tctatgaaaa ccttcaggtc caccctctcc cctttctgct cactccaaga aacttcttat 5520
   gctttgtact agagtgcgtg actttcttcc tcttttcccg gtaatggata cttctatcac 5580
   ataatttgcc atgaactgtt ggatgccttt ttataaatac atcccccatc cctgctccca 5640
   cctgcccctt tagttgtttt ctaacccgta ggctctctgg gcacgaggca gaaagcaggc 5700
   cgggcaccca tcctgagagg gccgcgctcc tctccccagc ctgccctcac agcattggag 5760
   cctgttacag tgcaagacat gatacaaact caggtcagaa aaacaaaggt taaatatttc 5820
   acacgtcttt gttcagtgtt tccactcacc gtggttgaga agcctcaccc tctctttccc 5880
   ttgcctttgc ttaggttgtg acacacatat atatatattt ttttaattct tgggtacaac 5940
   agcagtgtta accgcagaca ctaggcattt ggattactat ttttcttaat ggctatttaa 6000
   tccttccatc ccacgaaaaa cagctgctga gtccaaggga gcagcagagc gtggtccggc 6060
   agggcctgtt gtggccctcg ccacccccct caccggaccg actgacctgt ctttggaacc 6120
   agaacatccc aagggaactc cttcgcactg gcgttgagtg ggaccccggg atccaggctg 6180
   gcccagggcg gcaccctcag ggctgtgccc gctggagtgc taggtggagg cagcacagac 6240
   gccacggtgg cccaagagcc cctttgcttc ttgctggggg accagggctg tggtgctggc 6300
   ccactttccc tcggccagga atccaggtcc ttggggccca ggggtcttgt cttgtttcat 6360
   ttttagcact tctcaccaga gagatgacag cacaagagtt gcttctggga tagaaatgtt 6420
   taggagtaag aacaaagctg ggatacggtg attgctagtt gtgactgaag attcaacaca 6480
   gaaaagaaag tttatacggc ttttttgctg gtcagcagtt tgtcccactg ctttctctag 6540
   tctctatccc atagcgtgtt ccctttaaaa aaaaaaaaaa ggtattatat gtaggagttt 6600
   tcttttaatt tattttgtga taaattacca gtttcaatca ctgtagaaaa gccccattat 6660
   gaatttaaat ttcaaggaaa gggtgtgtgt gtgtgtatgt gtggggtgtg tgtgtgtgag 6720
   agtgatggga cagttcttga ttttttgggt tttttttccc ccaaacattt atctacctca 6780
   ctcttatttt ttatatgtgt atatagacaa aagaatacat ctcacctttc tcagcacctg 6840
   acaataggcc gttgatactg gtaacctcat ccacgccaca ggcgccacac ccaggtgatg 6900
   cagggggaag ccaggctgta ttccggggtc aaagcaacac taactcacct ctctgctcat 6960
   ttcagacagc ttgccttttt ctgagatgtc ctgttttgtg ttgctttttt tgttttgttt 7020
   tctatcttgg tttccaccaa ggtgttagat ttctcctcct cctagccagg tggccctgtg 7080
   aggccaacga gggcaccaga gcacacctgg gggagccacc aggctgtccc tggctggttg 7140
   tctttggaac aaactgcttc tgtgcagatg gaatgaccaa cacatttcgt ccttaagaga 7200
   gcagtggttc ctcaggttct gaggagagga aggtgtccag gcagcaccat ctctgtgcga 7260
   atccccaggg taaaggcgtg gggcattggg tttgctcccc ttgctgctgc tccatccctg 7320
   caggaggctc gcgctgaggc aggaccgtgc ggccatggct gctgcattca ttgagcacaa 7380
   aggtgcagct gcagcagcag ctggagagca agagtcaccc agcctgtgcg ccagaatgca 7440
   gaggctcctg acctcacagc cagtccctga tagaacacac gcaggagcag agtcccctcc 7500
   ccctccaggc tgccctctca acttctccct cacctccttc cctaggggta gacagagatg 7560
   taccaaacct tccggctgga aagcccagtg gccggcgccg aggctcgtgg cgtcacgccc 7620
   cccccgccag ggctgtacct ccgtctccct ggtcctgctg ctcacaggac agacggctcg 7680
   ctcccctctt ccagcagctg ctcttacagg cactgatgat ttcgctggga agtgtggcgg 7740
   gcagctttgc ctaagcgtgg atggctcctc ggcaattcca gcctaagtga aggcgctcag 7800
   gagcctcctg ctggaacgcg acccatctct cccaggaccc cggggatctt aaggtcattg 7860
   agaaatactg ttggatcagg gttttgttct tccacactgt aggtgacccc ttggaataac 7920
   ggcctctcct ctcgtgcaca tacctaccgg tttccacaac tggatttcta cagatcattc 7980
   agctggttat aagggttttg tttaaactgt ccgagttact gatgtcattt tgtttttgtt 8040
   ttatgtaggt agcttttaag tagaaaacac taacagtgta gtgcccatca tagcaaatgc 8100
   ttcagaaaca cctcaataaa agagaaaact tggcttgtgt gatggtgcag tcactttact 8160
   ggaccaaccc acccaccttg actataccaa ggcatcatct atccacagtt ctagcctaac 8220
   ttcatgctga tttctctgcc tcttgatttt tctctgtgtg ttccaaataa tcttaagctg 8280
   agttgtggca ttttccatgc aacctccttc tgccagcagc tcacactgct tgaagtcata 8340
   tgaaccactg aggcacatca tggaattgat gtgagcatta agacgttctc ccacacagcc 8400
   cttccctgag gcagcaggag ctggtgtgta ctggagacac tgttgaactt gatcaagacc 8460
   cagaccaccc caggtctcct tcgtgggatg tcatgacgtt tgacatacct ttggaacgag 8520
   cctcctcctt ggaagatgga agaccgtgtt cgtggccgac ctggcctctc ctggcctgtt 8580
   tcttaagatg cggagtcaca tttcaatggt acgaaaagtg gcttcgtaaa atagaagagc 8640
   agtcactgtg gaactaccaa atggcgagat gctcggtgca cattggggtg ctttgggata 8700
   aaagatttat gagccaacta ttctctggca ccagattcta ggccagtttg ttccactgaa 8760
   gcttttccca cagcagtcca cctctgcagg ctggcagccg aatggcttgc cagtggctct 8820
   gtggcaagat cacactgaga tcgatgggtg agaaggctag gatgcttgtc tagtgttctt 8880
   agctgtcacg ttggctcctt ccagggtggc cagacggtgt tggccactcc cttctaaaac 8940
   acaggcgccc tcctggtgac agtgacccgc cgtggtatgc cttggcccat tccagcagtc 9000
   ccagttatgc atttcaagtt tggggtttgt tcttttcgtt aatgttcctc tgtgttgtca 9060
   gctgtcttca tttcctgggc taagcagcat tgggagatgt ggaccagaga tccactcctt 9120
   aagaaccagt ggcgaaagac actttctttc ttcactctga agtagctggt ggtacaaatg 9180
   agaacttcaa gagaggatgt tatttagact gaacctctgt tgccagagat gctgaagata 9240
   cagaccttgg acaggtcaga gggtttcatt tttggccttc atcttagatg actggttgcg 9300
   tcatttggag aagtgagtgc tccttgatgg tggaatgacc gggtggtggg tacagaacca 9360
   ttgtcacagg gatcctggca cagagaagag ttacgagcag cagggtgcag ggcttggaag 9420
   gaatgtgggc aaggttttga acttgattgt tcttgaagct atcagaccac atcgaggctc 9480
   agcagtcatc cgtgggcatt tggtttcaac aaagaaacct aacatcctac tctggaaact 9540
   gatctcggag ttaaggcgaa ttgttcaaga acacaaacta catcgcactc gtcagttgtc 9600
   agttctgggg catgacttta gcgttttgtt tctgcgagaa cataacgatc actcattttt 9660
   atgtcccacg tgtgtgtgtc cgcatctttc tggtcaacat tgttttaact agtcactcat 9720
   tagcgttttc aatagggctc ttaagtccag tagattacgg gtagtcagtt gacgaagatc 9780
   tggtttacaa gaactaatta aatgtttcat tgcatttttg taagaacaga ataattttat 9840
   aaaatgtttg tagtttataa ttgccgaaaa taatttaaag acactttttt tttctctgtg 9900
   tgtgcaaatg tgtgtttgtg atccattttt tttttttttt tttaggacac ctgtttacta 9960
   gctagcttta caatatgcca aaaaaggatt tctccctgac cccatccgtg gttcaccctc 10020
   ttttcccccc atgctttttg ccctagttta taacaaagga atgatgatga tttaaaaagt 10080
   agttctgtat cttcagtatc ttggtcttcc agaaccctct ggttgggaag gggatcattt 10140
   tttactggtc atttcccttt ggagtgtagc tactttaaca gatggaaaga acctcattgg 10200
   ccatggaaac agccgaggtg ttggagccca gcagtgcatg gcaccgttcg gcatctggct 10260
   tgattggtct ggctgccgtc attgtcagca cagtgccatg gacatgggaa gacttgactg 10320
   cacagccaat ggttttcatg atgattacag catacacagt gatcacataa acgatgacag 10380
   ctatggggca cacaggccat ttgcttacat gcctcgtatc atgactgatt actgctttgt 10440
   tagaacacag aagagaccct attttattta aggcagaacc ccgaagatac gtatttccaa 10500
   tacagaaaag aatttttaat aaaaactata acatacacaa aaattggttt taaagttgac 10560
   tccacttcct ctaactccag tggattgttg gccatgtctc cccaactcca caatatctct 10620
   atcatgggaa acacctgggg tttttgcgct acataggaga aagatctgga aactatttgg 10680
   gttttgtttt caacttttca tttggatgtt tggcgttgca cacacacatc caccggtgga 10740
   agagacgccc ggtgaaaaca cctgtctgct ttctaagcca gtgaggttga ggtgagaggt 10800
   ttgccagagt ttgtctacct ctgggtatcc ctttgtctgg gataaaaaaa atcaaaccag 10860
   aaggcgggat ggaatggatg caccgcaaat aatgcatttt ctgagttttc ttgttaaaaa 10920
   aaaatttttt taagtaagaa aaaaaaaggt aataacatgg ccaatttgtt acataaaatg 10980
   actttctgtg tataaattat tcctaaaaaa tcctgtttat ataaaaaatc agtagatgaa 11040
   aaaaatttca aaatgttttt gtatattctg ttgtaagaat ttattcctgt tattgcgata 11100
   tactctggat tctttacata atggaaaaaa gaaactgtct attttgaatg gctgaagcta 11160
   aggcaacgtt agtttctctt actctgcttt tttctagtaa agtactacat ggtttaagtt 11220
   aaataaaata attctgtatg ca 11242

<210> 29
<211> 1660
<212> DNA
<213> Homo sapiens

<400> 29
ggtgcactag caaaacaaac ttattttgaa cactcagctc ctagcgtgcg gcgctgccaa 60

tcattaacct cctggtgcaa gtggcgcggc ctgtgccctt tataaggtgc gcgctgtgtc 120

cagcgagcat cggccaccgc catcccatcc agcgagcatc tgccgccgcg ccgccgccac 180

cctcccagag agcactggcc accgctccac catcacttgc ccagagtttg ggccaccgcc 240

cgccgccacc agcccagaga gcatcggccc ctgtctgctg ctcgcgcctg gagatgtcag 300

aggtccccgt tgctcgcgtc tggctggtac tgctcctgct gactgtccag gtcggcgtga 360

cagccggcgc tccgtggcag tgcgcgccct gctccgccga gaagctcgcg ctctgcccgc 420

cggtgtccgc ctcgtgctcg gaggtcaccc ggtccgccgg ctgcggctgt tgcccgatgt 480

gcgccctgcc tctgggcgcc gcgtgcggcg tggcgactgc acgctgcgcc cggggactca 540

gttgccgcgc gctgccgggg gagcagcaac ctctgcacgc cctcacccgc ggccaaggcg 600

cctgcgtgca ggagtctgac gcctccgctc cccatgctgc agaggcaggg agccctgaaa 660

gcccagagag cacggagata actgaggagg agctcctgga taatttccat ctgatggccc 720

cttctgaaga ggatcattcc atcctttggg acgccatcag tacctatgat ggctcgaagg 780

ctctccatgt caccaacatc aaaaaatgga aggagccctg ccgaatagaa ctctacagag 840

tcgtagagag tttagccaag gcacaggaga catcaggaga agaaatttcc aaattttacc 900

tgccaaactg caacaagaat ggattttatc acagcagaca gtgtgagaca tccatggatg 960

gagaggcggg actctgctgg tgcgtctacc cttggaatgg gaagaggatc cctgggtctc 1020

cagagatcag gggagacccc aactgccaga tatattttaa tgtacaaaac tgaaaccaga 1080

tgaaataatg ttctgtcacg tgaaatattt aagtatatag tatatttata ctctagaaca 1140

tgcacattta tatatatatg tatatgtata tatatatagt aactactttt tatactccat 1200

acataacttg atatagaaag ctgtttattt attcactgta agtttatttt ttctacacag 1260

taaaaacttg tactatgtta ataacttgtc ctatgtcaat ttgtatatca tgaaacactt 1320

ctcatcatat tgtatgtaag taattgcatt tctgctcttc caaagctcct gcgtctgttt 1380

ttaaagagca tggaaaaata ctgcctagaa aatgcaaaat gaaataagag agagtagttt 1440

ttcagctagt ttgaaggagg acggttaact tgtatattcc accattcaca tttgatgtac 1500

atgtgtaggg aaagttaaaa gtgttgatta cataatcaaa gctacctgtg gtgatgttgc 1560

cacctgttaa aatgtacact ggatatgttg ttaaacacgt gtctataatg gaaacattta 1620

caataaatat tctgcatgga aatactgtta aaaaaaaaaa 1660

<210> 30
<211> 2638
<212> DNA
<213> Homo sapiens

<400> 30
agatgcgagc actgcggctg ggcgctgagg atcagccgct tcctgcctgg attccacagc 60

ttcgcgccgt gtactgtcgc cccatccctg cgcgcccagc ctgccaagca gcgtgccccg 120

gttgcaggcg tcatgcagcg ggcgcgaccc acgctctggg ccgctgcgct gactctgctg 180

gtgctgctcc gcgggccgcc ggtggcgcgg gctggcgcga gctcggcggg cttgggtccc 240

gtggtgcgct gcgagccgtg cgacgcgcgt gcactggccc agtgcgcgcc tccgcccgcc 300

gtgtgcgcgg agctggtgcg cgagccgggc tgcggctgct gcctgacgtg cgcactgagc 360

gagggccagc cgtgcggcat ctacaccgag cgctgtggct ccggccttcg ctgccagccg 420

tcgcccgacg aggcgcgacc gctgcaggcg ctgctggacg gccgcgggct ctgcgtcaac 480

gctagtgccg tcagccgcct gcgcgcctac ctgctgccag cgccgccagc tccaggtgag 540

ccgcccgcgc caggaaatgc tagtgagtcg gaggaagacc gcagcgccgg cagtgtggag 600

agcccgtccg tctccagcac gcaccgggtg tctgatccca agttccaccc cctccattca 660

aagataatca tcatcaagaa agggcatgct aaagacagcc agcgctacaa agttgactac 720

gagtctcaga gcacagatac ccagaacttc tcctccgagt ccaagcggga gacagaatat 780

ggtccctgcc gtagagaaat ggaagacaca ctgaatcacc tgaagttcct caatgtgctg 840

agtcccaggg gtgtacacat tcccaactgt gacaagaagg gattttataa gaaaaagcag 900

tgtcgccctt ccaaaggcag gaagcggggc ttctgctggt gtgtggataa gtatgggcag 960

cctctcccag gctacaccac caaggggaag gaggacgtgc actgctacag catgcagagc 1020

aagtagacgc ctgccgcaag gttaatgtgg agctcaaata tgccttattt tgcacaaaag 1080

actgccaagg acatgaccag cagctggcta cagcctcgat ttatatttct gtttgtggtg 1140

aactgatttt ttttaaacca aagtttagaa agaggttttt gaaatgccta tggtttcttt 1200

gaatggtaaa cttgagcatc ttttcacttt ccagtagtca gcaaagagca gtttgaattt 1260

tcttgtcgct tcctatcaaa atattcagag actcgagcac agcacccaga cttcatgcgc 1320

ccgtggaatg ctcaccacat gttggtcgaa gcggccgacc actgactttg tgacttaggc 1380

ggctgtgttg cctatgtaga gaacacgctt cacccccact ccccgtacag tgcgcacagg 1440

ctttatcgag aataggaaaa cctttaaacc ccggtcatcc ggacatccca acgcatgctc 1500

ctggagctca cagccttctg tggtgtcatt tctgaaacaa gggcgtggat ccctcaacca 1560

agaagaatgt ttatgtcttc aagtgacctg tactgcttgg ggactattgg agaaaataag 1620

gtggagtcct acttgtttaa aaaatatgta tctaagaatg ttctagggca ctctgggaac 1680

ctataaaggc aggtatttcg ggccctcctc ttcaggaatc ttcctgaaga catggcccag 1740

tcgaaggccc aggatggctt ttgctgcggc cccgtggggt aggagggaca gagagacagg 1800

gagagtcagc ctccacattc agaggcatca caagtaatgg cacaattctt cggatgactg 1860

cagaaaatag tgttttgtag ttcaacaact caagacgaag cttatttctg aggataagct 1920

ctttaaaggc aaagctttat tttcatctct catcttttgt cctccttagc acaatgtaaa 1980

aaagaatagt aatatcagaa caggaaggag gaatggcttg ctggggagcc catccaggac 2040

actgggagca catagagatt cacccatgtt tgttgaactt agagtcattc tcatgctttt 2100

ctttataatt cacacatata tgcagagaag atatgttctt gttaacattg tatacaacat 2160

agccccaaat atagtaagat ctatactaga taatcctaga tgaaatgtta gagatgctat 2220

atgatacaac tgtggccatg actgaggaaa ggagctcacg cccagagact gggctgctct 2280

cccggaggcc aaacccaaga aggtctggca aagtcaggct cagggagact ctgccctgct 2340

gcagacctcg gtgtggacac acgctgcata gagctctcct tgaaaacaga ggggtctcaa 2400

gacattctgc ctacctatta gcttttcttt atttttttaa ctttttgggg ggaaaagtat 2460

ttttgagaag tttgtcttgc aatgtattta taaatagtaa ataaagtttt taccattaaa 2520

aaaatatctt tccctttgtt attgaccatc tctgggcttt gtatcactaa ttattttatt 2580

ttattatata ataattattt tattataata aaatcctgaa aggggaaaat aaaaaaaa 2638

<210> 31
<211> 4723
<212> DNA
<213> Homo sapiens

<400> 31
ggggggctgc gcggccgggt cggtgcgcac acgagaagga cgcgcggccc ccagcgctct 60

tgggggccgc ctcggagcat gacccccgcg ggccagcgcc gcgcgcctga tccgaggaga 120

ccccgcgctc ccgcagccat gggcaccggg ggccggcggg gggcggcggc cgcgccgctg 180

ctggtggcgg tggccgcgct gctactgggc gccgcgggcc acctgtaccc cggagaggtg 240

tgtcccggca tggatatccg gaacaacctc actaggttgc atgagctgga gaattgctct 300

gtcatcgaag gacacttgca gatactcttg atgttcaaaa cgaggcccga agatttccga 360

gacctcagtt tccccaaact catcatgatc actgattact tgctgctctt ccgggtctat 420

gggctcgaga gcctgaagga cctgttcccc aacctcacgg tcatccgggg atcacgactg 480

ttctttaact acgcgctggt catcttcgag atggttcacc tcaaggaact cggcctctac 540

aacctgatga acatcacccg gggttctgtc cgcatcgaga agaacaatga gctctgttac 600

ttggccacta tcgactggtc ccgtatcctg gattccgtgg aggataatca catcgtgttg 660

aacaaagatg acaacgagga gtgtggagac atctgtccgg gtaccgcgaa gggcaagacc 720

aactgccccg ccaccgtcat caacgggcag tttgtcgaac gatgttggac tcatagtcac 780

tgccagaaag tttgcccgac catctgtaag tcacacggct gcaccgccga aggcctctgt 840

tgccacagcg agtgcctggg caactgttct cagcccgacg accccaccaa gtgcgtggcc 900

tgccgcaact tctacctgga cggcaggtgt gtggagacct gcccgccccc gtactaccac 960

ttccaggact ggcgctgtgt gaacttcagc ttctgccagg acctgcacca caaatgcaag 1020

aactcgcgga ggcagggctg ccaccaatac gtcattcaca acaacaagtg catccctgag 1080

tgtccctccg ggtacacgat gaattccagc aacttgctgt gcaccccatg cctgggtccc 1140

tgtcccaagg tgtgccacct cctagaaggc gagaagacca tcgactcggt gacgtctgcc 1200

caggagctcc gaggatgcac cgtcatcaac gggagtctga tcatcaacat tcgaggaggc 1260

aacaatctgg cagctgagct agaagccaac ctcggcctca ttgaagaaat ttcagggtat 1320

ctaaaaatcc gccgatccta cgctctggtg tcactttcct tcttccggaa gttacgtctg 1380

attcgaggag agaccttgga aattgggaac tactccttct atgccttgga caaccagaac 1440

ctaaggcagc tctgggactg gagcaaacac aacctcacca ccactcaggg gaaactcttc 1500

ttccactata accccaaact ctgcttgtca gaaatccaca agatggaaga agtttcagga 1560

accaaggggc gccaggagag aaacgacatt gccctgaaga ccaatgggga caaggcatcc 1620

tgtgaaaatg agttacttaa attttcttac attcggacat cttttgacaa gatcttgctg 1680

agatgggagc cgtactggcc ccccgacttc cgagacctct tggggttcat gctgttctac 1740

aaagaggccc cttatcagaa tgtgacggag ttcgatgggc aggatgcgtg tggttccaac 1800

agttggacgg tggtagacat tgacccaccc ctgaggtcca acgaccccaa atcacagaac 1860

cacccagggt ggctgatgcg gggtctcaag ccctggaccc agtatgccat ctttgtgaag 1920

accctggtca ccttttcgga tgaacgccgg acctatgggg ccaagagtga catcatttat 1980

gtccagacag atgccaccaa cccctctgtg cccctggatc caatctcagt gtctaactca 2040

tcatcccaga ttattctgaa gtggaaacca ccctccgacc ccaatggcaa catcacccac 2100

tacctggttt tctgggagag gcaggcggaa gacagtgagc tgttcgagct ggattattgc 2160

ctcaaagggc tgaagctgcc ctcgaggacc tggtctccac cattcgagtc tgaagattct 2220

cagaagcaca accagagtga gtatgaggat tcggccggcg aatgctgctc ctgtccaaag 2280

acagactctc agatcctgaa ggagctggag gagtcctcgt ttaggaagac gtttgaggat 2340

tacctgcaca acgtggtttt cgtccccaga aaaacctctt caggcactgg tgccgaggac 2400

cctaggccat ctcggaaacg caggtccctt ggcgatgttg ggaatgtgac ggtggccgtg 2460

cccacggtgg cagctttccc caacacttcc tcgaccagcg tgcccacgag tccggaggag 2520

cacaggcctt ttgagaaggt ggtgaacaag gagtcgctgg tcatctccgg cttgcgacac 2580

ttcacgggct atcgcatcga gctgcaggct tgcaaccagg acacccctga ggaacggtgc 2640

agtgtggcag cctacgtcag tgcgaggacc atgcctgaag ccaaggctga tgacattgtt 2700

ggccctgtga cgcatgaaat ctttgagaac aacgtcgtcc acttgatgtg gcaggagccg 2760

aaggagccca atggtctgat cgtgctgtat gaagtgagtt atcggcgata tggtgatgag 2820

gagctgcatc tctgcgtctc ccgcaagcac ttcgctctgg aacggggctg caggctgcgt 2880

gggctgtcac cggggaacta cagcgtgcga atccgggcca cctcccttgc gggcaacggc 2940

tcttggacgg aacccaccta tttctacgtg acagactatt tagacgtccc gtcaaatatt 3000

gcaaaaatta tcatcggccc cctcatcttt gtctttctct tcagtgttgt gattggaagt 3060

atttatctat tcctgagaaa gaggcagcca gatgggccgc tgggaccgct ttacgcttct 3120

tcaaaccctg agtatctcag tgccagtgat gtgtttccat gctctgtgta cgtgccggac 3180

gagtgggagg tgtctcgaga gaagatcacc ctccttcgag agctggggca gggctccttc 3240

ggcatggtgt atgagggcaa tgccagggac atcatcaagg gtgaggcaga gacccgcgtg 3300

gcggtgaaga cggtcaacga gtcagccagt ctccgagagc ggattgagtt cctcaatgag 3360

gcctcggtca tgaagggctt cacctgccat cacgtggtgc gcctcctggg agtggtgtcc 3420

aagggccagc ccacgctggt ggtgatggag ctgatggctc acggagacct gaagagctac 3480

ctccgttctc tgcggccaga ggctgagaat aatcctggcc gccctccccc tacccttcaa 3540

gagatgattc agatggcggc agagattgct gacgggatgg cctacctgaa cgccaagaag 3600

tttgtgcatc gggacctggc agcgagaaac tgcatggtcg cccatgattt tactgtcaaa 3660

attggagact ttggaatgac cagagacatc tatgaaacgg attactaccg gaaagggggc 3720

aagggtctgc tccctgtacg gtggatggca ccggagtccc tgaaggatgg ggtcttcacc 3780

acttcttctg acatgtggtc ctttggcgtg gtcctttggg aaatcaccag cttggcagaa 3840

cagccttacc aaggcctgtc taatgaacag gtgttgaaat ttgtcatgga tggagggtat 3900

ctggatcaac ccgacaactg tccagagaga gtcactgacc tcatgcgcat gtgctggcaa 3960

ttcaacccca agatgaggcc aaccttcctg gagattgtca acctgctcaa ggacgacctg 4020

caccccagct ttccagaggt gtcgttcttc cacagcgagg agaacaaggc tcccgagagt 4080

gaggagctgg agatggagtt tgaggacatg gagaatgtgc ccctggaccg ttcctcgcac 4140

tgtcagaggg aggaggcggg gggccgggat ggagggtcct cgctgggttt caagcggagc 4200

tacgaggaac acatccctta cacacacatg aacggaggca agaaaaacgg gcggattctg 4260

accttgcctc ggtccaatcc ttcctaacag tgcctaccgt ggcgggggcg ggcaggggtt 4320

cccattttcg ctttcctctg gtttgaaagc ctctggaaaa ctcaggattc tcacgactct 4380

accatgtcca gtggagttca gagatcgttc ctatacattt ctgttcatct taaggtggac 4440

tcgtttggtt accaatttaa ctagtcctgc agaggattta actgtgaacc tggagggcaa 4500

ggggtttcca cagttgctgc tcctttgggg caacgacggt ttcaaaccag gattttgtgt 4560

tttttcgttc cccccacccg cccccagcag atggaaagaa agcacctgtt tttacaaatt 4620

cttttttttt tttttttttt tttttttttg ctggtgtctg agcttcagta taaaagacaa 4680

aacttcctgt ttgtggaaca aaatttcgaa agaaaaaacc aaa 4723

<210> 32
<211> 3017
<212> DNA
<213> Homo sapiens

<400> 32
   ggatccctga gcgtcacgcc gctgttgtgg agcgcgtgtt gacaacgtcg ccggggagac 60
   gggcgggggc ggggcccggg agagggggag gcgcggccct ggcggcgcgc gaggggccgg 120
   gctgtcagcg caaggcccag gccgccgcag tggccacggc cgctgccgcc cgccggctta 180
   tataccgcgg ctaaatttag gctgcgcccg gagctcgtcc ccatccggga cgcgtttccg 240
   ccgccgccgc tttggcccgg cccccgcgcg cgccgcgcct ataaggcttg ggcgggcccg 300
   gccgcggccc acagagccgt ccccgcccgc ccgcgccccg accagcccgg cctcgggcag 360
   ccactcaccg gtgtccccgt ccgcgtcctt cctccccggg tcccggccat ggcgctgagt 420
   gaacccatcc tgccgtcctt ttccactttc gccagcccgt gccgcgagcg cggcctgcag 480
   gaggtgaggg cggcggggac ggcggggcga ccgggaccgt ggcggcgggg ctcggggtag 540
   tagaacgtgg gctgcggggt gacaggacgc gaaggcgggg actgcagact caggagagga 600
   ggatgcgggc cacggggatc gcggacttag ggtggtaaaa ggcaagcagc gccccccgag 660
   ccccgccgcc cgctcacgcc cattgccctg tcgcccgcag cgctggccgc gcgccgaacc 720
   cgagtccggc ggcaccgacg acgacctcaa cagcgtgctg gacttcatcc tgtccatggg 780
   gctggatggc ctgggcgccg aggccgcccc ggagccgccg ccgccgcccc cgccgcctgc 840
   gttctattac cccgaacccg gcgcgccccc gccctacagc gcccccgcgg gtggcctggt 900
   gtctgagctg ctgcgacccg agctggatgc gccgccgggg cccgcactgc acggccgctt 960
   tctgctggcg ccgcccggcc gcctggtcaa ggccgagccc cctgaagcgg acggcggcgg 1020
   cggctacggc tgcgcccccg ggctgacccg tggaccgcgc ggcctcaagc gcgagggcgc 1080
   cccaggcccg gcggcttcgt gcatgcgagg tcccgggggc cgccccccgc cgccgcccga 1140
   cacaccgccg ctcagccccg acggccccgc gcgcctgccc gcgcccggtc cgcgcgcctc 1200
   cttcccgccg cctttcggtg gccctggttt cggcgcgccc gggcccggcc tgcattacgc 1260
   gccgcctgcg cccccagcct tcggtctttt cgacgacgcg gccgccgccg cggcagccct 1320
   gggcctggcg ccccccgccg cccgcggtct cctcacgccg cctgcgtccc cgctggagct 1380
   gctggaggcc aagccaaagc gcggccgccg ctcttggccc cgcaaacgca ccgccactca 1440
   cacctgcagc tacgcgggct gcggcaagac ctacaccaag agttcgcatc tgaaggcgca 1500
   tctgcgcacg cacacaggtg ggcggcacgc acgagccagg agcgcaggcg gggggacgcg 1560
   ggaggagagg tcggattccc agcgcgcgcc agaaaatgaa tttaggacct cccttggggc 1620
   gtggctcagg gggatctggc agtggtgcac gcttaggact ccccaggagc gtggctcggg 1680
   aggttggttg ggggggcaca caggaacact ccctaaggaa gtgtgatccg agaggttggg 1740
   gtgggggctt gcacgcttag gacgaggggg gcctccggag gttgggaaga gcacttagaa 1800
   aacctcctgg aggcgtggct agggagacag tctcagaaag ttggggaggg ggagcaggtt 1860
   taggagccgc tgggcacttg gctcagaatc cccggggctg aggctcaggt agttggggag 1920
   taggtgcgcg tttaggaacc ccggggagat gctgcgtctc aggaagttgg ggagggcgct 1980
   caggcttggg actcctctgg ggacaaggct caggaccttg gggagggagt gttcgctggg 2040
   aaaccttgag agattccgtg tcttagaatg ctggagagag gtgcatgctt aggaccgtcg 2100
   gggagcgtgg ctgacaacag tggggagtgg accttgcgct cctccgaccc cctgggggtg 2160
   aggatccgga ttgtgggggg agttggggat gtagggcaag gatccctcag gggcgcaaca 2220
   ctaccgcggg gagcgcgtca aggccctggt tagggatagg ttgcgctcgc cggggtagcc 2280
   atacgtgccc tgtcctggga ggggaactga cgcttactct cgccccctcc ctgcaggtga 2340
   gaagccctac cactgcaact gggacggctg cggctggaag tttgcgcgct cagacgagct 2400
   cacgcgccac taccgaaagc acacgggcca ccggccattc cagtgccatc tgtgcgatcg 2460
   tgccttctcg cgctccgatc acctggcgct gcacatgaaa cggcacatgt agccgggacg 2520
   cccccgccca cctgcgcgcg gccgtggcgg gtcccacgcg ccgggcgcgg ccccctccca 2580
   aactgtgact ggtatttatt ggacccagag aaccgggccg ggcacagcgt ggctacagag 2640
   ggtctccctc gatgacgacg acgacgacgc caccacccca gcccccgtct gtgactgaag 2700
   gcccggtggg aaaagaccac gatcctcctt gacgagtttt gtttttcaaa atggtgcaat 2760
   aatttaagtg gcatcttctc tcccaccggg tctacactag aggatcgagg cttgtgatgc 2820
   cttgtaagaa ataagggcct taatttgtac tgtctgcggc attttttata atattgtata 2880
   tagtgactga caaatattgt attactgtac atagagagac aggtgggcat ttttgggcta 2940
   cctggttcgt ttttataaga ttttgctggg ttggtttttt tttttattaa aaagttttgc 3000
   atcttttaaa aaaaaaa 3017

<210> 33
<211> 2949
<212> DNA
<213> Homo sapiens

<400> 33
agtttcccga ccagagagaa cgaacgtgtc tgcgggcgcg cggggagcag aggcggtggc 60

gggcggcggc ggcaccggga gccgccgagt gaccctcccc cgcccctctg gccccccacc 120

ctcccacccg cccgtggccc gcgcccatgg ccgcgcgcgc tccacacaac tcaccggagt 180

ccgcgccttg cgccgccgac cagttcgcag ctccgcgcca cggcagccag tctcacctgg 240

cggcaccgcc cgcccaccgc cccggccaca gcccctgcgc ccacggcagc actcgaggcg 300

accgcgacag tggtggggga cgctgctgag tggaagagag cgcagcccgg ccaccggacc 360

tacttactcg ccttgctgat tgtctatttt tgcgtttaca acttttctaa gaacttttgt 420

atacaaagga actttttaaa aaagacgctt ccaagttata tttaatccaa agaagaagga 480

tctcggccaa tttggggttt tgggttttgg cttcgtttct tctcttcgtt gactttgggg 540

ttcaggtgcc ccagctgctt cgggctgccg aggaccttct gggcccccac attaatgagg 600

cagccacctg gcgagtctga catggctgtc agcgacgcgc tgctcccatc tttctccacg 660

ttcgcgtctg gcccggcggg aagggagaag acactgcgtc aagcaggtgc cccgaataac 720

cgctggcggg aggagctctc ccacatgaag cgacttcccc cagtgcttcc cggccgcccc 780

tatgacctgg cggcggcgac cgtggccaca gacctggaga gcggcggagc cggtgcggct 840

tgcggcggta gcaacctggc gcccctacct cggagagaga ccgaggagtt caacgatctc 900

ctggacctgg actttattct ctccaattcg ctgacccatc ctccggagtc agtggccgcc 960

accgtgtcct cgtcagcgtc agcctcctct tcgtcgtcgc cgtcgagcag cggccctgcc 1020

agcgcgccct ccacctgcag cttcacctat ccgatccggg ccgggaacga cccgggcgtg 1080

gcgccgggcg gcacgggcgg aggcctcctc tatggcaggg agtccgctcc ccctccgacg 1140

gctcccttca acctggcgga catcaacgac gtgagcccct cgggcggctt cgtggccgag 1200

ctcctgcggc cagaattgga cccggtgtac attccgccgc agcagccgca gccgccaggt 1260

ggcgggctga tgggcaagtt cgtgctgaag gcgtcgctga gcgcccctgg cagcgagtac 1320

ggcagcccgt cggtcatcag cgtcagcaaa ggcagccctg acggcagcca cccggtggtg 1380

gtggcgccct acaacggcgg gccgccgcgc acgtgcccca agatcaagca ggaggcggtc 1440

tcttcgtgca cccacttggg cgctggaccc cctctcagca atggccaccg gccggctgca 1500

cacgacttcc ccctgggcgg gcagctcccc agcaggacta ccccgaccct gggtcttgag 1560

gaagtgctga gcagcaggga ctgtcaccct gccctgccgc ttcctcccgg cttccatccc 1620

cacccggggc ccaattaccc atccttcctg cccgatcaga tgcagccgca agtcccgccg 1680

ctccattacc aagagctcat gccacccggt tcctgcatgc cagaggagcc caagccaaag 1740

aggggaagac gatcgtggcc ccggaaaagg accgccaccc acacttgtga ttacgcgggc 1800

tgcggcaaaa cctacacaaa gagttcccat ctcaaggcac acctgcgaac ccacacaggt 1860

gagaaacctt accactgtga ctgggacggc tgtggatgga aattcgcccg ctcagatgaa 1920

ctgaccaggc actaccgtaa acacacgggg caccgcccgt tccagtgcca aaaatgcgac 1980

cgagcatttt ccaggtcgga ccacctcgcc ttacacatga agaggcattt ttaaatccca 2040

gacagtggat atgacccaca ctgccagaag agaattcagt attttttact tttcacactg 2100

tcttcccgat gagggaagga gcccagccag aaagcactac aatcatggtc aagttcccaa 2160

ctgagtcatc ttgtgagtgg ataatcagga aaaatgagga atccaaaaga caaaaatcaa 2220

agaacagatg gggtctgtga ctggatcttc tatcattcca attctaaatc cgacttgaat 2280

attcctggac ttacaaaatg ccaagggggt gactggaagt tgtggatatc agggtataaa 2340

ttatatccgt gagttggggg agggaagacc agaattccct tgaattgtgt attgatgcaa 2400

tataagcata aaagatcacc ttgtattctc tttaccttct aaaagccatt attatgatgt 2460

tagaagaaga ggaagaaatt caggtacaga aaacatgttt aaatagccta aatgatggtg 2520

cttggtgagt cttggttcta aaggtaccaa acaaggaagc caaagttttc aaactgctgc 2580

atactttgac aaggaaaatc tatatttgtc ttccgatcaa catttatgac ctaagtcagg 2640

taatatacct ggtttacttc tttagcattt ttatgcagac agtctgttat gcactgtggt 2700

ttcagatgtg caataatttg tacaatggtt tattcccaag tatgccttaa gcagaacaaa 2760

tgtgtttttc tatatagttc cttgccttaa taaatatgta atataaattt aagcaaacgt 2820

ctattttgta tatttgtaaa ctacaaagta aaatgaacat tttgtggagt ttgtattttg 2880

catactcaag gtgagaatta agttttaaat aaacctataa tattttatct gaaaaaaaaa 2940

aaaaaaaaa 2949

<210> 34
<211> 2073
<212> DNA
<213> Homo sapiens

<400> 34
agatgccacg ccccatagct ccaccagtca ccgcggcaca gtggccctta agcgaggagc 60

ggcggcgccc gcagcaatca cagcagtgcc gacgtcgtgg gtgtttggtg tgaggctgcg 120

agccgccgcg agttctcacg gtcccgccgg cgccaccacc gcggtcactc accgccgccg 180

ccgccaccac tgccaccacg gtcgcctgcc acaggtgtct gcaattgaac tccaaggtgc 240

agaatggttt ggaaagtagc tgtattcctc agtgtggccc tgggcattgg tgccgttcct 300

atagatgatc ctgaagatgg aggcaagcac tgggtggtga tcgtggcagg ttcaaatggc 360

tggtataatt ataggcacca ggcagacgcg tgccatgcct accagatcat tcaccgcaat 420

gggattcctg acgaacagat cgttgtgatg atgtacgatg acattgctta ctctgaagac 480

aatcccactc caggaattgt gatcaacagg cccaatggca cagatgtcta tcagggagtc 540

ccgaaggact acactggaga ggatgttacc ccacaaaatt tccttgctgt gttgagaggc 600

gatgcagaag cagtgaaggg cataggatcc ggcaaagtcc tgaagagtgg cccccaggat 660

cacgtgttca tttacttcac tgaccatgga tctactggaa tactggtttt tcccaatgaa 720

gatcttcatg taaaggacct gaatgagacc atccattaca tgtacaaaca caaaatgtac 780

cgaaagatgg tgttctacat tgaagcctgt gagtctgggt ccatgatgaa ccacctgccg 840

gataacatca atgtttatgc aactactgct gccaacccca gagagtcgtc ctacgcctgt 900

tactatgatg agaagaggtc cacgtacctg ggggactggt acagcgtcaa ctggatggaa 960

gattcggacg tggaagatct gactaaagag accctgcaca agcagtacca cctggtaaaa 1020

tcgcacacca acaccagcca cgtcatgcag tatggaaaca aaacaatctc caccatgaaa 1080

gtgatgcagt ttcagggtat gaaacgcaaa gccagttctc ccgtccccct acctccagtc 1140

acacaccttg acctcacccc cagccctgat gtgcctctca ccatcatgaa aaggaaactg 1200

atgaacacca atgatctgga ggagtccagg cagctcacgg aggagatcca gcggcatctg 1260

gatgccaggc acctcattga gaagtcagtg cgtaagatcg tctccttgct ggcagcgtcc 1320

gaggctgagg tggagcagct cctgtccgag agagccccgc tcacggggca cagctgctac 1380

ccagaggccc tgctgcactt ccggacccac tgcttcaact ggcactcccc cacgtacgag 1440

tatgcgttga gacatttgta cgtgctggtc aacctttgtg agaagccgta tccgcttcac 1500

aggataaaat tgtccatgga ccacgtgtgc cttggtcact actgaagagc tgcctcctgg 1560

aagcttttcc aagtgtgagc gccccaccga ctgtgtgctg atcagagact ggagaggtgg 1620

agtgagaagt ctccgctgct cgggccctcc tggggagccc ccgctccagg gctcgctcca 1680

ggaccttctt cacaagatga cttgctcgct gttacctgct tccccagtct tttctgaaaa 1740

actacaaatt agggtgggaa aagctctgta ttgagaaggg tcatatttgc tttctaggag 1800

gtttgttgtt ttgcctgtta gttttgagga gcaggaagct catgggggct tctgtagccc 1860

ctctcaaaag gagtctttat tctgagaatt tgaagctgaa acctctttaa atcttcagaa 1920

tgattttatt gaagaggccc gcaagcccca aatggaaaac tgtttttaga aaatatgatg 1980

atttttgatt gcttttgtat ttaattctgc aggtgttcaa gtcttaaaaa ataaagattt 2040

ataacagaac ccaaataaaa aaaaaaaaaa aaa 2073

<210> 35
<211> 3470
<212> DNA
<213> Homo sapiens

<400> 35
   atacacacag actcacagcg agaccgacac acactcccat acactcacac acacaactgc 60
   aggcagcgag gctcgggaag tcaggccggc ttttcgcccc ggcgccttct ctgctccagc 120
   cggccgggtc tccctggggg cccggagctc ggccgggccg cgcagccccg ttagaggacg 180
   agctcggcgg acccccgctc ctccatgggc aaacgcgggc ggccgcgcaa ggaggcgcgc 240
   tgcgagggcg cggggctggc ccccgccgcg cccccggctg tgccccccgc cgtggccgcg 300
   ccccagcccc cggccctgcc cgaggacccc gctggggcca agcccaggtg ccccttctca 360
   gacattttca acaccagcga gaactcgatg gagaagcaca tcaacacttt tctgcagaac 420
   gtgcagattc tgctcgaggc cgccagctac ctggagcaga tcgagaaaga aaacaaaaag 480
   tgtgaacatg gctacgcctc ttcattcccg tccatgccga gcccccgact gcagcattca 540
   aagcccccac ggaggttgag ccgggcacag aaacacagca gcgggagcag caacaccagc 600
   actgccaaca gatctacaca caatgagctg gaaaagaatc gacgagctca tctgcgcctt 660
   tgtttagaac gcttaaaagt tctgattcca ctaggaccag actgcacccg gcacacaaca 720
   cttggtttgc tcaacaaagc caaagcacac atcaagaaac ttgaagaagc tgaaagaaaa 780
   agccagcacc agctcgagaa tttggaacga gaacagagat ttttaaagtg gcgactggaa 840
   cagctgcagg gtcctcagga gatggaacga atacgaatgg acagcattgg atcaactatt 900
   tcttcagatc gttctgattc agagcgagag gagattgaag tggatgttga aagcacagag 960
   ttctcccatg gagaagtgga caatataagt accaccagca tcagtgacat tgatgaccac 1020
   agcagcctgc cgagtattgg gagtgacgag ggttactcca gtgccagtgt caaactttca 1080
   ttcacttcat agaacccagc atgacataac agtgcagggc aaaatattca ctgggccaat 1140
   tcaatacaaa caatctctta aattgggttc atgatgcagt ctcctcttta aaacaaaaca 1200
   aaacaaaaca aaactatact tgaacaaaag ggtcagagga cctgtattta agcaaatact 1260
   tagcaaaaag tggggcagag cctcccaagg agaacaaata ttcagaatat tcatattgga 1320
   aaaatcacaa tttttaatgg cagcagaaaa cttgtgtgaa attttcttga tttgagttga 1380
   ttgagaagag gacattggag atgccatcct ctttctcttt tctagtttgc tcatactaca 1440
   ttgagtagac acatttaagg atggggttat gaacccttcc tgagctttat ggtcctaaaa 1500
   gcaaaataaa aactattcga atgaaaagac aagaaaatca ggtattaatc ttggatagct 1560
   aataatgagc tattaaaact cagcctggga cagtttatca tgaagcctgt ggatgatcaa 1620
   tcctttatta ttattttttt tttttgaaaa aagctcattt catgctctgc aaaaggagag 1680
   actcccatga agccttttga aagggatcat catgcagctc aactttctgt tggattccat 1740
   gctaagcaag ctaaccttat cctgcattgt tagcactagg cacccagctg ccacctctcc 1800
   atcctgctgc ccttaggcca catgggagca gtccatgcat gacagcctct atcctacaag 1860
   gcctatgagt atggattggg ggggccaaaa ggaaaaagct ccatgtgcct ctttgtctgc 1920
   gtgggtcaga agagttgtgc acgcagatta gcaggccaag gtctgagcca cagcagcatt 1980
   tttatttcag attttgataa ctgtttatat gtgttgaaaa ccaaaatgac atctttttaa 2040
   agcttatcca taaaaaaaaa tagatgtctt ttatagtgga aaaacacatg gggaaaaaaa 2100
   tcatctattt tgatgcagca tttgataatg ataaaacacc tcacacctca ctctttatag 2160
   tgcacaaaat gaatgaggtc tgggctaggt agaaaaaggg tcaatgctat ttttgttttt 2220
   agaatcatta ccttttacca gcttttaacc atctgatatc tatagtagac acactatcat 2280
   agttaacata gtaagttcag cacttgtctc attttaatgt aaagatttgc ttccattttc 2340
   ctacaggcag tctctctctt cctcacagtc ccactgtgca ggtgctattg ttactcttac 2400
   gaatattttc agtaatgtta ttttcttcta agtgaaattt ctagcctgca ctttgatgtc 2460
   atgtgttccc tttgtctttc aaactccaag gttcccttgt ggccctctcc cttaccctgg 2520
   gaaggcctct tggagacctt acccctggct gtttggactt tgtatacttt aaataattta 2580
   actaccctta attacttaaa aaaaaaaaaa agctttatga ttttcataac ttattgctga 2640
   ttttaatgga ttgttaattt cagtcctgta gttttatttt atgtttagat agggctgggc 2700
   aaggaaaaag aaaataaaga caaccatatt tagcagtgca gttgagttgt gtgttaatgt 2760
   tagactatcc ctttgtgagt gacactttaa cagcattcac tgcttctata tatagtgtac 2820
   catcttggtc atacattacg cctcaacata tacttgtgct cttcctttgc ctccagaaga 2880
   agtttttcct tgattgtgct atgtttcagt ggaagaaatt ctttgaagta gatgtgagtg 2940
   aaaaactgca tgcctttaga agcccagtat cagaacttgc tacgtttcag gtgctaggga 3000
   cttaatgaaa aacaggacaa aacaattcct ttttgtggcc caggtaaatt atttctggtt 3060
   tcacttataa ttactaatgg ctgagtcaag atgttgtctc tgtgtttgct tactcttgat 3120
   caagtgtgag acagtttgaa gactgtgcta ccatacaaag tgaatgaagc cagtgactaa 3180
   gcttctgttt gttttgttat tctcatggcc ttcgcttgca ttatttgggc cttcattcag 3240
   atgaacttga ggtgccattt tgttgcatat gtacaggatt atgggctgga aagcatttgt 3300
   tataaaccta tagtgcacat tttaactgcc ccctaaatta cccttccctg ggtttgtttt 3360
   ccttggggtg gtgtagattg tatgagtaag aagtattaat tttttaaaag acaaatcaac 3420
   tttgaagaca caaaagttaa ttggaagaaa taaaaactgt gaacgaagaa 3470

<210> 36
<211> 1823
<212> DNA
<213> Homo sapiens

<400> 36
   gagaagctag gggtgaggaa gccctggggc gctgccgccg ctttccttaa ccacaaatca 60
   ggccggacag gagagggagg ggtgggggac agtgggtggg cattcagact gccagcactt 120
   tgctatctac agccggggct cccgagcggc agaaagttcc ggccactctc tgccgcttgg 180
   gttgggcgaa gccaggaccg tgccgcgcca ccgccaggat atggagctac tgtcgccacc 240
   gctccgcgac gtagacctga cggcccccga cggctctctc tgctcctttg ccacaacgga 300
   cgacttctat gacgacccgt gtttcgactc cccggacctg cgcttcttcg aagacctgga 360
   cccgcgcctg atgcacgtgg gcgcgctcct gaaacccgaa gagcactcgc acttccccgc 420
   ggcggtgcac ccggccccgg gcgcacgtga ggacgagcat gtgcgcgcgc ccagcgggca 480
   ccaccaggcg ggccgctgcc tactgtgggc ctgcaaggcg tgcaagcgca agaccaccaa 540
   cgccgaccgc cgcaaggccg ccaccatgcg cgagcggcgc cgcctgagca aagtaaatga 600
   ggcctttgag acactcaagc gctgcacgtc gagcaatcca aaccagcggt tgcccaaggt 660
   ggagatcctg cgcaacgcca tccgctatat cgagggcctg caggctctgc tgcgcgacca 720
   ggacgccgcg ccccctggcg ccgcagccgc cttctatgcg ccgggcccgc tgcccccggg 780
   ccgcggcggc gagcactaca gcggcgactc cgacgcgtcc agcccgcgct ccaactgctc 840
   cgacggcatg atggactaca gcggcccccc gagcggcgcc cggcggcgga actgctacga 900
   aggcgcctac tacaacgagg cgcccagcga acccaggccc gggaagagtg cggcggtgtc 960
   gagcctagac tgcctgtcca gcatcgtgga gcgcatctcc accgagagcc ctgcggcgcc 1020
   cgccctcctg ctggcggacg tgccttctga gtcgcctccg cgcaggcaag aggctgccgc 1080
   ccccagcgag ggagagagca gcggcgaccc cacccagtca ccggacgccg ccccgcagtg 1140
   ccctgcgggt gcgaacccca acccgatata ccaggtgctc tgaggggatg gtggccgccc 1200
   acccgcccga gggatggtgc ccctagggtc cctcgcgccc aaaagattga acttaaatgc 1260
   ccccctccca acagcgcttt aaaagcgacc tctcttgagg taggagaggc gggagaactg 1320
   aagtttccgc cccgccccca cagggcaagg acacagcgcg gtttttccca cgcagcaccc 1380
   ttctcggaga cccattgcga tggccgctcc gtgttcctcg gtgggccaga gctgaacctt 1440
   gaggggctag gttcagcttt ctcgcgccct cccccatggg ggtgagaccc tcgcagacct 1500
   aagccctgcc ccgggatgca ccggttattt gggggggcgt gagacccagt gcactccggt 1560
   cccaaatgta gcaggtgtaa ccgtaaccca cccccaaccc gtttcccggt tcaggaccac 1620
   tttttgtaat acttttgtaa tctattcctg taaataagag ttgctttgcc agagcaggag 1680
   cccctggggc tgtatttatc tctgaggcat ggtgtgtggt gctacaggga atttgtacgt 1740
   ttataccgca ggcgggcgag ccgcgggcgc tcgctcaggt gatcaaaata aaggcgctaa 1800
   tttataaaaa aaaaaaaaaa aaa 1823

<210> 37
<211> 4345
<212> DNA
<213> Homo sapiens

<400> 37
actgaaacta ggggcaagga gacgaagaga acatgaaagt taaactttaa gatgaagaac 60

aaagctgaac atactgatgc attggatctt tggagaggat ctcagaactc attgtactta 120

atttacaggc taaaacctta gaagaggaat ttattatatc ctacacaaga ctccagggaa 180

gcacatggcc ttggactgaa ggctggcatc tggaagctgt cagccaccag caccttctgc 240

agcaggaaaa ggccagggct ctgctggagc aggcagcaga gtggacgcac agtaacatgg 300

gcaacttgaa gagcgtggcc caggagcctg ggccaccctg cggcctgggg ctggggctgg 360

gccttgggct gtgcggcaag cagggcccag ccaccccggc ccctgagccc agccgggccc 420

cagcatccct actcccacca gcgccagaac acagcccccc gagctccccg ctaacccagc 480

ccccagaggg gcccaagttc cctcgtgtga agaactggga ggtggggagc atcacctatg 540

acaccctcag cgcccaggcg cagcaggatg ggccctgcac cccaagacgc tgcctgggct 600

ccctggtatt tccacggaaa ctacagggcc ggccctcccc cggccccccg gcccctgagc 660

agctgctgag tcaggcccgg gacttcatca accagtacta cagctccatt aagaggagcg 720

gctcccaggc ccacgaacag cggcttcaag aggtggaagc cgaggtggca gccacaggca 780

cctaccagct tagggagagc gagctggtgt tcggggctaa gcaggcctgg cgcaacgctc 840

cccgctgcgt gggccggatc cagtggggga agctgcaggt gttcgatgcc cgggactgca 900

ggtctgcaca ggaaatgttc acctacatct gcaaccacat caagtatgcc accaaccggg 960

gcaaccttcg ctcggccatc acagtgttcc cgcagcgctg ccctggccga ggagacttcc 1020

gaatctggaa cagccagctg gtgcgctacg cgggctaccg gcagcaggat ggctctgtgc 1080

ggggggaccc agccaacgtg gagatcaccg agctctgcat tcagcacggc tggaccccag 1140

gaaacggtcg cttcgacgtg ctgcccctgc tgctgcaggc cccagatgat cccccagaac 1200

tcttccttct gccccccgag ctggtccttg aggtgcccct ggagcacccc acgctggagt 1260

ggtttgcagc cctgggcctg cgctggtacg ccctcccggc agtgtccaac atgctgctgg 1320

aaattggggg cctggagttc cccgcagccc ccttcagtgg ctggtacatg agcactgaga 1380

tcggcacgag gaacctgtgt gaccctcacc gctacaacat cctggaggat gtggctgtct 1440

gcatggacct ggatacccgg accacctcgt ccctgtggaa agacaaggca gcagtggaaa 1500

tcaacgtggc cgtgctgcac agttaccagc tagccaaagt caccatcgtg gaccaccacg 1560

ccgccacggc ctctttcatg aagcacctgg agaatgagca gaaggccagg gggggctgcc 1620

ctgcagactg ggcctggatc gtgcccccca tctcgggcag cctcactcct gttttccatc 1680

aggagatggt caactatttc ctgtccccgg ccttccgcta ccagccagac ccctggaagg 1740

ggagtgccgc caagggcacc ggcatcacca ggaagaagac ctttaaagaa gtggccaacg 1800

ccgtgaagat ctccgcctcg ctcatgggca cggtgatggc gaagcgagtg aaggcgacaa 1860

tcctgtatgg ctccgagacc ggccgggccc agagctacgc acagcagctg gggagactct 1920

tccggaaggc ttttgatccc cgggtcctgt gtatggatga gtatgacgtg gtgtccctcg 1980

aacacgagac gctggtgctg gtggtaacca gcacatttgg gaatggggat cccccggaga 2040

atggagagag ctttgcagct gccctgatgg agatgtccgg cccctacaac agctcccctc 2100

ggccggaaca gcacaagagt tataagatcc gcttcaacag catctcctgc tcagacccac 2160

tggtgtcctc ttggcggcgg aagaggaagg agtccagtaa cacagacagt gcaggggccc 2220

tgggcaccct caggttctgt gtgttcgggc tcggctcccg ggcatacccc cacttctgcg 2280

cctttgctcg tgccgtggac acacggctgg aggaactggg cggggagcgg ctgctgcagc 2340

tgggccaggg cgacgagctg tgcggccagg aggaggcctt ccgaggctgg gcccaggctg 2400

ccttccaggc cgcctgtgag accttctgtg tgggagagga tgccaaggcc gccgcccgag 2460

acatcttcag ccccaaacgg agctggaagc gccagaggta ccggctgagc gcccaggccg 2520

agggcctgca gttgctgcca ggtctgatcc acgtgcacag gcggaagatg ttccaggcta 2580

caatccgctc agtggaaaac ctgcaaagca gcaagtccac gagggccacc atcctggtgc 2640

gcctggacac cggaggccag gaggggctgc agtaccagcc gggggaccac ataggtgtct 2700

gcccgcccaa ccggcccggc cttgtggagg cgctgctgag ccgcgtggag gacccgccgg 2760

cgcccactga gcccgtggca gtagagcagc tggagaaggg cagccctggt ggccctcccc 2820

ccggctgggt gcgggacccc cggctgcccc cgtgcacgct gcgccaggct ctcaccttct 2880

tcctggacat cacctcccca cccagccctc agctcttgcg gctgctcagc accttggcag 2940

aagagcccag ggaacagcag gagctggagg ccctcagcca ggatccccga cgctacgagg 3000

agtggaagtg gttccgctgc cccacgctgc tggaggtgct ggagcagttc ccgtcggtgg 3060

cgctgcctgc cccactgctc ctcacccagc tgcctctgct ccagccccgg tactactcag 3120

tcagctcggc acccagcacc cacccaggag agatccacct cactgtagct gtgctggcat 3180

acaggactca ggatgggctg ggccccctgc actatggagt ctgctccacg tggctaagcc 3240

agctcaagcc cggagaccct gtgccctgct tcatccgggg ggctccctcc ttccggctgc 3300

cacccgatcc cagcttgccc tgcatcctgg tgggtccagg cactggcatt gcccccttcc 3360

ggggattctg gcaggagcgg ctgcatgaca ttgagagcaa agggctgcag cccactccca 3420

tgactttggt gttcggctgc cgatgctccc aacttgacca tctctaccgc gacgaggtgc 3480

agaacgccca gcagcgcggg gtgtttggcc gagtcctcac cgccttctcc cgggaacctg 3540

acaaccccaa gacctacgtg caggacatcc tgaggacgga gctggctgcg gaggtgcacc 3600

gcgtgctgtg cctcgagcgg ggccacatgt ttgtctgcgg cgatgttacc atggcaacca 3660

acgtcctgca gaccgtgcag cgcatcctgg cgacggaggg cgacatggag ctggacgagg 3720

ccggcgacgt catcggcgtg ctgcgggatc agcaacgcta ccacgaagac attttcgggc 3780

tcacgctgcg cacccaggag gtgacaagcc gcatacgcac ccagagcttt tccttgcagg 3840

agcgtcagtt gcggggcgca gtgccctggg cgttcgaccc tcccggctca gacaccaaca 3900

gcccctgaga gccgcctggc tttcccttcc agttccggga gagcggctgc ccgactcagg 3960

tccgcccgac caggatcagc cccgctcctc ccctcttgag gtggtgcctt ctcacatctg 4020

tccagaggct gcaaggattc agcattattc ctccaggaag gagcaaaacg cctcttttcc 4080

ctctctaggc ctgttgcctc gggcctgggt ccgccttaat ctggaaggcc cctcccagca 4140

gcggtacccc agggcctact gccacccgct tcctgtttct tagtcgaatg ttagattcct 4200

cttgcctctc tcaggagtat cttacctgta aagtctaatc tctaaatcaa gtatttatta 4260

ttgaagattt accataaggg actgtgccag atgttaggag aactactaaa gtgcctaccc 4320

cagctcatgt ggattacaaa aaaaa 4345

<210> 38
<211> 2692
<212> DNA
<213> Homo sapiens

<400> 38
tttaaagctg ggaggttctg ccaccaagca cggccttccc actgggaaca caaacttgct 60

ggcgggaaga gcccggaaag aaacctgtgg atctcccttc gagatcatcc aaagagaaga 120

aaggtgacct cacattcgtg ccccttagca gcactctgca gaaatgcctc ctcagctgca 180

aaacggcctg aacctctcgg ccaaagttgt ccagggaagc ctggacagcc taccccaggc 240

agtgagggag tttctcgaga ataacgctga gctgtgtcag cctgatcaca tccacatctg 300

tgacggctct gaggaggaga atggcggctt tctgggccag atggaggaag agggcatcct 360

caggcggctg aagaagtatg acaactgctg gttggctctc actgacccca gggatgtggc 420

caggatcgaa agcaagacgg ttatcgtcac ccaagagcaa agagacacag tgcccatccc 480

caaaacaggc ctcagccagc tcggtcgctg gatgtcagag gaggattttg agaaagcgtt 540

caatgccagg ttcccagggt gcatgaaagg tcgcaccatg tacgtcatcc cattcagcat 600

ggggccgctg ggctcgcctc tgtcaaagat cggcatcgag ctgacggatt caccctacgt 660

ggtggccagc atgcggatca tgacgcggat gggcacgccc gtcctggaag cagtgggcga 720

tggggagttt gtcaaatgcc tccattctgt ggggtgccct ctgcctttac aaaagccttt 780

ggtcaacaac tggccctgca acccggagct gacgctcatc gcccacctgc ctgaccgcag 840

agagatcatc tcctttggca gtgggtacgg cgggaactcg ctgctcggga agaagtgctt 900

tgctctcagg atggccagcc ggctggccaa ggaggaaggg tggctggcag agcacatgct 960

gattctgggt ataaccaacc ctgagggtga gaagaagtac ctggcggccg catttcccag 1020

cgcctgcggg aagaccaacc tggccatgat gaaccccagc ctccccgggt ggaaggttga 1080

gtgcgtcggg gatgacattg cctggatgaa gtttgacgca caaggtcatt taagggccat 1140

caacccagaa aatggctttt tcggtgtcgc tcctgggact tcagtgaaga ccaaccccaa 1200

tgccatcaag accatccaga agaacacaat ctttaccaat gtggccgaga ccagcgacgg 1260

gggcgtttac tgggaaggca ttgatgagcc gctagcttca ggtgtcacca tcacgtcctg 1320

gaagaataag gagtggagct cagaggatgg ggaaccttgt gcccacccca actcgaggtt 1380

ctgcacccct gccagccagt gccccatcat tgatgctgcc tgggagtctc cggaaggtgt 1440

tcccattgaa ggcattatct ttggaggccg tagacctgct ggtgtccctc tagtctatga 1500

agctctcagc tggcaacatg gagtctttgt gggggcggcc atgagatcag aggccacagc 1560

ggctgcagaa cataaaggca aaatcatcat gcatgacccc tttgccatgc ggcccttctt 1620

tggctacaac ttcggcaaat acctggccca ctggcttagc atggcccagc acccagcagc 1680

caaactgccc aagatcttcc atgtcaactg gttccggaag gacaaggaag gcaaattcct 1740

ctggccaggc tttggagaga actccagggt gctggagtgg atgttcaacc ggatcgatgg 1800

aaaagccagc accaagctca cgcccatagg ctacatcccc aaggaggatg ccctgaacct 1860

gaaaggcctg gggcacatca acatgatgga gcttttcagc atctccaagg aattctggga 1920

gaaggaggtg gaagacatcg agaagtatct ggaggatcaa gtcaatgccg acctcccctg 1980

tgaaatcgag agagagatcc ttgccttgaa gcaaagaata agccagatgt aatcagggcc 2040

tgagtgcttt acctttaaaa tcattccctt tcccatccat aaggtgcagt aggagcaaga 2100

gagggcaagt gttcccaaat tgacgccacc ataataatca tcaccacacc gtgagcagat 2160

ctgaaaggca cactttgatt tttttaagga taagaaccac agaacactgg gtagtagcta 2220

atgaaattga gaagggaaat cttagcatgc ctccaaaaat tcacatccaa tgcatagttt 2280

gttcaaattt aaggttactc aggcattgat cttttcagtg ttttttcact ttagctatgt 2340

ggattagcta gaatgcacac caaaaaaata cttgagctgt atatatatat gtgtgtgtgt 2400

gtgtgtgtgt gtgtgtgtgt gtgtgcatgt atgtgcacat gtgtctgtgt ggtatatttg 2460

tgtatgtgta tttgtatgta ctgttattga aaatatattt aatacctttg gaaaaatctt 2520

gggcaagatg acctactagt tttccttgaa aaaaagttgc tttgttatta atattgtgct 2580

taaattattt ttatacacca ttgttcctta cctttacata attgcaatat ttccccctta 2640

ctacttcttg gaaaaaaatt acaaaatgaa gttttataga aaagaaaaaa aa 2692

<210> 39
<211> 3710
<212> DNA
<213> Homo sapiens

<400> 39
aggacgcgtt tccaagttcc agtgactcct cctgtttggg actcgggggg agagtgcggg 60

gagacaaata aaacctcggg cggcggcggc tggtgggaag acttgaactt gaatctcgaa 120

ccactgcatc tccgactctg cccagactct tcactccgcg gcaccctcaa accccagccc 180

aggccggggc gcacaagcca gccagcgcac ctgcagtcct cgcccggacg cgccgcgccc 240

cctcggaacc aggctctgct ccgagcagcc ttcgcccctc aagccagcca cagtccccgc 300

caggccgggt gggcgtcaag atgaaggcgg cccgcttcgt gctgcgcagc gctggctcgc 360

tcaacggcgc cggcctggtg ccccgagagg tggagcattt ctcgcgctac agcccgtccc 420

cgctgtccat gaagcagcta ctggactttg gttcagaaaa tgcatgtgaa agaacttctt 480

ttgcattttt gcgacaagaa ttgcctgtga gactcgccaa cattctgaag gaaattgata 540

tcctcccgac ccaattagta aatacctctt cagtgcaatt ggttaaaagc tggtatatac 600

agagcctgat ggatttggtg gaattccatg agaaaagccc agatgaccag aaagcattat 660

cagactttgt agatacactc atcaaagttc gaaatagaca ccataatgta gtccctacaa 720

tggcacaagg aatcatagag tataaagatg cctgtacagt tgacccagtc accaatcaaa 780

atcttcaata tttcttggat cgattttaca tgaaccgtat ttctactcgg atgctgatga 840

accagcacat tcttatattt agtgactcac agacaggaaa cccaagccac attggaagca 900

ttgatcctaa ctgtgatgtg gtagcagtgg tccaagatgc ctttgagtgt tcaaggatgc 960

tctgtgatca gtattattta tcatctccag aattaaagct tacacaagtg aatggaaaat 1020

ttccagacca accaattcac atcgtgtatg ttccttctca cctccatcat atgctctttg 1080

aactatttaa gaatgcaatg cgggcaacag ttgaacacca ggaaaatcag ccttccctta 1140

caccaataga ggttattgtt gtcttgggaa aagaagacct taccattaag atttcagaca 1200

gaggaggtgg tgttcccctg agaattattg accgcctctt tagttataca tactccactg 1260

caccaacgcc tgtgatggat aattcccgga atgctccttt ggctggtttt ggttacggct 1320

tgccaatttc tcgtctgtat gcaaagtact ttcaaggaga tctgaatctc tactctttat 1380

caggatatgg aacagatgct atcatctact taaaggcttt gtcttctgag tctatagaaa 1440

aacttccagt ttttaacaag tcagccttca aacattatca gatgagctct gaggctgatg 1500

actggtgtat cccaagcagg gaaccaaaga acctggcaaa agaagtggcc atgtgaagag 1560

ggacactcag gacactttac gggatcaaag tgggtctaca ccagtgctgc ttcctgaatg 1620

tttgtgtgtg aacccttgtt tcctccaaaa caaacgacag caacgaaaac tccttaatca 1680

gaacactgat ccaatgagga atggagcttg tttctgtgac ccaggagaac ttagtgcaag 1740

actacaggag ttaacagatg gccagctcct tattttttaa tgtagaataa ctcctgagtt 1800

tatatcaaat cctgaagaaa taagcctcag ttttccatct gtttttgata agaataagaa 1860

agggagtgag tgtgaagatg gtggttagca gtttcactaa gactgatatt ttaggcctct 1920

tgttcacatc aaaagatatt ggtgtcagaa taccagcatt ttcctgccat gcaaaggatt 1980

aaaacttagt ttacactatg tggttacaaa tatatgtcaa tgtacatttt gaacatattt 2040

atgtgctatg gaaggaaatg ctggtgacta aaataaggtt tactctgaaa gaggaggaat 2100

tttattcaaa gcattcaaac attttattca agtgtttcaa aattcaaagc attgtattca 2160

aagttgcagt gaaggcatca acttatgtaa aaactcagaa ggaaggctcc tctgataaaa 2220

acacagctcc tttattatgc tgcttttctt gttcacttta cacactaagt aaacacttat 2280

tgtcaggtgc ctagtcttga gtgaattgtt agatgtgcac tgaactcggg atgttgggga 2340

ttggagagag agaattgcca aagtaacagc aaaaatatct cttactttgc tttgtttata 2400

aataaattag tagattggaa aaactagtgt tagggaaaga aatcacatgt tcagagccta 2460

attcagtagg aagggctttt ctctaccctg aaatgaaggt aatccaaagg catccatttt 2520

ctaggcttaa aagatatatt tttgatatat ttaattatat tctctacact ccagcattaa 2580

tatgtctgtt taaaaattac taattctcaa atggctcaag aacattagaa tttaagtacc 2640

ttttagagta attattttaa gcaaatagcc tggacgtaag agattctcat gccagcatgc 2700

tttcatttgt cagttgttgt gactgagaga taatgaatga cacctgaaat gcatatggta 2760

tttttgggag agttaaggta taatttgaag gttggcagac cagttgcgct gattactctt 2820

agagaagaag aaatggaaaa atgaaagaag gcaggaagga aagaaaggat ataggaagag 2880

agggaagcag aaggcaggca tttttctatt ttccccacaa attatttcaa aaaaaatctg 2940

tattttctgg gatatgtcat tggcaagagg aagaactggt gttttgaaag cagtatggat 3000

tctttaaatg cctctcactc ttacaagata gtaggctttg agataataaa cttacccgtg 3060

tcaattaaca tttaaactgg catatagaaa aaaaggagga tttttctgca ttgtaaaata 3120

atcagtatgg tttatatgtt gaatttgaca tttgtgtgta atttcatggt ggcctagtgt 3180

tgtggtgctt ctggtaatgg taatagaagc tcaactattt ttttgtggat ttcagttttt 3240

atcatcagaa gtcctagaca gtgacatttc ttaatggtgg gagtccagct catgcatttc 3300

tgattataca aaacagtttg cagtaggtta tttgtcattt cagtttttta ctgaaatttg 3360

agctaaacat ttttacatgt aaatacttgt atttaccaaa gatttaaatc agttgattaa 3420

ttaattaact caaatactgt gaactatctc taaaacacta gaaaaaagaa atgttagtat 3480

ctcaattaca ccaactgtgc aaatgaactt tgataaaata gaaataatct acattggcct 3540

ttgtgaaatc tggggaagag ctttaggatt ctagtagatg gatactgaat actcaggccc 3600

acttaaatta ttaatgtata cattgtgttt ttgtctttat gctatgtaca gagaaatgtg 3660

ataatttttt ataataaata ttttttatga tgataaaaga aaaaaaaaaa 3710

<210> 40
<211> 1295
<212> DNA
<213> Homo sapiens

<400> 40
ccttcccctg gcccggggag ctgctccttg tgctgccggg aaggtcaaag tcccgcgccc 60

accaggagag ctcggcaagt atataaggac agaggagcgc gggaccaagc ggcggcgaag 120

gaggggaaga agagccgcga ccgagagagg ccgccgagcg tccccgccct cagagagcag 180

cctcccgaga caggcacttg ctggattctc caaaagtatc tgcagtggct gttccaccag 240

gagagcctca gcctgcctgg aagatgccga gatcgtgctg cagccgctcg ggggccctgt 300

tgctggcctt gctgcttcag gcctccatgg aagtgcgtgg ctggtgcctg gagagcagcc 360

agtgtcagga cctcaccacg gaaagcaacc tgctggagtg catccgggcc tgcaagcccg 420

acctctcggc cgagactccc atgttcccgg gaaatggcga cgagcagcct ctgaccgaga 480

acccccggaa gtacgtcatg ggccacttcc gctgggaccg attcggccgc cgcaacagca 540

gcagcagcgg cagcagcggc gcagggcaga agcgcgagga cgtctcagcg ggcgaagact 600

gcggcccgct gcctgagggc ggccccgagc cccgcagcga tggtgccaag ccgggcccgc 660

gcgagggcaa gcgctcctac tccatggagc acttccgctg gggcaagccg gtgggcaaga 720

agcggcgccc agtgaaggtg taccctaacg gcgccgagga cgagtcggcc gaggccttcc 780

ccctggagtt caagagggag ctgactggcc agcgactccg ggagggagat ggccccgacg 840

gccctgccga tgacggcgca ggggcccagg ccgacctgga gcacagcctg ctggtggcgg 900

ccgagaagaa ggacgagggc ccctacagga tggagcactt ccgctggggc agcccgccca 960

aggacaagcg ctacggcggt ttcatgacct ccgagaagag ccagacgccc ctggtgacgc 1020

tgttcaaaaa cgccatcatc aagaacgcct acaagaaggg cgagtgaggg cacagcgggg 1080

ccccagggct accctccccc aggaggtcga ccccaaagcc ccttgctctc ccctgccctg 1140

ctgccgcctc ccagcctggg gggtcgtggc agataatcag cctcttaaag ctgcctgtag 1200

ttaggaaata aaacctttca aatttcacat ccacctctga ctttgaatgt aaactgtgtg 1260

aataaagtaa aaatacgtag ccgtcaaata acagc 1295

<210> 41
<211> 6318
<212> DNA
<213> Homo sapiens

<400> 41
tagtaagaca ggtgccttca gttcactctc agtaaggggc tggttgcctg catgagtgtg 60

tgctctgtgt cactgtggat tggagttgaa aaagcttgac tggcgtcatt caggagctgg 120

atggcgtggg acatgtgcaa ccaggactct gagtctgtat ggagtgacat cgagtgtgct 180

gctctggttg gtgaagacca gcctctttgc ccagatcttc ctgaacttga tctttctgaa 240

ctagatgtga acgacttgga tacagacagc tttctgggtg gactcaagtg gtgcagtgac 300

caatcagaaa taatatccaa tcagtacaac aatgagcctt caaacatatt tgagaagata 360

gatgaagaga atgaggcaaa cttgctagca gtcctcacag agacactaga cagtctccct 420

gtggatgaag acggattgcc ctcatttgat gcgctgacag atggagacgt gaccactgac 480

aatgaggcta gtccttcctc catgcctgac ggcacccctc caccccagga ggcagaagag 540

ccgtctctac ttaagaagct cttactggca ccagccaaca ctcagctaag ttataatgaa 600

tgcagtggtc tcagtaccca gaaccatgca aatcacaatc acaggatcag aacaaaccct 660

gcaattgtta agactgagaa ttcatggagc aataaagcga agagtatttg tcaacagcaa 720

aagccacaaa gacgtccctg ctcggagctt ctcaaatatc tgaccacaaa cgatgaccct 780

cctcacacca aacccacaga gaacagaaac agcagcagag acaaatgcac ctccaaaaag 840

aagtcccaca cacagtcgca gtcacaacac ttacaagcca aaccaacaac tttatctctt 900

cctctgaccc cagagtcacc aaatgacccc aagggttccc catttgagaa caagactatt 960

gaacgcacct taagtgtgga actctctgga actgcaggcc taactccacc caccactcct 1020

cctcataaag ccaaccaaga taaccctttt agggcttctc caaagctgaa gtcctcttgc 1080

aagactgtgg tgccaccacc atcaaagaag cccaggtaca gtgagtcttc tggtacacaa 1140

ggcaataact ccaccaagaa agggccggag caatccgagt tgtatgcaca actcagcaag 1200

tcctcagtcc tcactggtgg acacgaggaa aggaagacca agcggcccag tctgcggctg 1260

tttggtgacc atgactattg ccagtcaatt aattccaaaa cagaaatact cattaatata 1320

tcacaggagc tccaagactc tagacaacta gaaaataaag atgtctcctc tgattggcag 1380

gggcagattt gttcttccac agattcagac cagtgctacc tgagagagac tttggaggca 1440

agcaagcagg tctctccttg cagcacaaga aaacagctcc aagaccagga aatccgagcc 1500

gagctgaaca agcacttcgg tcatcccagt caagctgttt ttgacgacga agcagacaag 1560

accggtgaac tgagggacag tgatttcagt aatgaacaat tctccaaact acctatgttt 1620

ataaattcag gactagccat ggatggcctg tttgatgaca gcgaagatga aagtgataaa 1680

ctgagctacc cttgggatgg cacgcaatcc tattcattgt tcaatgtgtc tccttcttgt 1740

tcttctttta actctccatg tagagattct gtgtcaccac ccaaatcctt attttctcaa 1800

agaccccaaa ggatgcgctc tcgttcaagg tccttttctc gacacaggtc gtgttcccga 1860

tcaccatatt ccaggtcaag atcaaggtct ccaggcagta gatcctcttc aagatcctgc 1920

tattactatg agtcaagcca ctacagacac cgcacgcacc gaaattctcc cttgtatgtg 1980

agatcacgtt caagatcgcc ctacagccgt cggcccaggt atgacagcta cgaggaatat 2040

cagcacgaga ggctgaagag ggaagaatat cgcagagagt atgagaagcg agagtctgag 2100

agggccaagc aaagggagag gcagaggcag aaggcaattg aagagcgccg tgtgatttat 2160

gtcggtaaaa tcagacctga cacaacacgg acagaactga gggaccgttt tgaagttttt 2220

ggtgaaattg aggagtgcac agtaaatctg cgggatgatg gagacagcta tggtttcatt 2280

acctaccgtt atacctgtga tgcttttgct gctcttgaaa atggatacac tttgcgcagg 2340

tcaaacgaaa ctgactttga gctgtacttt tgtggacgca agcaattttt caagtctaac 2400

tatgcagacc tagattcaaa ctcagatgac tttgaccctg cttccaccaa gagcaagtat 2460

gactctctgg attttgatag tttactgaaa gaagctcaga gaagcttgcg caggtaacat 2520

gttccctagc tgaggatgac agagggatgg cgaatacctc atgggacagc gcgtccttcc 2580

ctaaagacta ttgcaagtca tacttaggaa tttctcctac tttacactct ctgtacaaaa 2640

acaaaacaaa acaacaacaa tacaacaaga acaacaacaa caataacaac aatggtttac 2700

atgaacacag ctgctgaaga ggcaagagac agaatgatat ccagtaagca catgtttatt 2760

catgggtgtc agctttgctt ttcctggagt ctcttggtga tggagtgtgc gtgtgtgcat 2820

gtatgtgtgt gtgtatgtat gtgtgtggtg tgtgtgcttg gtttagggga agtatgtgtg 2880

ggtacatgtg aggactgggg gcacctgacc agaatgcgca agggcaaacc atttcaaatg 2940

gcagcagttc catgaagaca cgcttaaaac ctagaacttc aaaatgttcg tattctattc 3000

aaaaggaaat atatatatat atatatatat atatatatat atatataaat taaaaaggaa 3060

agaaaactaa caaccaacca accaaccaac caaccacaaa ccaccctaaa atgacagccg 3120

ctgatgtctg ggcatcagcc tttgtactct gtttttttaa gaaagtgcag aatcaacttg 3180

aagcaagctt tctctcataa cgtaatgatt atatgacaat cctgaagaaa ccacaggttc 3240

catagaacta atatcctgtc tctctctctc tctctctctc tctctttttt ttttcttttt 3300

ccttttgcca tggaatctgg gtgggagagg atactgcggg caccagaatg ctaaagtttc 3360

ctaacatttt gaagtttctg tagttcatcc ttaatcctga cacccatgta aatgtccaaa 3420

atgttgatct tccactgcaa atttcaaaag ccttgtcaat ggtcaagcgt gcagcttgtt 3480

cagcggttct ttctgaggag cggacaccgg gttacattac taatgagagt tgggtagaac 3540

tctctgagat gtgttcagat agtgtaattg ctacattctc tgatgtagtt aagtatttac 3600

agatgttaaa tggagtattt ttattttatg tatatactat acaacaatgt tcttttttgt 3660

tacagctatg cactgtaaat gcagccttct tttcaaaact gctaaatttt tcttaatcaa 3720

gaatattcaa atgtaattat gaggtgaaac aattattgta cactaacata tttagaagct 3780

gaacttactg cttatatata tttgattgta aaaacaaaaa gacagtgtgt gtgtctgttg 3840

agtgcaacaa gagcaaaatg atgctttccg cacatccatc ccttaggtga gcttcaatct 3900

aagcatcttg tcaagaaata tcctagtccc ctaaaggtat taaccacttc tgcgatattt 3960

ttccacattt tcttgtcgct tgtttttctt tgaagtttta tacactggat ttgttagggg 4020

aatgaaattt tctcatctaa aatttttcta gaagatatca tgattttatg taaagtctct 4080

caatgggtaa ccattaagaa atgtttttat tttctctatc aacagtagtt ttgaaactag 4140

aagtcaaaaa tctttttaaa atgctgtttt gttttaattt ttgtgatttt aatttgatac 4200

aaaatgctga ggtaataatt atagtatgat ttttacaata attaatgtgt gtctgaagac 4260

tatctttgaa gccagtattt ctttcccttg gcagagtatg acgatggtat ttatctgtat 4320

tttttacagt tatgcatcct gtataaatac tgatatttca ttcctttgtt tactaaagag 4380

acatatttat cagttgcaga tagcctattt attataaatt atgagatgat gaaaataata 4440

aagccagtgg aaattttcta cctaggatgc atgacaattg tcaggttgga gtgtaagtgc 4500

ttcatttggg aaattcagct tttgcagaag cagtgtttct acttgcacta gcatggcctc 4560

tgacgtgacc atggtgttgt tcttgatgac attgcttctg ctaaatttaa taaaaacttc 4620

agaaaaacct ccattttgat catcaggatt tcatctgagt gtggagtccc tggaatggaa 4680

ttcagtaaca tttggagtgt gtattcaagt ttctaaattg agattcgatt actgtttggc 4740

tgacatgact tttctggaag acatgataca cctactactc aattgttctt ttcctttctc 4800

tcgcccaaca cgatcttgta agatggattt cacccccagg ccaatgcagc taattttgat 4860

agctgcattc atttatcacc agcatattgt gttctgagtg aatccactgt ttgtcctgtc 4920

ggatgcttgc ttgatttttt ggcttcttat ttctaagtag atagaaagca ataaaaatac 4980

tatgaaatga aagaacttgt tcacaggttc tgcgttacaa cagtaacaca tctttaatcc 5040

gcctaattct tgttgttctg taggttaaat gcaggtattt taactgtgtg aacgccaaac 5100

taaagtttac agtctttctt tctgaatttt gagtatcttc tgttgtagaa taataataaa 5160

aagactatta agagcaataa attattttta agaaatcgag atttagtaaa tcctattatg 5220

tgttcaagga ccacatgtgt tctctatttt gcctttaaat ttttgtgaac caattttaaa 5280

tacattctcc tttttgccct ggattgttga catgagtgga atacttggtt tcttttctta 5340

cttatcaaaa gacagcacta cagatatcat attgaggatt aatttatccc ccctaccccc 5400

agcctgacaa atattgttac catgaagata gttttcctca atggacttca aattgcatct 5460

agaattagtg gagcttttgt atcttctgca gacactgtgg gtagcccatc aaaatgtaag 5520

ctgtgctcct ctcattttta tttttatttt tttgggagag aatatttcaa atgaacacgt 5580

gcaccccatc atcactggag gcaaatttca gcatagatct gtaggatttt tagaagaccg 5640

tgggccattg ccttcatgcc gtggtaagta ccacatctac aattttggta accgaactgg 5700

tgctttagta atgtggattt ttttcttttt taaaagagat gtagcagaat aattcttcca 5760

gtgcaacaaa atcaattttt tgctaaacga ctccgagaac aacagttggg ctgtcaacat 5820

tcaaagcagc agagagggaa ctttgcacta ttggggtatg atgtttgggt cagttgataa 5880

aaggaaacct tttcatgcct ttagatgtga gcttccagta ggtaatgatt atgtgtcctt 5940

tcttgatggc tgtaatgaga acttcaatca ctgtagtcta agacctgatc tatagatgac 6000

ctagaatagc catgtactat aatgtgatga ttctaaattt gtacctatgt gacagacatt 6060

ttcaataatg tgaactgctg atttgatgga gctactttaa gatttgtagg tgaaagtgta 6120

atactgttgg ttgaactatg ctgaagaggg aaagtgagcg attagttgag cccttgccgg 6180

gccttttttc cacctgccaa ttctacatgt attgttgtgg ttttattcat tgtatgaaaa 6240

ttcctgtgat tttttttaaa tgtgcagtac acatcagcct cactgagcta ataaagggaa 6300

acgaatgttt caaatcta 6318

<210> 42
<211> 4790
<212> DNA
<213> Homo sapiens

<400> 42
ggggaagcgc agtgcgcagg cgcaactgcc tggctctgct cgctccggcg ctccggccca 60

gctctcgcgg acaagtccag acatcgcgcg cccccccttc tccgggtccg ccccctcccc 120

cttctcggcg tcgtcgaaga taaacaatag ttggccggcg agcgcctagt gtgtctcccg 180

ccgccggatt cggcgggctg cgtgggaccg gcgggatccc ggccagccgg ccatggcggg 240

gctgtactcg ctgggagtga gcgtcttctc cgaccagggc gggaggaagt acatggagga 300

cgttactcaa atcgttgtgg agcccgaacc gacggctgaa gaaaagccct cgccgcggcg 360

gtcgctgtct cagccgttgc ctccgcggcc gtcgccggcc gcccttcccg gcggcgaagt 420

ctcggggaaa ggcccagcgg tggcagcccg agaggctcgc gaccctctcc cggacgccgg 480

ggcctcgccg gcacctagcc gctgctgccg ccgccgttcc tccgtggcct ttttcgccgt 540

gtgcgacggg cacggcgggc gggaggcggc acagtttgcc cgggagcact tgtggggttt 600

catcaagaag cagaagggtt tcacctcgtc cgagccggct aaggtttgcg ctgccatccg 660

caaaggcttt ctcgcttgtc accttgccat gtggaagaaa ctggcggaat ggccaaagac 720

tatgacgggt cttcctagca catcagggac aactgccagt gtggtcatca ttcggggcat 780

gaagatgtat gtagctcacg taggtgactc aggggtggtt cttggaattc aggatgaccc 840

gaaggatgac tttgtcagag ctgtggaggt gacacaggac cataagccag aacttcccaa 900

ggaaagagaa cgaatcgaag gacttggtgg gagtgtaatg aacaagtctg gggtgaatcg 960

tgtagtttgg aaacgacctc gactcactca caatggacct gttagaagga gcacagttat 1020

tgaccagatt ccttttctgg cagtagcaag agcacttggt gatttgtgga gctatgattt 1080

cttcagtggt gaatttgtgg tgtcacctga accagacaca agtgtccaca ctcttgaccc 1140

tcagaagcac aagtatatta tattggggag tgatggactt tggaatatga ttccaccaca 1200

agatgccatc tcaatgtgcc aggaccaaga ggagaaaaaa tacctgatgg gtgagcatgg 1260

acaatcttgt gccaaaatgc ttgtgaatcg agcattgggc cgctggaggc agcgtatgct 1320

ccgagcagat aacactagtg ccatagtaat ctgcatctct ccagaagtgg acaatcaggg 1380

aaactttacc aatgaagatg agttatacct gaacctgact gacagccctt cctataatag 1440

tcaagaaacc tgtgtgatga ctccttcccc atgttctaca ccaccagtca agtcactgga 1500

ggaggatcca tggccaaggg tgaattctaa ggaccatata cctgccctgg ttcgtagcaa 1560

tgccttctca gagaattttt tagaggtttc agctgagata gctcgagaga atgtccaagg 1620

tgtagtcata ccctcaaaag atccagaacc acttgaagaa aattgcgcta aagccctgac 1680

tttaaggata catgattctt tgaataatag ccttccaatt ggccttgtgc ctactaattc 1740

aacaaacact gtcatggacc aaaaaaattt gaagatgtca actcctggcc aaatgaaagc 1800

ccaagaaatt gaaagaaccc ctccaacaaa ctttaaaagg acattagaag agtccaattc 1860

tggccccctg atgaagaagc atagacgaaa tggcttaagt cgaagtagtg gtgctcagcc 1920

tgcaagtctc cccacaacct cacagcgaaa gaactctgtt aaactcacca tgcgacgcag 1980

acttaggggc cagaagaaaa ttggaaatcc tttacttcat caacacagga aaactgtttg 2040

tgtttgctga aatgcatctg ggaaatgagg tttttccaaa cttaggatat aagaggcttt 2100

tttaaatttg gtgccgatgt tgaacttttt ttaaggggag aaaattaaaa gaaatataca 2160

gtttgacttt ttggaattca gcagttttat cctggccttg tacttgcttg tattgtaaat 2220

gtggattttg tagatgttag ggtataagtt gctgtaaaat ttgtgtaaat ttgtatccac 2280

acaaattcag tctctgaata cacagtattc agagtctctg atacacagta attgtgacaa 2340

tagggctaaa tgtttaaaga aatcaaaaga atctattaga ttttagaaaa acatttaaac 2400

tttttaaaat acttattaaa aaatttgtat aagccacttg tcttgaaaac tgtgcaactt 2460

tttaaagtaa attattaagc agactggaaa agtgatgtat tttcatagtg acctgtgttt 2520

cacttaatgt ttcttagagc caagtgtctt ttaaacatta ttttttattt ctgatttcat 2580

aattcagaac taaatttttc atagaagtgt tgagccatgc tacagttagt cttgtcccaa 2640

ttaaaatact atgcagtatc tcttacatca gtagcatttt tctaaaacct tagtcatcag 2700

atatgcttac taaatcttca gcatagaagg aagtgtgttt gcctaaaaca atctaaaaca 2760

attcccttct ttttcatccc agaccaatgg cattattagg tcttaaagta gttactccct 2820

tctcgtgttt gcttaaaata tgtgaagttt tccttgctat ttcaataaca gatggtgctg 2880

ctaattccca acatttctta aattatttta tatcatacag ttttcattga ttatatgggt 2940

atatattcat ctaataaatc agtgaactgt tcctcatgtt gctgaatttg tagttgttgg 3000

tttattttaa tggtatgtac aagttgagta tcccttatcc aaaatgcttg ggaccagaag 3060

tgtttcagat tttttaaaat tttggaatat ttgctttata ctgagctttt gagtgttccc 3120

aatctgaaat tcaaaatgct ctaatgagca tttcctttga gcatcatgcc tgctctgaaa 3180

aagtttctga ttctggagca ttttggattt tggattttca gattagggat gcttaacctg 3240

gattaacatt ctgttgtgcc atgatcatgc tttacagtga gtgtatttta tttatttatt 3300

attttgtttg tttgtttgag atggagtctc actctgtcat ccaggctaga gtgcagtggc 3360

gtgatctcgg ctgactgcaa cctctgcctc ccgggttcaa gtgattctcc tgcctcaatc 3420

tctctcccca gaagctggga ttacaggtgt gtgccaccac acccggctaa tttttttttt 3480

tttttttgag atggagtcta gctctgtcat ccaggctgga gtgcagtggt gtgatctcgg 3540

ctccctgcaa cctctgcctt ctgggttcct gcgattctcc tgcctcagcc tcctgagtag 3600

ctgagattac aggcacgcgc cactgtgccc agccaatttt tgtattttta gtagagatgg 3660

ggtttcacat gtcagtcatg ctggtcttga tctcctgacc tcgtgatcca cccgcctcga 3720

cctcccaaag tactgggatt acaggcgtga gccaccgcat ccggcctgag ttttatgctt 3780

tcaatgtatt tcttacattt cagttcaagt gattttcatg tctcagcctc ctgagtagct 3840

ggaactacag gtgcgtgcca ccatgcctgg ctaagttttg tatttttagt agagatgggt 3900

tttcatcatg ttggccaaga tggtcttgat ctcttgacct catgatccac cagcctaggc 3960

ctcccaaagt gctgggatta caggtgtgag ccaccgtgcc cagccaacta tgccattatt 4020

taaccatgtc cacacattct ggttattttc aatattttgc agaagataat tcttgatcgg 4080

tgtgtcttat gccacaagga ttaaaatatg tattcattgc tacaaaacaa tatctcgaaa 4140

tttagcagtt taaaacaaca aatattatct ccagtttctg agcctcagaa atctgagagt 4200

ggtttagctg ggtgatagtc tcgtggtttt ggtcaagcta ccaaccaggg ctacaatctt 4260

tcgaaggtgt cattggggct agaagatctg cttcccgcaa gactcacagc tgttggcagg 4320

agacctcagt ttgttgccac atgttcccct ccagagggcc tctcacaaca tggcagttat 4380

ttgtccccag agcaagcaac accggagggc aaggaagaag ccatgatgtt ttttgtaacc 4440

tagcctctga aagtgtcata ccaattctgt attttgttgg tcacacagac caagtcaact 4500

acaacgtggg agactcctac acaaggcatg aattctagga ggtgggcatt tttaagtgtc 4560

atctggaagg aggctgtcac aacctggaag ttaaaagcat tgatattctg aaatacagcg 4620

tgtataacat tgttttagta gggtgtgcaa tagttatgtt ttggtaatag cattaatgaa 4680

caatgttatt ttcatcttcc agacatctgg aagattgctc tagtggagta aaacatctta 4740

atgtattttg tccctaaata aactatctca ctaacaaaaa aaaaaaaaaa 4790

<210> 43
<211> 1637
<212> DNA
<213> homo sapiens

<400> 43
agagggcccg ctcaccaccc cgtaggcccc gcccctgcgt ctctgcccgc cccgtggcgc 60

ccgagtgcac tgaagatggc ggctgctgta ggacggttgc tccgagcgtc ggttgcccga 120

catgtgagtg ccattccttg gggcatttct gccactgcag ccctcaggcc tgctgcatgt 180

ggaagaacga gcttgacaaa tttattgtgt tctggttcca gtcaagcaaa attattcagc 240

accagttcct catgccatgc acctgctgtc acccagcatg caccctattt taagggtaca 300

gccgttgtca atggagagtt caaagaccta agccttgatg actttaaggg gaaatatttg 360

gtgcttttct tctatccttt ggatttcacc tttgtgtgtc ctacagaaat tgttgctttt 420

agtgacaaag ctaacgaatt tcacgacgtg aactgtgaag ttgtcgcagt ctcagtggat 480

tcccacttta gccatcttgc ctggataaat acaccaagaa agaatggtgg tttgggccac 540

atgaacatcg cactcttgtc agacttaact aagcagattt cccgagacta cggtgtgctg 600

ttagaaggtt ctggtcttgc actaagaggt ctcttcataa ttgaccccaa tggagtcatc 660

aagcatttga gcgtcaacga tctcccagtg ggccgaagcg tggaagaaac cctccgcttg 720

gtgaaggcgt tccagtatgt agaaacacat ggagaagtct gcccagcgaa ctggacaccg 780

gattctccta cgatcaagcc aagtccagct gcttccaaag agtactttca gaaggtaaat 840

cagtagatca cccatgtgta tctgcacctt ctcaactgag agaagaacca cagttgaaac 900

ctgcttttat cattttcaag atggttattt gtagaaggca aggaaccaat tatgcttgta 960

ttcataagta ttactctaaa tgttttgttt ttgtaattct ggctaagacc ttttaaacat 1020

ggttagttgc tagtacaagg aatcctttat tggtaacatc ttggtggctg gctagctagt 1080

ttctacagaa cataatttgc ctctatagaa ggctattctt agatcatgtc tcaatggaaa 1140

cactcttctt tcttagcctt acttgaatct tgcctataat aaagtagagc aacacacatt 1200

gaaagcttct gatcaacggt cctgaaattt tcatcttgaa tgtctttgta ttaaactgaa 1260

ttttctttta agctaacaaa gatcataatt ttcaatgatt agccgtgtaa ctcctgcaat 1320

gaatgtttat gtgattgaag caaatgtgaa tcgtattatt ttaaaaagtg gcagagtgac 1380

ttaactgatc atgcatgatc cctcatccct gaaattgagt ttatgtagtc attttactta 1440

ttttattcat tagctaactt tgtctatgta tatttctaga tattgattag tgtaatcgat 1500

tataaaggat atttatcaaa tccagggatt gcattttgaa attataatta ttttctttgc 1560

tgaagtattc attgtaaaac atacaaaata aacatatttt aaaacatttg cattttacca 1620

ccaaaaaaaa aaaaaaa 1637

<210> 44
<211> 6582
<212> DNA
<213> Homo sapiens

<400> 44
agagggcaag gagagagcag agaacacact ttgccttctc tttggtattg agtaatatca 60

accaaattgc agacatctca acactttggc caggcagcct gctgagcaag gtacctcagc 120

cagcatggca gcctctttcc cacccacctt gggactcagt tctgccccag atgaaattca 180

gcacccacat attaaatttt cagaatggaa atttaagctg ttccgggtga gatcctttga 240

aaagacacct gaagaagctc aaaaggaaaa gaaggattcc tttgagggga aaccctctct 300

ggagcaatct ccagcagtcc tggacaaggc tgatggtcag aagccagtcc caactcagcc 360

attgttaaaa gcccacccta agttttcaaa gaaatttcac gacaacgaga aagcaagagg 420

caaagcgatc catcaagcca accttcgaca tctctgccgc atctgtggga attcttttag 480

agctgatgag cacaacagga gatatccagt ccatggtcct gtggatggta aaaccctagg 540

cctttacgaa aagaaggaaa agagagctac ttcctggccg gacctcattg ccaaggtttt 600

ccggatcgat gtgaaggcag atgttgactc gatccacccc actgagttct gccataactg 660

ctggagcatc atgcacagga agtttagcag tgccccatgt gaggtttact tcccgaggaa 720

cgtgaccatg gagtggcacc cccacacacc atcctgtgac atctgcaaca ctgcccgtcg 780

gggactcaag aggaagagtc ttcagccaaa cttgcagctc agcaaaaaac tcaaaactgt 840

gcttgaccaa gcaagacaag cccgtcagcg caagagaaga gctcaggcaa ggatcagcag 900

caaggatgtc atgaagaaga tcgccaactg cagtaagata catcttagta ccaagctcct 960

tgcagtggac ttcccagagc actttgtgaa atccatctcc tgccagatct gtgaacacat 1020

tctggctgac cctgtggaga ccaactgtaa gcatgtcttt tgccgggtct gcattctcag 1080

atgcctcaaa gtcatgggca gctattgtcc ctcttgccga tatccatgct tccctactga 1140

cctggagagt ccagtgaagt cctttctgag cgtcttgaat tccctgatgg tgaaatgtcc 1200

agcaaaagag tgcaatgagg aggtcagttt ggaaaaatat aatcaccaca tctcaagtca 1260

caaggaatca aaagagattt ttgtgcacat taataaaggg ggccggcccc gccaacatct 1320

tctgtcgctg actcggagag ctcagaagca ccggctgagg gagctcaagc tgcaagtcaa 1380

agcctttgct gacaaagaag aaggtggaga tgtgaagtcc gtgtgcatga ccttgttcct 1440

gctggctctg agggcgagga atgagcacag gcaagctgat gagctggagg ccatcatgca 1500

gggaaagggc tctggcctgc agccagctgt ttgcttggcc atccgtgtca acaccttcct 1560

cagctgcagt cagtaccaca agatgtacag gactgtgaaa gccatcacag ggagacagat 1620

ttttcagcct ttgcatgccc ttcggaatgc tgagaaggta cttctgccag gctaccacca 1680

ctttgagtgg cagccacctc tgaagaatgt gtcttccagc actgatgttg gcattattga 1740

tgggctgtct ggactatcat cctctgtgga tgattaccca gtggacacca ttgcaaagag 1800

gttccgctat gattcagctt tggtgtctgc tttgatggac atggaagaag acatcttgga 1860

aggcatgaga tcccaagacc ttgatgatta cctgaatggc cccttcactg tggtggtgaa 1920

ggagtcttgt gatggaatgg gagacgtgag tgagaagcat gggagtgggc ctgtagttcc 1980

agaaaaggca gtccgttttt cattcacaat catgaaaatt actattgccc acagctctca 2040

gaatgtgaaa gtatttgaag aagccaaacc taactctgaa ctgtgttgca agccattgtg 2100

ccttatgctg gcagatgagt ctgaccacga gacgctgact gccatcctga gtcctctcat 2160

tgctgagagg gaggccatga agagcagtga attaatgctt gagctgggag gcattctccg 2220

gactttcaag ttcatcttca ggggcaccgg ctatgatgaa aaacttgtgc gggaagtgga 2280

aggcctcgag gcttctggct cagtctacat ttgtactctt tgtgatgcca cccgtctgga 2340

agcctctcaa aatcttgtct tccactctat aaccagaagc catgctgaga acctggaacg 2400

ttatgaggtc tggcgttcca acccttacca tgagtctgtg gaagaactgc gggatcgggt 2460

gaaaggggtc tcagctaaac ctttcattga gacagtccct tccatagatg cactccactg 2520

tgacattggc aatgcagctg agttctacaa gatcttccag ctagagatag gggaagtgta 2580

taagaatccc aatgcttcca aagaggaaag gaaaaggtgg caggccacac tggacaagca 2640

tctccggaag aagatgaacc tcaaaccaat catgaggatg aatggcaact ttgccaggaa 2700

gctcatgacc aaagagactg tggatgcagt ttgtgagtta attccttccg aggagaggca 2760

cgaggctctg agggagctga tggatcttta cctgaagatg aaaccagtat ggcgatcatc 2820

atgccctgct aaagagtgcc cagaatccct ctgccagtac agtttcaatt cacagcgttt 2880

tgctgagctc ctttctacga agttcaagta taggtatgag ggaaaaatca ccaattattt 2940

tcacaaaacc ctggcccatg ttcctgaaat tattgagagg gatggctcca ttggggcatg 3000

ggcaagtgag ggaaatgagt ctggtaacaa actgtttagg cgcttccgga aaatgaatgc 3060

caggcagtcc aaatgctatg agatggaaga tgtcctgaaa caccactggt tgtacacctc 3120

caaatacctc cagaagttta tgaatgctca taatgcatta aaaacctctg ggtttaccat 3180

gaaccctcag gcaagcttag gggacccatt aggcatagag gactctctgg aaagccaaga 3240

ttcaatggaa ttttaagtag ggcaaccact tatgagttgg tttttgcaat tgagtttccc 3300

tctgggttgc attgagggct tctcctagca ccctttactg ctgtgtatgg ggcttcacca 3360

tccaagaggt ggtaggttgg agtaagatgc tacagatgct ctcaagtcag gaatagaaac 3420

tgatgagctg attgcttgag gcttttagtg agttccgaaa agcaacagga aaaatcagtt 3480

atctgaaagc tcagtaactc agaacaggag taactgcagg ggaccagaga tgagcaaaga 3540

tctgtgtgtg ttggggagct gtcatgtaaa tcaaagccaa ggttgtcaaa gaacagccag 3600

tgaggccagg aaagaaattg gtcttgtggt tttcattttt ttcccccttg attgattata 3660

ttttgtattg agatatgata agtgccttct atttcatttt tgaataattc ttcattttta 3720

taattttaca tatcttggct tgctatataa gattcaaaag agctttttaa atttttctaa 3780

taatatctta catttgtaca gcatgatgac ctttacaaag tgctctcaat gcatttaccc 3840

attcgttata taaatatgtt acatcaggac aactttgaga aaatcagtcc ttttttatgt 3900

ttaaattatg tatctattgt aaccttcaga gtttaggagg tcatctgctg tcatggattt 3960

ttcaataatg aatttagaat acacctgtta gctacagtta gttattaaat cttctgataa 4020

tatatgttta cttagctatc agaagccaag tatgattctt tatttttact ttttcatttc 4080

aagaaattta gagtttccaa atttagagct tctgcataca gtcttaaagc cacagaggct 4140

tgtaaaaata taggttagct tgatgtctaa aaatatattt catgtcttac tgaaacattt 4200

tgccagactt tctccaaatg aaacctgaat caatttttct aaatctaggt ttcatagagt 4260

cctctcctct gcaatgtgtt attctttcta taatgatcag tttactttca gtggattcag 4320

aattgtgtag caggataacc ttgtattttt ccatccgcta agtttagatg gagtccaaac 4380

gcagtacagc agaagagtta acatttacac agtgcttttt accactgtgg aatgttttca 4440

cactcatttt tccttacaac aattctgagg agtaggtgtt gttattatct ccatttgatg 4500

ggggtttaaa tgatttgctc aaagtcattt aggggtaata aatacttggc ttggaaattt 4560

aacacagtcc ttttgtctcc aaagcccttc ttctttccac cacaaattaa tcactatgtt 4620

tataaggtag tatcagaatt tttttaggat tcacaactaa tcactatagc acatgacctt 4680

gggattacat ttttatgggg caggggtaag caagttttta aatcatttgt gtgctctggc 4740

tcttttgata gaagaaagca acacaaaagc tccaaagggc cccctaaccc tcttgtggct 4800

ccagttattt ggaaactatg atctgcatcc ttaggaatct gggatttgcc agttgctggc 4860

aatgtagagc aggcatggaa ttttatatgc tagtgagtca taatgatatg ttagtgttaa 4920

ttagtttttt cttcctttga ttttattggc cataattgct actcttcata cacagtatat 4980

caaagagctt gataatttag ttgtcaaaag tgcatcggcg acattatctt taattgtatg 5040

tatttggtgc ttcttcaggg attgaactca gtatctttca ttaaaaaaca cagcagtttt 5100

ccttgctttt tatatgcaga atatcaaagt catttctaat ttagttgtca aaaacatata 5160

catattttaa cattagtttt tttgaaaact cttggttttg tttttttgga aatgagtggg 5220

ccactaagcc acactttccc ttcatcctgc ttaatccttc cagcatgtct ctgcactaat 5280

aaacagctaa attcacataa tcatcctatt tactgaagca tggtcatgct ggtttataga 5340

ttttttaccc atttctactc tttttctcta ttggtggcac tgtaaatact ttccagtatt 5400

aaattatcct tttctaacac tgtaggaact attttgaatg catgtgacta agagcatgat 5460

ttatagcaca acctttccaa taatccctta atcagatcac attttgataa accctgggaa 5520

catctggctg caggaatttc aatatgtaga aacgctgcct atggtttttt gcccttactg 5580

ttgagactgc aatatcctag accctagttt tatactagag ttttattttt agcaatgcct 5640

attgcaagtg caattatata ctccagggaa attcaccaca ctgaatcgag catttgtgtg 5700

tgtatgtgtg aagtatatac tgggacttca gaagtgcaat gtatttttct cctgtgaaac 5760

ctgaatctac aagttttcct gccaagccac tcaggtgcat tgcagggacc agtgataatg 5820

gctgatgaaa attgatgatt ggtcagtgag gtcaaaagga gccttgggat taataaacat 5880

gcactgagaa gcaagaggag gagaaaaaga tgtctttttc ttccaggtga actggaattt 5940

agttttgcct cagatttttt tcccacaaga tacagaagaa gataaagatt tttttggttg 6000

agagtgtggg tcttgcatta catcaaacag agttcaaatt ccacacagat aagaggcagg 6060

atatataagc gccagtggta gttgggagga ataaaccatt atttggatgc aggtggtttt 6120

tgattgcaaa tatgtgtgtg tcttcagtga ttgtatgaca gatgatgtat tcttttgatg 6180

ttaaaagatt ttaagtaaga gtagatacat tgtacccatt ttacattttc ttattttaac 6240

tacagtaatc tacataaata tacctcagaa atcatttttg gtgattattt tttgttttgt 6300

agaattgcac ttcagtttat tttcttacaa ataaccttac attttgttta atggcttcca 6360

agagcctttt ttttttttgt atttcagaga aaattcaggt accaggatgc aatggattta 6420

tttgattcag gggacctgtg tttccatgtc aaatgttttc aaataaaatg aaatatgagt 6480

ttcaatactt tttatatttt aatatttcca ttcattaata ttatggttat tgtcagcaat 6540

tttatgtttg aatatttgaa ataaaagttt aagatttgaa aa 6582

<210> 45
<211> 2457

<212>  DNA
<213>  Homo sapiens

<400>  45
attagatcag tgttcataag aacatctgta ggcacacata cacactctct ttacagtcag      60

ccttctgctt gccacagtca tagtgggcag tcagtgaatc ttccccaagt gctgacaatt     120

aatacctggt ttagcggcaa agattcagag aggcgtgagc agcccctctg gccttcagac     180

aaaaatctac gtaccatcag aaactatgtc tctgcagatg gtaacagtca gtaataacat     240

agccttaatt cagccaggct tctcactgat gaattttgat ggacaagttt tcttctttgg     300

acaaaaaggc tggcccaaaa gatcctgccc cactggagtt ttccatctgg atgtaaagca     360

taaccatgtc aaactgaagc ctacaatttt ctctaaggat tcctgctacc tccctcctct     420

tcgctaccca gccacttgca cattcaaagg cagcttggag tctgaaaagc atcaatacat     480

catccatgga gggaaaacac caaacaatga ggtttcagat aagatttatg tcatgtctat     540

tgtttgcaag aacaacaaaa aggttacttt tcgctgcaca gagaaagact tggtaggaga     600

tgttcctgaa gccagatatg gtcattccat taatgtggtg tacagccgag ggaaaagtat     660

gggtgttctc tttggaggac gctcatacat gccttctacc cacagaacca cagaaaaatg     720

gaatagtgta gctgactgcc tgccctgtgt tttcctggtg gattttgaat ttgggtgtgc     780

tacatcatac attcttccag aacttcagga tgggctatct tttcatgtct ctattgccaa     840

aaatgacacc atctatattt taggaggaca ttcacttgcc aataatatcc ggcctgccaa     900

cctgtacaga ataagggttg atcttcccct gggtagccca gctgtgaatt gcacagtctt     960

gccaggagga atctctgtct ccagtgcaat cctgactcaa actaacaatg atgaatttgt    1020

tattgttggt ggctatcagc ttgaaaatca aaaaagaatg atctgcaaca tcatctcttt    1080

agaggacaac aagatagaaa ttcgtgagat ggagacccca gattggaccc cagacattaa    1140

gcacagcaag atatggtttg gaagcaacat gggaaatgga actgtttttc ttggcatacc    1200

aggagacaat aaacaagttg tttcagaagg attctatttc tatatgttga aatgtgctga    1260

agatgatact aatgaagagc agacaacatt cacaaacagt caaacatcaa cagaagatcc    1320

aggggattcc actccctttg aagactctga agaattttgt ttcagtgcag aagcaaatag    1380

ttttgatggt gatgatgaat ttgacaccta taatgaagat gatgaagaag atgagtctga    1440

gacaggctac tggattacat gctgccctac ttgtgatgtg gatatcaaca cttgggtacc    1500

attctattca actgagctca acaaacccgc catgatctac tgctctcatg gggatgggca     1560

ctgggtccat gctcagtgca tggatctggc agaacgcaca ctcatccatc tgtcagcagg    1620

aagcaacaag tattactgca atgagcatgt ggagatagca agagctctac acactcccca    1680

aagagtccta cccttaaaaa agcctccaat gaaatccctc cgtaaaaaag gttctggaaa    1740

aatcttgact cctgccaaga aatcctttct tagaaggttg tttgattagt tttgcaaaag    1800

cctttcagat tcaggtgtat ggaatttttg aatctatttt taaaatcata acattgattt 1860

taaaaataca tttttgttta tttaaaatgc ctatgttttc ttttagttac atgaattaag 1920

ggccagaaaa aagtgtttat aatgcaatga taaataaagt cattctagac cctatacatt 1980

ttgaaaatat tttacccaaa tactcaattt actaatttat tcttcactga ggatttctga 2040

tctgattttt tattcaacaa accttaaaca cccagaagca gtaataatca tcgaggtatg 2100

tttatattta ttatataagt cttggtaaca aataacctat aaagtgttta tgacaaattt 2160

agccaataaa gaaattaaca cccaaaagaa ttaaattgat tattttgtgc aacataacaa 2220

ttcggcagtt ggccaaaact taaaagcaag atctactaca tcccacatta gtgttcttta 2280

tataccttca agcaaccctt tggattatgc ccatgaacaa gttagtttct catagcttta 2340

cagatgtaga tataaatata aatatatgta tacatataga tagataatgt tctccactga 2400

cacaaaagaa gaaataaata atctacatca aaaaaaaaaa aaaaaaaaaa aaaaaaa 2457

<210> 46
<211> 4903
<212> DNA
<213> Homo sapiens

<400> 46
gtcgtttgcg gcggcgcagg cgcggtgcgg gcggcggacg ggcgggcgct tcgccgtttg 60

aatggctgcg ggcccgggcc ctcacctcac ctgaggtccg gccgcccagg ggtgcgctat 120

gccgtcggga ggtgaccagt cgccaccgcc cccgcctccc cctccggcgg cggcagcctc 180

ggatgaggag gaggaggacg acggcgaggc ggaagacgcc gcgccgcctg ccgagtcgcc 240

cacccctcag atccagcagc ggttcgacga gctgtgcagc cgcctcaaca tggacgaggc 300

ggcgcgggcc gaggcctggg acagctaccg cagcatgagc gaaagctaca cgctggaggg 360

aaatgatctt cattggttag catgtgcctt atatgtggct tgcagaaaat ctgttccaac 420

tgtaagcaaa gggacagtgg aaggaaacta tgtatcttta actagaatcc tgaaatgttc 480

agagcagagc ttaatcgaat tttttaataa gatgaagaag tgggaagaca tggcaaatct 540

acccccacat ttcagagaac gtactgagag attagaaaga aacttcactg tttctgctgt 600

aatttttaag aaatatgaac ccatttttca ggacatcttt aaataccctc aagaggagca 660

acctcgtcag cagcgaggaa ggaaacagcg gcgacagccc tgtactgtgt ctgaaatttt 720

ccatttttgt tgggtgcttt ttatatatgc aaaaggtaat ttccccatga ttagtgatga 780

tttggtcaat tcttatcacc tgctgctgtg tgctttggac ttagtttatg gaaatgcact 840

tcagtgttct aatcgtaaag aacttgtgaa ccctaatttt aaaggcttat ctgaagattt 900

tcatgctaaa gattctaaac cttcctctga ccccccttgt atcattgaga aactgtgttc 960

cttacatgat ggcctagttt tggaagcaaa ggggataaag gaacatttct ggaaacccta 1020

tattaggaaa ctttatgaaa aaaagctcct taagggaaaa gaagaaaatc tcactgggtt 1080

tctagaacct gggaactttg gagagagttt taaagccatc aataaggcct atgaggagta 1140

tgttttatct gttgggaatt tagatgagcg gatatttctt ggagaggatg ctgaggagga 1200

aattgggact ctctcaaggt gtctgaacgc tggttcagga acagagactg ctgaaagggt 1260

gcagatgaaa aacatcttac agcagcattt tgacaagtcc aaagcactta gaatctccac 1320

accactaact ggtgttaggt acattaagga gaatagccct tgtgtgactc cagtttctac 1380

agctacgcat agcttgagtc gtcttcacac catgctgaca ggcctcagga atgcaccaag 1440

tgagaaactg gaacagattc tcaggacatg ttccagagat ccaacccagg ctattgctaa 1500

cagactgaaa gaaatgtttg aaatatattc tcagcatttc cagccagacg aggatttcag 1560

taattgtgct aaagaaattg ccagcaaaca ttttcgtttt gcggagatgc tttactataa 1620

agtattagaa tctgttattg agcaggaaca aaaaagacta ggagacatgg atttatctgg 1680

tattctggaa caagatgcgt tccacagatc tctcttggcc tgctgccttg aggtcgtcac 1740

tttttcttat aagcctcctg ggaattttcc atttattact gaaatatttg atgtgcctct 1800

ttatcatttt tataaggtga tagaagtatt cattagagca gaagatggcc tttgtagaga 1860

ggtggtaaaa caccttaatc agattgaaga acagatctta gatcatttgg catggaaacc 1920

agagtctcca ctctgggaaa aaattagaga caatgaaaac agagttccta catgtgaaga 1980

ggtcatgcca cctcagaacc tggaaagggc agatgaaatt tgcattgctg gctccccttt 2040

gactcccaga agggtgactg aagttcgtgc tgatactgga ggacttggaa ggagcataac 2100

atctccaacc acattatacg ataggtacag ctccccacca gccagcacta ccagaaggcg 2160

gctatttgtt gagaatgata gcccctctga tggagggacg cctgggcgca tgcccccaca 2220

gcccctagtc aatgctgtcc ctgtgcagaa tgtatctggg gagactgttt ctgtcacacc 2280

agttcctgga cagactttgg tcaccatggc aaccgccact gtcacagcca acaatgggca 2340

aacggtaacc attcctgtgc aaggtattgc caatgaaaat ggagggataa cattcttccc 2400

tgtccaagtc aatgttgggg ggcaggcaca agctgtgaca ggctccatcc agcccctcag 2460

tgctcaggcc ctggctggaa gtctgagctc tcaacaggtg acaggaacaa ctttgcaagt 2520

ccctggtcaa gtggccattc aacagatttc cccaggtggc caacagcaga agcaaggcca 2580

gtctgtaacc agcagtagta atagacccag gaagaccagc tctttatcgc ttttctttag 2640

aaaggtatac catttagcag ctgtccgcct tcgggatctc tgtgccaaac tagatatttc 2700

agatgaattg aggaaaaaaa tctggacctg ctttgaattc tccataattc agtgtcctga 2760

acttatgatg gacagacatc tggaccagtt attaatgtgt gccatttatg tgatggcaaa 2820

ggtcacaaaa gaagataagt ccttccagaa cattatgcgt tgttatagga ctcagccgca 2880

ggcccggagc caggtgtata gaagtgtttt gataaaaggg aaaagaaaaa gaagaaattc 2940

tggcagcagt gatagcagaa gccatcagaa ttctccaaca gaactaaaca aagatagaac 3000

cagtagagac tccagtccag ttatgaggtc aagcagcacc ttgccagttc cacagcccag 3060

cagtgctcct cccacaccta ctcgcctcac aggtgccaac agtgacatgg aagaagagga 3120

gaggggagac ctcattcagt tctacaacaa catctacatc aaacagatta agacatttgc 3180

catgaagtac tcacaggcaa atatggatgc tcctccactc tctccctatc catttgtaag 3240

aacaggctcc cctcgccgaa tacagttgtc tcaaaatcat cctgtctaca tttccccaca 3300

taaaaatgaa acaatgcttt ctcctcgaga aaagattttc tattacttca gcaacagtcc 3360

ttcaaagaga ctgagagaaa ttaatagtat gatacgcaca ggagaaactc ctactaaaaa 3420

gagaggaatt cttttggaag atggaagtga atcacctgca aaaagaattt gcccagaaaa 3480

tcattctgcc ttattacgcc gtctccaaga tgtagctaat gaccgtggtt cccactgagg 3540

ttagtctctt gtattaaact cttcacaaaa tctgtttagc agcagccttt aatgcatcta 3600

gattatggag cttttttcct taatccagct gatgagttac agcctgttag taacatgagg 3660

ggacattttg gtgagaaatg ggacttaact ccttccagtg tccttagaac attttaattc 3720

atcccaactg tctttttttc cctaccattc agtgattact gtcaaggctg cttagaatcc 3780

aaacttggat tttgactctg gcaaagcttt tagaaatac tgcaagaaaa tgatgtgtac 3840

ccaaacgtga gcataggagg cttctgttga cgtactccaa cagaagaact gtgtttcaag 3900

ttcaatccta cctgttttgt ggtcagctgt agtcctcata aaaagcaaaa caaaaattag 3960

gtattttgtc ctaaaacacc tggtaggagt gtgtgatttt ttgcattcct gacaaaggag 4020

agcacaccca ggtttggagg tcctaggtca ttagccctcg tctcccgttc cctttgtgca 4080

catcttccct ctccccattc ggtgtggtgc agtgtgaaaa gtccttgatt gttcgggtgt 4140

gcaatgtctg agtgaacctg tataagtgga ggcactttag ggctgtaaaa tgcatgattt 4200

tgtaacccag attttgctgt atatttgtga tagcactttc tacaatgtga actttattaa 4260

atacaaaact tccaggctaa acatccaata ttttctttaa tgcttttata tttttttaaa 4320

atgttaaaac ccctatagcc accttttggg aatgttttaa attctccagt tttttgttat 4380

atagggatca accagctaag aaaagatttt aatcaagttg aattgagggg attaatatga 4440

aaacttatga cctcttcctt taggagggag ttatctaaaa gaaatgtcta ttaaggtgat 4500

atatttaaaa atatttttgg gtgttcctgg cagtttaaaa aaattggttg gagaatttag 4560

gtttttatta gtaccatagt accatttata caaattagaa aatgttattt aacagctgaa 4620

ttatctatac atatctttat taatcactat tgttccagca gttttcaagt caaattaata 4680

atcttattag ggagaaaatt caattgtaaa ttgaatcagt ataaacaaag ttactaggta 4740

acttcatatt gctgagagaa atatggaact tacattgttc aattagaata gtgttctgca 4800

aaaatattta taaaacttct caagatactg ctactgtaat tttatatgaa gataagtgta 4860

tttttcaata aagcatttat aaattaaaaa aaaaaaaaaa aaa 4903

<210> 47
<211> 5189
<212> DNA
<213> Homo sapiens

<400> 47
ggactgcgaa aggagcaggg ttgcggagct agggctccag cctgcggccg cgcattcttg 60

cgtctggcca gccgcgagct ctaagggtcg gccccgcccg gtccgccccc gcggctccct 120

gccaggctct cgcgggcgcg ctcggggtgg ggcctcgcgg ctggcggaga tgcggccggg 180

gctgcgcggt ggtgatgcga gcctgctggg cggcgcgccg gggcagccgg agccgcgcgc 240

cgcggcgctg taatcggaca ccaagagcgc tcgcccccgg cctccggcca ctttccattc 300

actccgaggt gcttgattga gcgacgcgga gaagagctcc gggtgccgcg gcactgcagc 360

gctgagattc ctttacaaag aaactcagag gaccgggaag aaagaatttc acctttgcga 420

cgtgctagaa aataaggtcg tctgggaaaa ggactggaga cacaagcgca tccaaccccg 480

gtagcaaact gatgactttt ccgtgctgat ttctttcaac ctcggtattt tcccttggat 540

attaacttgc atatctgaag aaatggcatt ccggacaatt tgcgtgttgg ttggagtatt 600

tatttgttct atctgtgtga aaggatcttc ccagccccaa gcaagagttt atttaacatt 660

tgatgaactt cgagaaacca agacctctga atacttcagc ctttcccacc atcctttaga 720

ctacaggatt ttattaatgg atgaagatca ggaccggata tatgtgggaa gcaaagatca 780

cattctttcc ctgaatatta acaatataag tcaagaagct ttgagtgttt tctggccagc 840

atctacaatc aaagttgaag aatgcaaaat ggctggcaaa gatcccacac acggctgtgg 900

gaactttgtc cgtgtaattc agactttcaa tcgcacacat ttgtatgtct gtgggagtgg 960

cgctttcagt cctgtctgta cttacttgaa cagagggagg agatcagagg accaagtttt 1020

catgattgac tccaagtgtg aatctggaaa aggacgctgc tctttcaacc ccaacgtgaa 1080

cacggtgtct gttatgatca atgaggagct tttctctgga atgtatatag atttcatggg 1140

gacagatgct gctatttttc gaagtttaac caagaggaat gcggtcagaa ctgatcaaca 1200

taattccaaa tggctaagtg aacctatgtt tgtagatgca catgtcatcc cagatggtac 1260

tgatccaaat gatgctaagg tgtacttctt cttcaaagaa aaactgactg acaataacag 1320

gagcacgaaa cagattcatt ccatgattgc tcgaatatgt cctaatgaca ctggtggact 1380

gcgtagcctt gtcaacaagt ggaccacttt cttaaaggcg aggctggtgt gctcggtaac 1440

agatgaagac ggcccagaaa cacactttga tgaattagag gatgtgtttc tgctggaaac 1500

tgataacccg aggacaacac tagtgtatgg catttttaca acatcaagct cagttttcaa 1560

aggatcagcc gtgtgtgtgt atcatttatc tgatatacag actgtgttta atgggccttt 1620

tgcccacaaa gaagggccca atcatcagct gatttcctat cagggcagaa ttccatatcc 1680

tcgccctgga acttgtccag gaggagcatt tacacccaat atgcgaacca ccaaggagtt 1740

cccagatgat gttgtcactt ttattcggaa ccatcctctc atgtacaatt ccatctaccc 1800

aatccacaaa aggcctttga ttgttcgtat tggcactgac tacaagtata caaagatagc 1860

tgtggatcga gtgaacgctg ctgatgggag ataccatgtc ctgtttctcg gaacagatcg 1920

gggtactgtg caaaaagtgg ttgttcttcc tactaacaac tctgtcagtg gcgagctcat 1980

tctggaggag ctggaagtct ttaagaatca tgctcctata acaacaatga aaatttcatc 2040

taaaaagcaa cagttgtatg tgagttccaa tgaaggggtt tcccaggtat ctctgcaccg 2100

ctgccacatc tatggtacag cctgtgctga ctgctgcctg gcgcgggacc cttattgcgc 2160

ctgggatggc cattcctgtt ccagattcta cccaactggg aaacggagga gccgaagaca 2220

agatgtgaga catggaaacc cactgactca atgcagagga tttaatctaa aagcatacag 2280

aaatgcagct gaaattgtcc agtatggagt aaaaaataac accacttttc tggagtgtgc 2340

ccccaagtct ccgcaggcat ctatcaagtg gctgttacag aaagacaaag acaggaggaa 2400

agaggttaag ctgaatgaac gaataatagc cacttcacag ggactcctga tccgctctgt 2460

tcagggttct gaccaaggac tttatcactg cattgctaca gaaaatagtt tcaagcagac 2520

catagccaag atcaacttca aagttttaga ttcagaaatg gtggctgttg tgacggacaa 2580

atggtcccca tggacctggg ccagctctgt gagggcttta cccttccacc cgaaggacat 2640

catgggggca ttcagccact cagaaatgca gatgattaac caatattgca aagacactcg 2700

gcagcaacat cagcagggag atgaatcaca gaaaatgaga ggggactatg gcaagttaaa 2760

ggccctcatc aatagtcgga aaagtagaaa caggaggaat cagttgccag agtcataata 2820

ttttcttatg tgggtcttat gcttccatta acaaatgctc tgtcttcaat gatcaaattt 2880

tgagcaaaga aacttgtgct ttaccaaggg gaattactga aaaaggtgat tactcctgaa 2940

gtgagtttta cacgaactga aatgagcatg cattttcttg tatgatagtg actagcacta 3000

gacatgtcat ggtcctcatg gtgcatataa atatatttaa cttaacccag attttattta 3060

tatctttatt caccttttct tcaaaatcga tatggtggct gcaaaactag aattgttgca 3120

tccctcaatt gaatgagggc catatccctg tggtattcct ttcctgcttt ggggctttag 3180

aattctaatt gtcagtgatt ttgtatatga aaacaagttc caaatccaca gcttttacgt 3240

agtaaaagtc ataaatgcat atgacagaat ggctatcaaa agaaatagaa aaggaagaca 3300

gcatttaaag ttgtataaaa acatgagtta ttcataaaga gaaaatgatg agtttttatg 3360

gttccaatga aatatgttgg ggttttttta agattgtaaa aataatcagt tactggtatc 3420

tgtcactgac ctttgtttcc ttattcagga agataaaaat cagtaaccta ccccatgaag 3480

atatttggtg ggagttatat cagtgaagca gtttggttta tattcttatg ttatcacctt 3540

ccaaacaaaa gcacttactt tttttggaag ttatttattt tagactcaaa gaatataatc 3600

tggcactact cagttattac tgtttgttct cttattccct agtctgtgtg gcaaattaaa 3660

caatataaga aggaaaaatt tgaagtatta gacttctaaa taaggtgtga aatcatcaaa 3720

aagaaaaatc aaagtagaaa ctactaattt tttaagagga atttataaca aatatggcta 3780

gttttcaact tcagtactca aattcaatga ttcttccttt tattaaaacc agtctcagat 3840

atcatactga tttttaagtc aacactatat attttatgat cttttcagtg tgatggcaag 3900

gtgcttgtta tgtctagaaa gtaagaaaac aatatgagga gacattctgt ctttcaaaag 3960

gtaatggtac atacgttcac tggtctctaa gtgtaaaagt agtaaatttt gtgatgaata 4020

aaataattat ctcctaattg tatgttagaa taattttatt agaataattt catactgaaa 4080

ttattttctc caaataaaaa ttagatggaa aaatgtgaaa aaaattattc atgctctcat 4140

atatatttta aaaacactac ttttgctttt ttatttacct tttaagacat tttcatgctt 4200

ccaggtaaaa acagatattg taccatgtac ctaatccaaa tatcatataa acattttatt 4260

tatagttaat aatctatgat gaaggtaatt aaagtagatt atggcctttt taagtattgc 4320

agtctaaaac ttcaaaaact aaaatcattg tcaaaattaa tatgattatt aatcagaata 4380

tcagaatatg attcactatt taaactatga taaattatga taatatatga ggaggcctcg 4440

ctatagcaaa aatagttaaa atgctgacat aacaccaaac ttcatttttt aaaaaatctg 4500

ttgttccaaa tgtgtataat tttaaagtaa tttctaaagc agtttattat aatggtttgc 4560

ctgcttaaaa ggtataatta aacttctttt ctcttctaca ttgacacaca gaaatgtgtc 4620

aatgtaaagc caaaaccatc ttctgtgttt atggccaatc tattctcaaa gttaaaagta 4680

aaattgtttc agagtcacag ttccctttat ttcacataag cccaaactga tagacagtaa 4740

cggtgtttag ttttatacta tatttgtgct atttaattct ttctattttc acaattatta 4800

aattgtgtac actttcatta cttttaaaaa tgtagaaatt cttcatgaac ataactctgc 4860

tgaatgtaaa agaaaatttt ttttcaaaaa tgctgttaat gtatactact ggtggttgat 4920

tggttttatt ttatgtagct tgacaattca gtgacttaat atctattcca tttgtattgt 4980

acataaaatt ttctagaaat acactttttt ccaaagtgta agtttgtgaa tagattttag 5040

catgatgaaa ctgtcataat ggtgaatgtt caatctgtgt aagaaaacaa actaaatgta 5100

gttgtcacac taaaatttaa ttggatattg atgaaatcat tggcctggca aaataaaaca 5160

tgttgaattc cccaaaaaaa aaaaaaaaa 5189

<210> 48
<211> 2164

<212>  DNA
<213>  Homo sapiens

<400>  48
ataaatatca gagtgtgctg ctgtggcttt gtggagctgc cagagtaaag caaagagaaa      60

ggaagcaggc ccgttggaag tggttgtgac aaccccagca atgtggagaa gcctggggct     120

tgccctggct ctctgtctcc tcccatcggg aggaacagag agccaggacc aaagctcctt     180

atgtaagcaa cccccagcct ggagcataag agatcaagat ccaatgctaa actccaatgg     240

ttcagtgact gtggttgctc ttcttcaagc cagctgatac ctgtgcatac tgcaggcatc     300

taaattagaa gacctgcgag taaaactgaa gaaagaagga tattctaata tttcttatat     360

tgttgttaat catcaaggaa tctcttctcg attaaaatac acacatctta agaataaggt     420

ttcagagcat attcctgttt atcaacaaga agaaaaccaa acagatgtct ggactctttt     480

aaatggaagc aaagatgact tcctcatata tgatagatgt ggccgtcttg tatatcatct     540

tggtttgcct ttttccttcc taactttccc atatgtagaa gaagccatta agattgctta     600

ctgtgaaaag aaatgtggaa actgctctct cacgactctc aaagatgaag acttttgtaa     660

acgtgtatct ttggctactg tggataaaac agttgaaact ccatcgcctc attaccatca     720

tgagcatcat cacaatcatg gacatcagca ccttggcagc agtgagcttt cagagaatca     780

gcaaccagga gcaccaaatg ctcctactca tcctgctcct ccaggccttc atcaccacca     840

taagcacaag ggtcagcata ggcagggtca cccagagaac cgagatatgc cagcaagtga     900

agatttacaa gatttacaaa agaagctctg tcgaaagaga tgtataaatc aattactctg     960

taaattgccc acagattcag agttggctcc taggagctga tgctgccatt gtcgacatct    1020

gatatttgaa aaaacagggt ctgcaatcac ctgacagtgt aaagaaaacc tcccatcttt    1080

atgtagctga cagggacttc gggcagagga gaacataact gaatcttgtc agtgacgttt    1140

gcctccagct gcctgacaaa taagtcagca gcttataccc acagaagcca gtgccagttg    1200

acgctgaaag aatcaggcaa aaaagtgaga atgaccttca aactaaatat ttaaaatagg    1260

acatactccc caatttagtc tagacacaat ttcatttcca gcatttttat aaactaccaa    1320

attagtgaac caaaaataga aattagattt gtgcaaacat ggagaaatct actgaattgg    1380

cttccagatt ttaaatttta tgtcatagaa atattgactc aaaccatatt ttttatgatg    1440

gagcaactga aaggtgattg cagcttttgg ttaatatgtc tttttttttc tttttccagt    1500

gttctatttg ctttaatgag aatagaaacg taaactatga cctaggggtt tctgttggat    1560

aattagcagt ttagaatgga ggaagaacaa caaagacatg ctttccattt ttttctttac    1620

ttatctctca aaacaatatt actttgtctt ttcaatcttc tacttttaac taataaaata    1680

agtggatttt gtattttaag atccagaaat acttaacacg tgaatatttt gctaaaaaag    1740

catatataac tattttaaat atccatttat cttttgtata tctaagactc atcctgattt    1800

ttactatcac acatgaataa agcctttgta tctttctttc tctaatgttg tatcatactc 1860

ttctaaaact tgagtggctg tcttaaaaga tataagggga aagataatat tgtctgtctc 1920

tatattgctt agtaagtatt tccatagtca atgatggttt aataggtaaa ccaaacccta 1980

taaacctgac ctcctttatg gttaatacta ttaagcaaga atgcagtaca gaattggata 2040

cagtacggat ttgtccaaat aaattcaata aaaaccttaa agctgaaaaa aaaaaaaaaa 2100

aaaaaaaaaa aaaaaaaaaa aaaaaaaaaa aaaaaaaaaa aaaaaaaaaa aaaaaaaaaa 2160

aaaa 2164

<210> 49
<211> 3212
<212> DNA
<213> Homo sapiens

<400> 49
gaacccggtg gctgcacaga caaaaaagcc ccgaatggct ggagggcgtt cagctgttaa 60

cagccttttg gggcagagca cggatttgac agctccacaa cgtgaggata tccactgacc 120

ccgcgagacg gaggagaacg cttccccgaa attctctgcc caccaaagcc agcgctgcaa 180

ggttgcaact ttcaaacttt gtttttccag aaagaagact gccctttcgt gtacaaggag 240

agggtgagag ggtgacctag cttgtagatc ggctgaaggc accagtggtt ccaaatgtca 300

cccagatgtg tgttttcatg acgatttgat ttctctgatt ttatttttac atttttcatt 360

ttaaaaatac aaagcaattt ttttggggca tgctgaaagg taactgaaga ccgcaaagga 420

aaaactattg tcatggctga aggagagaat gaagtgagat gggatggact ctgcagcaga 480

gattcaacta ctagggagac agcattggaa aacattaggc aaaccatttt gaggaaaacc 540

gagtatcttc gttcggtgaa agaaacacct catcgtccat cagacgggct ttcaaatacc 600

gagtcttcgg atgggttgaa taagctactt gctcatctgc ttatgctttc taagaggtgt 660

cccttcaaag atgtgagaga gaaaagtgag tttattctga agagcatcca ggaacttggc 720

attagaattc ctcgaccact aggacaggga ccaagcagat tcatcccaga aaaggagatc 780

ctccaagtgg ggagtgaaga cgcacagatg catgctttat ttgcagattc ttttgctgct 840

ttgggccgtt tggataacat tacgttagtg atggttttcc acccacaata tttagaaagt 900

ttcttaaaaa ctcagcacta tctactgcaa atggatgggc cgttacccct acattatcgt 960

cactacattg gaataatggc tgcggcaaga catcagtgct cctacttagt gaacctgcat 1020

gtaaatgatt tccttcatgt tggtggggac cccaagtggc tcaatggttt agagaatgct 1080

cctcaaaaac tacagaattt aggagaactt aacaaagtgt tagcccatag accttggctt 1140

attaccaaag aacacattga gggactttta aaagctgaag agcacagctg gtcccttgcg 1200

gaattggtac atgcagtagt tttactcaca cactatcatt ctcttgcctc attcacattc 1260

ggctgtggaa tcagtccaga aattcattgt gatggtggcc acacattcag acctccttct 1320

gttagcaact actgcatctg tgacattaca aatggcaatc acagtgtgga tgagatgccg 1380

gtcaactcag cagaaaatgt ttctgtaagt gattctttct ttgaggttga agccctcatg 1440

gaaaagatga ggcagttaca ggaatgtcga gatgaagaag aggcaagtca ggaagagatg 1500

gcttcacgtt ttgaaataga aaaaagagag agtatgtttg tcttctcttc agatgatgaa 1560

gaagttacac cagcaagagc tgtatctcgt cattttgagg atactagtta tggctataaa 1620

gatttctcta gacatgggat gcatgttcca acatttcgtg tccaggacta ttgctgggaa 1680

gatcatggtt attctttggt aaatcgcctt tatccagatg tgggacagtt gattgatgaa 1740

aaatttcaca ttgcttacaa tcttacttat aatacaatgg caatgcacaa agatgttgat 1800

acctcaatgc ttagacgggc aatttggaac tatattcact gcatgtttgg aataagatat 1860

gatgattatg actatggtga aattaaccag ctattggatc gtagctttaa agtttatatc 1920

aaaactgttg tttgcactcc tgaaaaggtt accaaaagaa tgtatgatag cttctggagg 1980

cagttcaagc actctgagaa ggttcatgtt aatctgcttc ttatagaagc taggatgcaa 2040

gcagaactcc tttatgctct gagagccatt acccgctata tgacctgatg cctttccttc 2100

attaaagatg attctggaat gatcagcaga tatagtctac aagggggaag gtactaagcc 2160

ccaggaccaa tggtagacaa aataattcag aaatccattg tgccatgatt cctttagttt 2220

ctgctatttt tctgtggaaa accactgctg gcacaagcag tgactgtttg gcagcttcaa 2280

gtttagagct gtgaagacag gctgccattc acagtatttt gctttttgac agtacaagat 2340

gctgtgtaac tgttttaata cagcaaatag taactctcca aatcctgttg cttttatgtt 2400

aaataagata acaagaattg gagcatgcaa agaatgggac ttggataatg acttaagctt 2460

tatatgtaaa gaattttaga agatcttggt gctgctattc ctgctggagg aatgaataga 2520

tggctgtttc agttaagcta ttagtaataa aagtgaacat tgctactatc tgagcctaca 2580

tacataactt gtgtgatttc aaattaaact tgcattatgt gttaattttc ttgcatctaa 2640

aaaagcatag aattcctact cacacagctc agcaacaacc attttgatgg taacagttaa 2700

tttctttcat tagtttttta aattcagggt tctggatatt aaattaaaat ggcattctta 2760

aagattttct tcaaaaagca atcctaaatg aaagtgtgta aattataaga agctggcgat 2820

cttttgatat gctgtttcac aggatcctga cactggaggg cagctgtctt gtgcattact 2880

tgtgtttcca gcaccaaagt tgtgggacat gttgctgtag actgctgcgc agtcctgggt 2940

gcattcagtc tctctgcctc tgcctgcctc ctggtcccca ctttaaaggc tgtgcagctc 3000

cttaaataat aaagctggaa aatattttta gtcgggttat caaatttgat ttacaaaaac 3060

gctaactttg tttgaaatgc aaacaggttt gaaaatatgt attaagtact ttgtattctg 3120

gaagcgtgaa ttgcttttga agtctgtcag tattactggt atttttaaat aaagaagaat 3180

ttttctccaa ttttaaaaaa aaaaaaaaaa aa 3212

<210> 50
<211> 4086
<212> DNA
<213> Homo sapiens

<400> 50
gtcgagcggg agcagaggag gcgagggagg agggccagag aggcagttgg aagatggcgg 60

acgaggcggc cctcgccctt cagcccggcg gctccccctc ggcggcgggg gccgacaggg 120

aggccgcgtc gtcccccgcc ggggagccgc tccgcaagag gccgcggaga gatggtcccg 180

gcctcgagcg gagcccgggc gagcccggtg gggcggcccc agagcgtgag gtgccggcgg 240

cggccagggg ctgcccgggt gcggcggcgg cggcgctgtg gcgggaggcg gaggcagagg 300

cggcggcggc aggcggggag caagaggccc aggcgactgc ggcggctggg gaaggagaca 360

atgggccggg cctgcagggc ccatctcggg agccaccgct ggccgacaac ttgtacgacg 420

aagacgacga cgacgagggc gaggaggagg aagaggcggc ggcggcggcg attgggtacc 480

gagataacct tctgttcggt gatgaaatta tcactaatgg ttttcattcc tgtgaaagtg 540

atgaggagga tagagcctca catgcaagct ctagtgactg gactccaagg ccacggatag 600

gtccatatac ttttgttcag caacatctta tgattggcac agatcctcga acaattctta 660

aagatttatt gccggaaaca atacctccac ctgagttgga tgatatgaca ctgtggcaga 720

ttgttattaa tatcctttca gaaccaccaa aaaggaaaaa aagaaaagat attaatacaa 780

ttgaagatgc tgtgaaatta ctgcaagagt gcaaaaaaat tatagttcta actggagctg 840

gggtgtctgt ttcatgtgga atacctgact tcaggtcaag ggatggtatt tatgctcgcc 900

ttgctgtaga cttcccagat cttccagatc ctcaagcgat gtttgatatt gaatatttca 960

gaaaagatcc aagaccattc ttcaagtttg caaaggaaat atatcctgga caattccagc 1020

catctctctg tcacaaattc atagccttgt cagataagga aggaaaacta cttcgcaact 1080

atacccagaa catagacacg ctggaacagg ttgcgggaat ccaaaggata attcagtgtc 1140

atggttcctt tgcaacagca tcttgcctga tttgtaaata caaagttgac tgtgaagctg 1200

tacgaggaga tatttttaat caggtagttc ctcgatgtcc taggtgccca gctgatgaac 1260

cgcttgctat catgaaacca gagattgtgt tttttggtga aaatttacca gaacagtttc 1320

atagagccat gaagtatgac aaagatgaag ttgacctcct cattgttatt gggtcttccc 1380

tcaaagtaag accagtagca ctaattccaa gttccatacc ccatgaagtg cctcagatat 1440

taattaatag agaacctttg cctcatctgc attttgatgt agagcttctt ggagactgtg 1500

atgtcataat taatgaattg tgtcataggt taggtggtga atatgccaaa ctttgctgta 1560

accctgtaaa gctttcagaa attactgaaa aacctccacg aacacaaaaa gaattggctt 1620

atttgtcaga gttgccaccc acacctcttc atgtttcaga agactcaagt tcaccagaaa 1680

gaacttcacc accagattct tcagtgattg tcacactttt agaccaagca gctaagagta 1740

atgatgattt agatgtgtct gaatcaaaag gttgtatgga agaaaaacca caggaagtac 1800

aaacttctag gaatgttgaa agtattgctg aacagatgga aaatccggat ttgaagaatg 1860

ttggttctag tactggggag aaaaatgaaa gaacttcagt ggctggaaca gtgagaaaat 1920

gctggcctaa tagagtggca aaggagcaga ttagtaggcg gcttgatggt aatcagtatc 1980

tgtttttgcc accaaatcgt tacattttcc atggcgctga ggtatattca gactctgaag 2040

atgacgtctt atcctctagt tcttgtggca gtaacagtga tagtgggaca tgccagagtc 2100

caagtttaga agaacccatg gaggatgaaa gtgaaattga agaattctac aatggcttag 2160

aagatgagcc tgatgttcca gagagagctg gaggagctgg atttgggact gatggagatg 2220

atcaagaggc aattaatgaa gctatatctg tgaaacagga agtaacagac atgaactatc 2280

catcaaacaa atcatagtgt aataattgtg caggtacagg aattgttcca ccagcattag 2340

gaactttagc atgtcaaaat gaatgtttac ttgtgaactc gatagagcaa ggaaaccaga 2400

aaggtgtaat atttataggt tggtaaaata gattgttttt catggataat ttttaacttc 2460

attatttctg tacttgtaca aactcaacac taactttttt ttttttaaaa aaaaaaaggt 2520

actaagtatc ttcaatcagc tgttgggtca agactaactt tcttttaaag gttcatttgt 2580

atgataaatt catatgtgta tatataattt tttttgtttt gtctagtgag tttcaacatt 2640

tttaaagttt tcaaaaagcc atcggaatgt taaattaatg taaagggaca gctaatctag 2700

accaaagaat ggtattttca cttttctttg taacattgaa tggtttgaag tactcaaaat 2760

ctgttacgct aaacttttga ttctttaaca caattatttt taaacactgg cattttccaa 2820

aactgtggca gctaactttt taaaatctca aatgacatgc agtgtgagta gaaggaagtc 2880

aacaatatgt ggggagagca ctcggttgtc tttactttta aaagtaatac ttggtgctaa 2940

gaatttcagg attattgtat ttacgttcaa atgaagatgg cttttgtact tcctgtggac 3000

atgtagtaat gtctatattg gctcataaaa ctaacctgaa aaacaaataa atgctttgga 3060

aatgtttcag ttgctttaga aacattagtg cctgcctgga tccccttagt tttgaaatat 3120

ttgccattgt tgtttaaata cctatcactg tggtagagct tgcattgatc ttttccacaa 3180

gtattaaact gccaaaatgt gaatatgcaa agcctttctg aatctataat aatggtactt 3240

ctactgggga gagtgtaata ttttggactg ctgttttcca ttaatgagga gagcaacagg 3300

cccctgatta tacagttcca aagtaataag atgttaattg taattcagcc agaaagtaca 3360

tgtctcccat tgggaggatt tggtgttaaa taccaaactg ctagccctag tattatggag 3420

atgaacatga tgatgtaact tgtaatagca gaatagttaa tgaatgaaac tagttcttat 3480

aatttatctt tatttaaaag cttagcctgc cttaaaacta gagatcaact ttctcagctg 3540

caaaagcttc tagtctttca agaagttcat actttatgaa attgcacagt aagcatttat 3600

ttttcagacc atttttgaac atcactccta aattaataaa gtattcctct gttgctttag 3660

tatttattac aataaaaagg gtttgaaata tagctgttct ttatgcataa aacacccagc 3720

taggaccatt actgccagag aaaaaaatcg tattgaatgg ccatttccct acttataaga 3780

tgtctcaatc tgaatttatt tggctacact aaagaatgca gtatatttag ttttccattt 3840

gcatgatgtt tgtgtgctat agatgatatt ttaaattgaa aagtttgttt taaattattt 3900

ttacagtgaa gactgttttc agctcttttt atattgtaca tagtctttta tgtaatttac 3960

tggcatatgt tttgtagact gtttaatgac tggatatctt ccttcaactt ttgaaataca 4020

aaaccagtgt tttttacttg tacactgttt taaagtctat taaaattgtc atttgacttt 4080

tttctg 4086

<210> 51
<211> 11579
<212> DNA
<213> Homo sapiens

<400> 51
cggaccgtgc tttcgccgcc tgggagccgt ccggcgcagc agtttctagg tccccactgt 60

ccccgccgtc ccgccccttc gcgtcccggg aaccggctgg cttccgagcc gcactcgccg 120

atcctccagg catgccccgc tacgagctgg ctttaatcct gaaagccatg cagcggggtt 180

ggtacagtag gcttcactag acttagctgc aactcagaat ttctcctcca gcacctgagt 240

aaatgctgat ggtcttgtgg agagtggatt aagagtacga gctaagttct caatcccaat 300

taagaagcgg aaaatttaaa ctgtcttctt caaagtttat cacaaccacc accatcaaga 360

cagcaaacca aaggacaaag actttgaccc tgctgtgttg ctctgtgtag tccagttcac 420

gtatggttta cagacttggc tggggttact aaaaataaat aaaaagttgg acacttctgt 480

cattggagcg ctattattca caagttacca gaatgagagc tgtactggac acagcagaca 540

ttgccatagt ggccctgtat tttatcctgg tcatgtgcat tggttttttt gccatgtgga 600

aatctaatag aagcaccgtg agtggatact tcctggcggg gcgctctatg acctgggtaa 660

caattggtgc ctctctgttt gtgagcaata ttgggagtga gcacttcatt gggctggcag 720

gatctggagc tgcaagtgga tttgcagtgg gcgcatggga attcaatgcc ttactgcttt 780

tacaacttct gggatgggtt ttcatcccaa tttacatccg gtcaggggta tataccatgc 840

ctgaatactt gtccaagcga tttggtggcc ataggattca ggtctatttt gcagccttgt 900

ctctgattct ctatattttc accaagctct cggtggatct gtattcgggt gcccttttta 960

tccaggagtc tttgggttgg aatctttatg tgtctgtcat cctgctcatt ggcatgactg 1020

ctttgctgac tgtcaccgga ggccttgttg cagtgatcta cacagacact ctgcaggctc 1080

tgctcatgat cattggggca cttacactta tgattattag cataatggag attggcgggt 1140

ttgaggaagt taagagaagg tacatgttgg cctcacccga tgtcacttcc atcttattga 1200

catacaacct ttccaacaca aattcttgta atgtctcccc taagaaagaa gccctgaaaa 1260

tgctgcggaa tccaacagat gaagatgttc cttggcctgg attcattctt gggcagaccc 1320

cagcttcagt atggtactgg tgtgctgacc aagtcatcgt gcagagggtc cttgcagcca 1380

aaaacattgc tcatgccaaa ggctctactc ttatggctgg cttcttaaag ctcctgccaa 1440

tgtttatcat agttgtccca ggaatgattt ccaggatact gtttactgat gatatagctt 1500

gcatcaaccc agagcactgc atgctggtgt gtggaagcag agctggttgc tccaatattg 1560

cttacccacg cctggtgatg aagctggttc ctgtgggcct tcggggttta atgatggcag 1620

tgatgattgc agctctgatg agtgacttag actctatctt taacagtgcc agtaccatat 1680

tcaccctcga tgtgtacaaa cttatccgca agagcgcaag ctcccgggag ttaatgattg 1740

tggggaggat atttgtggca tttatggtgg tgatcagcat agcatgggtg ccaatcatcg 1800

tggagatgca aggaggccag atgtaccttt acattcagga ggtagcagat tacctgacac 1860

ccccagtggc agccttgttc ctgctggcaa ttttctggaa gcgctgcaat gaacaagggg 1920

ctttctatgg tggaatggct ggctttgttc ttggagcagt ccgtttgata ctggcctttg 1980

cctaccgtgc cccagaatgt gaccaacctg ataataggcc gggcttcatc aaagacatcc 2040

attatatgta tgtggccaca ggattgtttt gggtcacggg actcattact gtaattgtga 2100

gccttctcac accacctccc acaaaggaac agattcgaac caccaccttt tggtctaaga 2160

agaacctggt ggtgaaggag aactgctccc caaaagagga accataccaa atgcaagaaa 2220

agagcattct gagatgcagt gagaataatg agaccatcaa ccacatcatt cccaacggga 2280

aatctgaaga cagcattaag ggccttcagc ctgaagatgt taatctgttg gtaacctgca 2340

gagaggaggg caacccagtg gcatccttag gtcattcaga ggcagaaaca ccagttgacg 2400

cttactccaa tgggcaagca gctctcatgg gtgagaaaga gagaaagaaa gaaacggatg 2460

atggaggtcg gtactggaag ttcatagact ggttttgtgg ctttaaaagt aagagcctca 2520

gcaagaggag tctcagagac ctgatggaag aggaggctgt ttgtttacag atgctagaag 2580

agactcggca agttaaagta atactaaata ttggactttt tgctgtgtgt tcacttggaa 2640

ttttcatgtt tgtttatttc tccttatgaa cttaaggata tggtgagaca ctaacttaag 2700

acaatactga ctggtctttg gggaaaaaag ttatgtaact gtgcatctct caggcattgt 2760

ttacgctgta ggttttagcc aaattttact tagcagaaaa tcatctaatt acaagacttt 2820

attttcccag agatggatta aagtaaatct tcaacttaag tgaagccaaa cctaacagac 2880

tgaattgtgc aaatgtggtt ttaaattttg cataccaaag taagaagaga ccaattattc 2940

tcacagagca cttagagcag aatatatgtt aagttaccat gaattaaggt atactgtctg 3000

cactgccaag tcttggcaga ccttaccctg aagtagaaga tttgctcatt tctaaagttt 3060

tttttctgtc tctgtaatcc ctcctaccat taagaaaaac ttatttctta gacattgtac 3120

aatcagttat gtactgaaaa tcgaatgtgc ttgtgtgata cttgtttcag gacaagttca 3180

tttgccaggt tcattttgtt agcatgagcc tacggattct gatttcccaa agaaagaatg 3240

ttttcctgta ggtatttttg taccaccagt atatggaatg ttagggaaaa actttgttcc 3300

agttcctttt tttttttctt tctactttca agtttaagtg aaccatactg aaatgaccaa 3360

caagtctgcc tgtaaagtta catgtcatga ttgtgttgtt aaatgattat gggggagaaa 3420

atgaagtaaa tgttgctgat gatccccata tttattgatc atattaaggt tgtttatata 3480

gtttggaaat gaccagcccc ctaagcagtg tttgattaac ttatgctaat cagatgatta 3540

ctcatatatt ctgctaattt tctagcttta ttcttgttat ttggaaaaat tattagccaa 3600

atgccttcct aggtggatcc agttggaaga tatgtccaga aacctgaaga aaaattgacg 3660

ctgcctttgt gtgctggatt gctctacttg attagatcat gatatatcaa ggttgaattt 3720

ttagagggaa aatttaattc tgatatctta ttgcatcctt gataagtttt tccctgattt 3780

tttttttcct caaaagactt tccatctgta cacagcctct acatttttgt tgtagtgact 3840

tagagcataa ggatgtttca gtgcaaactg gccgtcggta acagaaaact cagtgcatac 3900

tttgctgttg ttaggttgtc aatatagtct ttctgtagga tggatagcat gtttgagagg 3960

tgccaaacaa gaacttttgg ggttagtagt gtgtcttgtg gagggtatta caggactgtg 4020

taattatagg actctaactt gacatggctt ggcacccact tgcagctagt gggtacaggg 4080

tacaaaagat gttagagaaa agctctacag attacgtact tctgtgtctt cgtatgctca 4140

acactgtcct ttgtcctcca tgaaagatga aggaagcaaa ttatgtatgt actttctttg 4200

accttcttta atctctgata ctttttagat tgcatgattt tactaggctt gtatttaggg 4260

aaattacttt cataaatact tttgtagatt ttgaatcaaa actcagtctt tttaattttt 4320

ttgtagtcta taaactagtt tcattatgat ggacttgatt agtccaaagt taattttaga 4380

aattgtcagg tagcatagtg tcttcccatg atcaggaggc tttctgaagg actgagtctg 4440

taaatgaaaa aataatttat gtatgaatag catgtatttc tgaagagctt agagtgcctt 4500

gtagaatttt tttctcaatt ttattcttga ggtttataat ttgggggcca aatagataga 4560

gctcatcatt ttcttgtttg gaagttgagg ctgcgacatg tccaaggtta tgaagtctct 4620

tttgggaaga acagaaacca ggtctccaaa tctggactca tggtttgttc agatgtgtct 4680

ggacaaatgg ttgtcaatgt tttgtcctgt tttttcaaag gaactgttct tcctttggga 4740

caaccttttg gtgtttggga aagtaataag atcttggatt tttcaaatta acattaagtt 4800

gtaagaacta aaatttcttt tgaaccacat tactgtgtaa ttcactgata attgacatat 4860

tggctgggca gcctatctct tccatatcca gcgtaaatga ataggaggtg tttgtgattt 4920

tttttttctc cctttattta acattgagtc ctagtagttt ggagaattag ggtccctcta 4980

ccttctttct gctcttgtct tagtaagata cataaggtac atcatcttgt gtctgtgtgt 5040

atatagcagt aggtcaagtt tagagtacta aagtctgtaa ataaggaatg actattagca 5100

tattcattag aattgtttat tcttgccagt ataaacatca ttttatttag actaaagtcc 5160

ctgaagcttg tctttcttat tgcttcccag taatagataa tgtgctcgag taagtttgtg 5220

aattgctgat tgcaacttaa ttcagggacc agtcttcaat ctatatttca ttagaatgat 5280

tgttcctgga atgatcatac atggactgtc ttaagctagc aaaatgttca tactttacac 5340

tgactaaatg ggtcctaaat gatgacattg gtctttagac attaacatgt gtatattttt 5400

atattagctc aagctaaggt tcagaattga agcttgatat tgactagaat agctaaaagt 5460

caaaatgagg tgaggacact ggtcttggaa ggtagagaaa aataaatgtc ttaccaggtg 5520

ttaatggtat ccccagttct tagacttttg tcttctcagg caattttcat ctcaagatct 5580

gatgagaagg gcatattaca ttggtatgca ggatgattat tgcatatttt gtgggacctc 5640

taatttccct ggtcatcttt cagaatattc tgttctgcca cccccagaga gtaaacactt 5700

gagccgattt cttcttcccc agctattctt tcctgggggt aattatgctt tgtctttaga 5760

ttagagaagc atcaagcaat agcaatggtg ctgtgtcctt cggcctaaat tcaatagatc 5820

tcatctccta gggcttcctt ttcacttggc tcaaaggatc cattgtattt tggcacaaag 5880

agcctggcca gggtcatgta gccatagctc ttagggatga tacctcaaga aattagctgg 5940

gacccatcac tctgtgaaac ttcacatttt aagaactgag ttgagggggt tgttatgcac 6000

ttctgtaact tgaggctaag caaggggtta actcttgtga gagccaatag agtgtgtctg 6060

tattcgcagt ccatggctca ttttctttat agtaggcata tggatcttcc cctctgactt 6120

tgaatatcat ttggtgtggc ctgtgggtta ttttcattct ttaccaccaa ataaagcggc 6180

ttattagcta ctcagttact tgctactcaa aggttaggtc ttccctgttc ctgcttggca 6240

gtgttaaagc ttacagggtt aacttatgat gattctcctg gctcattttc atcagaggca 6300

tgatgactgg aaagggatca catgggtcgt tggtggtgac acctcactgt ttcctaggtt 6360

tggatagaga gatgtataca agacctttcc tgttaaatta cgtgactaca gagacttgcc 6420

aggacaaaat tttcctaaga aatcagaaaa atgattaagt gagataagta cctgggtgac 6480

acagatatta gcccgttggt aaaagacaac aaatattagc ttaaaatctg catatgtaga 6540

atcattttca ttagatttag agcttgaagc accttggctc tcagctactt taaactcctc 6600

cccatataaa tcagggcacc aataaataag tttcagcttt ttaaaccctg gtttgatgtt 6660

aagcattata aagtacgaag tttgttacca cagtagagat aatttagtag aaaaatgctt 6720

tgaggcttca gtatttgtaa gattttgcat tagccagatg ctaggttgtt gaaggcattt 6780

cagtgttgat aatagcctga gcagacttct ttacaaatgg gatctgtttc tatatgtgta 6840

tatgcccact taccattcag agagactggt ctttctcttt gtcttccttc acattgctgt 6900

gtcagttcta cacctagtct tttcagcact tagcaaattc aaattttgat ttttttgtca 6960

gcttagttca ctttaaggca tattggcatg gtgtgtgaaa gtgatgtttt gccccagtat 7020

tgaggacttt tagatccaaa taatgactca ttaaatataa ttatgtttta agtatactga 7080

atttctgtta gcttaaaatg ttaattctca ggaatgattt tctcacactt tgtgttggct 7140

aataataaaa gcactgtttt attctcaaaa ctcctttttc aaaaattagg gagagagcag 7200

tagtgatcat ttatgtgagc ccctttgaaa tgatggtgtc agagtgcaga gaaacaatgg 7260

agttttgatg ccaaaaaggt ttttttgcag taaaagtaaa aatttggaat tagttggcat 7320

atagaggaac ccttttgtac tggaacgtat gaggctggat tgtgaaaagg taatctttcg 7380

attgctagac ttggttaact tagggctgca aatctttttc ttctgtcaag gtcacttaat 7440

atggaatgtt tttgtcagac tgtcctttgt tggaatactt tagctgttca gctactttga 7500

ctcctaggag agaatttagt taaggttcaa agtaattaac tggctttgcc agtggtgagt 7560

cccacaccat tattcactta gtagtcatat aaatgttttt atttaaactt ctctctcttc 7620

aatgctgaga ataaggcttt aaattactga ttcaccttta aaggaatgtt gtgagaattg 7680

atgtaatttc tgtttctgtt tccatctaaa cttctttata aaaagaggga ttagtttttt 7740

tgttttgggg taagcaccta atttatccag taaccaacaa ccctaaccat tggcatatat 7800

agtctttcac tcagaaataa acaaaaactg tttggtatat ctgtatcatt gctaatcttg 7860

tgcactttac tttttgggca gtaccataca tagtctgagg ctattgactt aaaccaataa 7920

ctgtacttta tgtaatgact cttaaatttg gttacctggg ttcacagctt gcttgaagag 7980

aaaggatgct agaataaagt aagcagctga agagcgagca aatcaagaca aaacacagtg 8040

gtctcagatt tttcgtagtg tgggaacagt ggttttgctc tataccactg aaaagcacta 8100

taacataatt gttgtccatg atactgaagc ttttcccctc acttctaggt tgtttacatt 8160

cagagctcta tcaataagag gaatacatat tacagtgaat tcgacaaccg cacaagttgg 8220

cagtaggtat ccccaaccta atttatcttg gtaaattcac cctgtttcct agtgctgctg 8280

gataaaagag tgtttacttt ttattgctct tagacagagt agtctagata agttttcaat 8340

ttatcaacat agcctagact tctgtaagtg gaatgttcat tagtaactca tctttttgtt 8400

gttataattg gaaacagaaa cgaggcttat tgctattgca gaaatcccaa actggcaaag 8460

gccagtatat atggtattcc ataatataac cagcttttga aatttatgtg tttggattag 8520

tgccttctgg ttaccagtat tgactctgct agtttgcacc tttccgttct taacagaaaa 8580

tttgtatttg ttattcctct taaattttgt cgtaactagt gaaggaagta aaaaaaaaaa 8640

aaaaacatgc attacattga catactttat gtgcagcctt tatttaggtt cagtgaaacc 8700

aggtagttct gtatttgtgt tgtagcctaa atgttgtttc ttttatatcc attaaaaact 8760

taaagttact tatgttctgt gatcttaatt ttgttgtgtt tccattgtag gttgataggt 8820

atatcgagaa caggtacgtg acaacagttt atattccatg atagaaagct aaagtccata 8880

gaaagcacaa aatcgtgttc acacattagt gtacccacac atagaaagca caagactaat 8940

agtattctct gtatcccaca agtgccagtc ataaaggcca ccaggtattt gtctcagagt 9000

tgctatgagc actacagtat tgataagccc aagacaatgc ggtatctaaa ctggtcctaa 9060

tggtaaggga cccaaaggaa taatctcaat aagtttgtac cacattgatg gagggagaga 9120

atataaatgt caagaatgcc aaaattatat ttgggggtta ctagctaaaa tggggtttga 9180

gggcttttta ctgcaacttg aaactggaga aatagggaca gatgtctagg tttttggtgg 9240

gtggaacagg tgacatattt ctgttttaag ctgtagtgtg attggggttt tttgtaaaaa 9300

atcttaaatc ttttaggaaa tattacctct taacagtgcc cccccaaaca tgcagaaagt 9360

catactttaa cagggcaaat actacttgtc tttgattttt tttgtgtacg tttgtatgtg 9420

agagatgaag ttacctttat ttttttccta tacttgactg tgcttcattt taataaagga 9480

taatttgatc tgagtgttct gagcatcaga ctaattctga agcatatttg ctagaggagc 9540

tactttgctt ttcacaatgg ggtggagagg attctttcac ttgtcccatt aaccctcttc 9600

tagtctagat gagatgaaat ctgttaatgt gtgtgtagaa gaaaacgtat gttcttctac 9660

tcagcattgc ccttttccac ctcctcactt cacctccgag tagcttgttt atcaagaatg 9720

aatgaatgtc tttgtcttaa attttgccca tgtgttaaaa gatgtaattc tcagaatggg 9780

agagaaatga ctacctttgt tcctactctt ttatataatt atccttttag ggaaagactt 9840

ggtcaactct aatatatcta gaaggaagac tatatctggt gtagactaat atgagatgtt 9900

ttagaagagt taacctgaac actttgaggg agagattatt cttgccagca aaaagctagc 9960

caggaatgag cctaccacat tatttgagaa tatcaaacct caggcctggg ggatgagggg 10020

gaagaagatt accagaagtg caggaaagag aagtttgagg aacacccttg gcttagcaac 10080

atgtgataat gcaaagctgt tataacctgt taatcctacg tactatgtgt tctgtacctt 10140

tacatgtttt taaatttaag atagtttgta agaactgtac aaaaaaatgc ttctggagat 10200

ttctttggca gaaatgcctt tcatctataa tttcatggag aactgcttta attagcctag 10260

gtgaaaagta gtcctagcag tgtaaatatg tataattaga gttttctaat ttcactgtga 10320

gatctctaac ttttgagtgg caaacagatc aagtcttttg ctcatagact tttctgtggg 10380

gttattaaaa tgcaaaagct ttattttttt taataatgcc atactccatt agtgtcagat 10440

gatggtatgg aatttgttcc cttgctttcc cccactgtta ctgcttcagt ttatagattg 10500

ccagcagagt tcagaaatag agcagggatt tacccgttct ttgcttggac atcccatttt 10560

cttttgtcca gacccatgtt ggcaatcatg tatgaactgt gttatacttc tcagtgcttt 10620

cttttttctt tttgataaga tggatatcaa aaatagttgc tgtgcaaaag ttagtagtct 10680

tcttcaagaa gaaaaccaat tctttttcta ataatatcct gtgaaattgc ttcattcatt 10740

catttatttt taagccaaat gtcagcagag tgctgctgct tttatctagt aattttgata 10800

tgtaagtatt aatgcatttt taaaagatgt ctacattgaa acatgttctt cccagtgtcc 10860

tgcttatgat gctttgttca gattttttgt aagagaccag ttagtacact gggggtgtat 10920

attgtgtaca tgtgtcattt tagttaggca ttgtaggcca aatgtgatta taaatgaagt 10980

tgatgaacat taattttgtt attagtgagt tttttgaatt gtaaatggat ttccagttta 11040

ccttctgttg tctacagctt ttttaatttt aaggtttgac taattgtatc catctcattg 11100

tacagtgttt tagttgcaag cagaaagtag aatttggtat aaagcaggtt atttctatat 11160

tgaaaggagt acagttgaaa ttgtagattt aagattgtta aaatcatgac aattctaact 11220

tgtctattct aacctattgt gtacaatctg attttttaaa attgtaaaca tgtatgatct 11280

tggtttcatg tgtttttgaa agtgttattg tttaaaaaat gaaaaaagca tatctgctaa 11340

agagctgtca gttttcatta ctgactctgt aaaatacact gttctttgtg tactgtgtgt 11400

tattttgcca gctgctgcat tagccttcaa aagtatttgg aaacttaaga tgaactacat 11460

ttcttgcaaa gtacattcct ttctgtggta ttttgtcctg taactgaagt atagtaatta 11520

ttttatggaa atgttagcaa ttctgtacca actttgaata aaatgaaaaa tttataaaa 11579

<210> 52
<211> 8789
<212> DNA
<213> Homo sapiens

<400> 52
atgctcagtg gcttctcgac aagttggcag caacaacacg gccctggtcg tcgtcgccgc 60

tgcggtaacg gagcggtttg ggtggcggag cctgcgttcg cgccttcccg ctctcctcgg 120

gaggcccttc ctgctctccc ctaggctccg cggccgccca gggggtggga gcgggtgagg 180

ggagccaggc gcccagcgag agaggccccc cgccgcaggg cggcccggga gctcgaggcg 240

gtccggcccg cgcgggcagc ggcgcggcgc tgaggagggg cggcctggcc gggacgcctc 300

ggggcggggg ccgaggagct ctccgggccg ccggggaaag ctacgggccc ggtgcgtccg 360

cggaccagca gcgcgggaga gcggactccc ctcgccaccg cccgagccca ggttatcctg 420

aatacatgtc taacaatttt ccttgcaacg ttagctgttg tttttcactg tttccaaagg 480

atcaaaattg cttcagaaat tggagacata tttgatttaa aaggaaaaac ttgaacaaat 540

ggacaatatg tctattacga atacaccaac aagtaatgat gcctgtctga gcattgtgca 600
tagtttgatg tgccatagac aaggtggaga gagtgaaaca tttgcaaaaa gagcaattga 660
aagtttggta aagaagctga aggagaaaaa agatgaattg gattctttaa taacagctat 720
aactacaaat ggagctcatc ctagtaaatg tgttaccata cagagaacat tggatgggag 780
gcttcaggtg gctggtcgga aaggatttcc tcatgtgatc tatgcccgtc tctggaggtg 840
gcctgatctt cacaaaaatg aactaaaaca tgttaaatat tgtcagtatg cgtttgactt 900
aaaatgtgat agtgtctgtg tgaatccata tcactacgaa cgagttgtat cacctggaat 960
tgatctctca ggattaacac tgcagagtaa tgctccatca agtatgatgg tgaaggatga 1020
atatgtgcat gactttgagg gacagccatc gttgtccact gaaggacatt caattcaaac 1080
catccagcat ccaccaagta atcgtgcatc gacagagaca tacagcaccc cagctctgtt 1140
agccccatct gagtctaatg ctaccagcac tgccaacttt cccaacattc ctgtggcttc 1200
cacaagtcag cctgccagta tactgggggg cagccatagt gaaggactgt tgcagatagc 1260
atcagggcct cagccaggac agcagcagaa tggatttact ggtcagccag ctacttacca 1320
tcataacagc actaccacct ggactggaag taggactgca ccatacacac ctaatttgcc 1380
tcaccaccaa aacggccatc ttcagcacca cccgcctatg ccgccccatc ccggacatta 1440
ctggcctgtt cacaatgagc ttgcattcca gcctcccatt tccaatcatc ctgctcctga 1500
gtattggtgt tccattgctt actttgaaat ggatgttcag gtaggagaga catttaaggt 1560
tccttcaagc tgccctattg ttactgttga tggatacgtg gacccttctg gaggagatcg 1620
cttttgtttg ggtcaactct ccaatgtcca caggacagaa gccattgaga gagcaaggtt 1680
gcacataggc aaaggtgtgc agttggaatg taaaggtgaa ggtgatgttt gggtcaggtg 1740
ccttagtgac cacgcggtct ttgtacagag ttactactta gacagagaag ctgggcgtgc 1800
acctggagat gctgttcata agatctaccc aagtgcatat ataaaggtct ttgatttgcg 1860
tcagtgtcat cgacagatgc agcagcaggc ggctactgca caagctgcag cagctgccca 1920
ggcagcagcc gtggcaggaa acatccctgg cccaggatca gtaggtggaa tagctccagc 1980
tatcagtctg tcagctgctg ctggaattgg tgttgatgac cttcgtcgct tatgcatact 2040
caggatgagt tttgtgaaag gctggggacc ggattaccca agacagagca tcaaagaaac 2100
accttgctgg attgaaattc acttacaccg ggccctccag ctcctagacg aagtacttca 2160
taccatgccg attgcagacc cacaaccttt agactgaggt cttttaccgt tggggccctt 2220
aaccttatca ggatggtgga ctacaaaata caatcctgtt tataatctga agatatattt 2280
cacttttgtt ctgctttatc ttttcataaa gggttgaaaa tgtgtttgct gccttgctcc 2340
tagcagacag aaactggatt aaaacaattt tttttttcct cttcagaact tgtcaggcat 2400
ggctcagagc ttgaagatta ggagaaacac attcttatta attcttcacc tgttatgtat 2460

gaaggaatca ttccagtgct agaaaattta gccctttaaa acgtcttaga gccttttatc 2520

tgcagaacat cgatatgtat atcattctac agaataatcc agtattgctg attttaaagg 2580

cagagaagtt ctcaaagtta attcacctat gttattttgt gtacaagttg ttattgttga 2640

acatacttca aaaataatgt gccatgtggg tgagttaatt ttaccaagag taactttact 2700

ctgtgtttaa aaagtaagtt aataatgtat tgtaatcttt catccaaaat attttttgca 2760

agttatatta gtgaagatgg tttcaattca gattgtcttg caacttcagt tttatttttg 2820

ccaaggcaaa aaactcttaa tctgtgtgta tattgagaat cccttaaaat taccagacaa 2880

aaaaatttaa aattacgttt gttattccta gtggatgact gttgatgaag tatacttttc 2940

ccctgttaaa cagtagttgt attcttctgt atttctaggc acaaggttgg ttgctaagaa 3000

gcctataaga ggaatttctt ttccttcatt catagggaaa ggttttgtat tttttaaaac 3060

actaaaagca gcgtcactct acctaatgtc tcactgttct gcaaaggtgg caatgcttaa 3120

actaaataat gaataaactg aatattttgg aaactgctaa attctatgtt aaatactgtg 3180

cagaataatg gaaacattac agttcataat aggtagtttg gatatttttg tacttgattt 3240

gatgtgactt tttttggtat aatgtttaaa tcatgtatgt tatgatattg tttaaaattc 3300

agtttttgta tcttggggca agactgcaaa cttttttata tcttttggtt attctaagcc 3360

ctttgccatc aatgatcata tcaattggca gtgactttgt atagagaatt taagtagaaa 3420

agttgcagat gtattgactg taccacagac acaatatgta tgctttttac ctagctggta 3480

gcataaataa aactgaatct caacatacaa agttgaattc taggtttgat ttttaagatt 3540

ttttttttct tttgcacttt tgagtccaat ctcagtgatg aggtaccttc tactaaatga 3600

caggcaacag ccagttctat tgggcagctt tgtttttttc cctcacactc taccgggact 3660

tccccatgga cattgtgtat catgtgtaga gttggttttt ttttttttta atttttattt 3720

tactatagca gaaatagacc tgattatcta caagatgata aatagattgt ctacaggata 3780

aatagtatga aataaaatca aggattatct ttcagatgtg tttacttttg cctggagaac 3840

ttttagctat agaaacactt gtgtgatgat agtcctcctt atatcacctg gaatgaacac 3900

agcttctact gccttgctca gaaggtcttt taaatagacc atcctagaaa ccactgagtt 3960

tgcttatttc tgtgatttaa acatagatct tgatccaagc tacatgactt ttgtctttaa 4020

ataacttatc taccacctca tttgtactct tgattactta caaattcttt cagtaaacac 4080

ctaattttct tctgtaaaag tttggtgatt taagttttat tggcagtttt ataaaaagac 4140

atcttctcta gaaattgcta actttaggtc cattttactg tgaatgagga ataggagtga 4200

gttttagaat aacagatttt taaaaatcca gatgatttga ttaaaacctt aatcatacat 4260

tgacataatt cattgcttct tttttttgag atatggagtc ttgctgtgtt gcccaggcag 4320

gagtgcagtg gtatgatctc agctcactgc aacctctgcc tcccgggttc aactgattct 4380

cctgcctcag cctccctggt agctaggatt acaggtgccc gccaccatgc ctggctaact 4440

tttgtagttt tagtagagac ggggttttgc ctgttggcca ggctggtctt gaactcctga 4500

cctcaagtga tccatccacc ttggcctccc aaagtgctgg gattacgggc gtgagccact 4560

gtccctggcc tcattgttcc cttttctact ttaaggaaag ttttcatgtt taatcatctg 4620

gggaaagtat gtgaaaaata tttgttaaga agtatctctt tggagccaag ccacctgtct 4680

tggtttcttt ctactaagag ccataaagta tagaaatact tctagttgtt aagtgcttat 4740

atttgtacct agatttagtc acacgctttt gagaaaacat ctagtatgtt atgatcagct 4800

attcctgaga gcttggttgt taatctatat ttctatttct tagtggtagt catctttgat 4860

gaataagact aaagattctc acaggtttaa aattttatgt ctactttaag ggtaaaatta 4920

tgaggttatg gttctgggtg ggttttctct agctaattca tatctcaaag agtctcaaaa 4980

tgttgaattt cagtgcaagc tgaatgagag atgagccatg tacacccacc gtaagacctc 5040

attccatgtt tgtccagtgc ctttcagtgc attatcaaag ggaatccttc atggtgttgc 5100

ctttattttc cggggagtag atcgtgggat atagtctatc tcatttttaa tagtttaccg 5160

cccctggtat acaaagataa tgacaataaa tcactgccat ataaccttgc tttttccaga 5220

aacatggctg ttttgtattg ctgtaaccac taaataggtt gcctatacca ttcctcctgt 5280

gaacagtgca gatttacagg ttgcatggtc tggcttaagg agagccatac ttgagacatg 5340

tgagtaaact gaactcatat tagctgtgct gcatttcaga cttaaaatcc atttttgtgg 5400

ggcagggtgt ggtgtgtaaa ggggggtgtt tgtaatacaa gttgaaggca aaataaaatg 5460

tcctgtctcc cagatgatat acatcttatt atttttaaag tttattgcta attgtaggaa 5520

ggtgagttgc aggtatcttt gactatggtc atctggggaa ggaaaatttt acattttact 5580

attaatgctc cttaagtgtc tatggaggtt aaagaataaa atggtaaatg tttctgtgcc 5640

tggtttgatg gtaactggtt aatagttact caccatttta tgcagagtca cattagttca 5700

caccctttct gagagccttt tgggagaagc agttttattc tctgagtgga acagagttct 5760

ttttgttgat aatttctagt ttgctccctt cgttattgcc aactttactg gcattttatt 5820

taatgatagc agattgggaa aatggcaaat ttaggttacg gaggtaaatg agtatatgaa 5880

agcaattacc tctaaagcca gttaacaatt attttgtagg tggggtacac tcagcttaaa 5940

gtaatgcatt tttttttccc gtaaaggcag aatccatctt gttgcagata gctatctaaa 6000

taatctcata tcctcttttg caaagactac agagaatagg ctatgacaat cttgttcaag 6060

cctttccatt tttttccctg ataactaagt aatttctttg aacataccaa gaagtatgta 6120

aaaagtccat ggccttattc atccacaaag tggcatccta ggcccagcct tatccctagc 6180

agttgtccca gtgctgctag gttgcttatc ttgtttatct ggaatcactg tggagtgaaa 6240

ttttccacat catccagaat tgccttattt aagaagtaaa acgttttaat ttttagcctt 6300

tttttggtgg agttatttaa tatgtatatc agaggatata ctagatggta acatttcttt 6360

ctgtgcttgg ctatctttgt ggacttcagg ggcttctaaa acagacagga ctgtgttgcc 6420

tttactaaat ggtctgagac agctatggtt ttgaattttt agtttttttt ttttaaccca 6480

cttcccctcc tggtctcttc cctctctgat aattaccatt catatgtgag tgttagtgtg 6540

cctcctttta gcattttctt cttctctttc tgattcttca tttctgactg cctaggcaag 6600

gaaaccagat aaccaaactt actagaacgt tctttaaaac acaagtacaa actctgggac 6660

aggacccaag acactttcct gtgaagtgct gaaaaagacc tcattgtatt ggcatttgat 6720

atcagtttga tgtagcttag agtgcttcct gattcttgct gagtttcagg tagttgagat 6780

agagagaagt gagtcatatt catattttcc cccttagaat aatattttga aaggtttcat 6840

tgcttccact tgaatgctgc tcttacaaaa actggggtta caagggttac taaattagca 6900

tcagtagcca gaggcaatac cgttgtctgg aggacaccag caaacaacac acaacaaagc 6960

aaaacaaacc ttgggaaact aaggccattt gttttgtttt ggtgtcccct ttgaagccct 7020

gccttctggc cttactcctg tacagatatt tttgacctat aggtgccttt atgagaattg 7080

agggtctgac atcctgcccc aaggagtagc taaagtaatt gctagtgttt tcaggatttt 7140

taacatcaga ctggaatgaa tgaatgaaac tttttgtcct ttttttttct gttttttttt 7200

ttctaatgta gtaaggacta aggaaaacct ttggtgaaga caatcatttc tctctgttga 7260

tgtggatact tttcacaccg tttatttaaa tgctttctca ataggtccag agccagtgtt 7320

cttgttcaac ctgaaagtaa tggctctggg ttgggccaga cagttgcact ctctagtttg 7380

ccctctgcca caaatttgat gtgtgacctt tgggcaagtc atttatcttc tctgggcctt 7440

agttgcctca tctgtaaaat gagggagttg gagtagatta attattccag ctctgaaatt 7500

ctaagtgacc ttggctacct tgcagcagtt ttggatttct tccttatctt tgttctgctg 7560

tttgaggggg ctttttactt atttccatgt tattcaaagg agactaggct tgatatttta 7620

ttactgttct tttatggaca aaaggttaca tagtatgccc ttaagactta attttaacca 7680

aaggcctagc accaccttag gggctgcaat aaacacttaa cgcgcgtgcg cacgcgcgcg 7740

cgcacacaca cacacacaca cacacacaca cacaggtcag agtttaaggc tttcgagtca 7800

tgacattcta gcttttgaat tgcgtgcaca cacacacgca cgcacacact ctggtcagag 7860

tttattaagg ctttcgagtc atgacattat agcttttgag ttggtgtgtg tgacaccacc 7920

ctcctaagtg gtgtgtgctt gtaatttttt ttttcagtga aaatggattg aaaacctgtt 7980

gttaatgctt agtgatatta tgctcaaaac aaggaaattc ccttgaaccg tgtcaattaa 8040

actggtttat atgactcaag aaaacaatac cagtagatga ttattaactt tattcttggc 8100

tctttttagg tccattttga ttaagtgact tttggctgga tcattcagag ctctcttcta 8160

gcctaccctt ggatgagtac aattaatgaa attcatattt tcaaggacct gggagccttc 8220

cttggggctg ggttgagggt ggggggttgg ggagtcctgg tagaggccag ctttgtggta 8280

gctggagagg aagggatgaa accagctgct gttgcaaagg ctgcttgtca ttgatagaag 8340

gactcacggg cttggattga ttaagactaa acatggagtt ggcaaacttt cttcaagtat 8400

tgagttctgt tcaatgcatt ggacatgtga tttaagggaa aagtgtgaat gcttatagat 8460

gatgaaaacc tggtgggctg cagagcccag tttagaagaa gtgagttggg ggttggggac 8520

agatttggtg gtggtatttc ccaactgttt cctcccctaa attcagagga atgcagctat 8580

gccagaagcc agagaagagc cactcgtagc ttctgctttg gggacaactg gtcagttgaa 8640

agtcccagga gttcctttgt ggctttctgt atacttttgc ctggttaaag tctgtggcta 8700

aaaaatagtc gaacctttct tgagaactct gtaacaaagt atgtttttga ttaaaagaga 8760

aagccaacta aaaaaaaaaa aaaaaaaaa 8789

<210> 53
<211> 1593
<212> DNA
<213> Homo sapiens

<400> 53
gcggtgccct tgcggcgcag ctggggtcgc ggccctgctc cccgcgcttt cttaaggccc 60

gcgggcggcg caggagcggc actcgtggct gtggtggctt cggcagcggc ttcagcagat 120

cggcggcatc agcggtagca ccagcactag cagcatgttg agccgggcag tgtgcggcac 180

cagcaggcag ctggctccgg ttttggggta tctgggctcc aggcagaagc acagcctccc 240

cgacctgccc tacgactacg gcgccctgga acctcacatc aacgcgcaga tcatgcagct 300

gcaccacagc aagcaccacg cggcctacgt gaacaacctg aacgtcaccg aggagaagta 360

ccaggaggcg ttggccaagg gagatgttac agcccagata gctcttcagc ctgcactgaa 420

gttcaatggt ggtggtcata tcaatcatag cattttctgg acaaacctca gccctaacgg 480

tggtggagaa cccaaagggg agttgctgga agccatcaaa cgtgactttg gttcctttga 540

caagtttaag gagaagctga cggctgcatc tgttggtgtc caaggctcag gttggggttg 600

gcttggtttc aataaggaac ggggacactt acaaattgct gcttgtccaa atcaggatcc 660

actgcaagga acaacaggcc ttattccact gctggggatt gatgtgtggg agcacgctta 720

ctaccttcag tataaaaatg tcaggcctga ttatctaaaa gctatttgga atgtaatcaa 780

ctgggagaat gtaactgaaa gatacatggc ttgcaaaaag taaaccacga tcgttatgct 840

gagtatgtta agctctttat gactgttttt gtagtggtat agagtactgc agaatacagt 900

aagctgctct attgtagcat ttcttgatgt tgcttagtca cttatttcat aaacaactta 960

atgttctgaa taatttctta ctaaacattt tgttattggg caagtgattg aaaatagtaa 1020

atgctttgtg tgattgaatc tgattggaca ttttcttcag agagctaaat tacaattgtc 1080

atttataaaa ccatcaaaaa tattccatcc atatactttg gggacttgta gggatgcctt 1140

tctagtccta ttctattgca gttatagaaa atctagtctt ttgccccagt tacttaaaaa 1200

taaaatatta acactttccc aagggaaaca ctcggctttc tatagaaaat tgcacttttt 1260

gtcgagtaat cctctgcagt gatacttctg gtagatgtca cccagtggtt tttgttaggt 1320

caaatgttcc tgtatagttt ttgcaaatag agctgtatac tgtttaaatg tagcaggtga 1380

actgaactgg ggtttgctca cctgcacagt aaaggcaaac ttcaacagca aaactgcaaa 1440

aaggtggttt ttgcagtagg agaaaggagg atgtttattt gcagggcgcc aagcaaggag 1500

aattgggcag ctcatgcttg agacccaatc tccatgatga cctacaagct agagtattta 1560

aaggcagtgg taaatttcag gaaagcagaa gtt 1593

<210> 54
<211> 3286
<212> DNA
<213> Homo sapiens

<400> 54
gcgtgtcggg cgcggaaggg ggaggcggcc cggggcgccc gcgagtgagg cgcggggcgg 60

cgaagggagc gcgggtggcg gcacttgctg ccgcggcctt ggatgggctg ggcccccctc 120

gccgctccgc ctcctccaca cgcgcggcgg ccgcggcgag ggggacgcgc cgcccggggc 180

ccggcacctt cgggaacccc ccggcccgga gcctgcggcc tgcgccgcct cggccgccgg 240

gagccccgtg gagcccccgc cgccgcgccg ccccgcggac cggacgctga gggcactcgg 300

ggcggggcgc gcgctcgggc agacgtttgc ggggaggggg gcgcctgccg ggccccggcg 360

accaccttgg gggtcgcggg ccggctcggg gggcgcccag tgcgggccct cgcgggcgcc 420

gggcagcgac cagccctgag cggagctgtt ggccgcggcg ggaggcctcc cggacgcccc 480

cagccccccg aacgctcgcc cgggccggcg ggagtcggcg ccccccggga ggtccgctcg 540

gtcgtccgcg gcggagcgtt tgctcctggg acaggcggtg ggaccggggc gtcgccggag 600

acgcccccag cgaagttggg ctctccaggt gtgggggtcc cggggggtag cgacgtcgcg 660

gacccggcct gtgggatggg cggcccggag aagactgcgc tcggccgtgt tcatacttgt 720

ccgtgggcct gaggtccccg gaggatgacc tagcactgaa aagccccggc cggcctcccc 780

agggtccccg aggacgaagt tgaccctgac cgggccgtct cccagttctg aggcccgggt 840

cccactggaa ctcgcgtctg agccgccgtc ccggaccccc ggtgcccgcc ggtccgcaga 900

ccctgcaccg ggcttggact cgcagccggg actgacgtgt agaacaatcg tttctgttgg 960

aagaagggtt tttcccttcc ttttggggtt tttgttgcct tttttttttc ttttttcttt 1020

gtaaaatttt ggagaaggga agtcggaaca caaggaagga ccgctcaccc gcggactcag 1080

ggctggcggc gggactccag gaccctgggt ccagcatgga ggtggtggac ccgcagcagc 1140

tgggcatgtt cacggagggc gagctgatgt cggtgggtat ggacacgttc atccaccgca 1200

tcgactccac cgaggtcatc taccagccgc gccgcaagcg ggccaagctc atcggcaagt 1260

acctgatggg ggacctgctg ggggaaggct cttacggcaa ggtgaaggag gtgctggact 1320

cggagacgct gtgcaggagg gccgtcaaga tcctcaagaa gaagaagttg cgaaggatcc 1380

ccaacgggga ggccaacgtg aagaaggaaa ttcaactact gaggaggtta cggcacaaaa 1440

atgtcatcca gctggtggat gtgttataca acgaagagaa gcagaaaatg tatatggtga 1500

tggagtactg cgtgtgtggc atgcaggaaa tgctggacag cgtgccggag aagcgtttcc 1560

cagtgtgcca ggcccacggg tacttctgtc agctgattga cggcctggag tacctgcata 1620

gccagggcat tgtgcacaag gacatcaagc cggggaacct gctgctcacc accggtggca 1680

ccctcaaaat ctccgacctg ggcgtggccg aggcactgca cccgttcgcg gcggacgaca 1740

cctgccggac cagccagggc tccccggctt tccagccgcc cgagattgcc aacggcctgg 1800

acaccttctc cggcttcaag gtggacatct ggtcggctgg ggtcaccctc tacaacatca 1860

ccacgggtct gtaccccttc gaaggggaca acatctacaa gttgtttgag aacatcggga 1920

aggggagcta cgccatcccg ggcgactgtg gccccccgct ctctgacctg ctgaaaggga 1980

tgcttgagta cgaaccggcc aagaggttct ccatccggca gatccggcag cacagctggt 2040

tccggaagaa acatcctccg gctgaagcac cagtgcccat cccaccgagc ccagacacca 2100

aggaccggtg gcgcagcatg actgtggtgc cgtacttgga ggacctgcac ggcgcggacg 2160

aggacgagga cctcttcgac atcgaggatg acatcatcta cactcaggac ttcacggtgc 2220

ccggacaggt cccagaagag gaggccagtc acaatggaca gcgccggggc ctccccaagg 2280

ccgtgtgtat gaacggcaca gaggcggcgc agctgagcac caaatccagg gcggagggcc 2340

gggcccccaa ccctgcccgc aaggcctgct ccgccagcag caagatccgc cggctgtcgg 2400

cctgcaagca gcagtgaggc tggccgcctg cagcccgtgt ccaggagccc cgccaggtgc 2460

ccgcgccagg ccctcagtct tcctgccggt tccgcccgcc ctcccggaga ggtggccgcc 2520

atgcttctgt gccgaccacg ccccaggacc tccggagcgc cctgcagggc cgggcagggg 2580

gacagcaggg accgggcgca gccctccccc ctcggccgcc cggcagtgca cgcggcttgt 2640

tgacttcgca gccccgggcg gagccttccc gggcgggcgt gggaggaggg aggcggcctc 2700

catgcacttt atgtggagac tactggcccc gcccgtggcc tcgtgctccg cagggcgccc 2760

agcgccgtcc ggcggccccg ccgcagacca gctggcgggt gtggagacca ggctcctgac 2820

cccgccatgc atgcagcgcc acctggaagc cgcgcggccg ctttggtttt ttgtttggtt 2880

ggttccattt tctttttttc tttttttttt taagaaaaaa taaaaggtgg atttgagctg 2940

tggctgtgag gggtgtttgg gagctgctgg gtggcagggg ggctgtgggg tcgggctcac 3000

gtcgcggccg cctttgcgct ctcgggtcac cctgctttgg cggcccggcc ggagggcagg 3060

accctcacct ctcccccaag gccactgcgc tcttgggacc ccagagaaaa cccggagcaa 3120

gcaggagtgt gcggtcaata tttatatcat ccagaaaaga aaaacacgag aaacgccatc 3180

gcgggatggt gcagacgcgg cggggactcg gagggtgccg tgcgggcgag gccgcccaaa 3240

tttggcaata aataaagctt gggaagcttg gacctgaaaa aaaaaa 3286

<210> 55
<211> 5174
<212> DNA
<213> Homo sapiens

<400> 55
ggagagctcg ccagagcgct cgcatggcgg gccggtgatt gtagtcaatc tggccgtatt 60

ctcaggcagg gtcgcccggg gcggactaca tctcccggga tgctgcgcgg ccgccccgcg 120

gaagattgtg aatatgtatc agaatgttaa tgattagctg ctgctaaatt tggtcaaaga 180

agtcacctac acagagcgtg ttgttagagc tgtgctgagc gggtgtttgg gttgttggct 240

gctttcttcc ccctttctca cacacttgta tattattttg aggtggtgtt cgcagagttt 300

gaaaggagag agaattaaaa aaaaaagccg caagcgtttc actcttttat ttttataatc 360

cccttcaatt tggggttaaa aaaaagacaa gaaaacagga aggaagagaa ataaggaaat 420

gagatgtggt aaaagaagct aaaaggtgcc ttttaaaaga tcgttgctgt gaagtgaaaa 480

aaatctccag agaaaccaaa aagcaccgcc gagacctctt ccgaaccaaa ggagtttgtg 540

tttgctttta gggaagaaga aagatcattc attcggagga ataacaacca attaaaagac 600

aaataaaaaa agtttggagt gggacgcaga gcgagcgaga ggagctgccg gcgggcggtg 660

gggcgcggag cccgcacttt cccggccggg tgagcggcgg ccgcggcgcc gggctcggcg 720

ggtgcgcctc ggcggagcga acgtcggagc gttgccttgg gagacgcgcg ccggacaatg 780

cccgcggcgg gccagtgacg cccgcgggga atgcggagcg gcccggcagc cggcacccag 840

ccgccgccgc gcgttcctgc cgcccgtgtc acgcgagacc cggcgggggc cgggaccgcc 900

cgagccgccc ctcagaccga gccggccgcc tccgctgccg cggccgcctc ctcttcgggg 960

tcattaaagc caatgagccg cgcgcctctg ccgagcgcag ccaactaaat cggcttggat 1020

gattcgcgac ctgagcaaga tgtacccgca gaccagacac ccggcaccgc atcagcctgc 1080

tcaacccttt aaatttacaa tttccgaatc ctgtgatcgg attaaggaag agtttcagtt 1140

tttacaggct caataccaca gtctgaagct ggaatgtgag aaactcgcca gtgagaagac 1200

agagatgcag cggcattatg tcatgtatta tgaaatgtcc tatgggttga atatagaaat 1260

gcacaagcag gcagagattg tcaagaggct gaatgctatc tgtgcacaag tcattccttt 1320

cctgtcccaa gagcaccagc aacaagtggt gcaggctgtg gaacggccca agcaggtgac 1380

catggcagaa ctgaacgcca tcattgggca acaactccag gcccagcatt tatcacatgg 1440

acatggtctc cccgtacctc tgactccaca cccttcaggg ctccagcccc ctgccattcc 1500

acccatcggt agcagtgccg ggcttctggc cctctccagt gctctaggag gtcagtccca 1560

tcttccaatt aaagatgaga agaagcacca tgacaatgat caccaaagag acagagactc 1620

catcaagagc tcttcagtat ccccatcagc cagtttccga ggtgctgaga agcacagaaa 1680

ctccgcagac tactcctcag agagcaaaaa gcagaaaact gaagaaaagg aaattgcagc 1740

tcgttatgac agcgatggtg agaaaagtga tgacaacttg gtggttgacg tttccaatga 1800

ggatccatct tcccctcgag ggagcccagc acattccccc agagagaatg gcctagacaa 1860

gacacgcctg ctcaagaaag atgccccgat tagtccagcc tctattgcat cttccagcag 1920

tactccctcc tccaaatcca aagaacttag ccttaagagg gatatgggga aattgagtga 1980

aacacgtctt agcgaagatg aacaatgcac attggggtta cagagatggt tttgtcgcct 2040

gtggtttatg aatgaaaaat ctactactcc cgtctcaaag tccaataccc ctactccacg 2100

aactgatgcg cccaccccag gcagtaactc tactcccgga ttgaggcctg tacctggaaa 2160

accaccagga gttgaccctt tggcctcaag cctaaggacc ccaatggcag taccttgtcc 2220

atatccaact ccatttggga ttgtgcccca tgctggaatg aacggagagc tgaccagccc 2280

cggagcggcc tacgctgggc tccacaacat ctcccctcag atgagcgcag ctgctgccgc 2340

cgccgctgct gctgctgcct atgggagatc accagtggtg ggatttgatc cacaccatca 2400

catgcgtgtg ccagcaatac ctccaaacct gacaggcatt ccaggaggaa aaccagcata 2460

ctccttccat gttagcgcag atggtcagat gcagcctgtc ccttttccac ccgacgccct 2520

catcggacct ggaatccccc ggcatgctcg ccagatcaac accctcaacc acggggaggt 2580

ggtgtgcgcg gtgaccatca gcaaccccac gagacacgtg tacacgggtg ggaagggctg 2640

cgtcaaggtc tgggacatca gccacccagg caataagagt cctgtctccc agctcgactg 2700

tctgaacagg gataactaca tccgttcctg cagattgctc cctgatggtc gcaccctaat 2760

tgttggaggg gaagccagta ctttgtccat ttgggacctg gcggctccaa ccccacgcat 2820

caaggcagag ctgacatcct cggcccccgc ctgctatgcc ctggccatca gccccgattc 2880

caaggtctgc ttctcatgct gcagcgacgg caacatcgct gtgtgggatc tgcacaacca 2940

gaccttggtg aggcaattcc agggccacac agatggagcc agctgtattg acatttctaa 3000

tgatggcacc aagctctgga caggtggttt ggacaacacg gtcaggtcct gggacctgcg 3060

cgaggggcgg cagctgcagc agcacgactt cacctcccag atcttttctc tgggctactg 3120

cccaactgga gagtggcttg cagtggggat ggagaacagc aatgtggaag ttttgcatgt 3180

caccaagcca gacaaatacc aactacatct tcatgagagc tgtgtgctgt cgctcaagtt 3240

tgcccattgt ggcaaatggt ttgtaagcac tggaaaggac aaccttctga atgcctggag 3300

aacaccttat ggggccagta tattccagtc caaagaatcc tcatcggtgc ttagctgtga 3360

catctccgtg gacgacaaat acattgtcac tggctctggg gataagaagg ccacagttta 3420

tgaagttatt tattaaagac aaatcttcat gcagactgga cttctcctcc tggtagcact 3480

ttgctctgtc atcctttttg ttcaccccca tccccgcatc taaaaccaag gatttcagat 3540

actcattgca gttgtggagt ttaatcccct ttcttaacct cacttcccac ttgctattga 3600

attgtgaata gtcattaaaa acctgtgata ccaaatcttc agctgtctac ttggaagaac 3660

atggaataag catacttaac agtgaaaaga atctttaatt atgtattata tctgtaatat 3720

atttattttg tttaaagaag gctttctaac aatgactgac taaataaagc tgtctgctcc 3780

tgcattgata atgaaggtgc gttgtatttg atacccctcc cccccttttt ttggcaaagg 3840

aggggaaagg aaggtttaaa ataattgatt taaaatgtca ctaagtgtag actgatgact 3900

gtatagagat gtgaaatgta taattacaca tggaagcaat atgttgctgt gttgttatta 3960

ggtttttttt gtttttgttt tctacatctt ttaaagactt ttggaaattt ggctgaacaa 4020

ttagaacaca acaggccaac tcatactcat ttggatctat ttagacaacg ttaaccaata 4080

tatctatagc tttagattat attcgataaa agtaattgga ctttttttct ttttttgact 4140

cgttgacaag tgtctttgta atatgttttt agttcccttt ttttgttgta ttataggcag 4200

atgaacaaat taaatttggc ctcaaagaga gaacttactc ccttctggat atttttgcca 4260

catttctttg caaaaggaga tatatatatc tttagtcagt tttgttgtta tgagaaatta 4320

tgggttattt tgtggcatgc tctttgggag ctgcacagtt atggggagga ctcccactgc 4380

tgtgcaagtt aagtctttta caaaacaagg acagcagagg agggtttgca gagacctccc 4440

tctgaaaaac acaaagaatg gactctctcc tgggatgagg acttgctttc tttacctccg 4500

gttctttcca tgtcttagtt ggatgtccct gaaatggaca caggctgtgc cattgtgcca 4560

gaaacattgt gttatctttt atgttgttgt tgttgctgtt aaactataat atgtgacttc 4620

tttttttatt attttttgtt tgaatgcttt aaaaatcttt taagtctgtg gatctgctga 4680

tgtacagtgc ctttgctgct atggatcaaa atcaaaagaa ccgtgtagat atactttatt 4740

gtataagtag aaaattactt aatttcatac tagaaatgga tggatgctgc aagttgaaat 4800

ggactgtcca ttgacgttcc taatgtggta gcagaaaaaa aaaaatggtg tcttaagtgc 4860

ttagtgtttg atgtcattaa cagtttcgta aaactctaca gtgtagaaag attttgatac 4920

taaactgtgc gttgtacata gttctaatgc attgtattga ccaccagtac ttctataatg 4980

gtagattgtt tgtgaattca gacttttaag cattaaacat aaataacttc tagtatgctt 5040

atttttctaa ttctttgtct tgatgacatt agtttatttt ttatctttgg ctgtgccact 5100

cctatatatt aaaaatgcct agttttttca agggagattg ttgttaaagt aaagtggttt 5160

tttttgttgt taaa 5174

<210> 56
<211> 1769
<212> DNA
<213> Homo sapiens

<400> 56
cctcactgac tataaaagaa tagagaagga agggcttcag tgaccggctg cctggctgac 60

ttacagcagt cagactctga caggatcatg gctatgatgg aggtccaggg gggacccagc 120

ctgggacaga cctgcgtgct gatcgtgatc ttcacagtgc tcctgcagtc tctctgtgtg 180

gctgtaactt acgtgtactt taccaacgag ctgaagcaga tgcaggacaa gtactccaaa 240

agtggcattg cttgtttctt aaaagaagat gacagttatt gggaccccaa tgacgaagag 300

agtatgaaca gcccctgctg gcaagtcaag tggcaactcc gtcagctcgt tagaaagatg 360

attttgagaa cctctgagga aaccatttct acagttcaag aaaagcaaca aaatatttct 420

cccctagtga gagaaagagg tcctcagaga gtagcagctc acataactgg gaccagagga 480

agaagcaaca cattgtcttc tccaaactcc aagaatgaaa aggctctggg ccgcaaaata 540

aactcctggg aatcatcaag gagtgggcat tcattcctga gcaacttgca cttgaggaat 600

ggtgaactgg tcatccatga aaaagggttt tactacatct attcccaaac atactttcga 660

tttcaggagg aaataaaaga aaacacaaag aacgacaaac aaatggtcca atatatttac 720

aaatacacaa gttatcctga ccctatattg ttgatgaaaa gtgctagaaa tagttgttgg 780

tctaaagatg cagaatatgg actctattcc atctatcaag gggaatattt tgagcttaag 840

gaaaatgaca gaatttttgt ttctgtaaca aatgagcact tgatagacat ggaccatgaa 900

gccagttttt tcggggcctt tttagttggc taactgacct ggaaagaaaa agcaataacc 960

tcaaagtgac tattcagttt tcaggatgat acactatgaa gatgtttcaa aaaatctgac 1020

caaaacaaac aaacagaaaa cagaaaacaa aaaaacctct atgcaatctg agtagagcag 1080

ccacaaccaa aaaattctac aacacacact gttctgaaag tgactcactt atcccaagaa 1140

aatgaaattg ctgaaagatc tttcaggact ctacctcata tcagtttgct agcagaaatc 1200

tagaagactg tcagcttcca aacattaatg caatggttaa catcttctgt ctttataatc 1260

tactccttgt aaagactgta gaagaaagcg caacaatcca tctctcaagt agtgtatcac 1320

agtagtagcc tccaggtttc cttaagggac aacatcctta agtcaaaaga gagaagaggc 1380

accactaaaa gatcgcagtt tgcctggtgc agtggctcac acctgtaatc ccaacatttt 1440

gggaacccaa ggtgggtaga tcacgagatc aagagatcaa gaccatagtg accaacatag 1500

tgaaacccca tctctactga aagtgcaaaa attagctggg tgtgttggca catgcctgta 1560

gtcccagcta cttgagaggc tgaggcagga gaatcgtttg aacccgggag gcagaggttg 1620

cagtgtggtg agatcatgcc actacactcc agcctggcga cagagcgaga cttggtttca 1680

aaaaaaaaaa aaaaaaaaaa cttcagtaag tacgtgttat ttttttcaat aaaattctat 1740

tacagtatgt caaaaaaaaa aaaaaaaaa 1769

<210> 57
<211> 2979
<212> DNA
<213> Homo sapiens

<400> 57
gtggctcttc tggcccgggc tactatatag agacgtttcc gcctcctgct tgaaactaac 60

ccctcttttt ctccaaagga gtgcttgtgg agatcggatc ttttctccag caattggggg 120

aaagaaggct ttttctctga attagcttag tgtaaccagc ggcgtatatt ttttaggcgc 180

cttttcgaaa acctagtagt taatattcat ttgtttaaat cttattttat ttttaagctc 240

aaactgctta agaatacctt aattccttaa agtgaaataa ttttttgcaa aggggtttcc 300

tcgatttgga gctttttttt tcttccaccg tcatttctaa ctcttaaaac caactcagtt 360

ccatcatggt gatgttcaag aagatcaagt cttttgaggt ggtctttaac gaccctgaaa 420

aggtgtacgg cagtggcgag aaggtggctg gccgggtgat agtggaggtg tgtgaagtta 480

ctcgtgtcaa agccgttagg atcctggctt gcggagtggc taaagtgctt tggatgcagg 540

gatcccagca gtgcaaacag acttcggagt acctgcgcta tgaagacacg cttcttctgg 600

aagaccagcc aacaggtgag aatgagatgg tgatcatgag acctggaaac aaatatgagt 660

acaagttcgg ctttgagctt cctcaggggc ctctgggaac atccttcaaa ggaaaatatg 720

ggtgtgtaga ctactgggtg aaggcttttc ttgaccgccc gagccagcca actcaagaga 780

caaagaaaaa ctttgaagta gtggatctgg tggatgtcaa tacccctgat ttaatggcac 840

ctgtgtctgc taaaaaagaa aagaaagttt cctgcatgtt cattcctgat gggcgggtgt 900

ctgtctctgc tcgaattgac agaaaaggat tctgtgaagg tgatgagatt tccatccatg 960

ctgactttga gaatacatgt tcccgaattg tggtccccaa agctgccatt gtggcccgcc 1020

acacttacct tgccaatggc cagaccaagg tgctgactca gaagttgtca tcagtcagag 1080

gcaatcatat tatctcaggg acatgcgcat catggcgtgg caagagcctt cgggttcaga 1140

agatcaggcc ttctatcctg ggctgcaaca tccttcgagt tgaatattcc ttactgatct 1200

atgttagcgt tcctggatcc aagaaggtca tccttgacct gcccctggta attggcagca 1260

gatcaggtct aagcagcaga acatccagca tggccagccg aaccagctct gagatgagtt 1320

gggtagatct gaacatccct gataccccag aagctcctcc ctgctatatg gatgtcattc 1380

ctgaagatca ccgattggag agcccaacca ctcctctgct agatgacatg gatggctctc 1440

aagacagccc tatctttatg tatgcccctg agttcaagtt catgccacca ccgacttata 1500

ctgaggtgga tccctgcatc ctcaacaaca atgtgcagtg agcatgtgga agaaaagaag 1560

cagctttacc tacttgtttc tttttgtctc tcttcctgga cactcacttt ttcagagact 1620

caacagtctc tgcaatggag tgtgggtcca ccttagcctc tgacttccta atgtaggagg 1680

tggtcagcag gcaatctcct gggccttaaa ggatgcggac tcatcctcag ccagcgccca 1740

tgttgtgata caggggtgtt tgttggatgg gtttaaaaat aactagaaaa actcaggccc 1800

atccattttc tcagatctcc ttgaaaattg aggccttttc gatagtttcg ggtcaggtaa 1860

aaatggcctc ctggcgtaag cttttcaagg ttttttggag gctttttgta aattgtgata 1920

ggaactttgg accttgaact tacgtatcat gtggagaaga gccaatttaa caaactagga 1980

agatgaaaag ggaaattgtg gccaaaactt tgggaaaagg aggttcttaa aatcagtgtt 2040

tcccctttgt gcacttgtag aaaaaaaaga aaaaccttct agagctgatt tgatggacaa 2100

tggagagagc tttccctgtg attataaaaa aggaagctag ctgctctacg gtcatctttg 2160

cttagagtat actttaacct ggcttttaaa gcagtagtaa ctgccccacc aaaggtctta 2220

aaagccattt ttggagccta ttgcactgtg ttctcctact gcaaatattt tcatatggga 2280

ggatggtttt ctcttcatgt aagtccttgg aattgattct aaggtgatgt tcttagcact 2340

ttaattcctg tcaaattttt tgttctcccc ttctgccatc ttaaatgtaa gctgaaactg 2400

gtctactgtg tctctagggt taagccaaaa gacaaaaaaa attttactac ttttgagatt 2460

gccccaatgt acagaattat ataattctaa cgcttaaatc atgtgaaagg gttgctgctg 2520

tcagccttgc ccactgtgac ttcaaaccca aggaggaact cttgatcaag atgcccaacc 2580

ctgtgatcag aacctccaaa tactgccatg agaaactaga gggcaggtct tcataaaagc 2640

cctttgaacc cccttcctgc cctgtgttag gagataggga tattggcccc tcactgcagc 2700

tgccagcact tggtcagtca ctctcagcca tagcactttg ttcactgtcc tgtgtcagag 2760

cactgagctc cacccttttc tgagagttat tacagccaga aagtgtgggc tgaagatggt 2820

tggtttcatg tttttgtatt atgtatcttt ttgtatggta aagactatat tttgtactta 2880

accagatata tttttacccc agatggggat attctttgta aaaaatgaaa ataaagtttt 2940

tttaatggaa aaaaaaaaaa aaaaaaaaaa aaaaaaaaa 2979